# EUROPEAN PATENT APPLICATION

(11) **EP 3 385 373 A1**
(43) Date of publication of application: **10.10.2018**
(21) Application number: 17171166.6
(22) Date of filing: 15.05.2017
(51) Int. Cl.: C12N 5/0784, A61K 35/15

(54) **COMPOSITIONS FOR REPROGRAMMING CELLS INTO DENDRITIC CELLS OR ANTIGEN PRESENTING CELLS, METHODS AND USES THEREOF**

(30) Priority: 05.04.2017 PT 110012
(71) Applicant: Centro de Neurociências e Biologia Celular, 3004-504 Coimbra (PT)
(72) Inventor: RIBEIRO LEMOS PEREIRA, Carlos Filipe, 3000-016 Coimbra (PT); FERREIRA PIRES, Cristiana, 3770-352 Palhaça (PT); FIÚZA ROSA, Fábio Alexandre, 2410-205 Leiria (PT)
(74) Representative: Patentree

(57) **Abstract**

The present disclosure relates to compositions, nucleic acid constructs, methods and kits thereof for cell induction or reprogramming cells to the dendritic cell state or antigen presenting cell state, based, in part, on the surprisingly effect described herein of novel use and combinations of transcription factors that permit induction or reprogramming of differentiated or undifferentiated cells into dendritic cells or antigen presenting cells. Such compositions, nucleic acid constructs, methods and kits can be used for inducing dendritic cells in vitro, ex vivo, or in vivo, and these induced dendritic cells or antigen presenting cells can be used for immunotherapy applications.

## Description

### TECHNICAL FIELD

The present disclosure relates to the development of methods for making dendritic cells or antigen presenting cells with antigen presenting capacity from differentiated, multipotent or pluripotent stem cells by introducing and expressing isolated transcription factors. More particularly, the disclosure provides methods for redirecting differentiated, multipotent or pluripotent stem cells to a dendritic cell or antigen presenting cell state by direct cellular reprogramming with a surprisingly use of combinations of transcription factors.

### BACKGROUND

Cellular reprogramming relies on rewiring the epigenetic and transcriptional network of one cell state to that of a different cell type. Transcription factor (TF)-transduction experiments have highlighted the plasticity of adult somatic or differentiated cells, providing new technologies to generate any desired cell type. Through forced expression of TFs, it is possible to reprogram somatic or differentiated cells into induced pluripotent stem cells (iPSCs) that are remarkably similar to embryonic stem cells (1, 2). Alternatively, a somatic cell can also be converted into another specialized cell type (3). Direct lineage conversion has proven successful to reprogram mouse and human fibroblasts into several cell types, such as neurons, cardiomyocytes and hepatocytes, using TFs specifying the target-cell identity (4). Lineage conversions were also demonstrated in the hematopoietic system, where forced expression of TFs induced a macrophage fate in B cells and fibroblasts (5) and the direct reprogramming of mouse fibroblasts into clonogenic hematopoietic progenitors is achieved with Gata2, Gfi1b, cFos and Etv6 (6). These four TFs induce a dynamic, multi-stage hemogenic process that progresses through an endotheliallike intermediate, recapitulating developmental hematopoiesis *in vitro* (*7*).

Reprogrammed cells are very promising therapeutic tools for regenerative medicine, and cells obtained by differentiation of iPSCs are already being tested in clinical studies. For hematopoietic regeneration, however, approaches to generate mature blood cells from iPSCs are still lacking. Therefore, alternative strategies are needed to generate patient-specific definitive hematopoietic cells that can be used as blood products. Given the opportunity of direct cell reprogramming mediated by TFs, one can envision the generation of antigen-presenting cells (APCs) of the immune system such as Dendritic Cells (DCs).

DCs are professional APCs capable of activating T cell responses by displaying peptide antigens complexed with the major histocompatibility complex (MHC) on the surface, together with all of the necessary soluble and membrane associated co-stimulatory molecules. DCs induce primary immune responses, potentiate the effector functions of previously primed T-lymphocytes, and orchestrate communication between innate and adaptive immunity. DCs are found in most tissues, where they continuously sample the antigenic environment and use several types of receptors to monitor for invading pathogens. In steady state, and at an increased rate upon detection of pathogens, sentinel DC in non-lymphoid tissues migrate to the lymphoid organs where they present to T cells the antigens they have collected and processed. The phenotype acquired by the T cell depends on the context in which the DC presents its antigen. If the antigen is derived from a pathogen, or damaged self, DC receive danger signals, become activated and the T cells are then stimulated to become effectors, necessary to provide protective immunity.

The ability of DCs to induce adaptive immunity has boosted research on DC-vaccine strategies for bacterial, viral and parasitic pathogens and cancer immunotherapy. In fact, clinical trials are ongoing utilizing DC-mediated immunotherapy for several tumor types, including solid and hematological tumors (8). However, the clinical outcome has been inconsistent, probably associated with variable efficiency in generating DCs *in vitro:* autologous monocytes give origin to less-efficient DCs, and hematopoietic progenitors are isolated in very low numbers. In addition, these precursor cells are commonly compromised in cancer-bearing patients, resulting in the generation of dysfunctional DCs (8, 9). Cancer evasion mechanisms also may be underlying the lack of consistent therapeutic advantages in DC-based immunotherapies. During tumor progression cancer cells exploit several immunological processes to escape immune surveillance. These adaptations, together with cancer antigen heterogeneity, prevent the recognition of tumor antigens by the immune system and are consequently responsible for the reduced immunogenicity of tumor cells and current immunotherapies.

The generation of APCs by direct reprogramming opens new opportunities to a better understanding of DC specification and cellular identity, contributing to a more efficient control of immune responses using autologous-engineered cells.

These facts are disclosed in order to illustrate the technical problem addressed by the present disclosure.

### GENERAL DESCRIPTION

The present subject matter identifies several isolated transcription factors that surprisingly reprogram or induce differentiated cell, multipotent or pluripotent stem cell into dendritic cell, in vitro, ex vivo or in vivo.

DCs are professional APCs located throughout the body functioning at the interface of the innate and adaptive immune system. DCs are able to provide a crucial link between the external environment and the adaptive immune system through their ability to capture, process and present antigens to T cells, targeting them to different types of immune responses or to tolerance. Firstly, DCs have to capture antigens and process them through major histocompatibility complex (MHC) class I and MHC class II. Following their activation, DCs are able to migrate towards the local draining lymph nodes priming multiple B cell and T cell responses, a key feature of adaptive immunity. The early protective efficacy is primarily conferred by the induction of antigen-specific antibodies produced by B lymphocytes. The long-term protection against specific antigens requires the persistence of specific antibodies and the generation of immunological memory that could provide a rapid and efficient response after subsequent antigen exposure. DCs, as professional APCs, have the ability to cross-present antigens, meaning that, in addition to its classical ability to present exogenous antigens on MHC class II and endogenous antigens on MHC class I, they are also able to present exogenous antigens on MHC class I, a critical step for the generation of Cytotoxic T Lymphocyte responses (CTL).

The ontogeny and/or microenvironment in which DC are positioned may result in the expression of distinct combinations of surface receptors by DCs. For example, phenotypic criteria alone allow the classification of mouse DCs into different subpopulations. Of these, conventional DC (cDC) in lymphoid tissues are traditionally sub-divided into cDC1 and cDC2 subpopulations. It has been argued that different DC subsets may be involved in specific recognition of certain pathogens and/or regulate different immune responses, e.g. Th1 or Th2 (immunity) or regulatory T cells (tolerance). However, the phenotype and functional behavior of DCs is also significantly conditioned by external activating stimuli, denoting significant plasticity. cDC1 and cDC2 subsets differentially prime Th1 and Th2 responses in vivo. Immune therapy for cancer relies on using DCs to prime Th1 or cytotoxic T lymphocyte responses to promote tumor clearance.

Currently, DC-based immunotherapies rely on autologous DC precursors: either monocytes, which are associated with the production of less-efficient DCs, or hematopoietic progenitors, which are isolated in very low numbers. In addition, these precursor cells are commonly compromised in cancer-bearing patients, resulting in the generation of dysfunctional DCs. In contrast, non-hematopoietic cell-types such as fibroblasts are usually not affected. Human Dermal Fibroblasts (HDFs) also exhibit other competitive advantages, namely are easily obtained from a small skin punch biopsy, are easily expanded in vitro for several passages (15-20 million cells after 4 weeks) and can be conserved frozen and used on-demand. Given the fundamental role of DCs as APCs functioning at the interface of the innate and adaptive immune system, there remains a clinical need to find alternative strategies to generate functional DCs to prime antigen-specific immune responses.

An aspect of the present disclosure relates to an isolated transcription factor comprising
a sequence 90% identical to a sequence from a list consisting of: SEQ. ID. 1 to SEQ. ID. 66, and mixtures thereof;
as a reprogramming or inducing factor of a cell selected from a list consisting of: stem cell or a differentiated cell, or mixtures thereof,
into dendritic cell or antigen presenting cell in vitro, ex vivo or in vivo.

In some embodiments, polypeptide variants or family members having the same or a similar activity as the reference polypeptide encoded by the sequences provided in Table 1 can be used in the compositions, methods, and kits described herein. Generally, variants of a particular polypeptide encoding a DC inducing factor for use in the compositions, methods, and kits described herein will have at least about 90%, at least about 91%, at least about 92%, at least about 93%, at least about 94%, at least about 95%, at least about 96%, at least about 97%, at least about 98%, at least about 99% or more sequence identity to that particular reference polynucleotide or polypeptide as determined by sequence alignment programs and parameters described herein and known to those skilled in the art.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (over the whole the sequence) alignment of two sequences that maximizes the number of matches and minimizes the number of gaps. The BLAST algorithm (Altschul et al. (1990) J Mol Biol 215:403-10) calculates percent sequence identity and performs a statistical analysis of the similarity between the two sequences. The software for performing BLAST analysis is publicly available through the National Centre for Biotechnology Information (NCBI). Global percentages of similarity and identity may also be determined using one of the methods available in the MatGAT software package (Campanella et al., BMC Bioinformatics. 2003 Jul 10; 4:29. MatGAT: an application that generates similarity/identity matrices using protein or DNA sequences). Minor manual editing may be performed to optimise alignment between conserved motifs, as would be apparent to a person skilled in the art. The sequence identity values, which are indicated in the present subject-matter as a percentage were determined over the entire amino acid sequence, using BLAST with default parameters.

In an embodiment for better results, the isolated transcription factor may be selected from a list consisting of: BATF3 (SEQ. ID. 1, SEQ. ID. 2), SPIB (SEQ. ID. 3, SEQ. ID. 4), IRF8 (SEQ. ID. 5, SEQ. ID. 6), PU.1 (SEQ. ID. 7, SEQ. ID. 8), STAT3 (SEQ. ID. 11, SEQ. ID. 12), TCF4 (SEQ. ID. 13, SEQ. ID. 14), IKZF1 (SEQ. ID. 15, SEQ. ID. 16), ID2 (SEQ. ID. 17, SEQ. ID. 18), BCL11A (SEQ. ID. 19, SEQ. ID. 20), RELB (SEQ. ID. 21, SEQ. ID. 22), ZBTB46 (SEQ. ID. 23, SEQ. ID. 24), RUNX3 (SEQ. ID. 25, SEQ. ID. 26), GFI1 (SEQ. ID. 27, SEQ. ID. 28), IRF2 (SEQ. ID. 29, SEQ. ID. 30), NFIL3 (SEQ. ID. 31, SEQ. ID. 32), BCL6 (SEQ. ID. 33, SEQ. ID. 34), L-MYC (SEQ. ID. 35, SEQ. ID. 36), NR4A3 (SEQ. ID. 37, SEQ. ID. 38), and mixtures thereof. Preferably the isolated transcription factor may be selected from a list consisting of: BATF3 (SEQ. ID. 1 or SEQ. ID. 2), IRF8 (SEQ. ID. 5, SEQ. ID. 6), PU.1 (SEQ. ID. 7, SEQ. ID. 8), TCF4 (SEQ. ID. 13, SEQ. ID. 14), and mixtures thereof. More preferably, the isolated transcription factor may include the following combination: BATF3 (SEQ. ID. 1 or SEQ. ID. 2) and IRF8 (SEQ. ID. 5, SEQ. ID. 6); or BATF3 (SEQ. ID. 1, SEQ. ID. 2) and PU.1 (SEQ. ID. 7, SEQ. ID. 8); or IRF8 (SEQ. ID. 5, SEQ. ID. 6) and PU.1 (SEQ. ID. 7, SEQ. ID. 8); or TCF4 (SEQ. ID. 13, SEQ. ID. 14) and BATF3 (SEQ. ID. 1, SEQ. ID. 2); or TCF4 (SEQ. ID. 13, SEQ. ID. 14) and IRF8 (SEQ. ID. 5, SEQ. ID. 6); or TCF4 (SEQ. ID. 13, SEQ. ID. 14) and PU.1 (SEQ. ID. 7, SEQ. ID. 8); or BATF3 (SEQ. ID. 1, SEQ. ID. 2), IRF8 (SEQ. ID. 5, SEQ. ID. 6) and PU.1 (SEQ. ID. 7, SEQ. ID. 8); or TCF4 (SEQ. ID. 13, SEQ. ID. 14) BATF3 (SEQ. ID. 1, SEQ. ID. 2) and IRF8 (SEQ. ID. 5, SEQ. ID. 6); or TCF4 (SEQ. ID. 13, SEQ. ID. 14), IRF8 (SEQ. ID. 5, SEQ. ID. 6) and PU.1 (SEQ. ID. 7, SEQ. ID. 8); or TCF4 (SEQ. ID. 13, SEQ. ID. 14), BATF3 (SEQ. ID. 1, SEQ. ID.2), IRF8 (SEQ. ID. 5, SEQ. ID. 6) and PU.1 (SEQ. ID. 7, SEQ. ID. 8).

In an embodiment, the isolated transcription factor of the present disclosure may be used in veterinary or human medicine, in particular in immunotherapy, or in neurodegenerative diseases, or in cancer or in infectious diseases.

In an embodiment for better results the cell may be selected from a list consisting of: pluripotent stem cell, multipotent stem cell, differentiated cell, tumor cell, cancer cell, and mixtures thereof. In particular mammalian cell, more in particular a mouse or a human cell.

In an embodiment for better results, the isolated transcription factor of the present disclosure may be use as a reprogramming or inducing factor of a cell selected from a list consisting of: pluripotent stem cell, or multipotent stem cell, or differentiated cell, and mixtures thereof into dendritic cell.

In an embodiment for better results, the isolated transcription factor of the present disclosure may be use a reprogramming or inducing factor of a cell selected from a list consisting of: tumor cell, cancer cell, and mixtures thereof, into antigen presenting cell.

Another aspect of the present disclosure is the use of a sequence at least 90% identical, preferably at least 91%, 92%, 93%, 94%, 95%, 96 %, 97%, 98%, 99%, or identical, to a sequence from a list consisting of: SEQ. ID. 1 to SEQ. ID. 66, and mixtures thereof;
as a reprogramming or inducing factor of a cell selected from a list consisting of: stem cell or a differentiated cell, or mixtures thereof,
into dendritic cell or antigen presenting cell in vitro, ex vivo or in vivo.
Preferably, identical to a sequence from a list consisting of: SEQ. ID. 1 to SEQ. ID. 66, and mixtures thereof. More preferably BATF3 (SEQ. ID. 1, SEQ. ID. 2), SPIB (SEQ. ID. 3, SEQ. ID. 4), IRF8 (SEQ. ID. 5, SEQ. ID. 6), PU.1 (SEQ. ID. 7, SEQ. ID. 8), STAT3 (SEQ. ID. 11, SEQ. ID. 12), TCF4 (SEQ. ID. 13, SEQ. ID. 14), IKZF1 (SEQ. ID. 15, SEQ. ID. 16), ID2 (SEQ. ID. 17, SEQ. ID. 18), BCL11A (SEQ. ID. 19, SEQ. ID. 20), RELB (SEQ. ID. 21, SEQ. ID. 22), ZBTB46 (SEQ. ID. 23, SEQ. ID. 24), RUNX3 (SEQ. ID. 25, SEQ. ID. 26), GFI1 (SEQ. ID. 27, SEQ. ID. 28), IRF2 (SEQ. ID. 29, SEQ. ID. 30), NFIL3 (SEQ. ID. 31, SEQ. ID. 32), BCL6 (SEQ. ID. 33, SEQ. ID. 34), L-MYC (SEQ. ID. 35, SEQ. ID. 36), NR4A3 (SEQ. ID. 37, SEQ. ID. 38), and mixtures thereof. Preferably the isolated transcription factor may be selected from a list consisting of: BATF3 (SEQ. ID. 1 or SEQ. ID. 2), IRF8 (SEQ. ID. 5, SEQ. ID. 6), PU.1 (SEQ. ID. 7, SEQ. ID. 8), TCF4 (SEQ. ID. 13, SEQ. ID. 14), and mixtures thereof. More preferably, the isolated transcription may include the following combination: BATF3 (SEQ. ID. 1 or SEQ. ID. 2) and IRF8 (SEQ. ID. 5, SEQ. ID. 6); or BATF3 (SEQ. ID. 1, SEQ. ID. 2) and PU.1 (SEQ. ID. 7, SEQ. ID. 8); or IRF8 (SEQ. ID. 5, SEQ. ID. 6) and PU.1 (SEQ. ID. 7, SEQ. ID. 8); or TCF4 (SEQ. ID. 13, SEQ. ID. 14) and BATF3 (SEQ. ID. 1, SEQ. ID. 2); or TCF4 (SEQ. ID. 13, SEQ. ID. 14) and IRF8 (SEQ. ID. 5, SEQ. ID. 6); or TCF4 (SEQ. ID. 13, SEQ. ID. 14) and PU.1 (SEQ. ID. 7, SEQ. ID. 8); or BATF3 (SEQ. ID. 1, SEQ. ID. 2), IRF8 (SEQ. ID. 5, SEQ. ID. 6) and PU.1 (SEQ. ID. 7, SEQ. ID. 8); or TCF4 (SEQ. ID. 13, SEQ. ID. 14), BATF3 (SEQ. ID. 1, SEQ. ID. 2) and IRF8 (SEQ. ID. 5, SEQ. ID. 6); or TCF4 (SEQ. ID. 13, SEQ. ID. 14), IRF8 (SEQ. ID. 5, SEQ. ID. 6) and PU.1 (SEQ. ID. 7, SEQ. ID. 8); or TCF4 (SEQ. ID. 13, SEQ. ID. 14), BATF3 (SEQ. ID. 1, SEQ. ID.2), IRF8 (SEQ. ID. 5, SEQ. ID. 6) and PU.1 (SEQ. ID. 7, SEQ. ID. 8).

Another aspect of the present disclosure relates to a construct or a vector encoding at least one isolated transcription factor described in the present subject-matter. In an embodiment for better results, the transducing step further comprises at least one vector selected from a list consisting of: a nucleic acid sequence encoding IL12; nucleic acid sequence encoding GM-CSF; nucleic acid sequence encoding IL-7; nucleic acid sequence encoding siRNA targeting IL-10 RNA, and mixtures thereof.

In an embodiment for better results the transducing of step further comprises at least one vector comprising nucleic acids encoding immunostimulatory cytokines.

Another aspect of the present disclosure relates to a method for programming or inducing a stem cell or a differentiated cell into a dendritic cell or antigen presenting cell, comprising the following step:
transducing a cell selected from a list consisting of: a stem cell or a differentiated cell, and mixtures thereof,
with one or more vectors comprising a nucleic acid sequence encoding a sequence at least 90% identical to a sequence from a list consisting of SEQ. ID. 1 to SEQ. ID. 66, and mixtures thereof; preferably at least 91%, 92%, 93%, 94%, 95%, 96%, 97%, 98%, 99%, or identical;
culturing the transduced somatic cell in a cell media that supports growth of dendritic cells or antigen presenting cells.

Preferably for better results, identical to a sequence from a list consisting of: SEQ. ID. 1 to SEQ. ID. 66, and mixtures thereof. More preferably BATF3 (SEQ. ID. 1, SEQ. ID. 2), SPIB (SEQ. ID. 3, SEQ. ID. 4), IRF8 (SEQ. ID. 5, SEQ. ID. 6), PU.1 (SEQ. ID. 7, SEQ. ID. 8), STAT3 (SEQ. ID. 11, SEQ. ID. 12), TCF4 (SEQ. ID. 13, SEQ. ID. 14), IKZF1 (SEQ. ID. 15, SEQ. ID. 16), ID2 (SEQ. ID. 17, SEQ. ID. 18), BCL11A (SEQ. ID. 19, SEQ. ID. 20), RELB (SEQ. ID. 21, SEQ. ID. 22), ZBTB46 (SEQ. ID. 23, SEQ. ID. 24), RUNX3 (SEQ. ID. 25, SEQ. ID.2 6), GFI1 (SEQ. ID. 27, SEQ. ID. 28), IRF2 (SEQ. ID. 29, SEQ. ID. 30), NFIL3 (SEQ. ID. 31, SEQ. ID. 32), BCL6 (SEQ. ID. 33, SEQ. ID. 34), L-MYC (SEQ. ID. 35, SEQ. ID. 36), NR4A3 (SEQ. ID. 37, SEQ. ID. 38), and mixtures thereof. Preferably the isolated transcription factor may be selected from a list consisting of: BATF3 (SEQ. ID. 1, SEQ. ID. 2), IRF8 (SEQ. ID. 5, SEQ. ID. 6), PU.1 (SEQ. ID. 7, SEQ. ID. 8), TCF4 (SEQ. ID. 13, SEQ. ID. 14), and mixtures thereof. More preferably, the isolated transcription may the following combination: BATF3 (SEQ. ID. 1 or SEQ. ID. 2) and IRF8 (SEQ. ID. 5, SEQ. ID. 6); or BATF3 (SEQ. ID. 1, SEQ. ID. 2) and PU.1 (SEQ. ID. 7, SEQ. ID. 8); or IRF8 (SEQ. ID. 5, SEQ. ID. 6) and PU.1 (SEQ. ID. 7, SEQ. ID. 8); or TCF4 (SEQ. ID. 13, SEQ. ID. 14) and BATF3 (SEQ. ID. 1, SEQ. ID.2); or TCF4 (SEQ. ID. 13, SEQ. ID. 14) and IRF8 (SEQ. ID. 5, SEQ. ID. 6); or TCF4 (SEQ. ID. 13, SEQ. ID. 14) and PU.1 (SEQ. ID. 7, SEQ. ID. 8); or BATF3 (SEQ. ID. 1, SEQ. ID.2), IRF8 (SEQ. ID. 5, SEQ. ID. 6) and PU.1 (SEQ. ID. 7, SEQ. ID. 8); or TCF4 (SEQ. ID. 13, SEQ. ID. 14) BATF3 (SEQ. ID. 1, SEQ. ID.2) and IRF8 (SEQ. ID. 5, SEQ. ID. 6); or TCF4 (SEQ. ID. 13, SEQ. ID. 14), IRF8 (SEQ. ID. 5, SEQ. ID. 6) and PU.1 (SEQ. ID. 7, SEQ. ID. 8); or TCF4 (SEQ. ID. 13, SEQ. ID. 14), BATF3 (SEQ. ID. 1, SEQ. ID.2), IRF8 (SEQ. ID. 5, SEQ. ID. 6) and PU.1 (SEQ. ID. 7, SEQ. ID. 8).

In an embodiment for better results, cells may be transduced with a plurality of isolated transcription factors and cultured during at least 2 days, preferably at least 5 days, more preferably at least 8 days.

In an embodiment for better results, the transducing step may further comprises at least one vector selected from a list consisting of: a nucleic acid sequence encoding IL-12; nucleic acid sequence encoding GM-CSF; nucleic acid sequence encoding IL-7; nucleic acid sequence encoding siRNA targeting IL-10 RNA, and mixtures thereof.

In an embodiment for better results, the cell may be selected from the group consisting of pluripotent stem cell, or multipotent stem cell, differentiated cell, and mixtures thereof. In particular an endoderm derived cell, a mesoderm derived cell, or an ectoderm derived cell, a multipotent stem cell including mesenchymal stem cell, a hematopoietic stem cell, intestinal stem cell, a pluripotent stem cell, a tumor or cancer cell and cell lines.

In an embodiment for better results, the cell may be a non-human cell, preferably a mouse or a human cell, more preferably cell is a human or mouse fibroblast, or a mammal umbilical cord blood stem cell.

Another aspect of the present disclosure relates to induced dendritic cell or obtained by the method described in the present disclosure.

Another aspect of the present disclosure relates to induced antigen presenting cell by the method described in the present disclosure. In particular, an induced antigen presenting cell capable to present a cancer antigen, a self-antigen, an allergen, an antigen from a pathogenic and/or infectious organism.

Another aspect of the present disclosure relates to composition comprising at least one isolated transcription factor as described in the present disclosure, or an induced dendritic cell as described in the present disclosure, or an induced antigen presenting cell as described in the present disclosure, or mixtures thereof, in a therapeutically effective amount and a pharmaceutically acceptable excipient.

In a preferably embodiment, the composition may be use in veterinary or human medicine, in particular in immunotherapy, or in neurodegenerative diseases, or in cancer or in infectious diseases.

In a preferably embodiment, the composition may further comprises an anti-viral, an analgesic, an anti-inflammatory agent, a chemotherapy agent, a radiotherapy agent, an antibiotic, a diuretic, or mixtures thereof.

In a preferably embodiment, the composition may further comprises a filler, a binder, a disintegrant, or a lubricant, or mixtures thereof.

In a preferably embodiment, the composition may be use in intradermal and transdermal therapies.

In a preferably embodiment, the composition may be use an injectable formulation, in particular an in situ injection.

In a preferably embodiment, the composition may be use in veterinary or human medicine, in particular in immunotherapy, or in neurodegenerative diseases, or in cancer or in infectious diseases.

In a preferably embodiment, the composition may be use in the treatment, therapy or diagnostic of a central and peripheral nervous system disorder.

In a preferably embodiment, the composition may be use in the treatment therapy or diagnostic of neoplasia in particular cancer, namely solid or hematological tumors.

In a preferably embodiment, the composition may be use in the treatment, diagnostic or therapy of benign tumor, malignant tumor, early cancer, basal cell carcinoma, cervical dysplasia, soft tissue sarcoma, germ cell tumor, retinoblastoma, age-related macular degeneration, Hodgkin's lymphoma, blood cancer, prostate cancer, ovarian cancer, cervix cancer, uterus cancer, vaginal cancer, breast cancer, naso-pharynx cancer, trachea cancer, larynx cancer, bronchi cancer, bronchioles cancer, lung cancer, hollow organs cancer, esophagus cancer, stomach cancer, bile duct cancer, intestine cancer, colon cancer, colorectum cancer, rectum cancer, bladder cancer, ureter cancer, kidney cancer, liver cancer, gall bladder cancer, spleen cancer, brain cancer, lymphatic system cancer, bone cancer, pancreatic cancer, leukemia, skin cancer, or myeloma.

In a preferably embodiment, the composition may be use in the treatment, therapy or diagnostic of a fungal, viral, chlamydial, bacterial, nanobacterial or parasitic infectious disease.

In a preferably embodiment, the composition may be use in therapy or diagnostic of HIV, infection with SARS coronavirus, Asian flu virus, herpes simplex, herpes zoster, hepatitis, or viral hepatitis.

In a preferably embodiment, the composition may be use in the treatment, therapy or diagnostic of an amyloid disease in particular Amyloid A (AA) amyloidosis, Alzheimer's disease, Light-Chain (AL) amyloidosis, Type-2 Diabetes, Medullary Carcinoma of the Thyroid, Parkinson's disease, Polyneuropathy, or Spongiform Encephalopathy - Creutzfeldt Jakob disease.

Another aspect of the present disclosure relates to a vaccine for cancer comprising to composition comprising at least one isolated transcription factor as described in the present disclosure, or an induced dendritic cell as described in the present disclosure, or an induced antigen presenting cell as described in the present disclosure, or mixtures thereof.

A kit comprising at least one of the following components: a composition comprising at least one isolated transcription factor as described in the present disclosure, or an induced dendritic cell as described in the present disclosure, or a induced antigen presenting cell as described in the present disclosure, a composition as described in the present disclosure, or a vector as described in the present disclosure, or a construct as described in the present disclosure or mixtures thereof.

An aspect of the present disclosure relates to compositions, methods, and kits for dendritic cell induction or for reprogramming cells to antigen-presenting dendritic cells (DC). In some embodiments, the compositions comprise at least one DC inducing factor. Such compositions, methods and kits can be used for inducing dendritic cells in vitro, ex vivo, or in vivo, as described herein, and these induced dendritic cells (iDCs) can be used in immunotherapies.

The compositions, methods, and kits for dendritic cell induction or for reprogramming cells to dendritic cells of the present disclosure are based, in part, in the use of a novel combination of transcription factors that permit direct reprogramming of differentiated cells to the dendritic cell state. Such compositions, nucleic acid constructs, methods and kits can be used for inducing dendritic cells in vitro, ex vivo, or in vivo, as described herein, and these induced dendritic cells can be used in immunotherapies.

In an embodiment, the present disclosure relates to the regulation of the immune system, and in particular to the use of reprogrammed dendritic cells to prime immune responses to target antigens.

In an embodiment, the resulting dendritic cell is an antigen presenting cell which activates T cells against MHC class I-antigen targets. Cancer, viral, bacterial and parasitic infections are all ameliorated by the reprogrammed dendritic cells. As reprogrammed dendritic cells are capable of cross-presenting extracellular antigens via the MHC class I pathway, they are particularly suitable for generation of cytotoxic T lymphocyte responses.

In an embodiment isolated transcription factor (or exogenous transcription factor) selected from a list consisting of: BATF3 (SEQ. ID. 1, SEQ. ID. 2), IRF8 (SEQ. ID. 5, SEQ. ID. 6), PU.1 (SEQ. ID. 7, SEQ. ID. 8), TCF4 (SEQ. ID. 13, SEQ. ID. 14), and mixtures thereof, upon forced expression in fibroblasts induce activation of the Clec9a DC-specific reporter and DC morphology. Induced Dendritic Cells (iDCs) express major histocompatibility complex (MHC)-II at the cell surface and the co-stimulatory molecules CD80 and CD86. iDCs are able to engulf particles and upon challenge with LPS or poly I:C secrete inflammatory cytokines. iDCs present antigens to CD4+ T cells and cross-present antigens to CD8+ T cells.

In an embodiment isolated transcription factor (or exogenous transcription factor) selected from a list consisting of: BATF3 (SEQ. ID. 1, SEQ. ID. 2), IRF8 (SEQ. ID. 5, SEQ. ID. 6), PU.1 (SEQ. ID. 7, SEQ. ID. 8), TCF4 (SEQ. ID. 13, SEQ. ID. 14), upon forced expression in fibroblasts induce expression of CLEC9A and CD11c, typical DC markers, and DC morphology. Induced Dendritic Cells (iDCs) are able to engulf particles, soluble protein and dead cells. This disclosure provides powerful new treatments for cancers and cellular infections, as well as a variety of diagnostic and cell screening assays.

In an embodiment, CLEC9A is preferentially expressed on the subset cDC1 of dendritic cells. This is an important cell type because it is capable of processing antigens derived from outside the cell and presenting them to T cells via MHC class I molecules. This is in contrast to most antigen presenting cells, which present extracellularly-derived antigens via MHC class II molecules. Consequently, this mechanism of antigen presentation is sometimes referred to as "cross-presentation". These cells therefore play an important role in the generation and stimulation of CTL responses, which are an essential part of the immune response against intracellular pathogens, e.g. viruses and cancers.

In an embodiment, immune responses stimulated via iDCs involve proliferation of T cells, which may be CTL or helper T cells. Antigen presenting cells (and in particular iDCs) can induce proliferation of both CD8+ T cells and CD4+ T cells, and may stimulate proliferation of both types of T cells in any given immune response.

In an embodiment, iDCs may be implicated in at least Th1, Th2, and Th17-type immune responses. Thus the methods of the invention may be applied to stimulation of various types of immune response against any antigen. However these cells are believed to be particularly important in the generation of CTL responses, so the immune response to be stimulated is preferably a CTL response. The method may comprise determining production and/or proliferation of CTLs, which are typically T cells expressing CD8 and are capable of cytotoxic activity against cells displaying their cognate antigen in the context of MHC class I molecules.

It will therefore be further understood that iDCs may be used for the prophylaxis and/or treatment of any condition in which it is desirable to induce a CTL response, such as cancer, or infection by an intracellular parasite or pathogen, such as a viral infection.

Nevertheless, if modified, iDCs can result in proliferation of helper T cells as well as, or instead of, CTLs. Thus the method may additionally or alternatively comprise determining production and/or proliferation of helper T cells. The helper T cells may be CD4+ T cells, and may be of Th1, Th2, Th17 or Treg type.

Under certain conditions, it is believed that iDCs may be capable of stimulating regulatory T cell (Treg) proliferation. Treg cells are characterised by the expression of the Foxp3 (Forkhead box p3) transcription factor. Most Treg cells are CD4+ and CD25+, and can be regarded as a subset of helper T cells, although a small population may be CD8+. Thus the immune response, which is to be stimulated by a method of the present disclosure, may comprise inducing proliferation of Treg cells in response to an antigen. Given that Treg cells may be capable of modulating the response of other cells of the immune system against an antigen in other ways, e.g. inhibiting or suppressing their activity, the effect on the immune system as a whole may be to modulate (e.g. suppress or inhibit) the response against that antigen. Thus the methods of this aspect of the invention can equally be referred to as methods of modulating (e.g. inhibiting or suppressing) an immune response against an antigen. This may be particularly useful (for example) in the treatment of autoimmune disease.

iDCs will promote antigen-specific responses. The antigen may be any protein or fragment thereof against which it is desirable to raise an immune response, in particular a CTL response, but also a Th17 response or a Treg response. These may include antigens associated with, expressed by, displayed on, or secreted by cells against which it is desirable to stimulate a CTL response, including cancer cells and cells containing intracellular pathogens or parasites. For example, the antigen may be, or may comprise, an epitope peptide from a protein expressed by an intracellular pathogen or parasite (such as a viral protein) or from a protein expressed by a cancer or tumor cell. Thus the antigen may be a tumor-specific antigen. The term "tumor-specific" antigen should not be interpreted as being restricted to antigens from solid tumors, but to encompass antigens expressed specifically by any cancerous, transformed or malignant cell.

The invention therefore provides a primed antigen presenting cell or population thereof. By "primed" is meant that the cell has been contacted with an antigen, is presenting that antigen or an epitope thereof in the context of MHC molecules, preferably MHC I molecules, and is capable of activating or stimulating T cells to proliferate and differentiate into effector cells in response thereof.

The term "antigen" is well understood in the art and includes immunogenic substances as well as antigenic epitopes. It will be appreciated that the use of any antigen is envisioned for use in the present invention and thus includes, but is not limited to, a self-antigen (whether normal or disease-related), an infectious antigen (e.g., a microbial antigen, viral antigen, etc.), or some other foreign antigen (e.g., a food component, pollen, etc.). Loading the antigen-presenting cells with an antigen can be accomplished utilizing standard methods, for example, pulsing, transducing, transfecting, and/or electrofusing. It is envisioned that the antigen can be nucleic acids (DNA or RNA), proteins, protein lysate, whole cell lysate, or antigen proteins linked to other proteins, i.e., heat shock proteins. The antigens can be derived or isolated from a pathogenic microorganism such as viruses including HIV, influenza, Herpes simplex, human papilloma virus, Hepatitis B, Hepatitis C, EBV, Cytomegalovirus (CMV) and the like. The antigen may be derived or isolated from pathogenic bacteria such as from *Chlamydia, Mycobacteria, Legionella, Meningiococcus,* Group *AStreptococcus, Salmonella, Listeria, Hemophilus influenzae,* and the like. Still further, the antigen may be derived or isolated from pathogenic yeast including *Aspergillus,* invasive *Candida, Nocardia,* Histoplasmosis, *Cryptosporidia* and the like. The antigen may be derived or isolated from a pathogenic protozoan and pathogenic parasites including, but not limited to *Pneumocystis carinii, Trypanosoma, Leishmania, Plasmodium* and *Toxoplasma gondii.* In certain embodiments, the antigen includes an antigen associated with a preneoplastic or hyperplastic state. Antigens may also be associated with, or causative of cancer. Such antigens are tumor specific antigen, tumor associated antigen (TAA) or tissue specific antigen, epitope thereof, and epitope agonist thereof. Such antigens include but are not limited to carcinoembryonic antigen (CEA) and epitopes thereof such as CAP-1, CAP-1-6D, MART-1, MAGE-1, MAGE-3, GAGE, GP-100, MUC-1, MUC-2, point mutated ras oncogene, normal and point mutated p53 oncogenes, PSMA, tyrosinase, TRP-1 (gp75), NY-ESO-1, TRP-2, TAG72, KSA, CA-125, PSA, HER-2/neu/c-erb/B2, BRC-I, BRC-II, bcr-abl, pax3-fkhr, ews-fli-1, modifications of TAAs and tissue specific antigen, splice variants of TAAs, epitope agonists, and the like.

The term "agent" as used herein means any compound or substance such as, but not limited to, a small molecule, nucleic acid, polypeptide, peptide, drug, ion, etc. An "agent" can be any chemical, entity or moiety, including without limitation synthetic and naturally-occurring proteinaceous and non-proteinaceous entities. In some embodiments, an agent is nucleic acid, nucleic acid analogues, proteins, antibodies, peptides, aptamers, oligomer of nucleic acids, amino acids, or carbohydrates including without limitation proteins, oligonucleotides, ribozymes, DNAzymes, glycoproteins, siRNAs, lipoproteins, aptamers, and modifications and combinations thereof etc. In some embodiments, the nucleic acid is DNA or RNA, and nucleic acid analogues, for example can be PNA, pcPNA and LNA. A nucleic acid may be single or double stranded, and can be selected from a group comprising; nucleic acid encoding a protein of interest, oligonucleotides, PNA, etc. Such nucleic acid sequences include, for example, but not limited to, nucleic acid sequence encoding proteins that act as transcriptional repressors, antisense molecules, ribozymes, small inhibitory nucleic acid sequences, for example but not limited to RNAi, shRNAi, siRNA, micro RNAi (mRNAi), antisense oligonucleotides etc. A protein and/or peptide agent or fragment thereof, can be any protein of interest, for example, but not limited to; mutated proteins; therapeutic proteins; truncated proteins, wherein the protein is normally absent or expressed at lower levels in the cell. Proteins of interest can be selected from a group comprising; mutated proteins, genetically engineered proteins, peptides, synthetic peptides, recombinant proteins, chimeric proteins, antibodies, humanized proteins, humanized antibodies, chimeric antibodies, modified proteins and fragments thereof.

As used herein, the term "transcription factor" or "TF" refers to a protein that binds to specific parts of DNA using DNA binding domains and is part of the system that controls the transcription of genetic information from DNA to RNA.

The term "DC inducing factor," as used herein, refers to a developmental potential altering factor, as that term is defined herein, such as a protein, RNA, or small molecule, the expression of which contributes to the reprogramming of a cell, e.g. a somatic cell, to the DC state. A DC inducing factor can be, for example, transcription factors that can reprogram cells to the DC state, such as PU.1, IRF8, BATF3 and TCF4, and the like, including any gene, protein, RNA or small molecule that can substitute for one or more of these factors in a method of making iDCs in vitro. In some embodiments, exogenous expression of a DC inducing factor induces endogenous expression of one or more DC inducing factors, such that exogenous expression of the one or more DC inducing factor is no longer required for stable maintenance of the cell in the iDC state.

The term "an antigen-presenting cell" (APC) as used herein, refers to a cell that displays antigen complexed with major histocompatibility complexes (MHCs) on their surfaces; this process is known as antigen presentation. T cells may recognize these complexes using their T cell receptors (TCRs). These cells process antigens and present them to T-cells.

The term "a somatic cell" used herein, refers to any biological cell forming the body of an organism; that is, in a multicellular organism, any cell other than a gamete, germ cell, gametocyte or undifferentiated stem cell.

The expression of endogenous DC inducing factors can be induced by the use of DNA targeting systems able to modulate mammalian gene expression in a cell with or without the use of chromatin modifying drugs. In some embodiments, the DNA targeting system may comprise a Clustered Regularly Interspaced Short Palindromic Repeats (CRISPR)/CRISPR-associated (Cas) 9-based system (as described in WO2014197748 A2) that may include any modified protein, isolated polynucleotide or vector contacted with the cell and at least one guide RNA targeting a promoter region of at least one gene selected from the group consisting of PU.1, IRF8, BATF3 and TCF4. The DNA targeting system may comprise dCas9-VP64. In some embodiments, the DNA targeting system may comprise two or more transcription activator-like effector transcription factors (as described in US20140309177 A1) that bind to different target regions of at least one gene selected from the group consisting of PU.1, IRF8, BATF3 and TCF4.

In an embodiment, iDCs can be used as immunotherapy to induce specific immune responses in patients with cancer, such as melanoma, prostate cancer, glioblastoma, acute myeloid leukemia, among others.

In an embodiment, iDCs can also be used to treat infections caused by viral, bacterial and parasitic pathogens.

In an embodiment, iDCs can also be used as *in vitro* tools for vaccine immunogenicity testing.

The pluripotent stem cells used in the present disclosure are obtained without having to recur to a method necessarily involving the destruction of human embryos, namely with the use of induced pluripotent stem cells. Induced pluripotent stem cells (also known as iPS cells or iPSCs) are a type of pluripotent stem cell that can be generated directly from adult cells by cellular reprogramming.

The induced Dendritic Cells (iDCs) obtainable by this method express MHC-II at the cell surface and the co-stimulatory molecules CD80 and CD86.

In some embodiments, the composition may comprises the isolated transcription factor discloses in the present subject-matter, in an amount effective to improve the immunotherapy by at least 5%, at least 10%, at least 15%, at least 20%, at least 25%, at least 30%, at least 40%, at least 50%, at least 60%, at least 70%, at least 75%, at least 80%, at least 90%, at least 95%, at least 95.7%, at least 98%, or at least 99% in the subject.

In some embodiments, the composition may comprises the induced dendritic cells disclosure in the present subject-matter, in an amount effective to improve the immunotherapy by at least 5%, at least 10%, at least 15%, at least 20%, at least 25%, at least 30%, at least 40%, at least 50%, at least 60%, at least 70%, at least 75%, at least 80%, at least 90%, at least 95%, at least 95.7%, at least 98%, or at least 99% in the subject.

DC inducing factors of the present disclosure can be delivered to induce reprogramming *in vitro, ex vivo* or *in vivo.*

Differentiated cells of the present disclosure can be isolated from a subject in need, DC inducing factors can be introduced to induce reprogramming into iDCs. Generated iDCs can be infused back to the patient.

Alternatively, DC inducing factors can be delivered to induce reprogramming in vivo of, for example, cancer cells into iDC with ability to present cancer antigens.

Preferred routes of administration include but are not limited to oral, parenteral, intramuscular, intravenous, in situ injection, intranasal, sublingual, intratracheal, and inhalation.

In some embodiments, the dose or dosage form is administered to the subject once a day, twice a day, or three times a day. In other embodiments, the dose is administered to the subject once a week, once a month, once every two months, four times a year, three times a year, twice a year, or once a year.

The embodiments of the invention provide multiple applications, including kits for research use and methods for generation of cells useful for conducting small molecule screens for immune disorders. In addition, the invention provides commercially and medically useful methods to produce autologous dendritic cells and give them back to a patient in need.

For example, the methods described herein can be used to produce dendritic cells to treat diseases including hyperproliferative diseases, which can also be further defined as cancer. In still further embodiments, the cancer is melanoma, non-small cell lung, small-cell lung, lung, hepatocarcinoma, leukemia, retinoblastoma, astrocytoma, glioblastoma, gum, tongue, neuroblastoma, head, neck, breast, pancreatic, prostate, colorectal, Esophageal, Non-Hodgkin lymphoma, uterine, liver, thyroid, renal, skin, bone, testicular, ovarian, mesothelioma, cervical, gastrointestinal, lymphoma, brain, colon, sarcoma or bladder. The cancer may include a tumor comprised of tumor cells. For example, tumor cells may include, but are not limited to melanoma cell, a bladder cancer cell, a breast cancer cell, a lung cancer cell, a colon cancer cell, a prostate cancer cell, a liver cancer cell, a pancreatic cancer cell, a stomach cancer cell, a testicular cancer cell, a brain cancer cell, an ovarian cancer cell, a lymphatic cancer cell, a skin cancer cell, a brain cancer cell, a bone cancer cell, or a soft tissue cancer cell. In other embodiments, the hyperproliferative disease is rheumatoid arthritis, inflammatory bowel disease, osteoarthritis, leiomyomas, adenomas, lipomas, hemangiomas, fibromas, vascular occlusion, restenosis, atherosclerosis, pre-neoplastic lesions (such as adenomatous hyperplasia and prostatic intraepithelial neoplasia), carcinoma in situ, oral hairy leukoplakia, or psoriasis.

Accordingly, provided herein, in an embodiment are dendritic cell (DC) inducing composition comprising one or more expression vectors encoding at least one, two, three, four, five, six, seven, eight, or more DC inducing factors selected from: BATF3 (SEQ. ID. 1, SEQ. ID. 2), SPIB (SEQ. ID. 3, SEQ. ID. 4), IRF8 (SEQ. ID. 5, SEQ. ID. 6), PU.1 (SEQ. ID. 7, SEQ. ID. 8), STAT3 (SEQ. ID. 11, SEQ. ID. 12), TCF4 (SEQ. ID. 13, SEQ. ID. 14), IKZF1 (SEQ. ID. 15, SEQ. ID. 16), ID2 (SEQ. ID. 17, SEQ. ID. 18), BCL11A (SEQ. ID. 19, SEQ. ID. 20), RELB (SEQ. ID.21, SEQ. ID. 22), ZBTB46 (SEQ. ID. 23, SEQ. ID. 24), RUNX3 (SEQ. ID. 25, SEQ. ID. 26), GFI1 (SEQ. ID. 27, SEQ. ID. 28), IRF2 (SEQ. ID. 29, SEQ. ID. 30), NFIL3 (SEQ. ID. 31, SEQ. ID. 32), BCL6 (SEQ. ID. 33, SEQ. ID. 34), L-MYC (SEQ. ID. 35, SEQ. ID. 36), NR4A3 (SEQ. ID. 37, SEQ. ID. 38), or mixtures thereof.

Also provided herein, in some aspects, are dendritic cell (DC) inducing compositions comprising one or more expression vectors comprising: a nucleic acid sequence encoding TCF4 (SEQ. ID. 13, SEQ. ID. 14), BATF3 (SEQ. ID. 1, SEQ. ID.2), IRF8 (SEQ. ID. 5, SEQ. ID. 6), PU.1 (SEQ. ID. 7, SEQ. ID. 8) or mixtures thereof. In a particular embodiment the addition of increases the efficiency in at least 8%.

In some embodiments of these aspects and all such aspects described herein, the one or more expression vectors are retroviral vectors.

In some embodiments of these aspects and all such aspects described herein, the one or more expression vectors are lentiviral vectors. In some embodiments, the lentiviral vectors are inducible lentiviral vectors.

Also provided herein, in some aspects, are dendritic cell (DC) inducing compositions comprising modified mRNA sequences encoding at least one, two, three, four, five, six, seven, eight, or more DC inducing factors selected from BATF3 (SEQ. ID. 1, SEQ. ID. 2), SPIB (SEQ. ID. 3, SEQ. ID. 4), IRF8 (SEQ. ID. 5, SEQ. ID. 6), PU.1 (SEQ. ID. 7, SEQ. ID. 8), STAT3 (SEQ. ID. 11, SEQ. ID. 12), TCF4 (SEQ. ID. 13, SEQ. ID. 14), IKZF1 (SEQ. ID. 15, SEQ. ID. 16), ID2 (SEQ. ID. 17, SEQ. ID. 18), BCL11A (SEQ. ID. 19, SEQ. ID. 20), RELB (SEQ. ID. 21, SEQ. ID. 22), ZBTB46 (SEQ. ID. 23, SEQ. ID. 24), RUNX3 (SEQ. ID. 25, SEQ. ID. 26), GFI1 (SEQ. ID. 27, SEQ. ID. 28), IRF2 (SEQ. ID. 29, SEQ. ID. 30), NFIL3 (SEQ. ID. 31, SEQ. ID. 32), BCL6 (SEQ. ID. 33, SEQ. ID. 34), L-MYC (SEQ. ID. 35, SEQ. ID. 36), NR4A3 (SEQ. ID. 37, SEQ. ID. 38), or mixtures thereof, wherein each cytosine of each said modified mRNA sequence is a modified cytosine, each uracil of each said modified mRNA sequence is a modified uracil, or a combination thereof.

Provided herein, in some aspects, are dendritic cell (DC) inducing compositions comprising SEQ. ID. 1 to SEQ. ID. 66, in particular factors selected from TCF4 (SEQ. ID. 13, SEQ. ID. 14), BATF3 (SEQ. ID. 1, SEQ. ID. 2), IRF8 (SEQ. ID. 5, SEQ. ID. 6), PU.1 (SEQ. ID. 7, SEQ. ID. 8), or mixtures thereof, wherein each cytosine of each said modified mRNA sequence is a modified cytosine, each uracil of each said modified mRNA sequence is a modified uracil, or a combination thereof.

In some embodiments of these aspects and all such aspects described herein, the modified cytosine is 5-methylcytosine and the modified uracil is pseudouracil.

Also provided herein in some aspects, are methods for preparing an induced dendritic cell (iDC) from a somatic cell comprising:
o transducing the somatic cell with one or more vectors comprising a nucleic acid sequence encoding PU.1 (SEQ. ID. 7, SEQ. ID. 8), a nucleic acid sequence encoding IRF8 (SEQ. ID. 5, SEQ. ID. 6); a nucleic acid sequence encoding BATF3 (SEQ. ID. 1, SEQ. ID. 2); wherein each said nucleic acid sequence is operably linked to a promoter; and
o culturing the transduced somatic cell in a cell media that supports growth of dendritic cells, thereby preparing an iDC.

In some embodiments of these aspects and all such aspects described herein, the transducing step further comprises one or more vectors comprising one or more of: a nucleic acid sequence encoding TCF4 (SEQ. ID. 13, SEQ. ID. 14); a nucleic acid sequence encoding SPIB (SEQ. ID. 3, SEQ. ID. 4); a nucleic acid sequence encoding IRF4 (SEQ. ID. 9, SEQ. ID. 10); a nucleic acid sequence encoding STAT3 (SEQ. ID. 11, SEQ. ID. 12); a nucleic acid sequence encoding IKZF1 (SEQ. ID. 15, SEQ. ID. 16); a nucleic acid sequence encoding ID2 (SEQ. ID. 17, SEQ. ID. 18); a nucleic acid sequence encoding BCL11A (SEQ. ID. 19, SEQ. ID. 20); a nucleic acid sequence encoding RELB(SEQ. ID. 21, SEQ. ID. 22); a nucleic acid sequence encoding ZBTB46 (SEQ. ID. 23, SEQ. ID. 24); a nucleic acid sequence encoding RUNX3(SEQ. ID. 25, SEQ. ID. 26); a nucleic acid sequence encoding GFI1(SEQ. ID. 27, SEQ. ID. 28); a nucleic acid sequence encoding IRF2(SEQ. ID. 29, SEQ. ID. 30); a nucleic acid sequence encoding NFIL3(SEQ. ID. 31, SEQ. ID. 32); a nucleic acid sequence encoding BCL6(SEQ. ID. 33, SEQ. ID. 34); a nucleic acid sequence encoding L-MYC (SEQ. ID. 35, SEQ. ID. 36); a nucleic acid sequence encoding NR4A3 (SEQ. ID. 37, SEQ. ID. 38); a nucleic acid sequence encoding IL12; nucleic acid sequence encoding GM-CSF; nucleic acid sequence encoding IL-7; nucleic acid sequence encoding siRNA targeting IL-10 RNA.

In some embodiments of these aspects and all such aspects described herein, the transducing step further comprises one or more vectors comprising nucleic acids encoding immunostimulatory cytokines. Preferably, the cytokine is one of the interleukins (e.g., IL-1α, IL-1β, IL-2, IL-3, IL-4, IL-6, IL-7, IL-8, IL-9, IL-10, IL-12, IL-18, IL-19, IL-20), the interferons (e.g., IFN-α, IFN-β, IFN-γ), tumor necrosis factor (TNF), transforming growth factor-β (TGF-β), granulocyte colony stimulating factors (G-CSF), macrophage colony stimulating factor (M-CSF), granulocyte-macrophage colony stimulating factor (GM-CSF), the Flt-3 ligand or the kit ligand. The amino acid sequences of these cytokines are well known in the art. In the case of heterodimeric immunostimulatory cytokines (e.g., IL-12), induced dendritic cells (iDCs) shall be genetically modified to express both subunits of the cytokine molecule.

The additional vectors may also comprise nucleic acids encoding variants of these cytokines. For example, for those cytokines having both pro-forms and mature forms (e.g., before and after cleavage of a signal peptide, or before and after limited proteolysis to yield an active fragment), the APCs of the invention may be genetically modified to express either the pro- or mature form. Other variants, such as fusion proteins between an active fragment of a cytokine and a heterologous sequence (e.g., a heterologous signal peptide), may also be employed. Species variants may also be employed to the extent that they retain activity in a human subject. Thus, for example, human APCs may be genetically modified to express a murine, bovine, equine, ovine, feline, canine, non-human primate or other mammalian variant of a human cytokine if these species variants retain activity substantially similar to their human homologues.

It may be desirable also to administer further immunostimulatory agents in order to achieve maximal CTL stimulation and proliferation, and/or stimulation and proliferation of other T cell types. These may include agents capable of activating dendritic cells and stimulating their ability to promote T cell activation. Such an agent may be referred to as an adjuvant. The adjuvant may comprise an agonist for CD40 (such as soluble CD40 ligand, or an agonist antibody specific for CD40), an agonist of CD28, CD27 or OX40 (e.g. an agonist antibody specific for one of those molecules), a CTLA-4 antagonist (e.g. a blocking antibody specific for CTLA-4), and/or a Toll-like receptor (TLR) agonist, and/or any other agent capable of inducing dendritic cell activation. A TLR agonist is a substance that activates a Toll-like receptor. Preferably, the TLR agonist is an activator of TLR3, TLR4, TLR5, TLR7 or TLR9. A suitable TLR agonist is MPL (monophosphoryl lipid A), which binds TLR4. Other TLR agonists which may be used are LTA (lipoteichoic acid, which binds TLR2; Poly I:C (polyinosine-polycytidylic acid), which binds TLR3; flagellin, which binds TLR5; imiquimod or polyU RNA (1-(2-methylpropyl)-1H-imidazo(4,5-c)quinolin-4-amine), which binds TLR7 and CpG (DNA CpG motifs), which binds TLR9; or any other component which binds to and activates a TLR. For more details, see Reis e Sousa, Toll-like receptors and dendritic cells. Seminars in Immunology 16:27, 2004. Adjuvants which may not work via TLRs include 5' triphosphate RNA, poly I:C, and β-glucans such as curdlan (β-1,3-glucan).

In some embodiments of these aspects and all such aspects described herein, the culturing step further comprises the use of cell media that supports growth of dendritic cells or antigen presenting cells supplemented with at least one immunostimulatory recombinant cytokine selected from the group consisting of interleukins (e.g., IL-1α, IL-1β, IL-2, IL-3, IL-4, IL-6, IL-7, IL-8, IL-9, IL-10, IL-12, IL-18, IL-19, IL-20), interferons (e.g., IFN-α, IFN-β, IFN-γ), tumor necrosis factor (TNF), transforming growth factor-β (TGF-β), granulocyte colony stimulating factors (G-CSF), macrophage colony stimulating factor (M-CSF), granulocyte-macrophage colony stimulating factor (GM-CSF), Flt-3 ligand or kit ligand. In the case of heterodimeric immunostimulatory cytokines (e.g., IL-12), induced dendritic cells (iDCs) shall be cultured with both subunits of the cytokine molecule. Other pro-inflammatory cytokines may also be used as adjuvants.

In some embodiments of these aspects and all such aspects described herein, the somatic cell is a fibroblast cell.

In some embodiments of these aspects and all such aspects described herein, the somatic cell is a hematopoietic lineage cell.

In some embodiments of these aspects and all such aspects described herein, the hematopoietic lineage cell is selected from promyelocytes, neutrophils, eosinophils, basophils, reticulocytes, erythrocytes, mast cells, osteoclasts, megakaryoblasts, platelet producing megakaryocytes, platelets, monocytes, macrophages, lymphocytes, NK cells, NKT cells, innate lymphocytes, multipotent hematopoietic stem and progenitor cells, oligopotent hematopoietic progenitor cells, lineage restricted hematopoietic progenitors.

In some embodiments of these aspects and all such aspects described herein, the hematopoietic lineage cell is selected from a multi-potent progenitor cell (MPP), common myeloid progenitor cell (CMP), granulocyte-monocyte progenitor cells (GMP), common lymphoid progenitor cell (CLP), and pre-megakaryocyte-erythrocyte progenitor cell.

In some embodiments of these aspects and all such aspects described herein, the hematopoietic lineage cell is selected from a megakaryocyte-erythrocyte progenitor cell (MEP), a ProB cell, a PreB cell, a PreProB cell, a ProT cell, a double-negative T cell, a pro-NK cell, a pre-dendritic cell (pre-DC), pregranulocyte/macrophage cell, a granulocyte/macrophage progenitor (GMP) cell, and a pro-mast cell (ProMC).

Also provided herein, in some aspects, are kits for making induced dendritic cells (iDC), the kits comprising any of the DC inducing compositions comprising one or more expression vector components described herein.

Provided herein, in some aspects, are kits for making induced dendritic cells (iDC), the kits comprising any of the DC inducing compositions comprising modified mRNA sequence components described herein.

In some embodiments of these aspects and all such aspects described herein, the one or more expression vectors are lentiviral vectors. In some embodiments, the lentiviral vectors are inducible lentiviral vectors. In some embodiments, the lentiviral vectors are polycistronic inducible lentiviral vectors. In some embodiments, the polycistronic inducible lentiviral vectors express three or more nucleic acid sequences. In some embodiments, each of the nucleic acid sequences of the polycistronic inducible lentiviral vectors are separated by 2A peptide sequences.

The use of polycistronic viral expression systems can increase the in vivo reprogramming efficiency of somatic cells to iDCs. Accordingly, in some embodiments of the aspects described herein, a polycistronic lentiviral vector is used. In such embodiments, sequences encoding two or more of the DC inducing factors described herein, are expressed from a single promoter, as a polycistronic transcript. 2A peptide strategy can be used to make polycistronic vectors (see, e.g., Expert Opin Biol Ther. 2005 May; 5(5):627-38). Polycistronic expression vector systems can also use internal ribosome entry sites (IRES) elements to create multigene, or polycistronic, messages. IRES elements are able to bypass the ribosome scanning model of 5'-methylated Cap dependent translation and begin translation at internal sites (Pelletier and Sonenberg, 1988). IRES elements can be linked to heterologous open reading frames. Multiple open reading frames can be transcribed together, each separated by an IRES, thus creating polycistronic messages. By virtue of the IRES element, each open reading frame is accessible to ribosomes for efficient translation. Multiple genes can be efficiently expressed using a single promoter/enhancer to transcribe a single message. See, for example, U.S. Pat. Nos. 4,980,285; 5,925,565; 5,631,150; 5,707,828; 5,759,828; 5,888,783; 5,919,670; and 5,935,819; and Sambrook et al., Molecular Cloning: A Laboratory Manual, 2nd Ed., Cold Spring Harbor Press (1989).

### BRIEF DESCRIPTION OF THE DRAWINGS

The following figures provide preferred embodiments for illustrating the description and should not be seen as limiting the scope of invention.

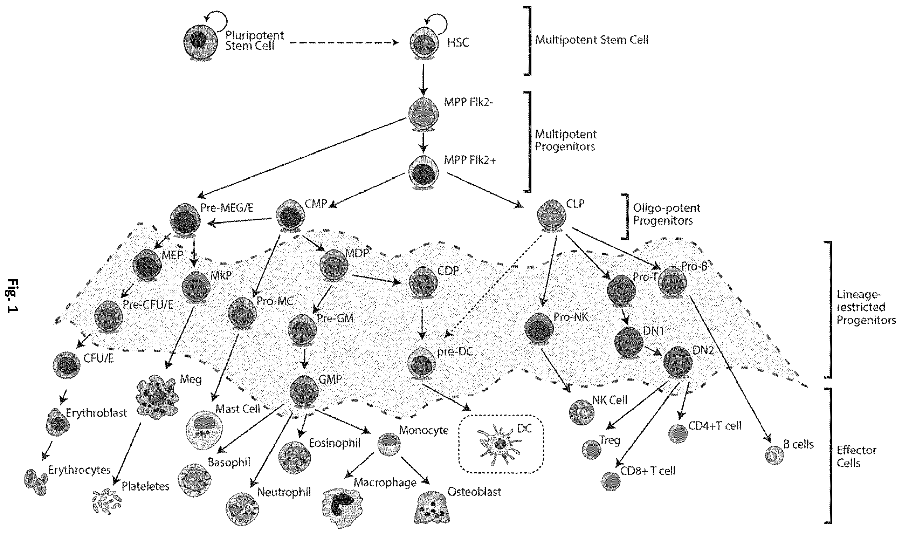

**Figure 1****.** Schematic representation of hematopoietic differentiation. Whereas hematopoietic differentiation normally proceeds from hematopoietic stem cells (HSCs) through progressively morerestricted progenitors into differentiated blood effector cells, such as dendritic cells (DCs) (highlighted with dashed-line box), the results described herein aim to utilize DC-enriched transcription factors to reprogram somatic differentiated cells from other lineages into the dendritic cell fate with antigen-presenting capacity. Multi-potent progenitor cells (MPPs), common myeloid progenitor cells (CMPs), common lymphoid progenitor cells (CLPs), granulocyte-monocyte progenitor cells (GMPs), pre-megakaryocyte-erythrocyte (pre-MEG/E) progenitor cell, megakaryocyte-erythrocyte progenitor cells (MEP), colony forming unit-erythroid (CFU-E), megakaryocytic progenitor MkP, pro-mast cells (ProMCs), Monocyte DC progenitors (MDP), Common Dendritic Cell Precursors (CDP), pre-dendritic cell (pre-DC), double negative T lineage precursors (DN1, DN2).

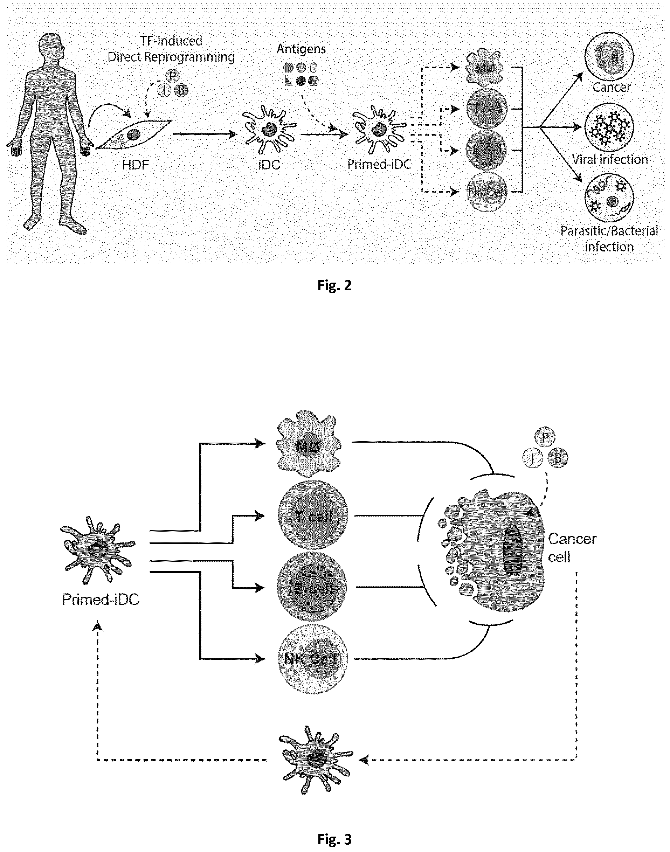

**Figure 2****.** Generating Antigen Presenting Cells by Direct Cellular Reprogramming. Observation of the effect of the TF combination disclosure in the present subject-matter for the induction of dendritic cells (iDCs) from mouse and human fibroblasts. Induced DCs can be applied to generate a personalized immunotherapy after loading with cell extracts or defined antigens (Primed-iDC). DCs are specialized in antigen presentation to Macrophages (Mo), T, B and NK cells. Induced DCs stimulate antigen-specific immune responses against cancer, viral, parasitic or bacterial infections.
**Figure 3****.** *In situ* or *ex vivo* direct reprogramming of cancer cells to stimulate antigen-specific immune responses. Effect of TF combination (PIB) for the induction of DC fate and antigen presenting capacity, when this cocktail is introduced directly into cancer cells *in vivo* or *in situ* or, *ex vivo* or *in vitro.* This strategy enable cancer cells to present their specific antigens (cancer-iDC) to CD4+ and CD8+ T-cells, triggering a targeted immune response against the tumor.

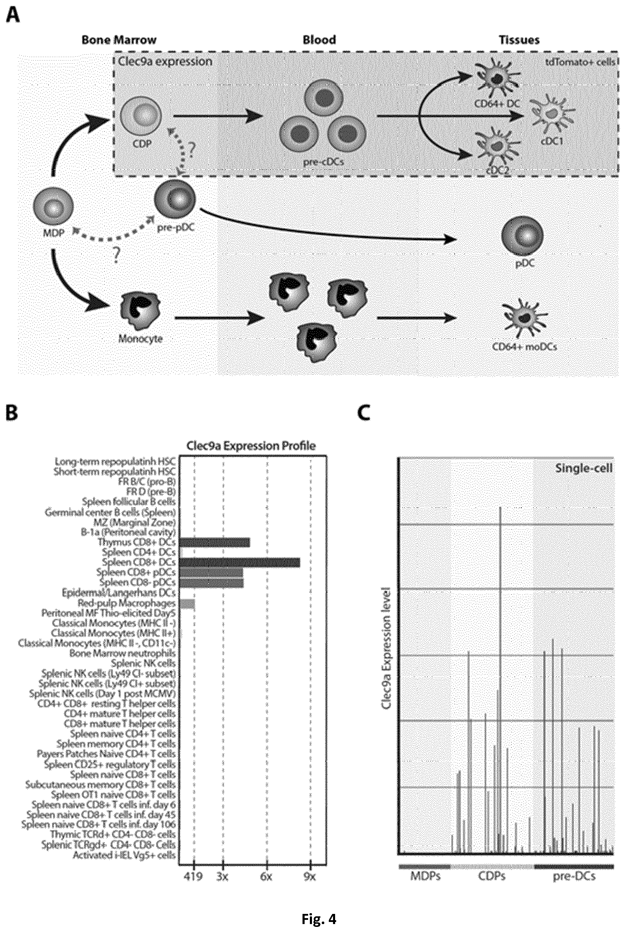

**Figure 4****.** Expression of Clec9a is specifically restricted to the conventional DC-lineage. (A) Clec9aCre X R26-stop-Tomato double transgenic mouse enables identification of conventional DCs and their committed precursors (CDP, Common Dendritic Cell Precursors), but not other leukocytes, due to restricted tdTomato expression. (B) Expression profile of Clec9a in DCs and several hematopoietic cell lineages obtained from data available in Immunological Genome Project (www.immgen.org). (C) Gene expression of the Clec9a gene in Monocyte DC progenitors (MDPs) and DC-committed precursors (CDPs and pre-DCs) at the single cell level (GSE60783).

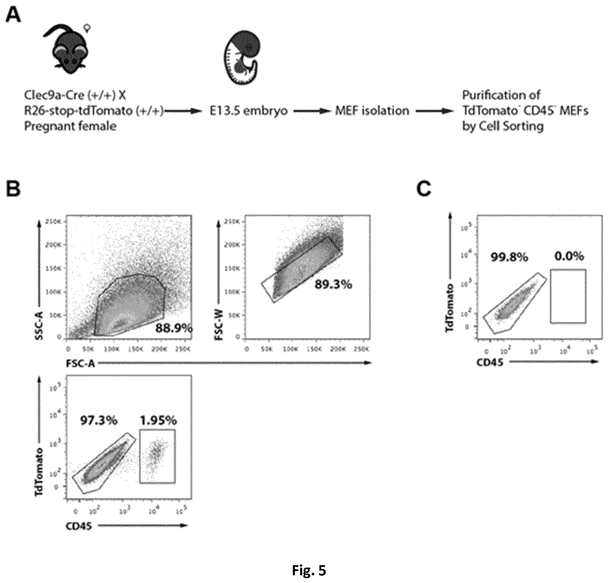

**Figure 5****.** Strategy to obtain Clec9a reporter MEFs to screen candidate TFs. (A) Double transgenic (Clec9a-Cre X R26-stop-Tomato) pregnant females were used to isolate MEFs at embryonic day E13.5. After removal of the head, fetal liver and all internal organs, MEFs were cultured until confluency. MEFs were sorted to remove residual CD45+ and tdTomato+ cells that could represent cells with hematopoietic potential. (B) Gating strategy to remove residual CD45+ and tdTomato+ cells. Double negative MEFs, approximately 97% of the population, were sorted. (C) Purity confirmation of the sorted population.

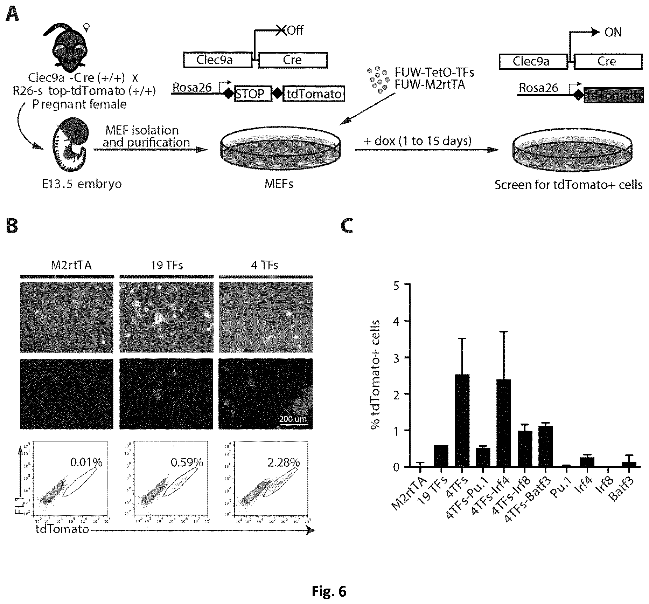

**Figure 6****.** Combinations of candidate DC-inducing TFs induce activation of the Clec9a-tdTomato reporter. (A) Experimental design to screen candidate TFs' ability to activate Clec9a reporter MEFs. Purified MEFs were transduced with different pools of inducible lentiviral vectors encoding DC-specific TFs. MEFs were cultured in the presence of Dox to induce expression of the TFs and monitored from day 1 to 15 for tdTomato expression. Activation of Clec9a promoter induces expression of Cre recombinase, which mediates excision of the Stop codon and consequent expression of tdTomato. (B) MEFs were transduced with M2rtTA (as control), all 19 candidate TFs or a pool of 4TFs (PU.1, IRF4, IRF8 and BATF3) and analysed by fluorescent microscopy and flow cytometry 5 days after addition of Dox. (C) Quantification of tdTomato+ cells after transduction with all 19 candidate TFs, pool of 4TFs, removal of individual TFs from the pool of 4TFs or their individual expression at day 8 after addition of Dox.

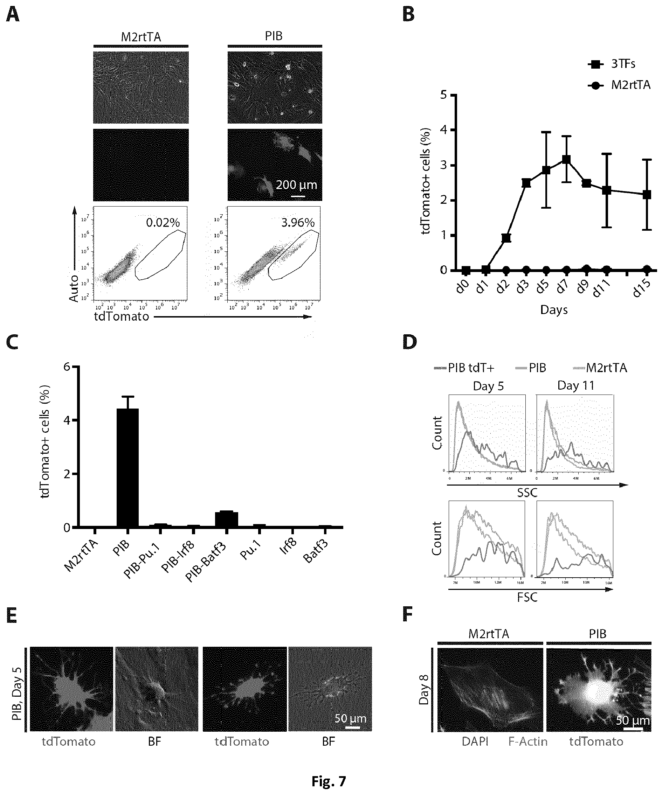

**Figure 7****.** Minimal transcription factor network activates Clec9a reporter and induce DC morphology in mouse fibroblasts. (A) MEFs were transduced with M2rtTA (as control) or PU.1, IRF8 and BATF3 (PIB - mixture of the 3 TF) and analysed by fluorescent microscopy and flow cytometry 5 days after addition of Dox. (B) Kinetics of Clec9a-tdTomato reporter activation analysed by flow cytometry. (C) Quantification of tdTomato+ cells after removal of individual TFs from the pool of PIB or their individual expression at day 5 after addition of Dox. (D) Flow cytometry histograms showing size (FSC) and complexity (SSC) in PIB transduced cells (gated in tdTomato+ or total population) and M2rtTA transduced cells. (E) Morphology of tdTomato+ cells at day 5 after the addition of Dox. (F) Immunofluorescence for F-actin at day 8 after addition of Dox.

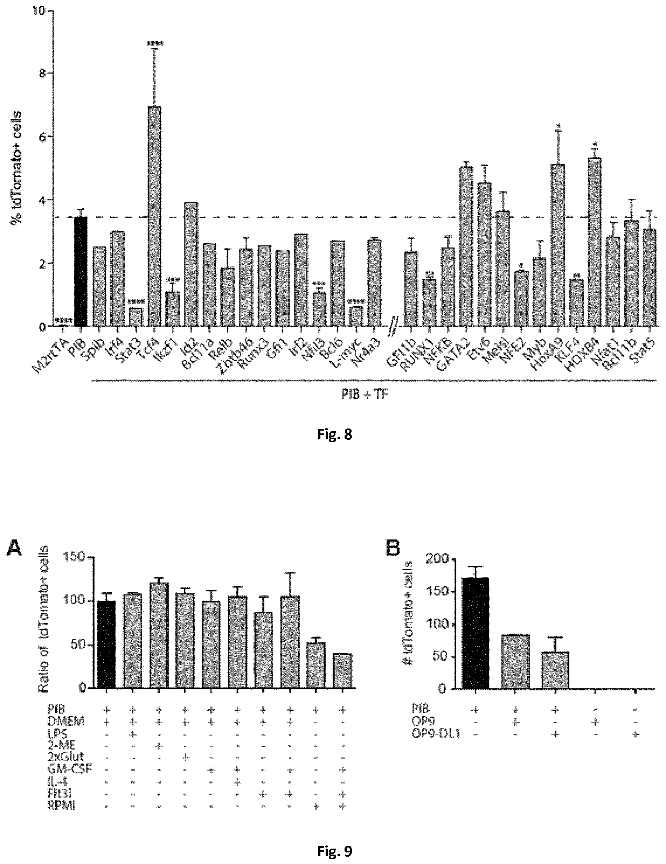

**Figure 8****.** TCF4 increases the efficiency of Clec9a reporter activation. MEFs were transduced with PIB (PU.1, IRF8 and BATF3, black bar) or PIB combined with individual TFs (grey bars). tdTomato+ cells were quantified 8 days after the addition of Dox. M2rtTA transduction was included as control. Mean ± SD; statistical analysis using one-way ANOVA with Bonferroni's multiple comparison test; ****p<0.0001, ***p<0.001, **p<0.01, *p<0.05.
**Figure 9****.** Optimal culture conditions to induce the activation of the Clec9a reporter. (A) Quantification of tdTomato+ cells in MEFs transduced with PIB (PU.1, IRF8 and BATF3) and cultured in different conditions at day 10 after addition of Dox. (B) Absolute numbers of tdTomato+ cells in MEFs transduced with PIB (black bar) and co-cultured with OP9 and OP9-DL1 cells at day 10 after addition of Dox. OP9 and OP9-DL1 cultures were included as controls.

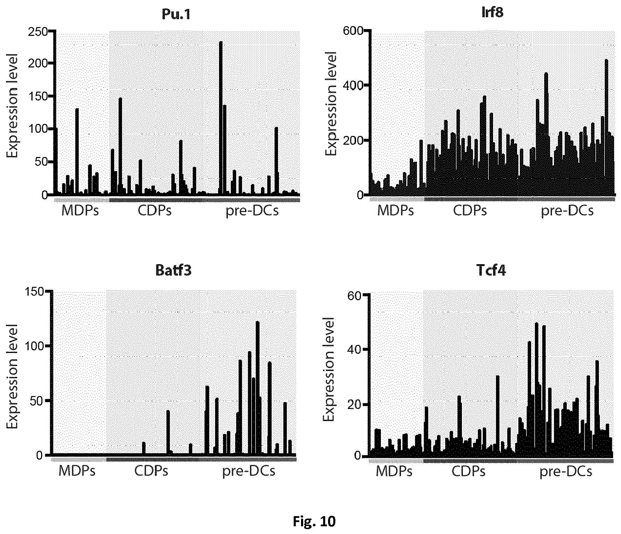

Figure 10. Expression profiles of PU.1, IRF8, BATF3 and TCF4 at the single cell level. Gene expression of PU.1, IRF8, BATF3 and TCF4 in single DC precursor cells (MDPs, CDPs, and pre-DCs) (GSE60783). Gene expression level is shown in reads per kilobase of exon model per million mapped reads (RPKM) values.

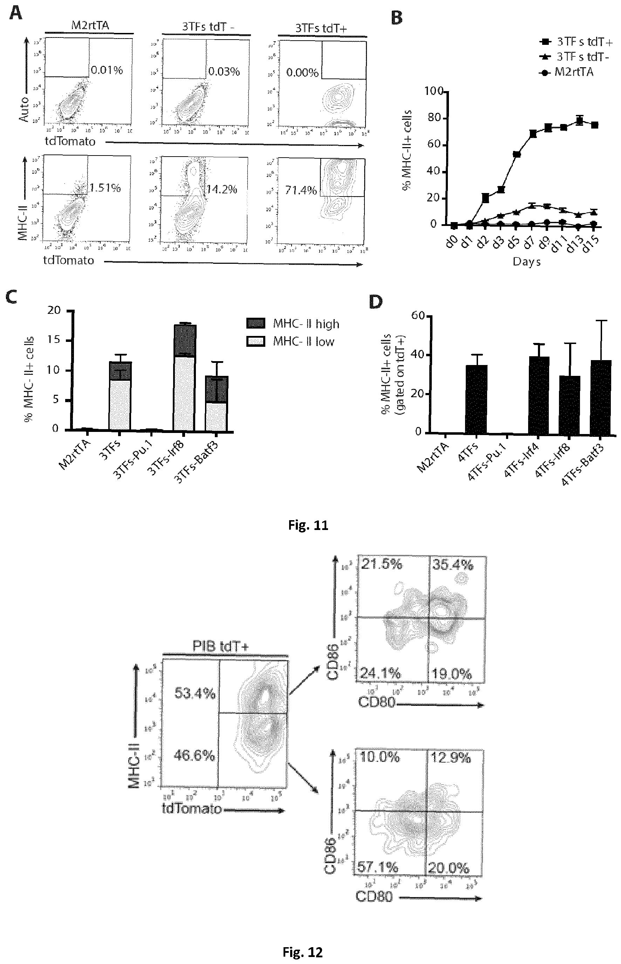

**Figure 11****.** Induced DCs express antigen-presenting machinery at the cell surface (A) Flow cytometry analysis of MHC-II expression in MEFs transduced with M2rtTA (as control) and PIB (PU.1, IRF8 and BATF3) 7 days after addition of Dox. TdTomato+ and tdTomato- populations are shown. (B) Kinetics of MHC-II surface expression in M2rtTA and PIB transduced cells. MEFs were analysed by flow cytometry from day 1 to 15 after addition of Dox. (C) Quantification of the percentage of cells expressing MHC-II in high and low levels in bulk cultures after removal of individual TF from the pool of PIB, at day 5 after addition of Dox. (D) Quantification of MHC-II+ cells at day 5 within tdTomato+ population after transduction with 4TFs or upon their individual removal.
**Figure 12****.** Induced DCs express co-stimulatory molecules at cell surface. Flow cytometry analysis of co-stimulatory molecules (CD80 and CD86) at cell surface of tdTomato+ population in PIB (PU.1, IRF8 and BATF3) transduced MEFs 5 days after addition of Dox. MHC-II+ and MHC-II- populations are shown.

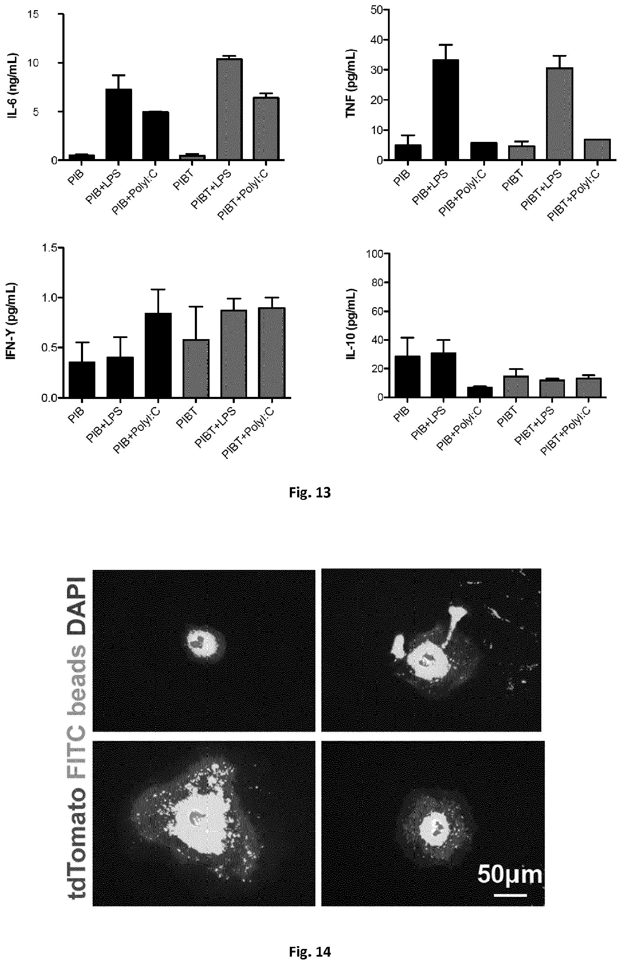

**Figure 13****.** Induced DCs secrete inflammatory cytokines upon TLR stimuli. (A) Secretion of cytokines by MEFs transduced with PIB (PU.1, IRF8 and BATF3) or PIBT (PU.1, IRF8, BATF3 and TCF4) with or without TLR4 (LPS) or TLR3 (Polyl:C) stimuli overnight. Supernatants of MEFs transduced with PIB or PIBT factors were collected at day 10 after addition of Dox and analysed for cytokine concentration using BD Cytometric Bead Array Mouse Inflammation Kit. Cytokine levels for untreated, 100 ng/mL LPS or 25 µg/mL of Polyl:C-treated cells overnight are shown; black or grey bars represent PIB or PIBT-transduced MEFs, respectively.
**Figure 14****.** Induced DCs are able to engulf small particles. MEFs transduced with PIB (PU.1, IRF8 and BATF3) were incubated overnight with FITC-labelled latex beads (1µm) and analysed by fluorescent microscopy at day 7 after addition of Dox.

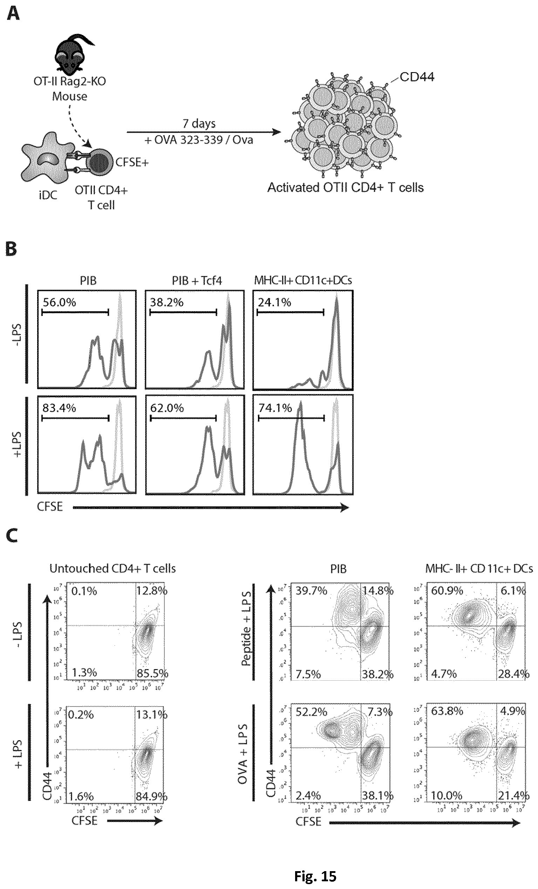

**Figure 15****.** Induced DCs capture and present antigens to CD4+ T cells. (A) Schematic representation of antigen presenting assay. iDCs at day 8 after addition of Dox were co-cultured with OT-II CD4+ T cells isolated from OT-II Rag2KO mice and labelled with CFSE in the presence of Ovalbumine (OVA) or OVA peptide 323-339. After 7 days, activation of CD4+ T cells was evaluated by CFSE dilution and expression of T cell activation marker CD44. (B) Flow cytometry plots of CFSE-labelled CD4+ T cells co-cultured with MEFs transduced with PIB or PIB plus TCF4, in the presence of OVA, stimulated or not with LPS. CD4+ T cells co-cultured with splenic MHC-II+ CD11c+ DCs were included as controls. Grey lines correspond to untouched CD4+T cells. (C) Flow cytometry plots showing CD44 expression of CFSE-labelled CD4+ T cells co-cultured with MEFs transduced with PIB, in the presence of LPS and OVA or OVA peptide.

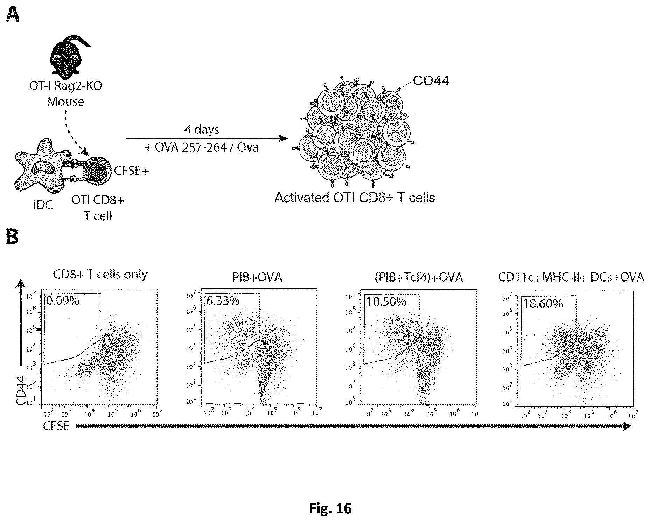

**Figure 16****.** Induced DCs capture and cross-present exogenous antigens to CD8+ T cells. (A) Schematic representation of cross-presentation assay. iDCs at day 8 after addition of Dox were co-cultured with OT-I CD8+ T cells isolated from OT-I Rag2KO mice and labelled with CFSE in the presence of Ovalbumine (OVA) or OVA 257-264 peptide. After 4 days, activation of CD4+T cells was evaluated by CFSE dilution and expression of the T cell activation marker CD44. (B) Flow cytometry plots showing CD44 expression of CFSE-labelled CD8+ T cells co-cultured with MEFs transduced with PIB or PIB plus TCF4, in the presence of OVA. CD8+T cells co-cultured with splenic MHC-II+ CD11c+ DCs were included as controls.

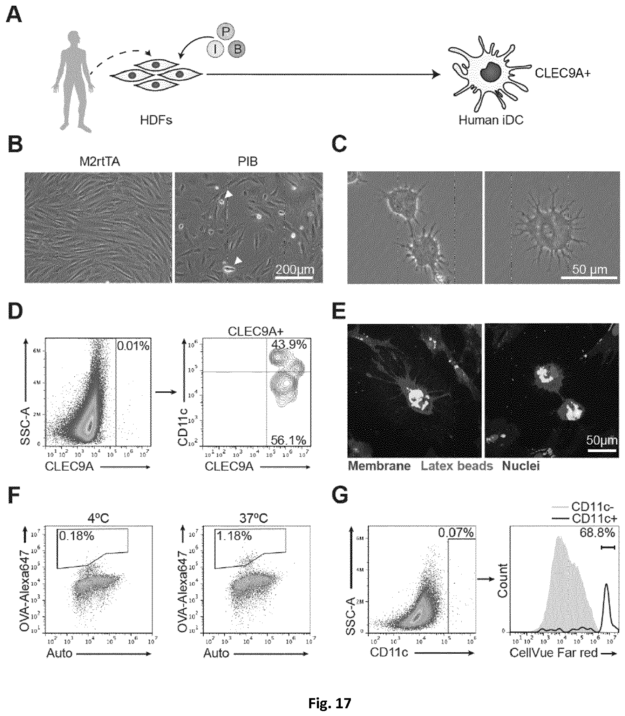

**Figure 17****.** PU.1, IRF8 and BATF3 induce DC-like morphology in human fibroblasts. (A) Human Dermal Fibroblasts (HDFs) were transduced with PIB (PU.1, IRF8, BATF3) and cultured in the presence of Dox. (B) Bright field images of HDFs transduced with PIB at day 3 after addition of Dox. White arrowheads mark cells with typical DC-like morphology. M2rtTA transduced HDFs are shown as control. (C) Higher magnification of bright field images of PIB-transduced HDFs with DC-like morphology. (D) Flow cytometry analysis of CLEC9A and CD11c expression in PIB-transduced HDFs at day 10 after addition of Dox. (E) HDFs transduced with PIB were incubated overnight with FITC-labelled latex beads (1µm) and analysed by fluorescent microscopy at day 7 after addition of Dox. CellVue Claret Far Red and DAPI were used to stain cellular membranes and nuclei, respectively. (F) Flow cytometry analysis of PIB-transduced HDFs after incubation with Ovalbumine-AlexaFluor647 for 20 minutes at 37 °C at day 7 after addition of Dox. Controls were kept on ice (4°C). (G) PIB-transduced HDFs were incubated overnight with dead cells labelled with CellVue Claret Far Red membrane staining, 10 days after addition of Dox. Flow cytometry analysis of dead cell incorporation within CD11c positive and negative populations.

### DETAILED DESCRIPTION

The present disclosure relates to compositions, nucleic acid constructs, methods and kits thereof for cell induction or reprogramming cell to the dendritic cell state or antigen presenting cell state, based, in part, on the surprisingly effect described herein of novel use and combinations of transcription factors that permit induction or reprogramming of differentiated or undifferentiated cells into dendritic cells or antigen presenting cells. Such compositions, nucleic acid constructs, methods and kits can be used for inducing dendritic cells in vitro, ex vivo, or in vivo, and these induced dendritic cells or antigen presenting cells can be used for immunotherapy applications.

Natural DCs are bone marrow-derived cells that are seeded in all tissues. DCs are poised to sample the environment and to transmit the gathered information to cells of the adaptive immune system (T cells and B cells). Upon antigen engulfment, DCs initiate an immune response by presenting the processed antigen, which is in the form of peptide-major histocompatibility complex (MHC) molecule complexes, to naive (that is, antigen inexperienced) T cells in lymphoid tissues. After activation, DCs typically overexpress co-stimulatory and MHC molecules in addition to secrete various cytokines responsible for initiating and/or enhancing many T and B lymphocyte responses, i.e. type I interferon, tumor necrosis factor (TNF)-α, IFN-γ, IL-12 and IL-6. Thus, DCs are generally identified by their high expression of major histocompatibility complex class II molecules (MHC-II), co-stimulatory molecules, such as CD80/86 and CD40, and integrin CD11c, as well as their superior capacity to secrete inflammatory cytokines and to migrate from non-lymphoid to lymphoid organs and stimulate naïve T cells. In mice and humans, distinct subsets of DCs can be variably defined by phenotype, ontogeny, and function. They include the conventional DC subset 1 (cDC1, also kwon as CD8α+ DC subset) found in mouse lymphoid organs and the related CD103+ DC subset in non-lymphoid tissues. Cells bearing a similar phenotype have recently been described in humans, humanized mice, and sheep, indicating cross-species conservation of the cDC1 family. This extended family has distinct functional properties, most notably a remarkable efficiency at capturing material from dead or dying cells, as well as processing exogenous antigens for cross-presentation on MHC class I. These two features allow cDC1 DCs to cross-present cell-associated antigens and trigger CTL responses against infectious agents or tumors. In addition to priming CD8+T cells, cDC1+ DCs have been implicated in the establishment of cross-tolerance to tissue-specific cell-associated antigens. The ability of cDC1 DCs to either cross-prime or cross-tolerize CD8+ T cells against cell-associated antigens implies that they can decode the context in which they encounter dead cells. DNGR-1, also known as CLEC9A, is a receptor for necrotic cells that favors cross-priming of CTLs to dead cell-associated antigens in mice. DNGR-1 is selectively expressed at high levels by mouse cDC1 DCs, CD103+ DCs and by their human equivalents, being responsible for recognizing an intracellular ligand exposed after cell death. Recently, expression of Clec9a was shown to allow the identification of DC precursors (CDPs) committed to the conventional DC lineage and their progeny in lymphoid tissues (10).

The successful identification of DC inducing factors capable of reprogramming differentiated cells to induced DCs, as described herein, can advance our basic understanding of DC biology in a number of ways. This work will provide thorough insight into DC minimal transcriptional networks. In addition, the identification of DC inducing factors offer unprecedented opportunities to understand how DC state is established and how key regulatory machinery is put into place.

Transcription factors play a critical role in the specification of all cell types during development. The success of direct reprogramming strategies using transcription factor-mediated reprogramming indicates that it is equally plausible to direct the differentiation of pluripotent ES/iPS cells or multipotent stem cells to specific fates using such factors. Accordingly, using the DC inducing factors identified herein, directed differentiation of ES/iPS cells to a definitive DC fate by expression of the DC-enriched transcription factors can be achieved. Additionally, using the DC inducing factors identified herein, directed differentiation of multipotent hematopoietic stem and progenitor cells to a definitive DC fate by expression of the DC-enriched transcription factors can be achieved (forcing differentiation along the hematopoietic tree depicted in Figure 1).

Typically, nucleic acids encoding the DC inducing factors, e.g., DNA or RNA, or constructs thereof, are introduced into a cell, using viral vectors or without viral vectors, via one or repeated transfections, and the expression of the gene products and/or translation of the RNA molecules result in cells that are morphologically, biochemically, and functionally similar to DCs, as described herein.

An aspect of the present disclosure is the use of TFs or the use of a combination of TFs in cancer cells *(in situ* or *ex vivo)* to force them to present their own antigens to immune cells (**Figure 3**). This method represents a feasible strategy to increase the clinical outcome of anticancer immunotherapies as it bypasses cancer evasion mechanisms and increases tumor immunogenicity.

In an embodiment, we have cloned a list of 19 candidate TFs individually in a reprogramming proven Doxycycline (Dox)-inducible lentiviral vector (6).

In an embodiment, for screening the effect of the new dendritic cell-inducing TFs and DC-inducing TF combinations by cellular reprogramming, we have started with Mouse Embryonic Fibroblasts (MEFs) harboring a DC-specific reporter (Clec9a-Cre X R26-stop-tdTomato) and used the activation of the reporter to shown DC-inducing TFs. In Clec9a-tomato reporter mouse, the tdTomato fluorescent protein is expressed exclusively by CDPs, pre-DCs and in cDCs (26). Macrophages, other immune lineages or monocyte-derived DCs in culture do not express Clec9a and therefore the tdTomato protein (**Figure 4A**). Within the immune system Clec9a gene expression is selectively restricted to CDPs and their progeny (pre-cDCs and cDCs) (**Figure 4B**). Results from single cell gene expression analysis of cDC and precursors also highlighted that Clec9a expression is acquired after commitment to cDC lineage in CDPs and pre-DCs and not before in Monocyte DC progenitors (MDPs) (**Figure 4C**) (11).

Double transgenic Clec9a-tdTomato reporter MEFs were isolated from E13.5 embryos and excluded from any contaminating tdTomato+ or CD45+ cell that could be already committed to the hematopoietic lineage (**Figure 5A and 5B**) by Fluorescent-Activated Cell Sorting (FACS). MEFs used for screening and in the following experiments were tdTomato- CD45- with a purity of 99.8% (**Figure 5C**).

In an embodiment, PU.1, IRF8 and BATF3 are sufficient for Clec9a activation and to impose dendritic cell morphology.

Clec9a reporter MEFs were transduced with combinations of candidate TFs and evaluated for tdTomato expression (**Figure 6A**). After transduction with a pool of selected 4TFs or all 19TFs, we observed the emergence of tdTomato+ cells 5 days after adding Dox (**Figure 6B**). The pool of 4TFs generated more tdTomato+ cells than the pool of all TFs (2.28% versus 0.59%, respectively) suggesting that the minimal combination of factors required to induced reporter activation is contained within the pool of 4TFs. TdTomato+ cells were not observed with control vectors. We then removed each of the 4 TFs individually from the pool of 4TFs (Figure 6C). Pu.1, Irf8 and Batf3 removal reduced reporter activation while removal of IRF4 did not have an impact. These results suggest that only 3 of the 4 TFs are important for DC specification.

In an embodiment when the combination of PU.1, IRF8 or BATF3 (PIB) was expressed in MEFs the Clec9-reporter is activated with an increased efficiency (approx. 3.96%, Figure 7A). In an embodiment was then evaluated the kinetics of reporter activation (**Figure 7B**). TdTomato+ cells start to be detected between day 1 and day 2 and peak between day 5 and day 7 (**Figure 7B**). In an embodiment removal of PU.1, IRF8 or BATF3 completely abolished reporter activation whereas their individual expression was not sufficient to generate tdTomato+ cells (**Figure 7C**). These data suggest that in this embodiment PIB constitute the minimal combination of TFs for Clec9a activation and induced Dendritic Cell (iDC) generation. Importantly, tdTomato+ cells display increased size and complexity (**Figure 7D**), consistent with the observed stellate morphology and the establishment of dendrites characteristic of DCs (**Figure 7E and 7F**).

In an embodiment was evaluated the impact of expressing additional factors to PIB. It was assessed the individual impact of each of the TFs from the candidate pool of 19 TFs as well as other hematopoietic TFs (**Figure 8**). From the 30 TFs tested we observed that STAT3, IKZF1, NFIL3, L-MYC, RUNX1, NFE2 and KLF4 negatively impact the numbers of tdTomato+ cells generated. The addition of TCF4 has the most dramatic increase in reporter activation (6.95 ± 1.84%).

In an effort to optimize the culture conditions for iDC generation it was tested the addition of the cytokines Granulocyte-macrophage colony-stimulating factor (GM-CSF), IL-4 and FMS-like tyrosine kinase 3 ligand (Flt3l) during the induction (**Figure 9A**) because of their important role during DC specification (12). We also tested adding lipopolysaccharides (LPS), different media compositions (RPMI, 2-mercaptoethanol (2-ME) and 4mM L-Glutamine (2xGlut) and co-culture with the stromal cells OP-9 and OP9-DL1 (**Figure 9B**) (13). These culture modifications did not increase the number of induced tdTomato+ cells.

In an embodiment, the *in vivo* expression patterns of the PIB and TCF4 were analysed. *PU.1, IRF8, BATF3* and *TCF4* transcripts are expressed in single DC precursor cells (**Figure 10**). While *Pu.1* is equally expressed in MDPs, CDPs and Pre-DCs, *IRF8* expression markedly increases in CDPs and is maintained in pre-DCs. *BATF3* and *TCF4* are only up-regulated at a later stage, in pre-DCs.

In an embodiment was evaluated, if the activation of the Clec9a-tdTomato reporter was reflected in the expression of key components of the antigen presentation machinery at the cell surface. Remarkably we observed that 71.4% of tdTomato+ cells at day 7 expressed MHC-II at the surface (**Figure** 11A), a key molecule for the establishment of APC functionality. The expression of MHC-II is gradually acquired starting between day 1 and day 2 and peak between day 7 and day 11 (Figure 11B). The kinetics of MHC-II activation resembles the activation of the Clec9a reporter (Figure 7B). At day 7 the tdTomato-compartment contained a lower percentage of MHC-II+cells (14.2%) (Figure 11A). This population of cells may represent partially reprogrammed cells that do not activate the reporter or a subset of DCs where Clec9a is not active. We then addressed whether expression of MHC-II was controlled directly by PIB factors. By excluding each of the factors individually we observed that PU.1 expression, but not IRF8 or BATF3 expression, is important for MHC-II activation (**Figure 14C**), consistent with the described Pu.1 role in regulating Class II Transactivator (CIITA) through its promoter I (CIITApl) (14, 15). CIITA is known as the master regulator of MHC Class II genes' expression, determining cell-type-specific, cytokine-induced and developmental-derived modulation of MHC-II expression through the differential usage of CIITA promoters (16). In DCs, CIITApl has been associated with regulation of MHC-II genes.

In an embodiment, due to the described involvement of IRF4 in inducing MHC-II expression through interaction with CIITA (17), it was evaluated whether IRF4 inclusion would increase the generation of MHC-II+ cells within the tdTomato+ population. We therefore assessed the expression of MHC-II in tdTomato+ cells generated by 4TFs or their individual exclusion (**Figure 11D**). Inclusion of IRF4 in the pool did not increase MHC-II expression on tdTomato+ cells. Accordingly, IRF4 and PU.1 were found to synergistically promote MHC-II expression through CIITA promoter III in B cells but not DCs (17). During reprogramming to iDCs, no synergism with PU.1 was observed, which was strictly required for MHC-II expression in tdTomato+ cells.

In an embodiment, it was evaluated the expression the co-stimulatory molecules CD80 and CD86, required for efficient antigen presentation (**Figure 12**). CD80 and CD86 are expressed in 35.2% of tdTomato+MHC-II+ cells in contrast to only 12.9% of tdTomato+ MHC-II- cells. This characterization of the expression of MHC-II, CD80 and CD86 at the cell surface of iDCs suggests that a cohort of tdTomato+ MHC-II+ cells would be competent in antigen presentation.

In an embodiment, in addition to the membrane associated co-stimulatory molecules, mature DCs express cytokines with a pro-inflammatory function that are important for the development of T-cell responses. These responses can be initiated by the triggering of at least 11 different Toll-like receptors (TLRs), allowing the specific recognition of distinct conserved microbial or viral structures. We asked whether iDCs secrete cytokines to the media when challenged with TLR3 (using Polyinosinic-polycytidylic acid (poly-I:C)) or TLR4 (Lipopolysaccharides (LPS)) stimulation (**Figure 13**). Upon LPS or polyl:C challenge of iDCs we observed an increase in the secretion of IL-6 (14-or 10-fold, respectively). An increase in the secretion of tumor necrosis factor TNF (7-fold) and interferon IFN-γ (2-fold) was also observed after LPS stimulation or polyl:C, respectively. Cells transduced with PIB plus TCF4 (PIBT) respond equivalently to stimulation displaying increase secretion of IL-6, TNF and IFN-γ. Importantly, upon stimulation of iDCs we did not observed increase in the secretion of the anti-inflammatory cytokine IL-10. These results suggest that iDCs underwent maturation towards a proinflammatory profile.

In an embodiment, it was evaluated the capacity of iDCs to mount an antigen-specific immune response. First it was evaluated whether iDCs would be able to engulf particles by incubation with µm FITC-labeled latex beads. After incubation tdTomato+ cells contained numerous fluorescent beads in the cytoplasm (**Figure 14**), suggesting that iDCs have established the competence for phagocytosis.

In an embodiment, it was evaluated the functional capacity of iDCs to promote antigen-specific proliferation of CD4 T-cells (**Figure 15A**). For this we employed MHC class II-restricted ovalbumin-specific T cells (OT-II cells) isolated from lymph nodes and spleen of OT-II Rag2 KO mice (17, 18). In this model T cells respond to the processed antigenic peptide (OVA 323-339) when shown in the context of MHC-II of DCs. Therefore OT-II CD4 T-cells were co-cultured with iDCs when given the Ovalbumine protein (OVA) or pre-processed antigenic peptide (OVA 323-339). Functional DCs are able to capture the protein, process and present the processed antigenic peptide in the context of MHC-II. Induced CD4+ T cell proliferation was measured by CFSE dilution and the activation of the T-cell activation marker CD44 after 7 days of co-culture. Remarkably, 56% of CD4+ T cells diluted CFSE when co-cultured with PIB-generated iDCs in the presence of OVA protein (**Figure 15B**). When co-cultured with MEFs transduced with PIB+TCF4, 38.2% of CD4+ T cells diluted CFSE content, which suggests that inclusion of TCF4 in the reprogramming pool does not increase the stimulatory ability of iDCs. Splenic MHC-II+CD11c+ DCs were used as controls and generated 24.1% of proliferative T-cells. Importantly, addition of LPS stimuli, which is commonly employed to induce DC "maturation", increased the antigen-specific stimulatory ability of MEFs transduced with PIB (1.5-fold) or PIB+TCF4 (2-fold) and also splenic DCs (3-fold) (Figure 15B). As expected, T-cells that were not co-cultured did not proliferate with or without LPS stimuli. To further assess the stimulatory ability of iDCs, it was evaluated if they were able to induce expression of T-cell activation markers, such as CD44. When given the pre-processed antigen, OT-II CD4 T cells diluted CFSE and upregulated the expression of CD44 when co-cultured with both PIB-generated iDCs and splenic MHC-II+CD11c+ DCs (**Figure 15C**). Importantly, when given OVA protein, iDCs display comparable ability to induce CD44 expression in OT-II T cells when compared with splenic DCs (52.2% versus 63.8%). This data supports iDCs' functional ability to incorporate and process OVA protein followed by presentation of processed Ovalbumin peptides in MHC-II complexes at cell surface. Collectively, our results highlight the functional capacity of iDCs and support the feasibility of using directly reprogrammed fibroblast to present antigens and inducing antigen-specific adaptive immune responses.

In an embodiment, it was evaluated if iDCs are able to cross-present antigens to CD8+ T cells. For this, we employed MHC class I-restricted ovalbumin-specific T cells (OT-I cells) isolated from lymph nodes and spleen of OT-I Rag2 KO mice (19). In this model T cells respond to the processed antigenic peptide (OVA 257-264) when shown in the context of MHC-I of DCs. Therefore OT-I CD8 T-cells were co-cultured with iDCs when given the Ovalbumine protein (OVA) or pre-processed antigenic peptide (OVA 257-264) (Figure 16A). Functional DCs are able to capture the exogenous protein, process and perform cross-presentation of the processed antigenic peptide in the context of MHC-I, inducing activation of CD8+ T cells. Induced CD8+ T cell proliferation was measured by CFSE dilution and the activation of the T-cell activation marker CD44 after 4 days of co-culture. Remarkably, in the presence of OVA protein, 6.33% or 10.5% of CD8+ T cells diluted CFSE and up-regulated CD44 expression when co-cultured with PIB or PIB+TCF4-generated iDCs, respectively (**Figure 16B**). Splenic MHC-II+CD11c+ DCs were used as controls and generated 18.60% of proliferative and CD44+ T-cells.

In an embodiment, Human Dermal Fibroblasts (HDFs) were transduced with PIB (Figure 17A). Importantly, it was observed morphologic alterations when PIB TFs were introduced in HDFs. Three days after transduction we observed that HDFs lost the characteristic bipolar and elongated shapes and acquired a stellate DC-like morphology (Figure 17B and 17C). PIB-transduced HDFs also express CLEC9A and CD11c, known specific human DC markers (Figure 17D), suggesting that PIB combination of DC-inducing factors is conserved between the mouse and human and is sufficient to generate human iDCs.

In an embodiment it was evaluated whether iDCs would be able to engulf particles by incubation with 1µm FITC-labeled latex beads. After incubation PIB-transduce HDFs contained numerous fluorescent beads in the cytoplasm (Figure **17E**), suggesting that iDCs have established the competence for phagocytosis. Additionally, the ability to incorporate proteins was evaluated by incubating iDCs with AlexaFluor647-labelled ovalbumine (Figure 17F). After incubation at 37°C, 1.18% of PIB-transduced HDFs contained labelled protein, suggesting that iDCs are able to actively engulf proteins. Dead cell incorporation was also evaluated by incubating PIB-transduced HDFs with cells labelled with membrane staining (Figure 17G). Specifically, CD11c+ population of PIB-transduced HDFs acquired the far red labelling supporting iDCs ability to incorporate dead cells, a key feature for antigen presentation and dendritic cell function.

In an embodiment, coding regions of each candidate TF were individually cloned into an inducible lentiviral pFUW-TetO vector (6) in which the expression of the TFs is under the control of the tetracycline operator and a minimal CMV promoter. A previously described lentiviral vector containing the reverse tetracycline transactivator M2rtTA under the control of a constitutively active human ubiquitin C promoter (FUW-M2rtTA) was used in combination. Human Embryonic Kidney (HEK) 293T cells were transfected with a mixture of TF-encoding plasmids, packaging constructs and the VSV-G envelope protein. Viral supernatants were harvested after 36, 48 and 60 hours, filtered (0.45 µm, Corning) and used fresh or concentrated 40-fold with Amicon ultra centrifugal filters (Millipore).

In some embodiments, polypeptide variants or family members having the same or a similar activity as the reference polypeptide encoded by the sequences provided in Table 1 can be used in the compositions, methods, and kits described herein. Generally, variants of a particular polypeptide encoding a DC inducing factor for use in the compositions, methods, and kits described herein will have at least about 95%, at least about 96%, at least about 97%, at least about 98%, at least about 99% or more sequence identity to that particular reference polynucleotide or polypeptide as determined by sequence alignment programs and parameters described herein and known to those skilled in the art.

*Homo sapiens* Basic Leucine Zipper ATF-Like Transcription Factor (BATF3), mRNA (SEQ. ID. 1) and a codon optimized, or different codons encoding the same amino acids, are naturally also contemplated to be covered by the reference to the nucleic acid as set forth herein.

*Homo sapiens* Spi-1 proto-oncogene (PU.1), mRNA (SEQ. ID. 7) and a codon optimized, or different codons encoding the same amino acids, are naturally also contemplated to be covered by the reference to the nucleic acid as set forth herein.

*Homo sapiens* Interferon Regulatory Factor 8 (IRF8), mRNA (SEQ. ID. 5) and a codon optimized, or different codons encoding the same amino acids, are naturally also contemplated to be covered by the reference to the nucleic acid as set forth herein.

*Homo sapiens* Transcription factor 4 (TCF4), mRNA (SEQ. ID. 13) and a codon optimized, or different codons encoding the same amino acids, are naturally also contemplated to be covered by the reference to the nucleic acid as set forth herein.

In some embodiments of the compositions, methods, and kids provided herein, the number of DC inducing factors used or selected to generate iDCs from a starting somatic cell, such as a fibroblast cell or hematopoietic lineage cell, a multipotent stem cell, an induced pluripotent stem cell, a cancer or tumor cell is at least three. In some embodiments, the number of DC inducing factors used or selected is at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least thirty, at least thirty three, at least thirty five, at least forty, or more.

Also provided herein, in various aspects of the compositions, methods, and kits, are isolated amino acid sequences, and isolated DNA or RNA nucleic acid sequences encoding one or more DC inducing factors for use in making iDCs.

In some embodiments of the compositions, methods, and kits described herein, the nucleic acid sequence or construct encoding the DC inducing factor(s), such as PU.1, IRF8, BATF3 and TCF4, is inserted or operably linked into a suitable expression vector for transfection of cells using standard molecular biology techniques. As used herein, a "vector" refers to a nucleic acid molecule, such as a dsDNA molecule that provides a useful biological or biochemical property to an inserted nucleotide sequence, such as the nucleic acid constructs or replacement cassettes described herein. Examples include plasmids, phages, autonomously replicating sequences (ARS), centromeres, and other sequences that are able to replicate or be replicated in vitro or in a host cell, or to convey a desired nucleic acid segment to a desired location within a host cell. A vector can have one or more restriction endonuclease recognition sites (whether type I, II or IIs) at which the sequences can be cut in a determinable fashion without loss of an essential biological function of the vector, and into which a nucleic acid fragment can be spliced or inserted in order to bring about its replication and cloning. Vectors can also comprise one or more recombination sites that permit exchange of nucleic acid sequences between two nucleic acid molecules. Vectors can further provide primer sites, e.g., for PCR, transcriptional and/or translational initiation and/or regulation sites, recombination signals, replicons, additional selectable markers, etc. A vector can further comprise one or more selectable markers suitable for use in the identification of cells transformed with the vector.

In some embodiments of the compositions, methods, and kits described herein, the expression vector is a viral vector. Some viral-mediated expression methods employ retrovirus, adenovirus, lentivirus, herpes virus, pox virus, and adeno-associated virus (AAV) vectors, and such expression methods have been used in gene delivery and are well known in the art.

In some embodiments of the compositions, methods, and kits described herein, the viral vector is a retrovirus. Retroviruses provide a convenient platform for gene delivery. A selected gene can be inserted into a vector and packaged in retroviral particles using techniques known in the art. The recombinant virus can then be isolated and delivered to target cells of the subject either in vivo or ex vivo. A number of retroviral systems have been described. See, e.g., U.S. Pat. No. 5,219,740; Miller and Rosman (1989) BioTechniques 7:980-90; Miller, A. D. (1990) Human Gene Therapy 1:5-14; Scarpa et al. (1991) Virology 180:849-52; Burns et al. (1993) Proc. Natl. Acad. Sci. USA 90:8033-37; Boris-Lawrie and Temin (1993) Curr. Opin. Genet. Develop. 3:102-09. In some embodiments of the compositions, methods, and kits described herein, the retrovirus is replication deficient. Retroviral vector systems exploit the fact that a minimal vector containing the 5' and 3' LTRs and the packaging signal are sufficient to allow vector packaging, infection and integration into target cells, provided that the viral structural proteins are supplied in trans in the packaging cell line. Fundamental advantages of retroviral vectors for gene transfer include efficient infection and gene expression in most cell types, precise single copy vector integration into target cell chromosomal DNA and ease of manipulation of the retroviral genome.

In some embodiments of the compositions, methods, and kits described herein, the viral vector is an adenovirus-based expression vector. Unlike retroviruses, which integrate into the host genome, adenoviruses persist extrachromosomally, thus minimizing the risks associated with insertional mutagenesis (Haj-Ahmad and Graham (1986) J. Virol. 57:267-74; Bett et al. (1993) J. Virol. 67:5911-21; Mittereder et al. (1994) Human Gene Therapy 5:717-29; Seth et al. (1994) J. Virol. 68:933-40; Barr et al. (1994) Gene Therapy 1:51-58; Berkner, K. L. (1988) BioTechniques 6:616-29; and Rich et al. (1993) Human Gene Therapy 4:461-76). Adenoviral vectors infect a wide variety of cells, have a broad host-range, exhibit high efficiencies of infectivity, direct expression of heterologous genes at high levels, and achieve long-term expression of those genes in vivo. The virus is fully infective as a cell-free virion so injection of producer cell lines is not necessary. With regard to safety, adenovirus is not associated with severe human pathology, and the recombinant vectors derived from the virus can be rendered replication defective by deletions in the early-region 1 ("E1") of the viral genome. Adenovirus can also be produced in large quantities with relative ease. Adenoviral vectors for use in the compositions, methods, and kits described herein can be derived from any of the various adenoviral serotypes, including, without limitation, any of the over 40 serotype strains of adenovirus, such as serotypes 2, 5, 12, 40, and 41. The adenoviral vectors used herein are preferably replication-deficient and contain the DC inducing factor of interest operably linked to a suitable promoter.

In some embodiments of the compositions, methods, and kits described herein, the nucleic acid sequences encoding the DC inducing factor(s), such as, PU.1, IRF8, BATF3 and TCF4 are introduced or delivered using one or more inducible lentiviral vectors. Control of expression of DC inducing factors delivered using one or more inducible lentiviral vectors can be achieved, in some embodiments, by contacting a cell having at least one DC inducing factor in an expression vector under the control of or operably linked to an inducible promoter, with a regulatory agent (e.g., doxycycline) or other inducing agent. When using some types of inducible lentiviral vectors, contacting such a cell with an inducing agent induces expression of the DC inducing factors, while withdrawal of the regulatory agent inhibits expression. When using other types of inducible lentiviral vectors, the presence of the regulatory agent inhibits expression, while removal of the regulatory agent permits expression. As used herein, the term "induction of expression" refers to the expression of a gene, such as a DC inducing factor encoded by an inducible viral vector, in the presence of an inducing agent, for example, or in the presence of one or more agents or factors that cause endogenous expression of the gene in a cell.

In some embodiments of the aspects described herein, a doxycycline (Dox) inducible lentiviral system is used. Unlike retroviruses, lentiviruses are able to transduce quiescent cells making them amenable for transducing a wider variety of hematopoietic cell types. For example, the pFUW-tetO lentivirus system has been shown to transduce primary hematopoietic progenitor cells with high efficiency.

In some embodiments of the methods described herein, the nucleic acid sequences encoding the DC inducing factor(s), such as PU.1 (SEQ. ID. 7, SEQ. ID. 8), IRF8 (SEQ. ID. 5, SEQ. ID. 6), BATF3 (SEQ. ID. 1, SEQ. ID. 2) and/or TCF4 (SEQ. ID. 13, SEQ. ID. 14), are introduced or delivered using a non-integrating vector (e.g., adenovirus). While integrating vectors, such as retroviral vectors, incorporate into the host cell genome and can potentially disrupt normal gene function, non-integrating vectors control expression of a gene product by extra-chromosomal transcription. Since non-integrating vectors do not become part of the host genome, non-integrating vectors tend to express a nucleic acid transiently in a cell population. This is due in part to the fact that the non-integrating vectors are often rendered replication deficient. Thus, non-integrating vectors have several advantages over retroviral vectors including, but not limited to: (1) no disruption of the host genome, and (2) transient expression, and (3) no remaining viral integration products. Some non-limiting examples of non-integrating vectors for use with the methods described herein include adenovirus, baculovirus, alphavirus, picornavirus, and vaccinia virus. In some embodiments of the methods described herein, the non-integrating viral vector is an adenovirus. Other advantages of non-integrating viral vectors include the ability to produce them in high titers, their stability in vivo, and their efficient infection of host cells.

Nucleic acid constructs and vectors for use in generating iDCs in the compositions, methods, and kits described herein can further comprise, in some embodiments, one or more sequences encoding selection markers for positive and negative selection of cells. Such selection marker sequences can typically provide properties of resistance or sensitivity to antibiotics that are not normally found in the cells in the absence of introduction of the nucleic acid construct. A selectable marker can be used in conjunction with a selection agent, such as an antibiotic, to select in culture for cells expressing the inserted nucleic acid construct. Sequences encoding positive selection markers typically provide antibiotic resistance, i.e., when the positive selection marker sequence is present in the genome of a cell, the cell is sensitive to the antibiotic or agent. Sequences encoding negative selection markers typically provide sensitivity to an antibiotic or agent, i.e., when the negative selection marker is present in the genome of a cell, the cell is sensitive to the antibiotic or agent.

Nucleic acid constructs and vectors for use in making iDCs in the compositions, methods, and kits thereof described herein can further comprise, in some embodiments, other nucleic acid elements for the regulation, expression, stabilization of the construct or of other vector genetic elements, for example, promoters, enhancers, TATA-box, ribosome binding sites, IRES, as known to one of ordinary skill in the art.

In some embodiments of the compositions, methods, and kits described herein, the DC inducing factor(s), such as PU.1 (SEQ. ID. 7, SEQ. ID. 8), IRF8 (SEQ. ID. 5, SEQ. ID. 6), BATF3 (SEQ. ID. 1, SEQ. ID. 2) and/or TCF4 (SEQ. ID. 13, SEQ. ID. 14), are provided as synthetic, modified RNAs, or introduced or delivered into a cell as a synthetic, modified RNA, as described in US Patent Publication 2012-0046346-A1, the contents of which are herein incorporated by reference in their entireties. In those embodiments where synthetic, modified RNAs are used to reprogram cells to iDCs according to the methods described herein, the methods can involve repeated contacting of the cells or involve repeated transfections of the synthetic, modified RNAs encoding DC inducing factors, such as for example, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, or more transfections.

In addition to one or more modified nucleosides, the modified mRNAs for use in the compositions, methods, and kits described herein can comprise any additional modifications known to one of skill in the art and as described in US Patent Publications 2012-0046346-A1 and 20120251618A1, and PCT Publication WO 2012/019168. Such other components include, for example, a 5' cap (e.g., the Anti-Reverse Cap Analog (ARCA) cap, which contains a 5'-5'-triphosphate guanine-guanine linkage where one guanine contains an N7 methyl group as well as a 3'-O-methyl group; caps created using recombinant Vaccinia Virus Capping Enzyme and recombinant 2'-O-methyltransferase enzyme, which can create a canonical 5'-5'-triphosphate linkage between the 5'-most nucleotide of an mRNA and a guanine nucleotide where the guanine contains an N7 methylation and the ultimate 5'-nucleotide contains a 2'-O-methyl generating the Cap1 structure); a poly(A) tail (e.g., a poly-A tail greater than 30 nucleotides in length, greater than 35 nucleotides in length, at least 40 nucleotides, at least 45 nucleotides, at least 55 nucleotides, at least 60 nucleotide, at least 70 nucleotides, at least 80 nucleotides, at least 90 nucleotides, at least 100 nucleotides, at least 200 nucleotides, at least 300 nucleotides, at least 400 nucleotides, at least 500 nucleotides, at least 600 nucleotides, at least 700 nucleotides, at least 800 nucleotides, at least 900 nucleotides, at least 1000 nucleotides, or more); a Kozak sequence; a 3' untranslated region (3' UTR); a 5' untranslated region (5' UTR); one or more intronic nucleotide sequences capable of being excised from the nucleic acid, or any combination thereof.

The modified mRNAs for use in the compositions, methods, and kits described herein can further comprise an internal ribosome entry site (IRES). An IRES can act as the sole ribosome binding site, or can serve as one of multiple ribosome binding sites of an mRNA. An mRNA containing more than one functional ribosome binding site can encode several peptides or polypeptides, such as the DC inducing factors described herein, that are translated independently by the ribosomes ("multicistronic mRNA"). When nucleic acids are provided with an IRES, further optionally provided is a second translatable region. Examples of IRES sequences that can be used according to the invention include without limitation, those from picornaviruses (e.g. FMDV), pest viruses (CFFV), polio viruses (PV), encephalomyocarditis viruses (ECMV), foot-and-mouth disease viruses (FMDV), hepatitis C viruses (HCV), classical swine fever viruses (CSFV), murine leukemia virus (MLV), simian immune deficiency viruses (SW) or cricket paralysis viruses (CrPV).

In some embodiments of the compositions, methods, and kits described herein, the synthetic, modified RNA molecule comprises at least one modified nucleoside. In some embodiments of the compositions, methods, and kits described herein, the synthetic, modified RNA molecule comprises at least two modified nucleosides.

In some embodiments of the compositions, methods, and kits described herein, the modified nucleosides are selected from the group consisting of 5-methylcytosine (5mC), N6-methyladenosine (m6A), 3,2'-O-dimethyluridine (m4U), 2-thiouridine (s2U), 2' fluorouridine, pseudouridine, 2'-O-methyluridine (Um), 2'deoxy uridine (2' dU), 4-thiouridine (s4U), 5-methyluridine (m5U), 2'-O-methyladenosine (m6A), N6,2'-O-dimethyladenosine (m6Am), N6,N6,2'-O-trimethyladenosine (m62Am), 2'-O-methylcytidine (Cm), 7-methylguanosine (m7G), 2'-O-methylguanosine (Gm), N2,7-dimethylguanosine (m2,7G), N2,N2,7-trimethylguanosine (m2,2,7G), and inosine (I). In some embodiments, the modified nucleosides are 5-methylcytosine (5mC), pseudouracil, or a combination thereof.

Modified mRNAs need not be uniformly modified along the entire length of the molecule. Different nucleotide modifications and/or backbone structures can exist at various positions in the nucleic acid. One of ordinary skill in the art will appreciate that the nucleotide analogs or other modification(s) can be located at any position(s) of a nucleic acid such that the function of the nucleic acid is not substantially decreased. A modification can also be a 5' or 3' terminal modification. The nucleic acids can contain at a minimum one and at maximum 100% modified nucleotides, or any intervening percentage, such as at least 50% modified nucleotides, at least 80% modified nucleotides, or at least 90% modified nucleotides.

In some embodiments, it is preferred, but not absolutely necessary, that each occurrence of a given nucleoside in a molecule is modified (e.g., each cytosine is a modified cytosine e.g., 5-methylcytosine, each uracil is a modified uracil, e.g., pseudouracil, etc.). For example, the modified mRNAs can comprise a modified pyrimidine such as uracil or cytosine. In some embodiments, at least 25%, at least 50%, at least 80%, at least 90% or 100% of the uracil in the nucleic acid are replaced with a modified uracil. It is also contemplated that different occurrences of the same nucleoside can be modified in a different way in a given synthetic, modified RNA molecule. The modified uracil can be replaced by a compound having a single unique structure, or can be replaced by a plurality of compounds having different structures (e.g., 2, 3, 4 or more unique structures). In some embodiments, at least 25%, at least 50%, at least 80%, at least 90% or 100% of the cytosine in the nucleic acid may be replaced with a modified cytosine. The modified cytosine can be replaced by a compound having a single unique structure, or can be replaced by a plurality of compounds having different structures (e.g., 2, 3, 4 or more unique structures) (e.g., some cytosines modified as 5mC, others modified as 2'-O-methylcytosine or other cytosine analog). Such multi-modified synthetic RNA molecules can be produced by using a ribonucleoside blend or mixture comprising all the desired modified nucleosides, such that when the RNA molecules are being synthesized, only the desired modified nucleosides are incorporated into the resulting RNA molecule encoding the DC inducing factor.

In certain embodiments it is desirable to intracellularly degrade a modified nucleic acid introduced into the cell, for example if precise timing of protein production is desired. Thus, in some embodiments of the compositions, methods, and kits described herein, provided herein are modified nucleic acids comprising a degradation domain, which is capable of being acted on in a directed manner within a cell.

While it is understood that iDCs can be generated by delivery of DC inducing factors in the form of nucleic acid (DNA or RNA) or amino acid sequences, in some embodiments of the compositions, methods, and kits described herein, iDC induction can be induced using other methods, such as, for example, by treatment of cells with an agent, such as a small molecule or cocktail of small molecules, that induce expression one or more of the DC inducing factors.

Detection of expression of DC inducing factors introduced into cells or induced in a cell population using the compositions, methods, and kits described herein, can be achieved by any of several techniques known to those of skill in the art including, for example, Western blot analysis, immunocytochemistry, and fluorescence-mediated detection.

In order to distinguish whether a given combination of DC inducing factors has generated iDCs, one or more DC activities or parameters can be measured, such as, in some embodiments, differential expression of surface antigens. The generation of induced DCs using the compositions, methods, and kits described herein preferably causes the appearance of the cell surface phenotype characteristic of endogenous DCs, such as CLEC9A, MHC-II, CD80, CD86, CD11c, CD103, FLT3 for example.

DCs are most reliably distinguished from other immune cells by their functional behavior. Functional aspects of DC phenotypes, or dendritic cell activities, such as the ability of a dendritic cell to induce antigen specific T cell responses, can be easily determined by one of skill in the art using routine methods known in the art, and as described herein, for example, in the Drawings, i.e., FIGS. 1-17. In some embodiments of the aspects described herein, functional assays to identify reprogramming factors can be used. For example, in some embodiments, antigen presentation and antigen cross-presentation assays can be used to confirm antigen-specific induction of T cell responses (antigen presentation potential) of iDCs generated using the compositions, methods, and kits thereof. Cytokine secretion can be used to confirm immune-modulatory properties of iDCs generated using the compositions, methods, and kits described herein. Ability to engulf particles, proteins and dead cells of iDCs generated using the compositions, methods, and kits described herein can be evaluated by culturing transduced cells in the presence of labelled beads, ovalbumine or dead cells, followed by flow cytometry analysis, respectively.

As used herein, "cellular parameter," "DC parameter," or "antigen presentation activity" refer to measureable components or qualities of endogenous or natural DCs, particularly components that can be accurately measured. A cellular parameter can be any measurable parameter related to a phenotype, function, or behavior of a cell. Such cellular parameters include, changes in characteristics and markers of a DC or DC population, including but not limited to changes in viability, cell growth, expression of one or more or a combination of markers, such as cell surface determinants, such as receptors, proteins, including conformational or posttranslational modification thereof, lipids, carbohydrates, organic or inorganic molecules, nucleic acids, e.g. mRNA, DNA, global gene expression patterns, etc. Such cellular parameters can be measured using any of a variety of assays known to one of skill in the art. For example, viability and cell growth can be measured by assays such as Trypan blue exclusion, CFSE dilution, and 3H-thymidine incorporation. Expression of protein or polypeptide markers can be measured, for example, using flow cytometric assays, Western blot techniques, or microscopy methods. Gene expression profiles can be assayed, for example, using RNA-sequencing methodologies and quantitative or semi-quantitative real-time PCR assays. A cellular parameter can also refer to a functional parameter or functional activity. While most cellular parameters will provide a quantitative readout, in some instances a semi-quantitative or qualitative result can be acceptable. Readouts can include a single determined value, or can include mean, median value or the variance, etc. Characteristically a range of parameter readout values can be obtained for each parameter from a multiplicity of the same assays. Variability is expected and a range of values for each of the set of test parameters will be obtained using standard statistical methods with a common statistical method used to provide single values.

In some embodiments of the compositions, methods, and kits described herein, additional factors and agents can be used to enhance iDC reprogramming. For example, factors and agents that modify epigenetic pathways can be used to facilitate reprogramming into iDCs.

Essentially any primary somatic cell type can be used for producing iDCs or reprogramming somatic cells to iDCs according to the presently described compositions, methods, and kits. Such primary somatic cell types also include other stem cell types, including pluripotent stem cells, such as induced pluripotent stem cells (iPS cells); other multipotent stem cells; oligopotent stem cells; and (5) unipotent stem cells. Some non-limiting examples of primary somatic cells useful in the various aspects and embodiments of the methods described herein include, but are not limited to, fibroblast, epithelial, endothelial, neuronal, adipose, cardiac, skeletal muscle, hematopoietic or immune cells, hepatic, splenic, lung, circulating blood cells, gastrointestinal, renal, bone marrow, and pancreatic cells, as well as stem cells from which those cells are derived. The cell can be a primary cell isolated from any somatic tissue including, but not limited to, spleen, bone marrow, blood, brain, liver, lung, gut, stomach, intestine, fat, muscle, uterus, skin, spleen, endocrine organ, bone, etc. The term "somatic cell" further encompasses, in some embodiments, primary cells grown in culture, provided that the somatic cells are not immortalized. Where the cell is maintained under in vitro conditions, conventional tissue culture conditions and methods can be used, and are known to those of skill in the art. Isolation and culture methods for various primary somatic cells are well within the abilities of one skilled in the art.

In some embodiments of these aspects and all such aspects described herein, the somatic cell is a fibroblast cell.

In some embodiments of these aspects and all such aspects described herein, the somatic cell can be a hematopoietic lineage cell.

In some embodiments of these aspects and all such aspects described herein, the somatic cell can be a cancer cell or a tumor cell.

In some embodiments of the compositions, methods, and kits described herein, a somatic cell to be reprogrammed or made into an iDC cell is a cell of hematopoietic origin. As used herein, the terms "hematopoietic-derived cell," "hematopoietic-derived differentiated cell," "hematopoietic lineage cell," and "cell of hematopoietic origin" refer to cells derived or differentiated from a multipotent hematopoietic stem cell (HSC). Accordingly, hematopoietic lineage cells for use with the compositions, methods, and kits described herein include multipotent, oligopotent, and lineage-restricted hematopoietic progenitor cells, granulocytes (e.g., promyelocytes, neutrophils, eosinophils, basophils), erythrocytes (e.g., reticulocytes, erythrocytes), thrombocytes (e.g., megakaryoblasts, platelet producing megakaryocytes, platelets), monocytes (e.g., monocytes, macrophages), dendritic cells, and lymphocytes (e.g., T-lymphocytes, which carry T-cell receptors (TCRs), B-lymphocytes or B cells, which express immunoglobulin and produce antibodies, NK cells, NKT cells, and innate lymphocytes). As used herein, the term "hematopoietic progenitor cells" refer to multipotent, oligopotent, and lineage-restricted hematopoietic cells capable of differentiating into two or more cell types of the hematopoietic system, including, but not limited to, granulocytes, monocytes, erythrocytes, megakaryocytes, and lymphocytes B-cells and T-cells. Hematopoietic progenitor cells encompass multi-potent progenitor cells (MPPs), common myeloid progenitor cells (CMPs), common lymphoid progenitor cells (CLPs), granulocyte-monocyte progenitor cells (GMPs), and pre-megakaryocyte-erythrocyte progenitor cell. Lineage-restricted hematopoietic progenitor cells include megakaryocyte-erythrocyte progenitor cells (MEP), ProB cells, PreB cells, PreProB cells, ProT cells, double-negative T cells, pro-NK cells, pre-granulocyte/macrophage cells, granulocyte/macrophage progenitor (GMP) cells, and pro-mast cells (ProMCs). A differentiation chart of the hematopoietic lineage is provided at Figure 1.

Cells of hematopoietic origin for use in the compositions, methods, and kits described herein can be obtained from any source known to comprise these cells, such as fetal tissues, umbilical cord blood, bone marrow, peripheral blood, mobilized peripheral blood, spleen, liver, thymus, lymph, etc. Cells obtained from these sources can be expanded ex vivo using any method acceptable to those skilled in the art prior to use in with the compositions, methods, and kits for making iDCs described herein. For example, cells can be sorted, fractionated, treated to remove specific cell types, or otherwise manipulated to obtain a population of cells for use in the methods described herein using any procedure acceptable to those skilled in the art. Mononuclear lymphocytes may be collected, for example, by repeated lymphocytophereses using a continuous flow cell separator as described in U.S. Pat. No. 4,690,915, or isolated using an affinity purification step of CLP method, such as flow-cytometry using a cytometer, magnetic separation, using antibody or protein coated beads, affinity chromatography, or solid-support affinity separation where cells are retained on a substrate according to their expression or lack of expression of a specific protein or type of protein, or batch purification using one or more antibodies against one or more surface antigens specifically expressed by the cell type of interest. Cells of hematopoietic origin can also be obtained from peripheral blood. Prior to harvest of the cells from peripheral blood, the subject can be treated with a cytokine, such as e.g., granulocyte-colony stimulating factor, to promote cell migration from the bone marrow to the blood compartment and/or promote activation and/or proliferation of the population of interest. Any method suitable for identifying surface proteins, for example, can be employed to isolate cells of hematopoietic origin from a heterogeneous population. In some embodiments, a clonal population of cells of hematopoietic origin, such as lymphocytes, is obtained. In some embodiments, the cells of hematopoietic origin are not a clonal population.

Further, in regard to the various aspects and embodiments of the compositions, methods, and kits described herein, a somatic cell can be obtained from any mammalian species, with non-limiting examples including a murine, bovine, simian, porcine, equine, ovine, or human cell. In some embodiments, the somatic cell is a human cell. In some embodiments, the cell is from a non-human organism, such as a non-human mammal.

In general, the methods for making iDCs described herein involve culturing or expanding somatic cells, such as cells of hematopoietic origin, in any culture medium that is available and well-known to one of ordinary skill in the art. Such media include, but are not limited to, Dulbecco's Modified Eagle's Medium^{®} (DMEM), DMEM F12 Medium^{®}, Eagle's Minimum Essential Medium^{®}, F-12K Medium^{®}, Iscove's Modified Dulbecco's Medium^{®}, RPMI-1640 Medium^{®}, and serum-free medium for culture and expansion of DCs. Many media are also available as low-glucose formulations, with or without sodium. The medium used with the methods described herein can, in some embodiments, be supplemented with one or more immunostimulatory cytokine. Commonly used growth factors include, but are not limited to, G-CSF, GM-CSF, TNF-α, IL-4, the Flt-3 ligand and the kit ligand. In addition, in preferred embodiments, the immunostimulatory cytokine is selected from the group consisting of the interleukins (e.g., IL-1α, IL-1β, IL-2, IL-3, IL-4, IL-6, IL-8, IL-9, IL-10, IL-12, IL-18, IL-19, IL-20), the interferons (e.g., IFN-α, IFN-β, IFN-γ), tumor necrosis factor (TNF), transforming growth factor-β (TGF-β), granulocyte colony stimulating factor (G-CSF), macrophage colony stimulating factor (M-CSF), granulocyte-macrophage colony stimulating factor (GM-CSF), the Flt-3 ligand and the kit ligand.

Cells in culture can be maintained either in suspension or attached to a solid support, such as extracellular matrix components or plating on feeder cells, for example. Cells being used in the methods described herein can require additional factors that encourage their attachment to a solid support, in some embodiments, such as type I and type II collagen, chondroitin sulfate, fibronectin, "superfibronectin" and fibronectin-like polymers, gelatin, poly-D and poly-L-lysine, thrombospondin and vitronectin. In some embodiments, the cells are suitable for growth in suspension cultures. Suspension-competent host cells are generally monodisperse or grow in loose aggregates without substantial aggregation. Suspension-competent host cells include cells that are suitable for suspension culture without adaptation or manipulation (e.g., cells of hematopoietic origin, such as lymphoid cells) and cells that have been made suspension-competent by modification or adaptation of attachment-dependent cells (e.g., epithelial cells, fibroblasts).

In some embodiments of these aspects and all such aspects described herein, the isolated induced dendritic cells (iDCs) further comprise a pharmaceutically acceptable carrier for administration to a subject in need.

Also provided herein, in some aspects, are methods of treating a subject in need of treatment to induce antigen-specific immune responses to eliminate cancer cells or infectious agents using the DC inducing compositions and methods of preparing iDCs described herein, or using the isolated induced dendritic cells (iDCs) and cell clones thereof produced using any of the combinations of DC inducing factors, DC inducing compositions, or methods of preparing iDCs described herein. In such methods of treatment, somatic cells, such as fibroblast cells or hematopoietic lineage cells, can first be isolated from the subject, and the isolated cells transduced or transfected, as described herein with a DC inducing composition comprising expression vectors or synthetic mRNAs, respectively. The isolated induced dendritic cells (iDCs) produced using any of the combinations of DC inducing factors, DC inducing compositions, or methods of preparing iDCs described herein, can then be administered to the subject, such as via systemic injection of the iDCs to the subject.

Also provided herein, in some aspects, are methods of treating a subject in need of treatment to induce antigen-specific immune responses to eliminate cancer cells or infectious agents using the DC inducing compositions and any of the combinations of DC inducing factors described herein. In such methods of treatment, cancer cells are transduced, as described herein with a DC inducing composition comprising expression vectors. Cancer cells can be first isolated from the subject, transduced with a DC inducing composition comprising expression vectors and then administered to the subject, such as via systemic injection. Alternatively, cancers cells can be transduced in situ or in vivo with DC inducing composition comprising viral expression vectors. The modified cancer cell acquires antigen presentation ability, presenting their tumor antigens to T cells and eliciting cytotoxic responses against themselves.

The reprogrammed iDCs generated using the compositions, methods, and kits described herein can, in some embodiments of the methods of treatment described herein, be used directly or administered to subjects in need of immunotherapies. Accordingly, various embodiments of the methods described herein involve administration of an effective amount of a iDC or a population of iDCs, generated using any of the compositions, methods, and kits described herein, to an individual or subject in need of a cellular therapy. The cell or population of cells being administered can be an autologous population, or be derived from one or more heterologous sources. Further, such iDCs can be administered in a manner that permits them to migrate to lymph node and activate effector T cells.

A variety of means for administering cells to subjects are known to those of skill in the art. Such methods can include systemic injection, for example, i.v. injection, or implantation of cells into a target site in a subject. Cells may be inserted into a delivery device which facilitates introduction by injection or implantation into the subject. Such delivery devices can include tubes, e.g., catheters, for injecting cells and fluids into the body of a recipient subject. In one preferred embodiment, the tubes additionally have a needle, e.g., through which the cells can be introduced into the subject at a desired location. The cells can be prepared for delivery in a variety of different forms. For example, the cells can be suspended in a solution or gel or embedded in a support matrix when contained in such a delivery device. Cells can be mixed with a pharmaceutically acceptable carrier or diluent in which the cells remain viable.

Accordingly, the cells produced by the methods described herein can be used to prepare cells to treat or alleviate several cancers and tumors including, but not limited to, breast cancer, prostate cancer, lymphoma, skin cancer, pancreatic cancer, colon cancer, melanoma, malignant melanoma, ovarian cancer, brain cancer, primary brain carcinoma, head-neck cancer, glioma, glioblastoma, liver cancer, bladder cancer, non- small cell lung cancer, head or neck carcinoma, breast carcinoma, ovarian carcinoma, lung carcinoma, small-cell lung carcinoma, Wilms' tumor, cervical carcinoma, testicular carcinoma, bladder carcinoma, pancreatic carcinoma, stomach carcinoma, colon carcinoma, prostatic carcinoma, genitourinary carcinoma, thyroid carcinoma, esophageal carcinoma, myeloma, multiple myeloma, adrenal carcinoma, renal cell carcinoma, endometrial carcinoma, adrenal cortex carcinoma, malignant pancreatic insulinoma, malignant carcinoid carcinoma, choriocarcinoma, mycosis fungoides, malignant hypercalcemia, cervical hyperplasia, leukemia, acute lymphocytic leukemia, chronic lymphocytic leukemia, acute myelogenous leukemia, chronic myelogenous leukemia, chronic granulocytic leukemia, acute granulocytic leukemia, hairy cell leukemia, neuroblastoma, rhabdomyosarcoma, Kaposi's sarcoma, polycythemia vera, essential thrombocytosis, Hodgkin's disease, non- Hodgkin's lymphoma, soft-tissue sarcoma, osteogenic sarcoma, primary macroglobulinemia, and retinoblastoma, and the like.

In addition to the above, the methods of the invention can be used to prevent or eliminate infection by pathogens known to predispose to certain cancers. Pathogens of particular interest for use in the cancer vaccines provided herein include the hepatitis B virus (hepatocellular carcinoma), hepatitis C virus (heptomas), Epstein Barr virus (EBV) (Burkitt lymphoma, nasopharynx cancer, PTLD in immunosuppressed individuals), HTLVL (adult T cell leukemia), oncogenic human papilloma viruses types 16, 18, 33, 45 (adult cervical cancer), and the bacterium Helicobacter pylori (B cell gastric lymphoma). Other medically relevant microorganisms that may serve as antigens in mammals and more particularly humans are described extensively in the literature, e.g., C. G. A Thomas, Medical Microbiology, Bailliere Tindall, (1983).

In addition to the above, the methods of the invention can be used for viral infections. Exemplary viral pathogens include, but are not limited to, infectious virus that infect mammals, and more particularly humans. Examples of infectious virus include, but are not limited to: Retroviridae (e.g., human immunodeficiency viruses, such as HIV-I (also referred to as HTLV-III, LAV or HTLV-III/LAV, or HIV-III; and other isolates, such as HIV-LP; Picornaviridae (e.g. polio viruses, hepatitis A virus; enteroviruses, human Coxsackie viruses, rhinoviruses, echoviruses); Calciviridae (e.g. strains that cause gastroenteritis); Togaviridae (e.g. equine encephalitis viruses, rubella viruses); Flaviridae (e.g. dengue viruses, encephalitis viruses, yellow fever viruses); Coronoviridae (e.g. coronaviruses such as the SARS coronavirus); Rhabdoviradae (e.g. vesicular stomatitis viruses, rabies viruses); Filoviridae (e.g. ebola viruses); Paramyxoviridae (e.g. parainfluenza viruses, mumps virus, measles virus, respiratory syncytial virus); Orthomyxoviridae (e.g. influenza viruses); Bungaviridae (e.g. Hantaan viruses, bunga viruses, phleboviruses and Nairo viruses); Arena viridae (hemorrhagic fever viruses); Reoviridae (e.g. reoviruses, orbiviurses and rotaviruses); Bir-naviridae; Hepadnaviridae (Hepatitis B virus); Parvovirida (parvoviruses); Papovaviridae (papilloma viruses, polyoma viruses); Adenoviridae (most adenoviruses); Herpesviridae herpes simplex virus (HSV) 1 and 2, varicella zoster virus, cytomegalovirus (CMV), herpes virus; P.oxyiridae (variola viruses, vaccinia viruses, pox viruses); and Iridoviridae (e.g. African swine fever virus); and unclassified viruses (e.g. the etiological agents of Spongiform encephalopathies, the agent of delta hepatitis (thought to be a defective satellite of hepatitis B virus), the agents of non-A, non-B hepatitis (class I=interaally transmitted; class 2=parenterally transmitted (i.e. Hepatitis C); Norwalk and related viruses, and astro viruses).

In addition to the above, the methods of the invention can be used to target gram negative and gram positive bacteria in vertebrate animals. Such gram positive bacteria include, but are not limited to Pasteurella sp., Staphylococci sp., and Streptococcus sp. Gram negative bacteria include, but are not limited to, Escherichia coli, Pseudomonas sp. , and Salmonella sp. Specific examples of infectious bacteria include but are not limited to: Helicobacter pyloris, Borella burgdorferi, Legionella pneumophilia, Mycobacteria sp. (e.g. M. tuberculosis, M. avium, M. intracellular e, M. kansaii, M. gordonae), Staphylococcus aureus, Neisseria gonorrhoeae, Neisseria meningitidis, Listeria monocytogenes, Streptococcus pyogenes (Group A Streptococcus), Streptococcus agalactiae (Group B Streptococcus), Streptococcus (viridans group), Streptococcus faecalis, Streptococcus bovis, Streptococcus (anaerobic sps.), Streptococcus pneumoniae, pathogenic Campylobacter sp., Enterococcus sp., Haemophilus infuenzae, Bacillus antracis, Corynebacterium diphtheriae, Corynebacterium sp., Erysipelothrix rhusiopathiae, Clostridium perfringers, Clostridium tetani, Enterobacter aerogenes, Klebsiella pneumoniae, Pasturella multocida, Bacteroides sp., Fusobacterium nucleatum, Streptobacillus moniliformis, Treponema pallidium, Treponema pertenue, Leptospira, Rickettsia, and Actinomyces israelii.

In addition to the above, the methods of the invention can be used to target pathogens that include, but are not limited to, infectious fungi and parasites that infect mammals, and more particularly humans. Examples of infectious fungi include, but are not limited to: Cryptococcus neoformans, Histoplasma capsulatum, Coccidioides immitis, Blastomyces dermatitidis, Chlamydia trachomatis, and Candida albicans.

In addition to the above, the methods of the invention can be used to target parasites such as intracellular parasites and obligate intracellular parasites. Examples of parasites include but are not limited to Plasmodium- falciparum, Plasmodium ovale, Plasmodium malariae, Plasmdodium vivax, Plasmodium knowlesi, Babesia microti, Babesia divergens, Trypanosoma cruzi, Toxoplasma gondii, Trichinella spiralis, Leishmania major, Leishmania donovani, Leishmania braziliensis, Leishmania tropica, Trypanosoma gambiense, Trypanosoma rhodesiense, Wuchereria bancrofti, Brugia malayi, Brugia timori, Ascaris lumbricoides, Onchocerca volvulus and Schistosoma mansoni.

If modified induced dendritic cells can be used to induce a tolerogenic response including the suppression of a future or existing immune response, to one or more target antigens. Thus, induce DCs are useful for treating or preventing an undesirable immune response including, for example, transplant rejection, graft versus host disease, allergies, parasitic diseases, inflammatory diseases and autoimmune diseases. Examples of transplant rejection, which can be treated or prevented in accordance with the present invention, include rejections associated with transplantation of bone marrow and of organs such as heart, liver, pancreas, kidney, lung, eye, skin etc. Examples of allergies include seasonal respiratory allergies; allergy to aeroallergens such as hayfever; allergy treatable by reducing serum IgE and eosinophilia; asthma; eczema; animal allergies, food allergies; latex allergies; dermatitis; or allergies treatable by allergic desensitisation. Autoimmune diseases that can be treated or prevented by the present invention include, for example, psoriasis, systemic lupus erythematosus, myasthenia gravis, stiffman syndrome, thyroiditis, Sydenham chorea, rheumatoid arthritis, diabetes and multiple sclerosis. Examples of inflammatory disease include Crohn's disease, chronic inflammatory eye diseases, chronic inflammatory lung diseases and chronic inflammatory liver diseases, autoimmune haemolytic anaemia, idiopathic leucopoenia, ulcerative colitis, dermatomyositis, scleroderma, mixed connective tissue disease, irritable bowel syndrome, systemic lupus erythromatosus (SLE), multiple sclerosis, myasthenia gravis, Guillan-Barre syndrome (antiphospholipid syndrome), primary myxoedema, thyrotoxicosis, pernicious anaemia, autoimmune atrophic gastris, Addison's disease, insulin-dependent diabetes mellitus (IDDM), Goodpasture's syndrome, Behcet's syndrome, Sjogren's syndrome, rheumatoid arthritis, sympathetic ophthalmia, Hashimoto's disease/hypothyroiditis, celiac disease/dermatitis herpetiformis, and demyelinating disease primary biliary cirrhosis, mixed connective tissue disease, chronic active hepatitis, Graves' disease/hyperthyroiditis, scleroderma, chronic idiopathic thrombocytopenic purpura, diabetic neuropathy and septic shock.

Pharmaceutically acceptable carriers and diluents include saline, aqueous buffer solutions, solvents and/or dispersion media. The use of such carriers and diluents is well known in the art. The solution is preferably sterile and fluid. Preferably, prior to the introduction of cells, the solution is stable under the conditions of manufacture and storage and preserved against the contaminating action of microorganisms such as bacteria and fungi through the use of, for example, parabens, chlorobutanol, phenol, ascorbic acid, thimerosal, and the like.

It is preferred that the mode of cell administration is relatively non-invasive, for example by intravenous injection, pulmonary delivery through inhalation, topical, or intranasal administration. However, the route of cell administration will depend on the tissue to be treated and may include implantation. Methods for cell delivery are known to those of skill in the art and can be extrapolated by one skilled in the art of medicine for use with the methods and compositions described herein.

Also provided herein, in some aspects, are kits for making induced dendritic cells (iDCs), the kits comprising any of the DC inducing compositions comprising one or more expression vector components described herein.

Also provided herein, in some aspects, are kits comprising one or more of the DC inducing factors described herein as components for the methods of making the induced dendritic cells described herein.

Accordingly, in some aspects, provided herein, are kits for preparing induced dendritic cells comprising the following components: (a) one or more expression vectors encoding at least one, two, three, four, five, six, seven, eight, or more DC inducing factors selected from: BATF3 (SEQ. ID. 1, SEQ. ID. 2), SPIB (SEQ. ID. 3, SEQ. ID. 4), IRF8 (SEQ. ID. 5, SEQ. ID. 6), PU.1 (SEQ. ID. 7, SEQ. ID. 8), STAT3 (SEQ. ID. 11, SEQ. ID. 12), TCF4 (SEQ. ID. 13, SEQ. ID. 14), IKZF1 (SEQ. ID. 15, SEQ. ID. 16), ID2 (SEQ. ID. 17, SEQ. ID. 18), BCL11A (SEQ. ID. 19, SEQ. ID. 20), RELB (SEQ. ID. 21, SEQ. ID. 22), ZBTB46 (SEQ. ID. 23, SEQ. ID. 24), RUNX3 (SEQ. ID. 25, SEQ: ID. 26), GFI1 (SEQ. ID. 27, SEQ. ID. 28), IRF2 (SEQ. ID. 29, SEQ. ID. 30), NFIL3 (SEQ. ID. 31, SEQ. ID. 32), BCL6 (SEQ. ID. 33, SEQ. ID. 34), L-MYC (SEQ. ID. 35, SEQ. ID. 36), NR4A3 (SEQ. ID. 37, SEQ. ID. 38), and (b) packaging and instructions therefor.

The kits described herein, in some embodiments, can further provide the synthetic mRNAs or the one or more expression vectors encoding DC inducing factors in an admixture or as separate aliquots.

In some embodiments, the kits can further comprise an agent to enhance efficiency of reprogramming. In some embodiments, the kits can further comprise one or more antibodies or primer reagents to detect a cell-type specific marker to identify cells induced to the dendritic cell state.

In some embodiments, the kits can further comprise a buffer. In some such embodiments, the buffer is RNase-free TE buffer at pH 7.0. In some embodiments, the kit further comprises a container with cell culture medium.

All kits described herein can further comprise a buffer, a cell culture medium, a transduction or transfection medium and/or a media supplement. In preferred embodiments, the buffers, cell culture mediums, transfection mediums, and/or media supplements are DNAse and RNase-free. In some embodiments, the synthetic, modified RNAs provided in the kits can be in a non-solution form of specific quantity or mass, e.g., 20 µg, such as a lyophilized powder form, such that the end-user adds a suitable amount of buffer or medium to bring the components to a desired concentration, e.g., 100 ng/µl.

All kits described herein can further comprise devices to facilitate single-administration or repeated or frequent infusions of the cells generated using the kits components described herein, such as a non-implantable delivery device, e.g., needle, syringe, pen device, or an implantatable delivery device, e.g., a pump, a semi-permanent stent (e.g., intravenous, intraperitoneal, intracisternal or intracapsular), or a reservoir. In some such embodiments, the delivery device can include a mechanism to dispense a unit dose of a pharmaceutical composition comprising the iDCs. In some embodiments, the device releases the composition continuously, e.g., by diffusion. In some embodiments, the device can include a sensor that monitors a parameter within a subject. For example, the device can include pump, e.g., and, optionally, associated electronics.

In an embodiment, induced dendritic cells are made by the hand of man by, e.g., modifying the gene expression of at least one of the factors disclosed herein of a somatic cell, a pluripotent cell, a progenitor cell or a stem cell, or by exposing any one of these cell types to at least one protein or RNA that produces at least one protein as disclosed herein. The cells can further be made by exposing them to small molecules that turn on at least one of the factors disclosed herein. In some aspects at least two, three, four, five, six, seven, or eight factors are used to make the induced dendritic cells.

In an embodiment, the induced dendritic cells in some aspects of all the embodiments of disclosure, while similar in functional characteristics, differ significantly in their gene expression from the naturally occurring endogenous dendritic cells.

In an embodiment, the induced dendritic cells as described herein differ from naturally occurring dendritic cells by both their posttranslational modification signatures and their gene expression signatures.

In an embodiment, the induced dendritic cells as described herein differ from naturally occurring dendritic cells by their ability to growth in vitro as adherent cultures and to survive in culture for more than one month.

In an embodiment, induced dendritic cell is also defined as comprising a gene expression signature that differs from naturally occurring dendritic cells. One can experimentally show the difference by comparing the gene expression pattern of a naturally occurring dendritic cell to that of the induced dendritic cells. Therefore, in some aspects of all the embodiments of the invention, the induced dendritic cells comprise an expression signature that is about 1-5%, 5-10%, 5-15%, or 5-20% different from the expression signature of about 1-5%, 2-5%, 3-5%, up to 50%, up to 40%, up to 30%, up to 25%, up to 20%, up to 15%, or up to 10% of specific genes. For example, expression levels of DC inducing factor(s), such as PU.1, IRF8, BATF3 and TCF4, in iDCs are higher than in naturally occurring DCs as the DC inducing factors are being overexpressed.

In an embodiment, mouse Embryonic Fibroblasts (MEFs) were isolated and purified in the following way: Clec9aCre/Cre animals (10) were crossed with Rosa26-stopflox-tdTomato reporter mice (The Jackson Laboratory) to generate double homozygous Clec9a^{Cre/Cre} Rosa ^{tdTomato/tdTomato} (C9A-tdTomato) mice. C57BL/6 mice, Rag2 constitutive knock-out (KO)/OT-II random transgenic (Rag2KO/OT-II) mice and Rag2KO/OT-I random transgenic mice were acquired from Charles River and Taconic, respectively (17-19). All animals were housed under controlled temperature (23 ± 2 °C), subject to a fixed 12-h light/dark cycle, with free access to food and water.

In an embodiment, primary cultures of MEFs were isolated from E13.5 embryos of C9A-tdTomato or C57BL/6 mice (6, 10). Head, fetal liver and all internal organs were removed and the remaining tissue was mechanically dissociated. Dissected tissue was enzymatic digested using 0.12% trypsin/0.1 mM Ethylenediaminetetraacetic acid (EDTA) solution (3 mL per embryo), and incubation at 37°C for 15 min. Additional 3 mL of same solution per embryo were added, followed by another 15 min incubation period. A single cell suspension was obtained and plated in 0.1% gelatin-coated 10-cm tissue culture dishes in growth media. Cells were grown for 2-3 days until confluence, dissociated with Tryple Express and frozen in Fetal Bovine Serum (FBS) 10% dimethyl sulfoxide (DMSO). Before plating for lentiviral transduction, MEFs were sorted to remove residual CD45+ and tdTomato+ cells that could represent cells with hematopoietic potential. MEFs used for screening and in the following experiments were tdTomato-CD45- with a purity of 99.8% and expanded up to 4 passages.

In an embodiment, HEK293T cells, MEFs and Human Dermal Fibroblasts (HDFs, ScienCell) were maintained in growth medium [Dulbecco's modified eagle medium (DMEM) supplemented with 10% (v/v) FBS, 2mM L-Glutamine and antibiotics (10 µg/ml Penicillin and Streptomycin)], OP-9 and OP-9-DL1 cell lines were cultured in Minimum Essential Medium (MEM) Alpha containing 20% FBS, 1mM L-Glutamine and penicillin/streptomycin (10 µg/ml). OP-9 and OP-9-DL1 were routinely passaged at 80% confluency. All cells were maintained at 37°C and 5% (v/v) CO2. All tissue culture reagents were from Thermo Fisher Scientific unless stated otherwise.

In an embodiment, viral transduction and reprogramming experiments were performed in the following way: C9A-tdTomato MEFs were seeded at a density of 40,000 cells per well on 0.1% gelatin coated 6-well plates. Cells were incubated overnight with a ratio of 1:1 FUW-TetO-TFs and FUW-M2rtTA lentiviral particles in growth media supplemented with 8 µg/mL polybrene. When testing combinations of TFs, equal MOls of each individual viral particles were applied. Cells were transduced twice in consecutive days and after overnight incubation, media was replaced with fresh growth media. After the second transduction, growth media was supplemented with Doxycycline (1 µg/mL) - day 0. Media was changed every 2-3 days for the duration of the cultures. Emerging tdTomato+ cells were analyzed 1-15 days post-transduction. When stated, variations of culture conditions were applied, namely RPMI-1640, Lipopolysaccharide (LPS, 100 ng/ml, Sigma), 2-Mercaptoethanol (1x104 µM; 2-ME), L-glutamine (2 µmol/ml), GM-CSF (10 ng/ml, STEMCELL Technologies), IL-4 (20 ng/ml, STEMCELL Technologies) and Flt3l (100 ng/ml, STEMCELL Technologies).

In an embodiment, fluorescent microscopy and immunofluorescence was evaluated in the following way: C9A-driven tdTomato in MEFs and transduced HDFs were visualized directly on 6-well plates under an inverted microscope (Zeiss AxioVert 200M) and images processed with AxioVision and Adobe Photoshop software. DAPI (4',6-diamidino-2-phenylindole, 1 µg/mL, Sigma) and Phalloidin (50 µg/ml, Sigma) were used to stain nuclei and F-actin, respectively. For time-lapse microscopy fluorescent pictures were acquired after adding Dox every 1 hour for 6 days and 4 hours using an INCELL Analyzer 2200 (GE Healthcare). Movies were generated with ImageJ software (NIH).

In an embodiment, flow cytometry analysis was performed in the following way: Transduced C9A-tdTomato MEFs or transduced HDFs were dissociated with TrypLE Express, resuspended in 200 µL PBS 5% FBS and kept at 4°C prior analysis in BD Accuri C6 Flow Cytometer (BD Biosciences). Sample acquisition was performed with the configuration 3-blue-1-red (533/30 filter in FL1; 585/40 in FL2, 670 LP in FL3 and 675/25 in FL4). tdTomato fluorescence was analyzed in the FL2 channel. For the analysis of CD45 or MHC-II cell surface marker expression, dissociated cells were incubated with APC-Cy7 rat anti-mouse CD45 antibody or Alexa Fluor 647 rat anti-mouse I-A/I-E diluted in PBS 5% FBS at 4°C for 30 minutes in the presence of rat serum (1/100, GeneTex) to block unspecific binding. Cells were washed with PBS 5% FBS, resuspended in PBS 5% FBS and analyzed in a BD Accuri C6 Flow cytometer. CD45 APC-Cy7 and I-A/I-E Alexa Fluor 647 fluorescence were analyzed in FL4 channel. For the combined analysis of MHCII, CD80 and CD86 cell surface expression, dissociated cells were stained with Alexa Fluor 647 rat anti-mouse I-A/I-E, BV650 rat anti-mouse CD80 and PE-CY7 rat anti-mouse CD86 and analyzed in BD FACSAria III (BD Biosciences). For the analysis of transduced HDFs, dissociated cells were stained with APC mouse anti-human CLEC9A and PE-Cy7 mouse anti-human CD11c. To assess CD4+ and CD8+ T cell proliferation and activation after 7 days of co-culture with APCs, carboxyfluorescein succinimidyl ester (CFSE)-labeled T cells were incubated with PE rat anti-mouse CD44 and analyzed in BD Accuri C6. Flow cytometry data were analyzed using FlowJo software (FLOWJO, LLC, version 7.6).

In an embodiment, fluorescence activated cell sorting (FACS) was performed in the following way: To purify C9A-tdTomato MEFs, cells were incubated at 4°C for 30 minutes with APC-Cy7 anti-CD45 antibody diluted in PBS 5% FBS. Subsequently, MEFs were washed with PBS 5% FBS, resuspended in PBS 5% FBS and tdTomato- CD45- MEFs were purified in BD FACSAria III. When described tdTomato+ cells were purified using BD FACSAria III and cultured in the absence or presence of doxycycline. For the isolation of splenic DCs, splenic cells were incubated with Alexa Fluor 647 rat anti-mouse I-A/I-E and FITC rat anti-mouse CD11c. CD11c+MHCII+ MEFs were purified in BD FACSAria III (BD Biosciences). FACS data was processed in FlowJo software.

In an embodiment, bead incorporation assay was evaluated in the following way: transduced C9A-tdTomato MEF or transduced HDF cultures were incubated with 2.5% yellow-green fluorescent-coupled solid latex beads (carboxylate-modified polystyrene, Sigma) at 1:1000 ratio in growth medium. Sixteen hours later, cells were washed twice in PBS 5% FBS and analyzed under an inverted microscope. DAPI (1 µg/mL, Sigma) was used for nuclear staining.

In an embodiment, incorporation of labelled ovalbumin was evaluated in the following way: transduced HDF cultures were incubated with Alexa647-labelled ovalbumin (Life Technologies) for 20 minutes at 37°C or 4°C. After washing with PBS 5%FBS, cells were analysed in BD Accuri C6.

In an embodiment, incorporation of dead cells was evaluated in the following way: HEK293T cells were exposed to ultra-violet (UV) irradiation to induce cell death and labelled with CellVue^{®} Claret Far Red Fluorescent Cell Linker Kit (Sigma), according to manufacturer's instructions. Transduced HDFs were incubated with Far red-labelled dead cells overnight, stained with PE-Cy7 anti CD11c and analysed in BD Accuri C6.

In an embodiment, inflammatory cytokine assay was performed in the following way: Levels of the cytokines interleukin- 6 (IL-6), interleukin-10 (IL-10), interleukin-12p70 (IL-12p70), interferon-γ (IFN-γ) and tumor necrosis factor (TNF) were assessed in supernatants of iDCs cultures 10 days after Dox supplementation. At day 9, 100 ng/mL LPS or 25 µg/mL of Polyinosinic-polycytidylic acid (Polyl:C) (Invivogen) were added for overnight stimulation. 50 µL of culture supernatants from a 6-well plates well were collected and analyzed by CBA Mouse Inflammation Kit (BD Biosciences), according to manufacturer's instructions. Acquisition was performed with a BD Accuri C6 and data were analyzed using FCAP array software, version 3.0 (BD Biosciences). The limit of detection in CBA was: IL-6, 20.91 pg/ml; IL-10, 10.55 pg/ml; IFN-γ, 18.2 pg/ml; TNF, 18.13 pg/ml; IL-12p70, 20.05 pg/ml.

In an embodiment, splenic DC isolation was evaluated in the following way: Freshly isolated spleens were homogenized using the frosted ends of 2 sterile slides. Cells were harvested in PBS supplemented with 2% FBS and filtered through a 70 µm cell strainers (BD Biosciences). Red blood cells were lysed with BD Pharm Lyse (BD Biosciences) for 8 min at room temperature. MHC-II+CD11c+ DCs were purified by FACS (BD FACSAria III, BD Biosciences) and immediately used for antigen presenting assays.

In an embodiment, CD4+ T cell isolation and antigen presenting assays was evaluated in the following way: CD4+T cells from spleen of Rag2KO/OT-II mice were enriched using Dynabeads Untouched Mouse CD4 Cells Kit (BD Biosciences), according to manufacturer's instructions. Enriched CD4+ T cells were labeled with CFSE 5 µM at room temperature for 10 min, washed, and counted before cultured with APCs. iDCs cultures at day 8 after the addition of Dox or splenic CD11c+ MHC-II+ DCs cells were incubated with OVA protein (10 µg/mL) or OVA323-339 peptide (10 µg/mL) in the presence or absence of 100 ng/mL of LPS and co-cultured with untouched CFSE-labeled OT-II CD4+ T cells. iDC cultures (20000 cells) or 20000 splenic CD11c+ MHC-II+ DCs were incubated with 20000 CFSE-labeled CD4+ T cells in 96-well round-bottom tissue culture plates. T cell proliferation (dilution of CFSE staining) and activation (CD44 expression) were assessed by flow cytometry after 7 days of co-culture.

In an embodiment, CD8+ T cell isolation and antigen cross-presentation was evaluated in the following way: CD8+ T cells from spleen of Rag2KO/OT-I mice were enriched using Dynabeads Untouched Mouse CD8 Cells Kit (BD Biosciences), according to manufacturer's instructions. Enriched CD8+ T cells were labelled with CFSE 5 µM at room temperature for 10 min, washed, and counted before cultured with APCs. iDCs cultures at day 8 after the addition of Dox or splenic CD11c+ MHC-II+ DCs cells were incubated with OVA protein (10 µg/mL) or OVA257-264 peptide (10nM) in the presence or absence of 100 ng/mL of LPS or 25 µg/mL of polyl:C and co-cultured with untouched CFSE-labelled OT-I CD8+ T cells. iDC cultures (20000 cells) or 20000 splenic CD11c+ MHC-II+ DCs were incubated with 20000 CFSE-labelled CD8+ T cells in 96-well round-bottom tissue culture plates. T cell proliferation (dilution of CFSE staining) and activation (CD44 expression) were assessed by flow cytometry after 4 days of co-culture.

In an embodiment, comparisons among groups were performed by one-way ANOVA followed by Bonferroni's multiple comparison test with GraphPad Prism 5 software. P-values are shown when relevant (*p<0.05; **p<0.01, ***p<0.001, ****p<0.0001).

**Table I - Primary Antibodies Used in the analysis**

| Antibody/Antigen | Specie | Clone | Conjugate | Source |
|---|---|---|---|---|
| CD45 | Mouse | 30-F11 | PE | BD Pharmingen |
| CD4 | Mouse | GK1.5 | PE-CY7 | eBioscience |
| CD44 | Mouse | IM7 | PE | BD Pharmingen |
| MHC Class II (I-A/1-E) | Mouse | M5/114.15.2 | Alexa Fluor 647 | BD Pharmingen |
| CD80 (B7 1) | Mouse | 16-101A | BV650 | BD Horizon |
| CD86 (B7 2) | Mouse | GL1 | PE-CY7 | eBioscience |
| CD11c | Mouse | N418 | FITC | Biolegend |
| CD11c | Human | B-ly6 | PE-Cy7 | BD Pharmigen |
| CLEC9A | Human | 8F9 | APC | Biolegend |

Those skilled in the art will recognize, or be able to ascertain using no more than routine experimentation, many equivalents to the specific embodiments of the invention described herein. The scope of the present invention is not intended to be limited to the above description, but rather is as set forth in the appended claims.

Where singular forms of elements or features are used in the specification of the claims, the plural form is also included, and vice versa, if not specifically excluded. For example, the term "a cell" or "the cell" also includes the plural forms "cells" or "the cells," and vice versa. In the claims articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention also includes embodiments in which more than one, or all of the group members are present in, employed in, or otherwise relevant to a given product or process.

Furthermore, it is to be understood that the invention encompasses all variations, combinations, and permutations in which one or more limitations, elements, clauses, descriptive terms, etc., from one or more of the claims or from relevant portions of the description is introduced into another claim. For example, any claim that is dependent on another claim can be modified to include one or more limitations found in any other claim that is dependent on the same base claim. Furthermore, where the claims recite a composition, it is to be understood that methods of using the composition for any of the purposes disclosed herein are included, and methods of making the composition according to any of the methods of making disclosed herein or other methods known in the art are included, unless otherwise indicated or unless it would be evident to one of ordinary skill in the art that a contradiction or inconsistency would arise.

Where ranges are given, endpoints are included. Furthermore, it is to be understood that unless otherwise indicated or otherwise evident from the context and/or the understanding of one of ordinary skill in the art, values that are expressed as ranges can assume any specific value within the stated ranges in different embodiments of the invention, to the tenth of the unit of the lower limit of the range, unless the context clearly dictates otherwise. It is also to be understood that unless otherwise indicated or otherwise evident from the context and/or the understanding of one of ordinary skill in the art, values expressed as ranges can assume any subrange within the given range, wherein the endpoints of the subrange are expressed to the same degree of accuracy as the tenth of the unit of the lower limit of the range.

In addition, it is to be understood that any particular embodiment of the present invention may be explicitly excluded from any one or more of the claims. Where ranges are given, any value within the range may explicitly be excluded from any one or more of the claims. Any embodiment, element, feature, application, or aspect of the compositions and/or methods of the invention, can be excluded from any one or more claims. For purposes of brevity, all of the embodiments in which one or more elements, features, purposes, or aspects is excluded are not set forth explicitly herein.

The above described embodiments are combinable.

The following claims further set out particular embodiments of the present disclosure.

All references recited in this document are incorporated herein in their entirety by reference, as if each and every reference had been incorporated by reference individually.

### References

1. Takahashi K, Tanabe K, Ohnuki M, Narita M, Ichisaka T, Tomoda K, et al. Induction of pluripotent stem cells from adult human fibroblasts by defined factors. Cell. 2007;131(5):861-72. Epub 2007/11/24. doi: 10.1016/j.cell.2007.11.019. PubMed PMID: 18035408.
2. Takahashi K, Yamanaka S. Induction of pluripotent stem cells from mouse embryonic and adult fibroblast cultures by defined factors. Cell. 2006;126(4):663-76. Epub 2006/08/15. doi: 10.1016/j.cell.2006.07.024. PubMed PMID: 16904174.
3. Pereira CF, Lemischka IR, Moore K. Reprogramming cell fates: insights from combinatorial approaches. Ann NY Acad Sci. 2012;1266:7-17. Epub 2012/08/21. doi: 10.1111/j.1749-6632.2012.06508.x. PubMed PMID: 22901251.
4. Xu J, Du Y, Deng H. Direct lineage reprogramming: strategies, mechanisms, and applications. Cell Stem Cell. 2015;16(2):119-34. Epub 2015/02/07. doi: 10.1016/j.stem.2015.01.013. PubMed PMID: 25658369.
5. Xie H, Ye M, Feng R, Graf T. Stepwise reprogramming of B cells into macrophages. Cell. 2004;117(5):663-76. Epub 2004/05/28. PubMed PMID: 15163413.
6. Pereira CF, Chang B, Qiu J, Niu X, Papatsenko D, Hendry CE, et al. Induction of a hemogenic program in mouse fibroblasts. Cell Stem Cell. 2013;13(2):205-18. Epub 2013/06/19. doi: 10.1016/j.stem.2013.05.024. PubMed PMID: 23770078; PubMed Central PMCID: PMCPMC3735774.
7. Pereira CF, Chang B, Gomes A, Bernitz J, Papatsenko D, Niu X, et al. Hematopoietic Reprogramming In Vitro Informs In Vivo Identification of Hemogenic Precursors to Definitive Hematopoietic Stem Cells. Dev Cell. 2016;36(5):525-39. Epub 2016/03/10. doi: 10.1016/j.devcel.2016.02.011. PubMed PMID: 26954547; PubMed Central PMCID: PMCPMC4785845.
8. Datta J, Terhune JH, Lowenfeld L, Cintolo JA, Xu S, Roses RE, et al. Optimizing dendritic cell-based approaches for cancer immunotherapy. Yale J Biol Med. 2014;87(4):491-518. Epub 2014/12/17. PubMed PMID: 25506283; PubMed Central PMCID: PMCPMC4257036.
9. Subklewe M, Geiger C, Lichtenegger FS, Javorovic M, Kvalheim G, Schendel DJ, et al. New generation dendritic cell vaccine for immunotherapy of acute myeloid leukemia. Cancer Immunol Immunother. 2014;63(10):1093-103. Epub 2014/09/05. doi: 10.1007/s00262-014-1600-5. PubMed PMID: 25186611.
10. Schraml BU, van Blijswijk J, Zelenay S, Whitney PG, Filby A, Acton SE, et al. Genetic tracing via DNGR-1 expression history defines dendritic cells as a hematopoietic lineage. Cell. 2013;154(4):843-58. Epub 2013/08/21. doi: 10.1016/j.cell.2013.07.014. PubMed PMID: 23953115.
11. Schlitzer A, Sivakamasundari V, Chen J, Sumatoh HR, Schreuder J, Lum J, et al. Identification of cDC1- and cDC2-committed DC progenitors reveals early lineage priming at the common DC progenitor stage in the bone marrow. Nat Immunol. 2015;16(7):718-28. Epub 2015/06/10. doi: 10.1038/ni.3200. PubMed PMID: 26054720.
12. Merad M, Sathe P, Helft J, Miller J, Mortha A. The dendritic cell lineage: ontogeny and function of dendritic cells and their subsets in the steady state and the inflamed setting. Annu Rev Immunol. 2013;31:563-604. Epub 2013/03/23. doi: 10.1146/annurev-immunol-020711-074950. PubMed PMID: 23516985; PubMed Central PMCID: PMCPMC3853342.
13. Senju S, Hirata S, Matsuyoshi H, Masuda M, Uemura Y, Araki K, et al. Generation and genetic modification of dendritic cells derived from mouse embryonic stem cells. Blood. 2003;101(9):3501-8. Epub 2002/10/31. doi: 10.1182/blood-2002-07-2254. PubMed PMID: 12406878.
14. Kitamura N, Yokoyama H, Yashiro T, Nakano N, Nishiyama M, Kanada S, et al. Role of PU.1 in MHC class II expression through transcriptional regulation of class II transactivator pl in dendritic cells. J Allergy Clin Immunol. 2012;129(3):814-24 e6. Epub 2011/11/25. doi: 10.1016/j.jaci.2011.10.019. PubMed PMID: 22112519.
15. Smith MA, Wright G, Wu J, Tailor P, Ozato K, Chen X, et al. Positive regulatory domain I (PRDM1) and IRF8/PU.1 counter-regulate MHC class II transactivator (CIITA) expression during dendritic cell maturation. J Biol Chem. 2011;286(10):7893-904. Epub 2011/01/11. doi: 10.1074/jbc.M110.165431. PubMed PMID: 21216962; PubMed Central PMCID: PMCPMC3048676.
16. Reith W, LeibundGut-Landmann S, Waldburger JM. Regulation of MHC class II gene expression by the class II transactivator. Nat Rev Immunol. 2005;5(10):793-806. Epub 2005/10/04. doi: 10.1038/nri1708. PubMed PMID: 16200082.
17. van der Stoep N, Quinten E, Marcondes Rezende M, van den Elsen PJ. E47, IRF-4, and PU.1 synergize to induce B-cell-specific activation of the class II transactivator promoter III (CIITA-PIII). Blood. 2004;104(9):2849-57. Epub 2004/07/10. doi: 10.1182/blood-2004-03-0790. PubMed PMID: 15242870.
18. Shinkai Y, Rathbun G, Lam KP, Oltz EM, Stewart V, Mendelsohn M, et al. RAG-2-deficient mice lack mature lymphocytes owing to inability to initiate V(D)J rearrangement. Cell. 1992;68(5):855-67. Epub 1992/03/06. PubMed PMID: 1547487.
19. Hogquist KA, Jameson SC, Heath WR, Howard JL, Bevan MJ, Carbone FR. T cell receptor antagonist peptides induce positive selection. Cell. 1994;76(1):17-27. Epub 1994/01/14. PubMed PMID: 8287475.

## Claims

1. An isolated transcription factor comprising
a sequence at least 90% identical to a sequence from a list consisting of: SEQ. ID. 1 to SEQ. ID. 66, and mixtures thereof; preferably at least 95% identical to a sequence from a list consisting of: SEQ. ID. 1 to SEQ. ID. 66;
as a reprogramming or inducing factor of a cell selected from a list consisting of: stem cell or a differentiated cell, or mixtures thereof,
into dendritic cell or antigen presenting cell.

2. The isolated transcription factor according to the previous claim selected from a list consisting of: SEQ. ID. 1, SEQ. ID. 2, SEQ. ID. 5, SEQ. ID. 6, SEQ. ID. 7, SEQ. ID. 8, SEQ. ID. 13, SEQ. ID. 14 and mixtures thereof.

3. The isolated transcription factor according wherein the sequence is selected from the group consisting of:
SEQ. ID. 1 or SEQ. ID. 2 and SEQ. ID. 5 or SEQ. ID. 6;
SEQ. ID. 1 or SEQ. ID. 2 and SEQ. ID. 7 or SEQ. ID. 8;
SEQ. ID. 5 or SEQ. ID. 6 and SEQ. ID. 7 or SEQ. ID. 8;
SEQ. ID. 13 or SEQ. ID. 14 and SEQ. ID. 1 or SEQ. ID. 2;
SEQ. ID. 13 or SEQ. ID. 14 and SEQ. ID. 5 or SEQ. ID. 6;
SEQ. ID. 13 or SEQ. ID. 14 and SEQ. ID. 7or SEQ. ID. 8;
SEQ. ID. 1 or SEQ. ID. 2, SEQ. ID. 5or SEQ. ID. 6, and SEQ. ID. 7 or SEQ. ID. 8;
SEQ. ID. 13 or SEQ. ID. 14, SEQ. ID. 1 or SEQ. ID. 2 and IRF8 SEQ. ID. 5 or SEQ. ID. 6;
SEQ. ID. 13 or SEQ. ID. 14, SEQ. ID. 5 or SEQ. ID. 6 and PU.1 SEQ. ID. 7 or SEQ. ID. 8;
or SEQ. ID. 13 or SEQ. ID. 14, SEQ. ID. 1 or SEQ. ID. 2, SEQ. ID. 5 or SEQ. ID. 6 and SEQ. ID. 7 or SEQ. ID. 8.

4. The isolated transcription factor according to the previous claims, wherein the cell is selected from a list consisting of: of pluripotent stem cell, multipotent stem cell, differentiated cell, tumour cell, cancer cell, and mixtures thereof.

5. The isolated transcription factor according to the previous claims, as a reprogramming or inducing factor of a cell selected from a list consisting of: of pluripotent stem cell, or multipotent stem cell, differentiated cell, and mixtures thereof into dendritic cell.

6. The isolated transcription factor according to the previous claims, as a reprogramming or inducing factor of a cell selected from a list consisting of: tumour cell, cancer cell, and mixtures thereof, into antigen presenting cell.

7. The isolated transcription factor according to the previous claims, as reprogramming or inducing factors of antigen presenting cells, wherein the antigen is: a cancer antigen, a self-antigen, an allergen, an antigen from a pathogenic and/or infectious organism.

8. The isolated transcription factor according to the previous claims, for use in veterinary or human medicine, in particular in immunotherapy, or in neurodegenerative or ageing diseases, or in cancer or in infectious diseases, or as a drug screening.

9. Use of at least one isolated transcription factor comprising a sequence described in any of the previous claim as a reprogramming or inducing factor of a cell selected from a list consisting of: stem cell or a differentiated cell, or mixtures thereof, into dendritic cell or antigen presenting cell.

10. A construct or vector encoding at least one isolated transcription factor as described in any one of the claims 1-9.

11. An induced dendritic cell obtained by the method described in the preceding claim or an induced antigen presenting cell obtained by the method described in the preceding claim.

12. A composition comprising at least one isolated transcription factor as described in any one of the claims 1-9, or an induced dendritic cell as described in claim 11, or an induce antigen presenting cell as described in claim 11, or mixtures thereof, in a therapeutically effective amount and a pharmaceutically acceptable excipient.

13. The composition according to the previous claims, for use in veterinary or human medicine, in particular in immunotherapy, or in ageing diseases, or in neurodegenerative diseases, or in cancer or in infectious diseases, or as a drug screening.

14. The composition according to any of the previous claims 12-13, for use in the treatment, therapy or diagnostic of a central and peripheral nervous system disorder, or neoplasia in particular cancer, namely solid or hematological tumours.

15. A vaccine for cancer comprising at least one isolated transcription factor as described in any one of the claims 1-9, or an induced dendritic cell as described in claim 11, or an induce antigen presenting cell as described in claim 11, or mixtures thereof.


SEQUENCE LISTING

<110> CENTRO DE NEUROCIÊNCIAS E BIOLOGIA CELULAR

<120> COMPOSITIONS FOR REPROGRAMMING CELLS INTO DENDRITIC CELLS OR ANTIGEN
PRESENTING CELLS, METHODS AND USES THEREOF

<130> P466.7 EP

<150> PT110012
<151> 2017-04-05

<160> 66

<170> BiSSAP 1.3.6

<210> 1
<211> 992
<212> DNA
<213> Homo sapiens


<220>
<223> Human BATF3 (NM_018664.2)

<400> 1
ggggcagacg tgggacggga aggacggctg ccgggactgg cgcgcgggga cactgggccg 60

acgcgtggag tagcggggag agcgggaagc ctgagggggc ggggccggcg cgaggccgtg 120

ggtgcggcac gaggatgccg gcggcgggac agcgcccgta ggcagcccca cgggcagggc 180

gcgcgggcgg ggcggggcgg gccgggccag aggagcgccc ggcatgtcgc aagggctccc 240

ggccgccggc agcgtcctgc agaggagcgt cgcggcgccc gggaaccagc cgcagccgca 300

gccgcagcag cagagccctg aggatgatga caggaaggtc cgaaggagag aaaaaaaccg 360

agttgctgct cagagaagtc ggaagaagca gacccagaag gctgacaagc tccatgagga 420

atatgagagc ctggagcaag aaaacaccat gctgcggaga gagatcggga agctgacaga 480

ggagctgaag cacctgacag aggcactgaa ggagcacgag aagatgtgcc cgctgctgct 540

ctgccctatg aactttgtgc cagtgcctcc ccggccggac cctgtggccg gctgcttgcc 600

ccgatgaagc cggggacact cctctgccca gcaaggagcc ttggtcattt tcatacctgg 660

gaggaaggct tttccttcac aattgtatac agggggcacc tgtggccagg cctcctcctg 720

ggagctccag gaccagccag ctgtgttccc tgcagactgg gctcagcccg acatccaaca 780

ggcgccaaac tcacagagcc cttgtgcaga tccagcatgg aggccaccct caggagtgac 840

ttctcatcca ccctggcagc tagtaggttc tgctgttatg cagagccatt tcctctagaa 900

tttggataat aaagatgctt attgtctctc ccttctccag ttctgggaat ttacaggcac 960

aatacacttc cttttcctgg aaaaaaaaaa aa 992


<210> 2
<211> 712

<212> DNA
<213> Mus <mouse, genus>

<220>
<223> Mouse Batf3 (NM_030060.2)

<400> 2
agcagagccc cacgttcagg gcgccccgtg tggcgagcgc gcggcatgtc gcaagggccc 60

cccgcggtca gcgtgctgca gagaagcgtg gatgcgcccg ggaaccagcc gcagagcccc 120

aaggacgatg acaggaaagt tcgaaggaga gagaaaaacc gggttgcagc tcagaggagc 180

cggaagaagc agacccagaa ggctgacaag ctccacgagg agcacgagag cctggagcag 240

gagaactctg tgctgcgcag ggagatttcg aagctgaagg aggagctgcg tcacctgagc 300

gaggtgctga aggagcacga gaagatgtgc ccgctgctgc tgtgtcctat gaactttgtg 360

cagcttcggt cagaccccgt ggccagctgt ctaccacgat gacaccccag ctcactcctc 420

ctttgtccgg ctcggaacct tggtttgcac actcaggagt ttccctacac acctgtgtcc 480

aggggtacat gtggctgtgc ccttccaggg agctctgggg ccagcctggg ctcagcatcg 540

tgctctgggg catctgactt ccggcccctg ccttccggcc ccaggacaga ggatccagag 600

gccccagcaa gaaggccctt tgagtcgttc ctctctcact ttggcagcta gccggtgctg 660

tcctttggca ggatcttttc gtctagaact tggaaaataa agatagttac ct 712

<210> 3
<211> 3537
<212> DNA
<213> Homo sapiens

<220>
<223> Human SPIB (NM_003121.4)

<400> 3
ggcaaacagc ccgcccggca ccaccatgct cgccctggag gctgcacagc tcgacgggcc 60

acacttcagc tgtctgtacc cagatggcgt cttctatgac ctggacagct gcaagcattc 120

cagctaccct gattcagagg gggctcctga ctccctgtgg gactggactg tggccccacc 180

tgtcccagcc accccctatg aagccttcga cccggcagca gccgctttta gccaccccca 240

ggctgcccag ctctgctacg aaccccccac ctacagccct gcagggaacc tcgaactggc 300

ccccagcctg gaggccccgg ggcctggcct ccccgcatac cccacggaga acttcgctag 360

ccagaccctg gttcccccgg catatgcccc gtaccccagc cctgtgctat cagaggagga 420

agacttaccg ttggacagcc ctgccctgga ggtctcggac agcgagtcgg atgaggccct 480

cgtggctggc cccgagggga agggatccga ggcagggact cgcaagaagc tgcgcctgta 540

ccagttcctg ctggggctac tgacgcgcgg ggacatgcgt gagtgcgtgt ggtgggtgga 600

gccaggcgcc ggcgtcttcc agttctcctc caagcacaag gaactcctgg cgcgccgctg 660
gggccagcag aaggggaacc gcaagcgcat gacctaccag aagctggcgc gcgccctccg 720
aaactacgcc aagaccggcg agatccgcaa ggtcaagcgc aagctcacct accagttcga 780
cagcgcgctg ctgcctgcag tccgccgggc ctgagcacac ccgaggctcc cacctgcgga 840
gccgctgggg gacctcacgt cccagccagg atccccctgg aagaaaaagg gcgtccccac 900
actctaggtg ataggactta cgcatcccca ccttttgggg taaggggagt gctgccctgc 960
cataatcccc aagcccagcc cgggcctgtc tgggattccc cacttgtgcc tggggtccct 1020
ctgggatttc tttgtcatgt acagactccc tgggatcctc atgttttggg tgacaggacc 1080
tatggaccac tatactcggg gaggcagggt agcagttctt ccagaatccc aagagcttct 1140
ctgggatttt cttgtgatat ctgattcccc agtgaggcct gggacgtttt taagatcgct 1200
gtgtgtctgt aaaccctgaa tctcatctgg ggtgggggcc ctgctggcaa ccctgagccc 1260
tgtccaaggt tccctcttgt cagatctgag atttcctagt tatgtctggg gccctctggg 1320
agctgttatc atctcagatc tcttcgccca tctatggctg tgttgtcaca tctgtcccct 1380
catttttgag atcccccaat tctctggaac tattctgctg ccccttttta tgtgtctgga 1440
gttccccaat cacatctagg gctcctccaa gatccttttg tcatgtctga aatcactctt 1500
gagaggtctg gggtggagga tggggagtca gtgaaatgtg tcatgtctgg gccctgtcag 1560
ggacaccctt gttatatctg ggatcctcca atcacatctg agacctccta ggctctccat 1620
ctgatatgcc ctttcaggga ccccacaaag actgagttct catggggatc ctacccttcc 1680
tagtgccact ccctatggcc atgctgaaga ccactctggc cacgcgactg attttgggtg 1740
atcatggcag ctccccaccc atgtcatttc taaccagaag tctcaaggtc gtcacccccc 1800
tgccccccaa ccgaggcccc ggtcgctggt ggtggtctct ttagtgcact gtagcacttg 1860
gtggtggagg tgtgagggat ccacattaac agcaggccat cagctgggca atggctcaca 1920
cctgtaatcc cagcactttg ggaggcgagg cagggggaat ggcttgaacc caggcattca 1980
agaccagcct gggcaacata atgagacctc gtctctacaa aacataacaa aaacaattag 2040
ccgagcgtgg gggtgaacac ctgtggtccc agctgctcag gaggctgagg tgggaggatc 2100
tcttgagccc aggaagtagg aggctgtagt gagctgtaat cgtgccactg cactccagcc 2160
tgggcgacag agtgagacac cgtcttaaaa acaaaaacaa ggccgggcac ggtggctcat 2220
gcctgttgtc ccagcacttt gggaggccga ggcaggcgga tcacgaggtc gagagatcga 2280
gaccatcctg gccaacatgg tgaaaccctg tctctactaa aaatacagaa attagctggg 2340
cgtggtggca cgtgcctgta gtcccagcta ctcgggaggc tgaggcaaga gaatcgcttg 2400
aacgtgggag gcagaggttg cagtgagcct agattgtgcc actgcactcc agcctggggg 2460
acagagcgag actccgtctg aaaataaaaa caacaaaaac agcagaccat tcaaaatagg 2520

gagactttgc ataatccaga tttctgcctt cacttaaaac tttggacggt ctggagagag 2580

tcggccagtt ttcggtgggg ggtggggagc tggaacagga cagtagcctt tcctaatgag 2640

gcatttgttc tccaatctgc cccagtcgct gccatccctg gctatctcac cctagcagct 2700

tctcaagcct gttggcttta gaccactgta taaacccagc tggaactgaa gcctgggtgg 2760

actatggagc cctggttggg acccccaggg agtcaaaggc tgcgggccaa gaggccagag 2820

gtccttgagc ctgggtgggc aggtggatct agggtgcatg acttgctgct tcccaacctt 2880

agtttgtccc ttctgtgaaa aagggagaga aggaggagga agatctcaaa aagactttcc 2940

agcccagtgc ggtggctcac gcctgtaatc ccagcacttt gggaggccga tgcaggtgga 3000

tcacctgagg taggagttca agaccagcct gaccaacata gtgaagcccc ttctctacta 3060

aaaatacaaa attagctggg cgtggtggca tgtgcctgta ctcccagcta cttgggaggc 3120

tgaggcagga gaatcgcttg aacctgggag gcggaggttg tagtgagctg agatcacacc 3180

actgcacacc agcctgggcg acaagagcga aactccgtct caaaaaaaaa aaactgttgc 3240

agccccgttg agcctttgac accgcctgaa atccacccca ctcccaggag gaggaggagg 3300

aaggaatgcc aatgacctag agacacgaga agtccatgtg gaggcacaca gcagctgatg 3360

gcagagccca ggctgggacc tgcccttaag agaatgagtg ggaaggggga gggaggaagg 3420

gcaggtaaaa cgtcctcccc agggccccct gcaacgggga aggtactttt tacaaaagct 3480

atcattgtca ccctaaatgt ggaataaaat aagatgcatc gacgtagaca aaaaaaa 3537

<210> 4
<211> 2786
<212> DNA
<213> Mus <mouse, genus>

<220>
<223> Mouse SpiB (NM_019866.1)

<400> 4
agcctgctct gaaccaccat gcttgctctg gaggctgcac agctggatgg cccacactta 60

agctgtttgt acccagaagg agtcttctac gacctggaca gctgcaagcc cttcagttac 120

ccagattcag atgggggcct tgactctaca tggggctgga cagaggcccc gcctgctcct 180

gccatcgctc cctatgaagc cttcgatcct gctactgctg cctttagcca ctcccaaact 240

gttcagctct gttatagcca tggtcctaac ccctccacct atagccccat ggggaccctc 300

gacccagccc ccagcttgga ggccccaggg cctggcctcc aggtgtaccc cccagaggac 360

ttcaccagcc agaccctggg ctccttggct tatgctccgt accccagccc tgtgctatca 420

gaggaagaag acattatgct ggacagcccc gccctggagg tctcggacag tgagtcagac 480

gaggccctct tggctggctc cgaggggagg ggatctgagg caggtgcacg caagaagctg 540

cgcctgtacc agttcttgct ggggttgctc ctacgcgggg acatgcgcga gtgcgtgtgg 600

tgggtggagc caggtgccgg cgtcttccag ttctcctcca agcacaagga gttgttggct 660

cgccgctggg gccagcagaa gggcaaccgc aagcgcatga cgtatcagaa gctggcccga 720

gcgctgcgca actatgccaa gacaggcgaa atccgcaagg tcaaacgcaa actcacctac 780

cagtttgaca gcgcgctgct gccagcctcc cggcatgtct gagcactccg ctaaggaccc 840

ctttctggcc cctaagtccc atggagcccc atatgagggc agtcagggtt ctcagctctc 900

cctagagcct ccccagagtt tcctgtgccg tgtataggat tccaatctag gatggtcgtg 960

tttgagggag cactggccat tctacacggt ttcagaatgg caggtttctc gggggggggg 1020

gggatggggg agccctgatg tcgtctacgg ttccagaaac cgcagttctt gcgagtcctg 1080

tgagctcaca tgacatctca ccagcaggtg gcgctgtcta cagccccccc caaacccttg 1140

ttttgttggc cagataggtc ggtccctctg tactccccct gaagcccttg ttagatctga 1200

ggtctagtta tgtttggagc tctctgagaa ccctgtgcca cctgtgtgtg acttttctct 1260

gcgtccgttt atgacttttg tttgtttgag acagggtctc attatgtagc tcaggctggc 1320

cccccaactt ttaacaatcg tcctgcctcg gcctcctgag tgctgggatg acaagggtgc 1380

accatcacac caggtttttt ccttttttga gagattttac tatgtaaccc gggctgggct 1440

attctcaagc tagtggcagt cctcttgcct caggctcctc ttgcctcagg caccccttgg 1500

gaccctctgg gacctatgtc cgagatgaat ggctgggtaa ggtagggtgg gaggttcagt 1560

gaaccttata ggttgggccc ttccttctgg gatcccttga tcatatggga agttctctag 1620

gctctcagca gccctgcatt cacacactga ctgaggcgcg acctgtatgt tgtgtttgag 1680

ggggatgtgt ggcagaggta tggctgtggc aaggccggtg ccttttattc ttgagattga 1740

gtcttatgta gctcaggctg gtcttgaact cactctcact gtgtagctag ggaagacctt 1800

gagcccttgc ctcagccaga atgctcagat gtcaggcagc gcaccacgtg actgtttctt 1860

tccattgtct tgtctttttg ttgttgttgt tttcgagaca gggtttctct gtgtagccct 1920

ggccgttctg gaactcactc tgtagaccag gctggcctta aactcagaaa tccacctgcc 1980

tctgcctctc aaatgctggg attaaaggcg tgcaccacca cgcccttttt cctttttctt 2040

aagtcaaggt ctatttgtgt agcccaggct ggcttcaagc tcatgacact cttcctgcct 2100

ctgcgtctgg aactatgaac ataccttact actctgtgct tacccacgcc atgggtagac 2160

agacttctag acttggtcaa cccccaccca caaggcaggc aaattaagtc cctgcaggtg 2220

ctttctttgg aggaaagccc gctttcatag tgatctgtca agctagaaag cactcccgcc 2280

acccagatat ctaagtgtga atctttggac aactggtgac ttctggccag cttttctggg 2340

gccggcctct gtccaggtgt ctgcccagga ccctgctcag tgcctgtctg tctcacacca 2400

gtgacttcct cacacccgcc tgttcaggcc cagtcttttc cgttcaagtg ctacaggcca 2460

agtaggctca aactgtgggt ttgtcttggg catccacagc agaatcagaa cccagagctt 2520

tgaagcctga gtgaggggag gggggcactc aggcttccgt ctctctgaga aacagacgat 2580

gaagaggccc ttaaaactct ttgcaacccc atgagcctcc ccaatgagcc tgtgacacac 2640

cggaactcac ctctgtgggt ggccgggagg gggaacaggg tcatggaaga tccagatgtc 2700

catgtggtca aggctaaact gtactaaata aaattatttc tcatcaccac tatacatgca 2760

taataaataa agtgtacatc aaaatt 2786

<210> 5
<211> 2678
<212> DNA
<213> Homo sapiens

<220>
<223> Human IRF8 (NM_002163.2)

<400> 5
   agcgcggcag caagcgtggg aacgcgggcg gcgagacggc ggcaggacgg cggcaggatg 60
   tgtgaccgga atggtggtcg gcggcttcga cagtggctga tcgagcagat tgacagtagc 120
   atgtatccag gactgatttg ggagaatgag gagaagagca tgttccggat cccttggaaa 180
   cacgctggca agcaagatta taatcaggaa gtggatgcct ccatttttaa ggcctgggca 240
   gtttttaaag ggaagtttaa agaaggggac aaagctgaac cagccacttg gaagacgagg 300
   ttacgctgtg ctttgaataa gagcccagat tttgaggaag tgacggaccg gtcccaactg 360
   gacatttccg agccatacaa agtttaccga attgttcctg aggaagagca aaaatgcaaa 420
   ctaggcgtgg caactgctgg ctgcgtgaat gaagttacag agatggagtg cggtcgctct 480
   gaaatcgacg agctgatcaa ggagccttct gtggacgatt acatggggat gatcaaaagg 540
   agcccttccc cgccggaggc ctgtcggagt cagctccttc cagactggtg ggcgcagcag 600
   cccagcacag gcgtgccgct ggtgacgggg tacaccacct acgacgcgca ccattcagca 660
   ttctcccaga tggtgatcag cttctactat gggggcaagc tggtgggcca ggccaccacc 720
   acctgccccg agggctgccg cctgtccctg agccagcctg ggctgcccgg caccaagctg 780
   tatgggcccg agggcctgga gctggtgcgc ttcccgccgg ccgacgccat ccccagcgag 840
   cgacagaggc aggtgacgcg gaagctgttc gggcacctgg agcgcggggt gctgctgcac 900
   agcagccggc agggcgtgtt cgtcaagcgg ctgtgccagg gccgcgtgtt ctgcagcggc 960
   aacgccgtgg tgtgcaaagg caggcccaac aagctggagc gtgatgaggt ggtccaggtc 1020
   ttcgacacca gccagttctt ccgagagctg cagcagttct ataacagcca gggccggctt 1080
   cctgacggca gggtggtgct gtgctttggg gaagagtttc cggatatggc ccccttgcgc 1140
   tccaaactca ttctcgtgca gattgagcag ctgtatgtcc ggcaactggc agaagaggct 1200
   gggaagagct gtggagccgg ctctgtgatg caggcccccg aggagccgcc gccagaccag 1260
   gtcttccgga tgtttccaga tatttgtgcc tcacaccaga gatcattttt cagagaaaac 1320
   caacagatca ccgtctaagt gcgtcgcttg ggcgccccac cccgtctgcg tcctgcatcc 1380
   atctccctgt tacagtggcc cgcatcatga ttaaagaatg tggatccctc tgtctggggt 1440
   gggatgcctt actttgcact taatttaata agggcattct cggaggagta gacgtttaat 1500
   acgaagtggc ggcatagccc tgccgagatg tcggtgatgg cctggatgct gtaaccacaa 1560
   cctgtggcta aaaattttat tttctatcct ttacccgtca ttatcattag ttgctatgat 1620
   tctttctgca ttttcggtta actatcattt ccaaagactt gtcattcagt aatattagca 1680
   gatagctgct tcgataaagg aatttggagt ttaaaaatca acttgtgaaa acaaggttgt 1740
   ttttgtcttt atcgtttgtt agagttatag atttatgatt tcataggctt gattctatgt 1800
   gaaatatctt tttactttta tgcattttaa taagatttaa aaatatttag attaaagccc 1860
   cctttaatga gtacaagaaa aactcttggc ttgttagaag aaagtatatt ctttctagaa 1920
   tttggtgcag gaatatgtgt tcatatccag gcaaacgggt gtgtttttat cttcagacaa 1980
   tgaaaccttc tcctctgggg ctttgttgcc aggaagatta gaactaaatt tatttttttc 2040
   atttctgtca tgaaatcatt ccagatacct cttttcttct ttccaaatgg ttttcacatg 2100
   tgtttgaaat atttgtactt cgaattgtcg gattttccat gtcctccttt ctcctttgtg 2160
   cccagcctga gtcagcacca atcccgcatt cagaacctcc cagtgaaagg gcagccttca 2220
   ttttgagaag gtggaaggtg ttagggtttg ggagacagct catccaatct cccaagtctc 2280
   atggtggatt tgtgactgtg agagtttccg gtttaaaatc tgaaaagcca gatatgcctg 2340
   tttccttttc ccagcaccat gcctgtggag gggacagtca gacccagagg tcctttacgt 2400
   gtggatggag ttcacaggcg aatagaggag aggaccaggg gacgtggctt gtcccttttg 2460
   tccaacaaag cattatattt ttaagaatgg cagacctgtt tgctgaagtg ttcataagat 2520
   aacaataggc ttgaatctcc aattcaaatg aatgtcaaag cacatatctt taatatgctg 2580
   aatgaatatt tatttttgta tccattaaaa cagtatattg atctctttta ttctttatta 2640
   aaataaaatg ctctttttta aaaaaaaaaa aaaaaaaa 2678

<210> 6
<211> 3391
<212> DNA
<213> Mus <mouse, genus>

<220>
<223> Mouse Irf8 (NM_001301811.1)

<400> 6

ttccaagccc actggtggca gttagggctg ctgtgtggct aaaaccagcc ctggagagac 60

ctcatctccc tcctctcagg ctgctgccgt ctttcctggc cccactacca cctgcaagcg 120

gcctggagtt tctgaccctc aggcctcttt ccagcaatcc tgactttgtg ggacatgctg 180

gctctggatg tgactatgtt cttggggata tcttgacttc tcactgatga taacttcttt 240

tttttttttt tttccctacc ccaagaggtc attttagttg gaagactaga agtgctgtgt 300

tctggttttc agggtgggcg tctgtccaac tgctttgggg atttccaggc tgttctagta 360

aatgctggct gcaagtccct gtgcacaaat gccctctctc cccacgctgt aggaaaagca 420

gacccgagag tgtccaagtg tgccattagg tgtctgggga tggcgggcct ggatatggag 480

tccctgagct ggctttgtac tgtccctttg ttctcactga gggctgattg taagagggac 540

tccctgtgtt gttggaggag tcctctagga tacaggctct gattgcagga tgtgtgaccg 600

gaacggcggg cggcggctgc ggcagtggct gatcgaacag atcgacagca gcatgtaccc 660

ggggctgatc tgggaaaatg atgagaagac catgttccgt atcccctgga agcatgccgg 720

caagcaggat tacaatcagg aggtggatgc ttccatcttc aaggcctggg cagtttttaa 780

agggaagttt aaagagggag acaaagctga accagccacg tggaagacga ggttacgctg 840

tgctctgaac aagagcccag attttgaaga agtgactgac cggtcccagc tggacatttc 900

tgagccatat aaagtttacc gaattgtccc cgaggaagaa caaaaatgca agctgggcgt 960

ggcacctgca ggctgcatga gcgaagttcc tgagatggag tgtggccgct cagagattga 1020

ggagctgatc aaggaacctt ctgtggatga gtacatgggt atgaccaaga ggagcccatc 1080

cccaccagag gcctgcagga gccagatcct ccctgactgg tgggtccagc agcccagtgc 1140

aggcctgcca ctggtgaccg gatatgccgc ctatgacaca caccattcag ctttctccca 1200

gatggtcatc agcttctact acgggggcaa gctggtgggc caggccacca ccacctgcct 1260

tgaaggctgc cgtctctccc tgagccagcc ggggctgcct aagttgtatg ggccggatgg 1320

cctggaaccc gtgtgctttc cgacggccga caccatcccc agtgagcggc agaggcaggt 1380

gacccggaag ctgtttgggc acctggaacg tggcgtgcta ctgcacagca accgcaaggg 1440

cgtgttcgtg aagcggctgt gccagggccg cgtgttctgc agcggcaacg cggtggtgtg 1500

caagggcagg cccaacaagc tggagcggga cgaggtggtg caggtctttg acaccaacca 1560

gttcatccga gagctgcagc aattctacgc cacccagagc cgcctacctg acagcagggt 1620

ggtcctgtgc ttcggggagg agtttccgga cactgtgccc ttgcgctcca aactcattct 1680

ggtgcaggta gagcagctgt atgccaggca gctggtggag gaagcgggca agagctgcgg 1740

tgctggctcc ctgatgccag ccctggagga gccccagccg gaccaggctt tccgcatgtt 1800

tccggatatc tgtacctcac accagagacc cttttttaga gaaaatcaac agatcaccgt 1860

ctaagcctca gtccgggcac cccacctcgc ctgagctcaa gcttcaagag tctgtgacta 1920

agagaattcc gaaaggatgt ggagccctct gactggggtg ggcgggtgtc ctccaagggg 1980

cctccggaag cccacagagg gatgcgctcc tgctcaggca ggtgtcagaa gcttgcaggg 2040

gctgtggccg caacctgtga ttaaagcatt cctttcctgc gtttccccct tcaccactaa 2100

tggctggcct ttctgtgtgc tgaggtcttt cgacagttca aatcatctgg tggcagcaga 2160

ctcgcctttg cccttctgcg gccgagggcg gagatttatg actttctctg cttggttgga 2220

gaagaagaat ctttactatt cagcttcttt tctttttggc cagaactctg aaaaaaaaaa 2280

aaactctttt taagacaata tttgtattct cacaggctca gctgtcaatc acttgagacc 2340

ttccctgtaa agtggggcag attttaaata tgggtgtaga tactgcttgc agccttcgca 2400

ggaattttgg ttgtggttca ttgattcaca cagactctgt gtcagctgac agggctgtgt 2460

ggggcatcaa aggaggacca ggcactgtgg agaagaccca ttcactggca tctcaccctt 2520

ccttgtccag ctccataccc agtcctaaga cccagtgaaa agccacgtcc aaactgtgct 2580

ctgggctcat cagtgcccac ccacgtacca gggaaaggca cacaccctac ccagtgggca 2640

cagagcggaa tgtcccccta ccgcaccatt tgcgccccca atctggctgt ccaacctagt 2700

ttgtaagtaa tctaaatcag tgactatagc cccgcctaag ggacacttcc cggaggaggg 2760

agccgctgaa aaggagttag tttgagggtc agtacacaac aggggcagaa agccaagcag 2820

atgtgggggc agggagagtc atcatctgct tttgtctgag agaaggagag cttctccgtt 2880

tgttcaactt tgtaacaagc tgggttacat gctccacgca gctagagaag cctaggtgct 2940

ctgcattccc tggggaactg caggaaagcc ttacctgctg actgttgctc tggggaaaag 3000

cctgagggtc cagagcagct acaagctaca ggccatacct tacaacctga aaagctaagg 3060

accacggtga ccttcccggc tactgtgtga aggtgctggg tggggcctgc tcaacagaca 3120

gggtcgacag agtgtgtgat acatgcaaac agaatccttg gagtgtgtga tacatgcaaa 3180

cagaatcctg ggcccctgct tctccccctc agtcaaagca ggagtgtccc ttccgaagcc 3240

aggacaacct gttcacaagg ccccttgtca catgtcacct tccacctgcc tcaaggagtg 3300

ctagtgtcca aatatttatt tttgtattct cttaagaagt attgatttca tcctttatta 3360

aaaaaagttg ctctttcaca aaaaaaaaaa a 3391

<210> 7
<211> 1426
<212> DNA
<213> Homo sapiens

<220>
<223> Human PU.1 (NM_001080547.1)

<400> 7
gactatctcc cagcggcagg cccttcgata aaatcaggaa cttgtgctgg ccctgcaatg 60

tcaaggaggg ggctcaccc agggctcctg tagctcaggg ggcaggcctg agccctgcac 120

ccgccccacg accgtccagc ccctgacggg gcaccccatc ctgaggggct ctgcattggc 180

ccccaccgag gcaggggatc tgaccgactc ggagcccggc tggatgttac aggcgtgcaa 240

aatggaaggg tttcccctcg tcccccctca gccatcagaa gacctggtgc cctatgacac 300

ggatctatac caacgccaaa cgcacgagta ttacccctat ctcagcagtg atggggagag 360

ccatagcgac cattactggg acttccaccc ccaccacgtg cacagcgagt tcgagagctt 420

cgccgagaac aacttcacgg agctccagag cgtgcagccc ccgcagctgc agcagctcta 480

ccgccacatg gagctggagc agatgcacgt cctcgatacc cccatggtgc caccccatcc 540

cagtcttggc caccaggtct cctacctgcc ccggatgtgc ctccagtacc catccctgtc 600

cccagcccag cccagctcag atgaggagga gggcgagcgg cagagccccc cactggaggt 660

gtctgacggc gaggcggatg gcctggagcc cgggcctggg ctcctgcctg gggagacagg 720

cagcaagaag aagatccgcc tgtaccagtt cctgttggac ctgctccgca gcggcgacat 780

gaaggacagc atctggtggg tggacaagga caagggcacc ttccagttct cgtccaagca 840

caaggaggcg ctggcgcacc gctggggcat ccagaagggc aaccgcaaga agatgaccta 900

ccagaagatg gcgcgcgcgc tgcgcaacta cggcaagacg ggcgaggtca agaaggtgaa 960

gaagaagctc acctaccagt tcagcggcga agtgctgggc cgcgggggcc tggccgagcg 1020

gcgccacccg ccccactgag cccgcagccc ccgccgggcc ccgccaggcc tccccgctgg 1080

ccatagcatt aagccctcgc ccggcccgga cacagggagg acgctcccgg ggcccagagg 1140

caggactgtg gcgggccggg cctcgcctca cccgccccct ccccccactc caggccccct 1200

ccacatcccg cttcgcctcc ctccaggact ccaccccggc tcccggacgc cagctgggcg 1260

tcagacccca ccggggcaac cttgcagagg acgacccggg gtactgcctt gggagtctca 1320

agtccgtatg taaatcagat ctcccctctc acccctccca cccattaacc tcctcccaaa 1380

aaacaagtaa agttattctc aatccatcaa aaaaaaaaaa aaaaaa 1426

<210> 8
<211> 1473
<212> DNA
<213> Mus <mouse, genus>

<220>
<223> Mouse Pu.1 (NM_011355.2)

<400> 8
aagagattta tgcaaacggg ctggggcggt gatgtcaccc caaggggact atctcccagt 60

ggcaggccct tcgataaaat caggaacttg tgctggccct gcaatgtcaa gggagggggc 120

tcacccaggg ctcctgtagc tcagggggca ggcctgagcc ctgcgtctga cccacgaccg 180

tccagtcccc cgacggggca cctggtcctg agggggatcc gccttgatcc ccaccgaagc 240

aggggatctg accaacctgg agctcagctg gatgttacag gcgtgcaaaa tggaagggtt 300

ttccctcacc gcccctccat cggatgactt ggttacttac gattcagagc tataccaacg 360

tccaatgcat gactactact ccttcgtggg cagcgatgga gaaagccata gcgatcacta 420

ctgggatttc tccgcacacc atgtccacaa caacgagttt gagaacttcc ctgagaacca 480

cttcacagag ctgcagagtg tgcagccccc gcagctacag cagctctatc gccacatgga 540

gctggaacag atgcacgtcc tcgatactcc catggtgcca ccccacaccg gcctcagtca 600

ccaggtttcc tacatgcccc ggatgtgctt cccttatcaa accttgtccc cagcccacca 660

gcagagctca gatgaggagg agggtgagag gcagagccct cccctggagg tgtctgatgg 720

agaagctgat ggcttggagc ctgggccagg tcttctgcac ggggagacag gcagcaagaa 780

aaagattcgc ctgtaccagt tcctgctgga cctgctgcgc agcggcgaca tgaaggacag 840

catctggtgg gtggacaagg acaaaggtac cttccagttc tcgtccaagc acaaggaggc 900

gctggcgcac cgctggggca tccagaaggg caaccgcaag aagatgacct accagaagat 960

ggcgcgcgcg ctgcgcaact acggcaagac aggcgaggtg aagaaagtca agaagaagct 1020

cacctaccag ttcagcggcg aggtgctggg ccgtgggggc ctggccgagc ggcgcctccc 1080

gccccactga tcgcccgcag agaccgccag gctcctggac cccgccggcc atagcattaa 1140

cccgtcgccc ggcccggaca cagggaggac attcccaggg ccgaggcagg actgggggcc 1200

cggcctcgcc ctcccatgcc cggcctggcc cgccccaccc gctttgcctc ccaccaggac 1260

tctagcccgc tccaagggcc gcctgggcct cggacctcaa ccgagggtca gcctggctta 1320

gtggccacgg tgcttccttg ggagtctggc gctggcacct ttttgtatat tgaatgcttt 1380

ttaaaaagct cttcctcccc accccctcat tagtcactaa agacaagtaa aattattgac 1440

agctattctc ccagaaaaaa aaaaaaaaaa aaa 1473

<210> 9
<211> 5332
<212> DNA
<213> Homo sapiens

<220>
<223> Human IRF4 (NM_002460.3)

<400> 9
acctcgcact ctcagtttca ccgctcgatc ttgggaccca ccgctgccct cagctccgag 60

tccagggcga gtgcagagca gagcgggcgg aggaccccgg gcgcgggcgc ggacggcacg 120

cggggcatga acctggaggg cggcggccga ggcggagagt tcggcatgag cgcggtgagc 180

tgcggcaacg ggaagctccg ccagtggctg atcgaccaga tcgacagcgg caagtacccc 240

gggctggtgt gggagaacga ggagaagagc atcttccgca tcccctggaa gcacgcgggc 300

aagcaggact acaaccgcga ggaggacgcc gcgctcttca aggcttgggc actgtttaaa 360

ggaaagttcc gagaaggcat cgacaagccg gaccctccca cctggaagac gcgcctgcgg 420

tgcgctttga acaagagcaa tgactttgag gaactggttg agcggagcca gctggacatc 480

tcagacccgt acaaagtgta caggattgtt cctgagggag ccaaaaaagg agccaagcag 540

ctcaccctgg aggacccgca gatgtccatg agccacccct acaccatgac aacgccttac 600

ccttcgctcc cagcccagca ggttcacaac tacatgatgc cacccctcga ccgaagctgg 660

agggactacg tcccggatca gccacacccg gaaatcccgt accaatgtcc catgacgttt 720

ggaccccgcg gccaccactg gcaaggccca gcttgtgaaa atggttgcca ggtgacagga 780

accttttatg cttgtgcccc acctgagtcc caggctcccg gagtccccac agagccaagc 840

ataaggtctg ccgaagcctt ggcgttctca gactgccggc tgcacatctg cctgtactac 900

cgggaaatcc tcgtgaagga gctgaccacg tccagccccg agggctgccg gatctcccat 960

ggacatacgt atgacgccag caacctggac caggtcctgt tcccctaccc agaggacaat 1020

ggccagagga aaaacattga gaagctgctg agccacctgg agaggggcgt ggtcctctgg 1080

atggcccccg acgggctcta tgcgaaaaga ctgtgccaga gcaggatcta ctgggacggg 1140

cccctggcgc tgtgcaacga ccggcccaac aaactggaga gagaccagac ctgcaagctc 1200

tttgacacac agcagttctt gtcagagctg caagcgtttg ctcaccacgg ccgctccctg 1260

ccaagattcc aggtgactct atgctttgga gaggagtttc cagaccctca gaggcaaaga 1320

aagctcatca cagctcacgt agaacctctg ctagccagac aactatatta ttttgctcaa 1380

caaaacagtg gacatttcct gaggggctac gatttaccag aacacatcag caatccagaa 1440

gattaccaca gatctatccg ccattcctct attcaagaat gaaaaatgtc aagatgagtg 1500

gttttctttt tccttttttt tttttttttt ttgatacggg gatacggggt cttgctctgt 1560

ctcccaggct ggagtgcagt gacacaatct cagctcactg tgacctccgc ctcctgggtt 1620

caagagactc tcctgcctca gcctccctgg tagctgggat tacaggtgtg agccactgca 1680

cccacccaag acaagtgatt ttcattgtaa atatttgact ttagtgaaag cgtccaattg 1740

actgccctct tactgttttg aggaattcag aagtggagat ttcagttcag cggttgagga 1800

gaattgcggc gagacaagca tggaaaatca gtgacatctg attggcagat gagcttattt 1860

caaaaggaag ggtggctttg catttcttgt gttctgtaga ctgccatcat tgatgatcac 1920

tgtgaaaatt gaccaagtga tgtgtttaca tttactgaaa tgcgctcttt aatttgttgt 1980

agattaggtc ttgctggaag acagagaaaa cttgcctttc agtattgaca ctgactagag 2040

tgatgactgc ttgtaggtat gtctgtgcca tttctcaggg aagtaagatg taaattgaag 2100

aagcctcaca cgtaaaagaa atgtattaat gtatgtagga gctgcagttc ttgtggaaga 2160
cacttgctga gtgaaggaaa tgaatctttg actgaagccg tgcctgtagc cttgggaggg 2220
cccatccccc acctgccagc ggtttcctgg tgtgggtccc tctgccccgc cctccttccc 2280
attggctttc tctccttggc ctttcctgga agccagttag taaacttcct attttcttga 2340
gtcaaaaaac atgagcgcta ctcttggatg ggacattttt gtctgtccta caatctagta 2400
atgtctaagt aatggttaag ttttcttgtt tctgcatctt tttgaccctc attctttaga 2460
gatgctaaaa ttcttcgcat aaagaagaag aaattaagga acataaatct taatacttga 2520
actgttgccc ttctgtccaa gtacttaact atctgttccc ttcctctgtg ccacgctcct 2580
ctgtttgttt ggctgtccag cgatcagcca tggcgacact aaaggaggag gagccgggga 2640
ctcccaggct ggagagcact gccaggaccc accactggaa gcaggatgga gctgactacg 2700
gaactgcaca ctcagtgggc tgtttctgct tatttcatct gttctatgct tcctcgtgcc 2760
aattatagtt tgacagggcc ttaaaattac ttggcttttt ccaaatgctt ctatttatag 2820
aatcccaaag acctccactt gcttaagtat acctatcact tacatttttg tggttttgag 2880
aaagtacagc agtagactgg ggcgtcacct ccaggccgtt tctcatacta caggatattt 2940
actattactc ccaggatcag cagaagattg cgtagctctc aaatgtgtgt tcctgctttt 3000
ctaatggata ttttaaattc attcaacaag cacctagtaa gtgcctgctg tatccctaca 3060
ttacacagtt cagcctttat caagcttagt gagcagtgag cactgaaaca ttatttttta 3120
atgtttaaaa agtttctaat attaaagtca gaatattaat acaattaata ttaatattaa 3180
ctacagaaaa gacaaacagt agagaacagc aaaaaaataa aaaggatctc cttttttccc 3240
agcccaaatt ctcctctcta aaagtgtcca caagaagggg tgtttattct tccaacacat 3300
ttcacttttc tgtaaatata cataaactta aaaagaaaac ctcatggagt catcttgcac 3360
acactttcat gcagtgctct ttgtagctaa cagtgaagat ttacctcgtt ctgctcagag 3420
gccttgctgt ggagctccac tgccatgtac ccagtagggt ttgacatttc attagccatg 3480
caacatggat atgtattggg cagcagactg tgtttcgtga actgcagtga tgtatacatc 3540
ttatagatgc aaagtatttt ggggtatatt atcctaaggg aagataaaga tgatattaag 3600
aactgctgtt tcacggggcc cttacctgtg accctctttg ctgaagaata tttaacccca 3660
cacagcactt caaagaagct gtcttggaag tctgtctcag gagcaccctg tcttcttaat 3720
tctccaagcg gatgctccat ttcaattgct ttgtgacttc ttcttctttg tttttttaaa 3780
tattatgctg ctttaacagt ggagctgaat tttctggaaa atgcttcttg gctggggcca 3840
ctacctcctt tcctatcttt acatctatgt gtatgttgac tttttaaaat tctgagtgat 3900
ccagggtatg acctagggaa tgaactagct atgaaatact cagggttagg aatcctagca 3960
cttgtctcag gactctgaaa aggaacggct tcctcattcc ttgtcttgat aaagtggaat 4020

tggcaaacta gaatttagtt tgtactcagt ggacagtgct gttgaagatt tgaggacttg 4080

ttaaagagca ctgggtcata tggaaaaaat gtatgtgtct cccaggtgca tttcttggtt 4140

tatgtcttgt tcttgagatt ttgtatattt aggaaaacct caagcagtaa ttaatatctc 4200

ctggaacact atagagaacc aagtgaccga ctcatttaca actgaaacct aggaagcccc 4260

tgagtcctga gcgaaaacag gagagttagt cgccctacag aaaacccagc tagactattg 4320

ggtatgaact aaaaagagac tgtgccatgg tgagaaaaat gtaaaatcct acagtgaaat 4380

gagcagccct tacagtattg ttaccaccaa gggcaggtag gtattagtgt ttgaaaaagc 4440

tggtctttga gcgagggcat aaatacagct agccccaggg gtggaacaac tctgggagtc 4500

ttgggtactc gcacctcttg gctttgttga tgctccgcca ggaaggccac ttgtgtgtgc 4560

gtgtcagtta cttttttagt aacaattcag atccagtgta aacttccgtt cattgctctc 4620

cagtcacatg cccccacttc cccacaggtg aaagtttttc tgaaagtgtt gggattggtt 4680

aaggtcttta tttgtattac gtatctcccc aagtcctctg tggccagctg catctgtctg 4740

aatggtgcgt gaaggctctc agaccttaca caccattttg taagttatgt tttacatgcc 4800

ccgtttttga gactgatctc gatgcaggtg gatctccttg agatcctgat agcctgttac 4860

aggaatgaag taaaggtcag tttttttttg tattgatttt cacagctttg aggaacatgc 4920

ataagaaatg tagctgaagt agaggggacg tgagagaagg gccaggccgg caggccaacc 4980

ctcctccaat ggaaattccc gtgttgcttc aaactgagac agatgggact taacaggcaa 5040

tggggtccac ttccccctct tcagcatccc ccgtacccca ctttctgctg aaagaactgc 5100

cagcaggtag gaccccagag gcccccaaat gaaagcttga atttccccta ctggctctgc 5160

gttttgctga gatctgtagg aaaggatgct tcacaaactg aggtagataa tgctatgctg 5220

tcgttggtat acatcatgaa tttttatgta aattgctctg caaagcaaat tgatatgttt 5280

gataaattta tgtttttagg taaataaaaa cttttaaaaa tttgttaaaa aa 5332

<210> 10
<211> 4762
<212> DNA
<213> Mus <mouse, genus>

<220>
<223> Mouse Irf4 (NM_013674.2)

<400> 10
tttcctagtt tcaccacttg aacttgggac cctttgctgc cctcagctaa gagtgcgggt 60

gagcgcacaa gcccaggagg aggtccgcac gcgtcatgaa cttggagacg ggcagccggg 120

gctcagagtt cggcatgagc gcagtgagct gcggcaatgg gaaactccga cagtggttga 180

tcgaccagat cgacagcggc aagtaccccg ggctggtgtg ggagaacgag gagaagagcg 240

tcttccgcat cccgtggaaa cacgcgggca agcaggacta caatcgtgag gaggacgctg 300

ccctcttcaa ggcttgggca ttgtttaaag gcaagttccg agaagggatc gacaagccag 360

atcctcctac ttggaagaca agattacgat gtgctctgaa caagagcaat gactttgagg 420

aattggtcga gaggagccag ctggatatct ctgacccata caaggtgtac aggattgttc 480

cagagggagc caaaaaagga gcaaagcagc tcactttgga tgacacacag atggccatgg 540

gccaccccta ccccatgaca gcaccttatg gctctctgcc agcccagcag gttcataact 600

acatgatgcc accccatgac aggagctgga gggattatgc ccctgaccag tcacacccag 660

aaatcccata tcaatgtcct gtgacgtttg gcccacgagg ccaccactgg caaggcccat 720

cttgtgaaaa tggttgccag gtgacaggaa ccttttatgc ttgtgcccca cctgagtccc 780

aggctcctgg aatccccatt gagccaagca taaggtctgc tgaagccttg gcgctctcag 840

actgccggct gcatatctgc ctgtattacc gggacatcct cgtgaaagag ctgaccacga 900

cgagccctga aggctgccgg atctcccacg gacacaccta tgatgttagc aacctggacc 960

aggtcctgtt tccctacccg gacgacaatg gacagaggaa gaacattgag aagttgctga 1020

gccacctgga gaggggactg gtcctctgga tggctccaga tgggctttat gccaaaagac 1080

tctgccagag taggatctac tgggatgggc ccctggcact gtgcagcgat cggcccaaca 1140

agctagaaag agaccagact tgcaagctct ttgacacaca gcagtttcta tcagagctgc 1200

aagtgtttgc tcaccatggc cggccagcac cgagattcca ggtgactctg tgctttggtg 1260

aggagtttcc agaccctcag agacagagga agctcatcac agctcatgtg gaacctctgc 1320

tagccagaca actgtattac tttgctcaac aaaacactgg acatttcctg aggggctacg 1380

agttacctga acacgttacc actccagatt accaccgctc cctccgtcat tcttccatcc 1440

aagagtgaga agaaatactc tgacagggca gccggctgct gccctttctc tttggaagag 1500

ctaagaagtg agtgggtttc cacttgaaga caacaacagg gctttgtgag gaaaaacagc 1560

tgtatctgct caacagagga gcttccccca gaagagtgcc tgtcatccag gtcttgacaa 1620

gtgccaggac ttgggtgact gtgccctggc ttataactgt gaaacttgat cagtgttgtg 1680

tttacatgta cttgaatgct ggctttagcc tggtatagat ggacttttgc ttgaagactg 1740

aaaacctgtg ccagcatgaa tccctgacaa gagaagacat acgtattatt ggtccatttc 1800

tcagggaagt aaagtctaga ctagagacta cgctgtccac tcacagagaa acgcattcct 1860

ggatagagga gctgtaggtt gcccagatca tgtccactga gtgaagggac ttggttctta 1920

ggtccagtct aggctatcat gtcctgatcc tgcactctct ctgagtagac cctacatcct 1980

gtttgctccc tcttcttgcc cttccctaaa agccagctgg tggactcttg ttgccacata 2040

cttgagccaa aaaaccatca gggacatata agatgagaca tgtttgcatc tcctctgatc 2100

taacaacggc tacaaagttt tttgttgttg ttgtctcccc tttctgtttt gcctctcatt 2160

ctttagaatt gttgcttttt tgtaaagaaa ggaagagaga gagaccgaat atactagaga 2220

tagattttta aaattctgtg ccatttcccc ttctatttgt tttgttggcc atcagtggtc 2280

cccatggcaa cactggaagg gcggagcctt tgagcaggtt gggaactgtc ccggacacca 2340

gtggttcagt gtacagctgt agactcagtg agctgtttct gcttattttg tatattagat 2400

gcttccttgt gccaataata gtttgacaga gtctcaaagc taccgggctt cttttccaac 2460

aacacattct ttcacagaat cccaaagagt ctcacttgcc ttcacaaccg tctgatcctt 2520

tattgttact gttttgttta ggtggctttg ccatgctctg ggatttttct cctaccacaa 2580

catcattcct gactctttcg ggctcagaaa gagttaagcg ctcttgcaca cttggcactt 2640

acttcttttt aatttttttt ttaacatgcc actcttaata tttcttctta agtctttcag 2700

cagcatacat tcaataagca atggaccatt gggtgtctca tttcattgtt ttatttttaa 2760

aaaggaacct ttgacaaata atacacatta cagaaaatgg aagcattttt catagagaag 2820

gtaagaactg aaatgcattt tatgttcgaa tcctccttgc tagtattagc cccaagagtt 2880

cagtctgtcc tttaagcatg ttttagtttt caatacatat atatgaagtt gccctttctt 2940

agagagagag agagagatat gtgatgacac tcttatgtac ccaactatac agatttacct 3000

catcttttga gtggcatgtg gacttccact gctatgtgcc taggtgcagt ttagacatct 3060

tcttagctac ccatcccagc agcccactga gtctcgtgaa ctaaagtcac ttatacatct 3120

tatagatgca aaacattctg ggtatattta tcccaaggga agatgaaggc aaaatcggga 3180

agcactcttc ctgaggcccc aagccacagc tgtcctcctg gagtatctta accatatctg 3240

gcacttcacc aaggtccctt gcctggtcct gtatgaccat gactcttcag tccataagca 3300

gatgctttgt ctcaattgcc atgtgaagtc ttttggtttt tggttttttt ccttaatgtt 3360

aagctgtttt aacagtgaag ctgaattttc tggaaaatgc tgtatggttc gagcccccag 3420

ttcatgtttc actttacagc catgcatgct gatgccttcc atcttgacaa atggaatgat 3480

gggtaaacac tcctgacaca aagaggaaca ccttcctcgt cccttgctga aacaaagtgc 3540

aatgcacaca gtagaattta gtttgcttgc actggaagat gccgttgaag aggtaggctg 3600

aggaacactg tatcctaggg agaaaatatg caagtctcac gggtgctttc tgttggcttg 3660

tttctggtcc ttgagcaggc ttaagtatct agtttcaaac tagaagcccc aaagccctca 3720

gtcgttgtcc tggacagagt tggtagaccc cacctgtaga aatccagatg cactctggga 3780

ttggagcaca gagaggcttg ctctatcgtg agaatgccca gcctgcagag gcccaggtgc 3840

ggtgcctgag tggctgtatg ccagacacag gaacgccagc aggctgagtg tggtgactga 3900

tatgtgaggt gcccttgccc tgcagacagg ctgaatgtga gccccagcag cagaggattc 3960

tgagagtcag ggtggtgtca tgtcctacac aggaagagcc ttcaatatta ctttttaaac 4020

gaactttccc attgctgata ttttgataaa aacccacaca aggtttaatt acatgccccc 4080

tcatccctct ccacacagtc ttgcccatcc cactggtgaa ttgttgttgt tcttgttgtt 4140

gttttttgtt attggtgaaa attgtgaaat tgttaaggtc tctgtttttg ttatttatct 4200

ccctaaatca catggctgct gcatacattg caatgttgcc ctaccacatg tatgtttttg 4260

caagttgctt cttcactcat tgaagaagcc tgcaccgtaa gtgaaactca cttagtcctt 4320

gctaattgct gctcactgtc acgtgaacgc agagaaggct gattttcctg gacccatttt 4380

cagtgctttg aggaacatga gtccagagta tattgaagta gaagacagtg tttggcaagc 4440

cagttcttat tagacactgc tacaggcttc acaatcttca aggtggaccc acctcatgtg 4500

gaagggcatc attgcccctc tgctcccatg cgctgtgcac ccccttcaca gtgaaagacc 4560

tgctggggac caggactcag agatctctgg cttcgagctc atatcttggc ttcctgtcat 4620

gtctaagccc gacaggaaag tgtggcacca tcctgcctgc ggcgttggat ggataggcca 4680

tggattttgc tgtaaacttg cttcacaagg aaagctgtgt gttttaaaat atttgataaa 4740

taaaggattt ctagaaagat tg 4762

<210> 11
<211> 4978
<212> DNA
<213> Homo sapiens

<220>
<223> Human STAT3 (NM_139276.2)

<400> 11
ggtttccgga gctgcggcgg cgcagactgg gagggggagc cgggggttcc gacgtcgcag 60

ccgagggaac aagccccaac cggatcctgg acaggcaccc cggcttggcg ctgtctctcc 120

ccctcggctc ggagaggccc ttcggcctga gggagcctcg ccgcccgtcc ccggcacacg 180

cgcagccccg gcctctcggc ctctgccgga gaaacagttg ggacccctga ttttagcagg 240

atggcccaat ggaatcagct acagcagctt gacacacggt acctggagca gctccatcag 300

ctctacagtg acagcttccc aatggagctg cggcagtttc tggccccttg gattgagagt 360

caagattggg catatgcggc cagcaaagaa tcacatgcca ctttggtgtt tcataatctc 420

ctgggagaga ttgaccagca gtatagccgc ttcctgcaag agtcgaatgt tctctatcag 480

cacaatctac gaagaatcaa gcagtttctt cagagcaggt atcttgagaa gccaatggag 540

attgcccgga ttgtggcccg gtgcctgtgg gaagaatcac gccttctaca gactgcagcc 600

actgcggccc agcaaggggg ccaggccaac caccccacag cagccgtggt gacggagaag 660

cagcagatgc tggagcagca ccttcaggat gtccggaaga gagtgcagga tctagaacag 720

aaaatgaaag tggtagagaa tctccaggat gactttgatt tcaactataa aaccctcaag 780

agtcaaggag acatgcaaga tctgaatgga aacaaccagt cagtgaccag gcagaagatg 840

cagcagctgg aacagatgct cactgcgctg gaccagatgc ggagaagcat cgtgagtgag 900

ctggcggggc ttttgtcagc gatggagtac gtgcagaaaa ctctcacgga cgaggagctg 960

gctgactgga agaggcggca acagattgcc tgcattggag gcccgcccaa catctgccta 1020

gatcggctag aaaactggat aacgtcatta gcagaatctc aacttcagac ccgtcaacaa 1080

attaagaaac tggaggagtt gcagcaaaaa gtttcctaca aaggggaccc cattgtacag 1140

caccggccga tgctggagga gagaatcgtg gagctgttta gaaacttaat gaaaagtgcc 1200

tttgtggtgg agcggcagcc ctgcatgccc atgcatcctg accggcccct cgtcatcaag 1260

accggcgtcc agttcactac taaagtcagg ttgctggtca aattccctga gttgaattat 1320

cagcttaaaa ttaaagtgtg cattgacaaa gactctgggg acgttgcagc tctcagagga 1380

tcccggaaat ttaacattct gggcacaaac acaaaagtga tgaacatgga agaatccaac 1440

aacggcagcc tctctgcaga attcaaacac ttgaccctga gggagcagag atgtgggaat 1500

gggggccgag ccaattgtga tgcttccctg attgtgactg aggagctgca cctgatcacc 1560

tttgagaccg aggtgtatca ccaaggcctc aagattgacc tagagaccca ctccttgcca 1620

gttgtggtga tctccaacat ctgtcagatg ccaaatgcct gggcgtccat cctgtggtac 1680

aacatgctga ccaacaatcc caagaatgta aactttttta ccaagccccc aattggaacc 1740

tgggatcaag tggccgaggt cctgagctgg cagttctcct ccaccaccaa gcgaggactg 1800

agcatcgagc agctgactac actggcagag aaactcttgg gacctggtgt gaattattca 1860

gggtgtcaga tcacatgggc taaattttgc aaagaaaaca tggctggcaa gggcttctcc 1920

ttctgggtct ggctggacaa tatcattgac cttgtgaaaa agtacatcct ggccctttgg 1980

aacgaagggt acatcatggg ctttatcagt aaggagcggg agcgggccat cttgagcact 2040

aagcctccag gcaccttcct gctaagattc agtgaaagca gcaaagaagg aggcgtcact 2100

ttcacttggg tggagaagga catcagcggt aagacccaga tccagtccgt ggaaccatac 2160

acaaagcagc agctgaacaa catgtcattt gctgaaatca tcatgggcta taagatcatg 2220

gatgctacca atatcctggt gtctccactg gtctatctct atcctgacat tcccaaggag 2280

gaggcattcg gaaagtattg tcggccagag agccaggagc atcctgaagc tgacccaggt 2340

agcgctgccc catacctgaa gaccaagttt atctgtgtga caccaacgac ctgcagcaat 2400

accattgacc tgccgatgtc cccccgcact ttagattcat tgatgcagtt tggaaataat 2460

ggtgaaggtg ctgaaccctc agcaggaggg cagtttgagt ccctcacctt tgacatggag 2520

ttgacctcgg agtgcgctac ctcccccatg tgaggagctg agaacggaag ctgcagaaag 2580

atacgactga ggcgcctacc tgcattctgc cacccctcac acagccaaac cccagatcat 2640

ctgaaactac taactttgtg gttccagatt ttttttaatc tcctacttct gctatctttg 2700

agcaatctgg gcacttttaa aaatagagaa atgagtgaat gtgggtgatc tgcttttatc 2760

taaatgcaaa taaggatgtg ttctctgaga cccatgatca ggggatgtgg cggggggtgg 2820

ctagagggag aaaaaggaaa tgtcttgtgt tgttttgttc ccctgccctc ctttctcagc 2880

agctttttgt tattgttgtt gttgttctta gacaagtgcc tcctggtgcc tgcggcatcc 2940

ttctgcctgt ttctgtaagc aaatgccaca ggccacctat agctacatac tcctggcatt 3000

gcacttttta accttgctga catccaaata gaagatagga ctatctaagc cctaggtttc 3060

tttttaaatt aagaaataat aacaattaaa gggcaaaaaa cactgtatca gcatagcctt 3120

tctgtattta agaaacttaa gcagccgggc atggtggctc acgcctgtaa tcccagcact 3180

ttgggaggcc gaggcggatc ataaggtcag gagatcaaga ccatcctggc taacacggtg 3240

aaaccccgtc tctactaaaa gtacaaaaaa ttagctgggt gtggtggtgg gcgcctgtag 3300

tcccagctac tcgggaggct gaggcaggag aatcgcttga acctgagagg cggaggttgc 3360

agtgagccaa aattgcacca ctgcacactg cactccatcc tgggcgacag tctgagactc 3420

tgtctcaaaa aaaaaaaaaa aaaaaagaaa cttcagttaa cagcctcctt ggtgctttaa 3480

gcattcagct tccttcaggc tggtaattta tataatccct gaaacgggct tcaggtcaaa 3540

cccttaagac atctgaagct gcaacctggc ctttggtgtt gaaataggaa ggtttaagga 3600

gaatctaagc attttagact tttttttata aatagactta ttttcctttg taatgtattg 3660

gccttttagt gagtaaggct gggcagaggg tgcttacaac cttgactccc tttctccctg 3720

gacttgatct gctgtttcag aggctaggtt gtttctgtgg gtgccttatc agggctggga 3780

tacttctgat tctggcttcc ttcctgcccc accctcccga ccccagtccc cctgatcctg 3840

ctagaggcat gtctccttgc gtgtctaaag gtccctcatc ctgtttgttt taggaatcct 3900

ggtctcagga cctcatggaa gaagaggggg agagagttac aggttggaca tgatgcacac 3960

tatggggccc cagcgacgtg tctggttgag ctcagggaat atggttctta gccagtttct 4020

tggtgatatc cagtggcact tgtaatggcg tcttcattca gttcatgcag ggcaaaggct 4080

tactgataaa cttgagtctg ccctcgtatg agggtgtata cctggcctcc ctctgaggct 4140

ggtgactcct ccctgctggg gccccacagg tgaggcagaa cagctagagg gcctccccgc 4200

ctgcccgcct tggctggcta gctcgcctct cctgtgcgta tgggaacacc tagcacgtgc 4260

tggatgggct gcctctgact cagaggcatg gccggatttg gcaactcaaa accaccttgc 4320

ctcagctgat cagagtttct gtggaattct gtttgttaaa tcaaattagc tggtctctga 4380

attaaggggg agacgacctt ctctaagatg aacagggttc gccccagtcc tcctgcctgg 4440

agacagttga tgtgtcatgc agagctctta cttctccagc aacactcttc agtacataat 4500

aagcttaact gataaacaga atatttagaa aggtgagact tgggcttacc attgggttta 4560

aatcataggg acctagggcg agggttcagg gcttctctgg agcagatatt gtcaagttca 4620

tggccttagg tagcatgtat ctggtcttaa ctctgattgt agcaaaagtt ctgagaggag 4680

ctgagccctg ttgtggccca ttaaagaaca gggtcctcag gccctgcccg cttcctgtcc 4740

actgccccct ccccatcccc agcccagccg agggaatccc gtgggttgct tacctaccta 4800

taaggtggtt tataagctgc tgtcctggcc actgcattca aattccaatg tgtacttcat 4860

agtgtaaaaa tttatattat tgtgaggttt tttgtctttt tttttttttt ttttttttgg 4920

tatattgctg tatctacttt aacttccaga aataaacgtt atataggaac cgtaaaaa 4978

<210> 12
<211> 4520
<212> DNA
<213> Mus <mouse, genus>

<220>
<223> Mouse Stat3 (NM_213659.3)

<400> 12
aattatgcat ggaggcgtgt cttggccagt ggcggctggg tggggattgg ctggaggggc 60

tgtaattcag cggtttccgg agctgcagtg tagacaggga gggggaacct ggggttccga 120

cgtcgcggcg gagggaacga gccctaaccg gatcgctgag gtacaacccc gctcggtgtc 180

gcctgaccgc gtcggctagg agaggccagg cggccctcgg gagcccagca gctcgcgcct 240

ggagtcagcg caggccggcc agtcgggcct cagccccgga gacagtcgag acccctgact 300

gcagcaggat ggctcagtgg aaccagctgc agcagctgga cacacgctac ctggagcagc 360

tgcaccagct gtacagcgac agcttcccca tggagctgcg gcagttcctg gcaccttgga 420

ttgagagtca agactgggca tatgcagcca gcaaagagtc acatgccacg ttggtgtttc 480

ataatctctt gggtgaaatt gaccagcaat atagccgatt cctgcaagag tccaatgtcc 540

tctatcagca caaccttcga agaatcaagc agtttctgca gagcaggtat cttgagaagc 600

caatggaaat tgcccggatc gtggcccgat gcctgtggga agagtctcgc ctcctccaga 660

cggcagccac ggcagcccag caagggggcc aggccaacca cccaacagcc gccgtagtga 720

cagagaagca gcagatgttg gagcagcatc ttcaggatgt ccggaagcga gtgcaggatc 780

tagaacagaa aatgaaggtg gtggagaacc tccaggacga ctttgatttc aactacaaaa 840

ccctcaagag ccaaggagac atgcaggatc tgaatggaaa caaccagtct gtgaccagac 900

agaagatgca gcagctggaa cagatgctca cagccctgga ccagatgcgg agaagcattg 960

tgagtgagct ggcggggctc ttgtcagcaa tggagtacgt gcagaagaca ctgactgatg 1020

aagagctggc tgactggaag aggcggcagc agatcgcgtg catcggaggc cctcccaaca 1080

tctgcctgga ccgtctggaa aactggataa cttcattagc agaatctcaa cttcagaccc 1140

gccaacaaat taagaaactg gaggagctgc agcagaaagt gtcctacaag ggcgacccta 1200

tcgtgcagca ccggcccatg ctggaggaga ggatcgtgga gctgttcaga aacttaatga 1260

agagtgcctt cgtggtggag cggcagccct gcatgcccat gcacccggac cggcccttag 1320

tcatcaagac tggtgtccag tttaccacga aagtcaggtt gctggtcaaa tttcctgagt 1380

tgaattatca gcttaaaatt aaagtgtgca ttgataaaga ctctggggat gttgctgccc 1440

tcagagggtc tcggaaattt aacattctgg gcacgaacac aaaagtgatg aacatggagg 1500

agtctaacaa cggcagcctg tctgcagagt tcaagcacct gacccttagg gagcagagat 1560

gtgggaatgg aggccgtgcc aattgtgatg cctccttgat cgtgactgag gagctgcacc 1620

tgatcacctt cgagactgag gtgtaccacc aaggcctcaa gattgaccta gagacccact 1680

ccttgccagt tgtggtgatc tccaacatct gtcagatgcc aaatgcttgg gcatcaatcc 1740

tgtggtataa catgctgacc aataacccca agaacgtgaa cttcttcact aagccgccaa 1800

ttggaacctg ggaccaagtg gccgaggtgc tcagctggca gttctcgtcc accaccaagc 1860

gggggctgag catcgagcag ctgacaacgc tggctgagaa gctcctaggg cctggtgtga 1920

actactcagg gtgtcagatc acatgggcta aattctgcaa agaaaacatg gctggcaagg 1980

gcttctcctt ctgggtctgg ctagacaata tcatcgacct tgtgaaaaag tatatcttgg 2040

ccctttggaa tgaagggtac atcatgggtt tcatcagcaa ggagcgggag cgggccatcc 2100

taagcacaaa gcccccgggc accttcctac tgcgcttcag cgagagcagc aaagaaggag 2160

gggtcacttt cacttgggtg gaaaaggaca tcagtggcaa gacccagatc cagtctgtag 2220

agccatacac caagcagcag ctgaacaaca tgtcatttgc tgaaatcatc atgggctata 2280

agatcatgga tgcgaccaac atcctggtgt ctccacttgt ctacctctac cccgacattc 2340

ccaaggagga ggcatttgga aagtactgta ggcccgagag ccaggagcac cccgaagccg 2400

acccaggtag tgctgccccg tacctgaaga ccaagttcat ctgtgtgaca ccaacgacct 2460

gcagcaatac cattgacctg ccgatgtccc cccgcacttt agattcattg atgcagtttg 2520

gaaataacgg tgaaggtgct gagccctcag caggagggca gtttgagtcg ctcacgtttg 2580

acatggatct gacctcggag tgtgctacct cccccatgtg aggagctgaa accagaagct 2640

gcagagacgt gacttgagac acctgccccg tgctccaccc ctaagcagcc gaaccccata 2700

tcgtctgaaa ctcctaactt tgtggttcca gatttttttt tttaatttcc tacttctgct 2760

atctttgggc aatctgggca ctttttaaaa tagagaaatg agtgagtgtg ggtgataaac 2820

tgttatgtaa agaggagagc acctctgagt ctggggatgg ggctgagagc agaagggagc 2880

aaggggaaca cctcctgtcc tgcccgcctg ccctcctttt tcagcagctc ggggttggtt 2940

gttagacaag tgcctcctgg tgcccatggc atcctgttgc cccactctgt gagctgatac 3000

cccaggctgg gaactcctgg ctctgcactt tcaaccttgc taatatccac atagaagcta 3060

ggactaagcc cagaggttcc tctttaaatt aaaaaaaaaa aaaataagaa ttaaagggca 3120

aaacacactg acacagcata gcctttccat atcaaggaat actcagttaa cagcctctcc 3180

agcgctgtct tcaggctgat catctatata aaccctggaa tggttgcaga tcaaatctgt 3240

aaaagagatc cgagagctgt ggcttggcct ctggttcaaa cacaaaggct agagagaacc 3300

tagatatccc tgggttttgt ttacccagta tgcttgtcgg ttggaggtgt gaggtaggcc 3360

aagggcactg gaaagccttt gtcatcaccc tactccctcc ccaacccaga ctccagaccc 3420

tgtttcaggg tcagcctgcc ctgtgggtgc cttactgggc ctagggtcaa cctgccttcc 3480

tttcccactt gaccttgctg gtagtatgtc cccttcccat gtccaaaggc cctctgtcct 3540

gcttctattg ggaatccctg cctcaggacc ttgtgtcgag agggattgcc ttacaggttt 3600

gaacctgcct cagactacag gccctcagca aagctcaggg agtatggtcc ttattctatg 3660

cgcttggttc ccagggatat ctgtaaccac agggcaaaag ctgacatata ctccaggtct 3720

gccctcatat gagtggtgta ttcttggcct cccctgagac tggcaactgt ctgctcccca 3780

ttgggtctcc caggtgaggt ggaacacagt tcctgcacct actgtggcct ccatgtcgct 3840

tgcttgcttc gctcactcag cttactggaa cactgagtgt tcaaggcaag cctttcctga 3900

cagaggcatg gctagattca gtgactcaaa gccacctcat tcagctgatc agtgtctgtg 3960

gaattgtttc cttccagtta accagtgtct gaattaaggg cagtgaggac attgtctcca 4020

agacgaactg cttgccttga ccaccccagc cttctgcttc gagacagtta ctgctctccc 4080

accccatcaa tgttctttag ttatacaata agctgaactt ataaactgaa aggtatttta 4140

ggaaggcaag gcttgggcat ttttatggct ttcaatcctg gggacccagg aacaaggtga 4200

gggcttctct ggggctggtg ttgtacctca ggggctctgg gaagtctgtg tgcctgggtt 4260

aaccacccat agtgagcccc tggaactgcc cactttccct ctccttggcc ccacttggcc 4320

ccagcctcac ccagcctgca gactgcttag cctttcagtg cagtggcttg tgttctggcc 4380

actgcactca gattccaatg taaactttct agtgtaaaaa tttatattat tgtgggttgt 4440

tttttgttgt tgtttgtttt tgtatattgc tgtaactact ttaacttcca gaaataaaga 4500

ttatatagga actgtctggc 4520

<210> 13
<211> 8332
<212> DNA
<213> Homo sapiens

<220>
<223> Human TCF4 (NM_001083962.1)

<400> 13

gtgtgtggat gtgtgagtga gagggaacga gagtaagaga aagaaagaag tgaggggatg 60
taaactcgaa taaatttcaa agtgcctccg agggatgcaa cgggcaaaaa ctgaactgtt 120
caggcttcag attgtaactg acgatctgag gaaaaatgag gtgctcgatg aattttcgtt 180
tgtatttttt ggcgaggcgg gggaggtgtt gagatttttt ttttttcccc tcggggtggg 240
tgcgaggggg atgcatccta gcctgcccga cccggagcaa gtcgcgtctc cccgccggag 300
cccccccacc catttctttg ctgaacttgc aattccgtgc gcctcggcgt gtttccccct 360
ccccccttcc ctccgtcccc tcccctcccc ggagaagaga gttggtgtta agagtcaggg 420
atcttggctg tgtgtctgcg gatctgtagt ggcggcggcg gcggcggcgg cggggaggca 480
gcaggcgcgg gagcgggcgc aggagcaggc ggcggcggtg gcggcggcgg ttagacatga 540
acgccgcctc ggcgccggcg gtgcacggag agccccttct cgcgcgcggg cggtttgtgt 600
gattttgcta aaatgcatca ccaacagcga atggctgcct tagggacgga caaagagctg 660
agtgatttac tggatttcag tgcgatgttt tcacctcctg tgagcagtgg gaaaaatgga 720
ccaacttctt tggcaagtgg acattttact ggctcaaatg tagaagacag aagtagctca 780
gggtcctggg ggaatggagg acatccaagc ccgtccagga actatggaga tgggactccc 840
tatgaccaca tgaccagcag ggaccttggg tcacatgaca atctctctcc accttttgtc 900
aattccagaa tacaaagtaa aacagaaagg ggctcatact catcttatgg gagagaatca 960
aacttacagg gttgccacca gcagagtctc cttggaggtg acatggatat gggcaaccca 1020
ggaacccttt cgcccaccaa acctggttcc cagtactatc agtattctag caataatccc 1080
cgaaggaggc ctcttcacag tagtgccatg gaggtacaga caaagaaagt tcgaaaagtt 1140
cctccaggtt tgccatcttc agtctatgct ccatcagcaa gcactgccga ctacaatagg 1200
gactcgccag gctatccttc ctccaaacca gcaaccagca ctttccctag ctccttcttc 1260
atgcaagatg gccatcacag cagtgaccct tggagctcct ccagtgggat gaatcagcct 1320
ggctatgcag gaatgttggg caactcttct catattccac agtccagcag ctactgtagc 1380
ctgcatccac atgaacgttt gagctatcca tcacactcct cagcagacat caattccagt 1440
cttcctccga tgtccacttt ccatcgtagt ggtacaaacc attacagcac ctcttcctgt 1500
acgcctcctg ccaacgggac agacagtata atggcaaata gaggaagcgg ggcagccggc 1560
agctcccaga ctggagatgc tctggggaaa gcacttgctt cgatctattc tccagatcac 1620
actaacaaca gcttttcatc aaacccttca actcctgttg gctctcctcc atctctctca 1680
gcaggcacag ctgtttggtc tagaaatgga ggacaggcct catcgtctcc taattatgaa 1740
ggacccttac actctttgca aagccgaatt gaagatcgtt tagaaagact ggatgatgct 1800
attcatgttc tccggaacca tgcagtgggc ccatccacag ctatgcctgg tggtcatggg 1860
gacatgcatg gaatcattgg accttctcat aatggagcca tgggtggtct gggctcaggg 1920

tatggaaccg gccttctttc agccaacaga cattcactca tggtggggac ccatcgtgaa 1980

gatggcgtgg ccctgagagg cagccattct cttctgccaa accaggttcc ggttccacag 2040

cttcctgtcc agtctgcgac ttcccctgac ctgaacccac cccaggaccc ttacagaggc 2100

atgccaccag gactacaggg gcagagtgtc tcctctggca gctctgagat caaatccgat 2160

gacgagggtg atgagaacct gcaagacacg aaatcttcgg aggacaagaa attagatgac 2220

gacaagaagg atatcaaatc aattactagg tcaagatcta gcaataatga cgatgaggac 2280

ctgacaccag agcagaaggc agagcgtgag aaggagcgga ggatggccaa caatgcccga 2340

gagcgtctgc gggtccgtga catcaacgag gctttcaaag agctcggccg catggtgcag 2400

ctccacctca agagtgacaa gccccagacc aagctcctga tcctccacca ggcggtggcc 2460

gtcatcctca gtctggagca gcaagtccga gaaaggaatc tgaatccgaa agctgcgtgt 2520

ctgaaaagaa gggaggaaga gaaggtgtcc tcagagcctc cccctctctc cttggccggc 2580

ccacaccctg gaatgggaga cgcatcgaat cacatgggac agatgtaaaa gggtccaagt 2640

tgccacattg cttcattaaa acaagagacc acttccttaa cagctgtatt atcttaaacc 2700

cacataaaca cttctcctta acccccattt ttgtaatata agacaagtct gagtagttat 2760

gaatcgcaga cgcaagaggt ttcagcattc ccaattatca aaaaacagaa aaacaaaaaa 2820

aagaaagaaa aaagtgcaac ttgagggacg actttcttta acatatcatt cagaatgtgc 2880

aaagcagtat gtacaggctg agacacagcc cagagactga acggcaatct ttccacactg 2940

tggaacaatg catttgtgcc taaacttctt ttggaaaaaa aaaatataat taatttgtaa 3000

gtctgaaaaa aaaatattta atttaaaaaa aattgtaaac ttgcaataat gaaaaagtgt 3060

acttctgaag aaaactacat gaacgttttt gttggtattc aagtcagcta gtgtttataa 3120

ttactggata ttgaattagg ggaagctcgg ctgccctagt aacaaaacca gcaaacgtcc 3180

tgatgacaac gaagtgatga cattagccat tccttagggt aggaggaaca gatggatctt 3240

atagacctat gacaaatata tatataaata tatatataaa tatatattaa aaatttagtg 3300

actatggtaa gcttttgttc atttgtttca gacttttttc tcctgtaaaa aaatagtact 3360

gattaacttt tttaaaagaa agattttact gtaaatatgg attttttttt ttttggtctt 3420

atttctgtcc ctttccctgg tttgttatcg taacctgtag tgccaactct gcttccagag 3480

gggtagtgca ggatgaaatg ctgaccctga tgttgcttct cattcataaa taagtagaaa 3540

gttgtttctc cagtcttttg ggaacacagg acttaaaagt cacatcatgt gtagatatta 3600

caagcagcat taccaagaca tggcaaaaag agtttgtctg aattgtaatg ttgcgtttgt 3660

gaacctattc tgggattttc agaggtacaa ggttagaatg ctacaatgtt accactgtgc 3720

cttccaatgt ttatatcatc ggaaacataa cataatcaaa gtggctgtga tttaacaaaa 3780

tgattaaagt gttacctacc tgtgtagccg aagtagtgtg cagtgaggcg tttctgaata 3840

catggtcaga tttttggaaa aaaacaaaaa caaaaaaaac aagtaaagtt caaaaaccgt 3900

caaatgagaa aattgcaagt agtgtgacag agctgattga ttttgttgct ttcttgattt 3960

tttttttcaa aatgggttta ctaaaatgta gatgacttaa ctgcctcctc cttcgtctga 4020

aaaatgccaa tattcaatca tcatgcagca ttataacaag ccttataagt cctaaagcat 4080

taagttgcac ttttttgagg aggggtagtg cagtatttct ctggccagta tgaatgaagt 4140

ttatacttac catatttgat agaaacatag atcaagctat ggcacagcga ctcatcagat 4200

agctagcttt gacgtctggg cacaattgaa ccaacttcca tcgtgaatct ttataatgat 4260

tgactttggt gtatagtgca gtaaacaaat agtgctccta gttaagtatt tgtcagcatc 4320

cttttgtctc taacttgttt ctatttttac agccacacaa ttcttggcat gtattaagaa 4380

aaaaaaaaat ccctgttcaa gtagtttttc cacctatcag cactgagtaa atgccataaa 4440

tccattgaaa tggtctaaat gttccatctg ttctcctgtt ttgccagtta tatagtaatg 4500

aaatacattt gtaaatttta tgcaacaaat ggcaaacgta tcattatttt gaaattgtgt 4560

atgtaaaagt tatattttta catgtagact cttgttatta tgtgttttaa tacattgtat 4620

cagtttttgt ttttttttaa aaactgtggt ttaaaaagaa gtctcattta aatgaaatag 4680

ctacaagaat cagaatttta tgttcatttc tgaaaatgta agaacaaata agatagttac 4740

cacgtggtca tcttttacaa acccataaac attttgatta gctgtgtgtg tgttgaaaaa 4800

ctgtaaatat gttcagtagc gataaaacta aaataacttt gatttgttga taagttccta 4860

aaatgtggag gtggattaaa accttaggag aatagcagaa atcaaacttc atgaaaagtt 4920

attttggggc tttcctgtga aatgtatgaa caaagaggct cagagaagga catggaagac 4980

aataatgtat actctctcct cctccctgaa taatgaaaac catgtgtatt tgttccctcc 5040

gtatgttaaa gatttccttt tagtggtaca ttctgcactc attttgtata gtctaccaag 5100

gcgggtatcc ctaggaacaa tattatatag gaagcaggta tactctgatc acattcagga 5160

taagtgtaca gaagaaaata cggtgtttac tctttaggga actggaaaca ctccctgcat 5220

tgatgtacat tttaagaatg gcacttttga tacatgttat cataaaggtg cttaatagag 5280

ctgaattaaa gtttttcaaa tctgtaaaca aagcaaaaaa gtaaattgta gtcatttgat 5340

tattttttaa attggtgctt tatattttgt tctcactcag agtaaaagct gcaatttatt 5400

gttcaccagc tttgatgtat tcattactca gtaatgcaat acctctattg ttgaattccc 5460

tttggaaata agtgaaaatt ctaacggcca ctgaaagctg ctcgctaggt tttgcttggt 5520

ggagaaacat aatctgcacc tatccatatt aattgggttg tatccccatt aaaaaagaaa 5580

aaaagggaat gtggcctttt tagtgtgttt tttattgttg ttgttttgta attatcaaac 5640

ccaggtaaga tattggtatc ctgcactgga ttttcaaatg aagttcagca gaagacagtt 5700

aagattaaag tactatacaa aaatttcaaa agggtccata ctacgctatc tgtatgacga 5760

cacttaggct ggggatctct ttcagaaact cggactttaa aagcaacttg gagcagttga 5820

tccacctcca cattcaagta atttatgaat atgcagaata gggatctgtt catctagaaa 5880

tttttaccat ttgtcttctg tgtagctgca aggaacacta atgtttatac aactgtcagt 5940

ccacccagtg gtgcaactgg ttctgattca gtcttccgat tcctttttat ttttcacttt 6000

ttcctatttc tgaatttttt tttttatttg tgatcttgat tttgatgagg ggttggggag 6060

tggggaggga gtcgaaccaa gacttggagt taagaggatt ttcatctttt gcatccaaca 6120

ggcagaatat gatctgtgtc caaaagtgaa cttgagtcag gaatgaatca atttcagcat 6180

aaacaagcac aaaaatttag tctgctggct gactggaagc aaaaaagtca agatggaata 6240

tgatgaattc caacacaatg gggcaccaag gcctttaggc ctctcttttt attttgcttt 6300

ggttttgttt gtttttcttt agagacatgc tctttctcat gggacttgaa gtggactcat 6360

ctttgtgcag tgctggtttt gccatactca tttcaagtat tatagacata tgtaatggtg 6420

aaaatatatg aactgtggcc tttttcattc ttgttacttg tgatgcaatt aagtgaagat 6480

aagaaaaaaa aaaaaaaagc agagatttac catgtatcag tgcctggctt tttgttataa 6540

agctttgttt gtctagtgct cttttgctat aaaatagact gtagtacacc ctagtaggaa 6600

aaaaaaaaaa ctaaatttaa aaataaaaaa tatatttggc ttatttttcg caggagcaat 6660

ccttttatac catgaatatt acaaaaaaat tgtcagattc tgaatatttc ttctttgtag 6720

atttttggaa tcattatgag taaaagtttg ttactttatt ttactattta aaagatgtta 6780

ttttaccatg tgttaccaag atgaaactgt atgggtagct tttttgtttg ttttttgttt 6840

tgtttttgtt tttgtttttg tttttagttg taggtcgcag cggggaaatt ttttgcgact 6900

gtacacatag ctgcagcatt aaaaacttaa aaaaattgtt aaaaaaaaaa aaagggaaaa 6960

catttcaaaa aaaaaaaaaa agataaacag ttacaccttg ttttcaatgt gtggctgagt 7020

gcctcgattt tttcatgttt ttggtgtatt tctgatttgt agaagtgtcc aaacaggttg 7080

tgtgctggag ttccttcaag acaaaaacaa acccagcttg gtcaaggcca ttacctgttt 7140

cccatctgta gttattcgat gaagtcatgt acatgaccgt tctgtagcaa taaatgtgcc 7200

atttttataa actgtttctg acacttgttt catttcattt tgcattgtcc atatagctat 7260

gattctcttc tgtaagtaaa acgcatctat atttcatttt ccaagtgttg gaggtattga 7320

cagcttaaca aacaaaacat acaaaaaaaa tcacaaaaac aaattgaaaa gcaaagcaca 7380

tgattgatca aggaagagat gcccttaatg aaaatggaac gggatgcatg caaaacaaaa 7440

agaaaactgt ctagaggatt aactaattga aggaatataa ttaatgtgtg tgtaacactg 7500

aagctatgca tttgaagagc tctgaactgc accagtgttt tcggttgtgc tgcaggttgc 7560

taagtcaagt cagccttaac cttttgcacc agttggtcgg ctgtttggca gaacattctc 7620

agatcttttc agtcaaaaat ctaagatgat ttattttgta tcactttgtt aaaagctgaa 7680

tattgttaac tacagttaat attaacactg tatttatact ttctcaaact acatccgccc 7740

caccacttct ggttgcctct gttgactatt aatccagatg taaacaacca gatgtttttt 7800

tctaacttgt acaaactgac gtgtgtcaac tatcatggaa ggaaaaaaat gtacagatta 7860

aaattattca gtgttatgta ctgtaagtta atatttttgt agaatggaca tcaatctact 7920

ttgcaaaatt tggaggctat ttcaacattg cactgtagaa atgtaaagta atgtatgcaa 7980

tgtaaaggaa agcccgcggt agctgagcgc ttcataacag aatgttctaa tcaagtacgt 8040

ggtatttggg gatgtctcca atattgctct tgtattcttt ctaattgggt ttagtgacta 8100

gttgaaggaa aatgttataa cgccatttgg ttcacatgtg aagtgccctc catagccaaa 8160

tgttgggatt tttttttttt tcgtttttgg ttggactgtt tgcagatatt taaattttat 8220

gaaatttcca aagattttgg ttgataaccc ccttttacct tctaaatgat ttgagatgtt 8280

cttatgttct tactgtgtgt tttaaatata tataaaagag ccacaagcat tt 8332

<210> 14
<211> 7462
<212> DNA
<213> Mus <mouse, genus>

<220>
<223> Mouse Tcf4 (NM_013685.2)

<400> 14
ggagcagccg cggccgcagc gccttctctt tataagccgc agtgcccgga tgtgaatgga 60

ttacaatgta tctttcaggg aaacctatta ttatcaatgt gactcctcgg gggagtcaat 120

gatggtgttg gggaggagga tgatgatgag acgcctctaa acttggaaca agtttaggac 180

tttgaaagag aagagaaaaa aaaatacaac caacaagacc gaagaacaat tataactatc 240

cagtgttgat tatttttata aacaatacga aaaagttgtc ggattttttt ttttaatgat 300

tactttttgg ggggagggaa ttttgttaca gtttgatgat ggaaaatgca aaaaccgagc 360

caggtgcata atcttgtaat ctgtggctaa ccctggaaca ggactgactt ctatttaaaa 420

tactcttttg ggggaacact catgtgagac actaagttct tgcagaagat ttttgtctct 480

ctttttaaag tctctttcct tggaatattg tgagcatatt tgtggccatt gaaggtttgt 540

gtgattttgc taaaatgcat caccaacagc gaatggctgc cttagggacg gacaaagagc 600

tgagtgattt actggatttc agtgcgatgt tttcgcctcc tgtaagcagt gggaaaaatg 660

gaccaacttc tttggcgagt ggacatttca ctggctcaaa tgtagaagac agaagtagct 720

cagggtcctg gggaactgga ggccatccaa gcccgtccag gaactatgga gatgggactc 780

cctatgacca catgactagc agggatcttg ggtcacatga caatctctct ccaccttttg 840

tcaattccag aatacaaagt aaaacagaaa ggggctcata ctcatcttat gggagagaaa 900

acgttcaggg ttgccaccag cagagtctcc tcggagggga catggatatg ggcaatccag 960

gaaccctttc gcccaccaaa cctggctccc agtactatca gtattcaagc aataatgccc 1020

gccggaggcc tcttcacagt agtgccatgg aggtacagac aaagaaagtc cgaaaagttc 1080

ctccgggttt gccgtcttca gtctacgctc cttcagccag cactgccgac tacaacaggg 1140

actcgccagg ctatccttcc tccaagccag cagccagcac tttccctagc tccttcttca 1200

tgcaagatgg ccatcacagc agcgaccctt ggagctcctc cagcgggatg aatcagcccg 1260

gctacggagg gatgctgggc aattcttctc atatcccaca gtccagcagc tactgtagcc 1320

tgcatccaca tgaacgtttg agctatccat cccactcctc ggcagacatc aactccagtc 1380

ttcctccgat gtccacgttc catcgtagtg gcacaaacca ttacagcacc tcttcctgca 1440

caccccctgc caacggaaca gacagtataa tggcaaacag aggaactggg gcagcaggca 1500

gctcgcagac tggagacgct ctagggaaag ccctagcttc gatctattct cctgaccaca 1560

cgaacaacag cttttcctcc aatccttcaa ctcctgtggg ctcccctcct tcactctcag 1620

caggcacagc tgtttggtct agaaatggag gacaggcctc gtcatctccc aattatgaag 1680

gacccttgca ctcactgcaa agccgaatcg aagaccgttt ggaaagactg gacgatgcga 1740

ttcatgttct ccggaaccac gcagtgggcc cgtccacagc tgtgcctggt ggccatgggg 1800

acatgcatgg gatcatggga ccctcccaca acggagcgat gggtagcctg ggctcagggt 1860

acggaactag tcttctctca gccaacagac actcgctcat ggttggggcc caccgtgaag 1920

atggcgtggc tctgagaggc agccattctc tcctgccaaa ccaggttccg gtcccacaac 1980

ttccggtcca gtctgcaact tcccctgact tgaacccacc ccaagaccct tacagaggga 2040

tgccaccagg cctccagggc cagagcgtgt cttctggtag ctctgagatc aaatccgacg 2100

acgagggcga tgagaacctg caagacacaa aatcttctga ggacaagaaa ttagatgacg 2160

acaagaagga tatcaaatca attactaggt caagatctag caataacgat gatgaggacc 2220

tgaccccaga gcagaaggct gagcgcgaga aggaacggag gatggccaat aatgcccgtg 2280

agcgcctgag ggtccgagat atcaacgagg ctttcaagga gcttggccgt atggtgcagc 2340

tccacctgaa gagcgacaag ccccagacca agctcctgat tctccaccag gccgtggctg 2400

tcatcctcag cctggagcag caagttcgag aaaggaatct gaacccgaaa gctgcctgtc 2460

tgaaaagaag ggaggaagag aaggtgtcct cagagcctcc cccactctcc ttggctggcc 2520

cacaccctgg gatgggagac gcagcgaatc acatggacag atgtgaaaa ggtccaagtt 2580

gctaccttgc ttcattaaac aagagaccac ttccttaaca gctgtattac cctaaaccca 2640

cataaacact gctccttaac cccgtttttt tttgtaatat aagacaagtc tgagtagtta 2700

tgaatcgcag acgcaagagg tttcagcatt cccaattatc aaaaaacaga aaaacaaaca 2760

aaaaaatgaa tgaaagaaag aaagaaagaa aaaaatgcaa cttgagggac gacttcttta 2820

acatatcact ctgaatgtgc gaagcggtat gtacaggctg agacacagcc cagagactga 2880

atggcaatcc tcccacactg tggagcaatg catttgtgcc taaacttctt ttggaaaaaa 2940

aaaatataat taatttgtaa gtctgaaaaa aatatttaat ttaaaaaaaa attgtaaact 3000

tgcaataatg aaaaagtgta cttctgaaga aaacgacatg aacgtttttg ttggtattca 3060

cgtcagctag tgtttctaat taccggatat tgaatagggg aagcccggct gccctcgtaa 3120

caaaaccagc aaacgtcctg atggcaacga agtgatgaca ttagccattc cttagggtag 3180

gagggacaga tggatgttat agacctatga caaatatata tataaatata tatataaata 3240

tatattaaaa atttagtgac tatggtaagc ttttgttgat ttgtttcaga cttttttctc 3300

ctgtaaaaaa atagtactga ttaacttttt taaaagaaag attttactgt aaatatggat 3360

tttttttttt gtctgatttt tgtcccttcc cccggtttgt tatcgtaacc tgtagtgcca 3420

actctgcttc cggaggggca gtgcaggacg aaatgctgac cctgaagttg cttctcattc 3480

acaaatagta aaaagttgtt tctccagtct tttgggaaca caggacttaa aagtcacatc 3540

atgtgtagga attacatgca gcattgcccg ggcgaggcaa aaagcgtttg tctggcttgt 3600

ggcgctgccc ttgttaccct cccctgggat tttcagaggt acacggttag aatgctacaa 3660

tgttaccact gtgccttcca atgtttatat catcggaaac ataacataat caaagtggct 3720

gtgatttaac aaaaaaaacg attcaagtgt tacctacctg tgtagccgaa gtagtgtgca 3780

gtgaccgaga cgtttctgaa tacatggtca gatttttttt ggaaaaaata caaaaattaa 3840

aaaaaaaaaa aaaaaagaaa aaaagaaaaa aaaactaaaa aagaaagtca agttcaaaaa 3900

ccgtcaaatg agaaaattgc aaggagtgtg acagagctga ttgatttttg ttgctttctt 3960

gatttttttt tcaaaatggg tttactaaaa agtagatgac ttacctgcct cctccttcgt 4020

ctgaaaaaaa aaaaaaatgc caaccaatca tgcagcatta taacaagcct tataagccct 4080

aaagcattaa gttgcacttt tgtgaggagg ggtaacgcag tattctctct ggccagtatg 4140

agtgaagttt atacttaaca tttgatagaa acatagatca aactacggca cagcaactca 4200

tcagatagct agcgttgact ctgggcacaa ttgaaccaat tcccatcgta agtctttcca 4260

acaatggact ttggtgtgta gtgcagtgaa cacataggac tcctacgtaa gcatttgtca 4320

gcatcctttt gtctctaact cggttctgtt ttgacagcca cacaatcttg gcatgtatta 4380

aggaaaaaaa aaatccctgt tgaagtagtt tttccaccta tcagcactga gtaaatgcca 4440

taactccgcg gaaatggtct aaatacccca tttgttgtcc tgaactacca gttacatagt 4500

gacgaaacac atctgtaaat tttatgcaac caatggcaaa cctatcatta ttttgaaact 4560

gtgtatgtaa aagttatatt tttacatgta gactcttgtt attatgtgtt ttaatacatt 4620

gtatcagttt tttgtttttt tttaaactgt gtggttttaa aaagtcattt aaatgaaata 4680

gtgagctaca agaatctgaa atttatgttc atttctgaaa atgtaagaac aaataagata 4740

gttaccacgt ggtcaccttt tacaaaccca tgaacatttt gattagctgt gtgcatgtgt 4800

gtgcgtgtgt gctcttgcgc gtgtgtgtgt gtgtgtgtgt gtgtgtatgt gtgtgtgttg 4860

aaaattgtaa atatgttcag tagcgataaa actaaaatgc tttgatttgt tgagaagttc 4920

ctaaaatgtg gaggtggatt gaaaatgtag gggaatagca ggaatcaaat ctcataaaaa 4980

gttcttcggg gactttgctg tgatgcggtg gcacaaaggt gctccaagaa gggcaaagaa 5040

gaaagacagt aacttcccct cctccctgaa cgatggaaaa catgtgtacg tggtctctca 5100

ccatgttaaa gatttctttt ccgtgataca ttctgcactc attttgtata gtctgccaag 5160

gcgggtatcc ctaggaacaa tattattata taggaaacag gtatcgtccg atcacattca 5220

ggagaagcgt atagaaaaga atatggtgtt tactctttag ggaactggaa atcctccccg 5280

cattgatgga tatcttcaga gtggcacttt tgatacatgt tatcataaag gtgcttacta 5340

aagcagaatc aaagtttttc aactctgtaa acaaagcaaa aaattaaatt tttaaattaa 5400

atcatttgag attttttttt tcaattggtg cttttatatt ttgttctcac tcggacagag 5460

aaaaagctgc aatttcatgt tctcaccagc tttgatgtat tcattacttg gtaatgtaat 5520

atggctattg tcaaattcct tttggaaata agcaaaagac tccccaaagg ccagcagcag 5580

ctgctggcta ctttctgctt ggtgcggaaa cctgatctga ccctctttgt agtaatcagg 5640

tgtatctcca gttttaaaaa gaaggagaaa ggaaatgtgg ccgttttaac gtgttggttt 5700

tgttttgttt tgttttgttt tgttttcctt gttgttattt tgtaattatc aaacccaggt 5760

aagatattgg tattcctgca ctggattttt gaaagaaact tagcagaagt caggattaaa 5820

atactacaca aacatttcat aagtgttcat cctatactag acatacacga aggcggtgag 5880

gcttaggtgc aggatcggtt taagagactt tgaaagcaac ttggaacagt tgatccacct 5940

ccacattaaa gtaaattatg aatatgcaga attagggatc tgtccatctt ggagttgtta 6000

ctctttttgt cttctgtatg gcagtgatga acactaatgc tttgacagct ttcagcccac 6060

ccagtggtcc aactgattcc aattcagtct tccgattcct ggttttgttt tgtctttcca 6120

cttttttttt cctatcttga ttttattgtt attgttattg ttattattat tattattatt 6180

attattaata ttcatgatgt ttgttttgat gagggattgg gaagtgggag ggagtcgaac 6240

tgagactagg ggctgagagg attttttttt ttccatcttt gcatccaaca ggcagaatat 6300

ggtctgtgtc caaaacggaa cttaagtcag gaaggaaacc attcagcata aacaagcaca 6360

acatgtagtc tgccggctga ctggaagcat aaataaataa ataaataaca catacggagg 6420

tgaagatgga atgtgctgga ttccaagaca atggggcacc aaggcctgag ggcctcctct 6480

tgactttgct ttggttatgt ttgttttctt tagagatgtc ttttctcatg ggacttgaag 6540

tgactcatct ctgtgcagta ctggtttcgc catatgctca tttcaagtat tatagacata 6600

tgtaatggtg aaatatatga actgtggcct ttttcattct tgttacttgt gatgcaatta 6660

agtgaagata agaaaaaaaa aaagcagaga tttaccatgt atcagtgcct ggctttttgt 6720

tataaagctt cgtctgtcta gtgctctttt tgctataaaa aatagactgt agtacaccct 6780

agtaggaaaa aaaaactaaa tttaaaaata aaaaaatata tttggcttat ttttcgcagg 6840

agtaatcctt ttataccatg gatattacaa aaaaaaatgt cagattctga gtatttcttc 6900

tttgtagatt tttggaatca ttatgagtaa aagtttgtta ctttattttg ctatttaaaa 6960

gatgttattt taccatgtgt tactgaaagg aaactgtatg gtagagcttc tgtttgtttt 7020

tgttttcagg ttttttttgt ttgtttgttt ttagttgtag gtcgcagtat gaactttttt 7080

tttgtttctc gttatgtttt gttttgtttt tgcgactgta cacatagctg cagcattaaa 7140

aacttttaaa aaattgttta aaaaaaaaga aaaaaaggga aaacgtttta aaaaaaaaga 7200

taaacagtta caccttgttt tcaatgtgtg gctgagtgcc tcaatttttt catgtttttg 7260

gtgtatttct gatttgtaga agtgtccaaa caggttgtgt gccggacttc cttcaagagg 7320

cccacagccc agcttggtct agacctgttc ccatctgtag ttactcgatg aagtcatgta 7380

catgaccgtt ctgtagcaat aaatgtgcca tttttataaa ctgtctctga cacttttttc 7440

atttcacctc tcgtgcacat ag 7462

<210> 15
<211> 6317
<212> DNA
<213> Homo sapiens

<220>
<223> Human IKZF1 (NM_006060.5)

<400> 15
attgtgaaag aaagctggga agagctccgc ggccaagtta gcaggacact ctaacaagtg 60

actgcgcggc ccgcgcccgg ggcggtgact gcggcaagcc ccctgggtcc ccgcgcggcg 120

catcccagcc tgggcgggac gctcggccgc ggcgaggcgg gcaagcctgg cagggcagag 180

ggagccccgg ctccgaggtt gctcttcgca cccgaggatc agtcttggcc ccaaagcgcg 240

acgcacaaat ccacataacc tgaggaccat ggatgctgat gagggtcaag acatgtccca 300

agtttcaggg aaggaaagcc cccctgtaag cgatactcca gatgagggcg atgagcccat 360

gccgatcccc gaggacctct ccaccacctc gggaggacag caaagctcca agagtgacag 420

agtcgtggcc agtaatgtta aagtagagac tcagagtgat gaagagaatg ggcgtgcctg 480

tgaaatgaat ggggaagaat gtgcggagga tttacgaatg cttgatgcct cgggagagaa 540

aatgaatggc tcccacaggg accaaggcag ctcggctttg tcgggagttg gaggcattcg 600

acttcctaac ggaaaactaa agtgtgatat ctgtgggatc atttgcatcg ggcccaatgt 660

gctcatggtt cacaaaagaa gccacactgg agaacggccc ttccagtgca atcagtgcgg 720

ggcctcattc acccagaagg gcaacctgct ccggcacatc aagctgcatt ccggggagaa 780

gcccttcaaa tgccacctct gcaactacgc ctgccgccgg agggacgccc tcactggcca 840

cctgaggacg cactccgttg gtaaacctca caaatgtgga tattgtggcc gaagctataa 900

acagcgaagc tctttagagg aacataaaga gcgctgccac aactacttgg aaagcatggg 960

ccttccgggc acactgtacc cagtcattaa agaagaaact aatcacagtg aaatggcaga 1020

agacctgtgc aagataggat cagagagatc tctcgtgctg gacagactag caagtaacgt 1080

cgccaaacgt aagagctcta tgcctcagaa atttcttggg gacaagggcc tgtccgacac 1140

gccctacgac agcagcgcca gctacgagaa ggagaacgaa atgatgaagt cccacgtgat 1200

ggaccaagcc atcaacaacg ccatcaacta cctgggggcc gagtccctgc gcccgctggt 1260

gcagacgccc ccgggcggtt ccgaggtggt cccggtcatc agcccgatgt accagctgca 1320

caagccgctc gcggagggca ccccgcgctc caaccactcg gcccaggaca gcgccgtgga 1380

gaacctgctg ctgctctcca aggccaagtt ggtgccctcg gagcgcgagg cgtccccgag 1440

caacagctgc caagactcca cggacaccga gagcaacaac gaggagcagc gcagcggtct 1500

catctacctg accaaccaca tcgccccgca cgcgcgcaac gggctgtcgc tcaaggagga 1560

gcaccgcgcc tacgacctgc tgcgcgccgc ctccgagaac tcgcaggacg cgctccgcgt 1620

ggtcagcacc agcggggagc agatgaaggt gtacaagtgc gaacactgcc gggtgctctt 1680

cctggatcac gtcatgtaca ccatccacat gggctgccac ggcttccgtg atccttttga 1740

gtgcaacatg tgcggctacc acagccagga ccggtacgag ttctcgtcgc acataacgcg 1800

aggggagcac cgcttccaca tgagctaaag ccctcccgcg cccccacccc agaccccgag 1860

ccaccccagg aaaagcacaa ggactgccgc cttctcgctc ccgccagcag catagactgg 1920

actggaccag acaatgttgt gtttggattt gtaactgttt tttgtttttt gtttgagttg 1980

gttgattggg gtttgatttg cttttgaaaa gatttttatt tttagaggca gggctgcatt 2040

gggagcatcc agaactgcta ccttcctaga tgtttcccca gaccgctggc tgagattccc 2100

tcacctgtcg cttcctagaa tccccttctc caaacgatta gtctaaattt tcagagagaa 2160

atagataaaa cacgccacag cctgggaagg agcgtgctct accctgtgct aagcacgggg 2220

ttcgcgcacc aggtgtcttt ttccagtccc cagaagcaga gagcacagcc cctgctgtgt 2280

gggtctgcag gtgagcagac aggacaggtg tgccgccacc caagtgccaa gacacagcag 2340

ggccaacaac ctgtgcccag gccagcttcg agctacatgc atctagggcg gagaggctgc 2400

acttgtgaga gaaaatacta tttcaagtca tattctgcgt aggaaaatga attggttggg 2460
gaaagtcgtg tctgtcagac tgccctgggt ggagggagac gccgggctag agcctttggg 2520
atcgtcctgg attcactggc tttgcggagg ctgctcagat ggcctgagcc tcccgaggct 2580
tgctgccccg taggaggaga ctgtcttccc gtgggcatat ctggggagcc ctgttccccg 2640
ctttttcact cccatacctt taatggcccc caaaatctgt cactacaatt taaacaccag 2700
tcccgaaatt tggatcttct ttctttttga atctctcaaa cggcaacatt cctcagaaac 2760
caaagcttta tttcaaatct cttccttccc tggctggttc catctagtac cagaggcctc 2820
ttttcctgaa gaaatccaat cctagccctc attttaatta tgtacatctg tttgtagcca 2880
caagcctgaa tttctcagtg ttggtaagtt tctttaccta ccctcactat atattattct 2940
cgttttaaaa cccataaagg agtgatttag aacagtcatt aattttcaac tcaatgaaat 3000
atgtgaagcc cagcatctct gttgctaaca cacagagctc acctgtttga aaccaagctt 3060
tcaaacatgt tgaagctctt tactgtaaag gcaagccagc atgtgtgtcc acacatacat 3120
aggatggctg gctctgcacc tgtaggatat tggaatgcac agggcaattg agggactgag 3180
ccagaccttc ggagagtaat gccaccagat cccctaggaa agaggaggca aatggcactg 3240
caggtgagaa ccccgcccat ccgtgctatg acatggaggc actgaagccc gaggaaggtg 3300
tgtggagatt ctaatcccaa caagcaaggg tctccttcaa gattaatgct atcaatcatt 3360
aaggtcatta ctctcaacca cctaggcaat gaagaatata ccatttcaaa tatttacagt 3420
acttgtcttc accaacactg tcccaaggtg aaatgaagca acagagagga aattgtacat 3480
aagtacctca gcatttaatc caaacagggg ttcttagtct cagcactatg acattttggg 3540
ctgactactt atttgttagg cgggagctct cctgtgcatt gtaggataat tagcagtatc 3600
cctggtggct acccaataga cgccagtagc accccgaatt gacaacccaa actctccaga 3660
catcaccaac tgtcccctgc gaggagaaat cactcctggg ggagaaccac tgacccaaat 3720
gaattctaaa ccaatcaaat gtctgggaag ccctccaaga aaaaaaatag aaaagcactt 3780
gaagaatatt cccaatattc ccggtcagca gtatcaaggc tgacttgtgt tcatgtggag 3840
tcattataaa ttctataaat caattattcc ccttcggtct taaaaatata tttcctcata 3900
aacatttgag ttttgttgaa aagatggagt ttacaaagat accattcttg agtcatggat 3960
ttctctgctc acagaagggt gtggcatttg gaaacggaaa taaacaaaat tgctgcacca 4020
atgcactgag tgaaggaaga gagacagagg atcaagggct ttagacagca ctccttcaat 4080
atgcaatcac agagaaagat gcgccttatc caagttaata tctctaaggt gagagccttc 4140
ttagagtcag tttgttgcaa atttcaccta ctctgttctt ttccatccat ccccctgagt 4200
cagttggttg aagggagtta ttttttcaag tggaattcaa acaaagctca aaccagaact 4260
gtaaatagtg attgcaggaa ttcttttcta aactgctttg ccctttcctc tcactgcctt 4320

ttatagccaa tataaatgtc tctttgcaca ccttttgttg tggttttata ttgtaacacc 4380

attttctttt gaaactattg tatttaaagt aaggtttcat attatgtcag caagtaatta 4440

acttatgttt aaaaggtggc catatcatgt accaaaagtt gctgaagttt ctcttctagc 4500

tggtaaagta ggagtttgca tgacttcaca ctttttttgc gtagtttctt ctgttgtatg 4560

atggcgtgag tgtgtgtctt gggtaccgct gtgtactact gtgtgcctag attccatgca 4620

ctctcgttgt gtttgaagta aatattggag accggagggt aacaggttgg cctgttgatt 4680

acagctagta atcgctgtgt cttgttccgc cccctccctg acaccccagc ttcccaggat 4740

gtggaaagcc tggatctcag ctccttgccc catatccctt ctgtaatttg tacctaaaga 4800

gtgtgattat cctaattcaa gagtcactaa aactcatcac attatcattg catatcagca 4860

aagggtaaag tcctagcacc aattgcttca cataccagca tgttccattt ccaatttaga 4920

attagccaca taataaaatc ttagaatctt ccttgagaaa gagctgcctg agatgtagtt 4980

ttgttatatg gttccccacc gaccattttt gtgctttttt cttgttttgt tttgttttga 5040

ctgcactgtg agttttgtag tgtcctcttc ttgccaaaac aaacgcgaga tgaactggac 5100

ttatgtagac aaatcgtgat gccagtgtat ccttcctttc ttcagttcca gcaataatga 5160

atggtcaact tttttaaaat ctagatctct ctcattcatt tcaatgtatt tttactttaa 5220

gatgaaccaa aattattaga cttatttaag atgtacaggc atcagaaaaa agaagcacat 5280

aatgcttttg gtgcgatggc actcactgtg aacatgtgta accacatatt aatatgcaat 5340

attgtttcca atactttcta atacagtttt ttataatgtt gtgtgtggtg attgttcagg 5400

tcgaatctgt tgtatccagt acagctttag gtcttcagct gcccttctgg cgagtacatg 5460

cacaggattg taaatgagaa atgcagtcat atttccagtc tgcctctatg atgatgttaa 5520

attattgctg tttagctgtg aacaagggat gtaccactgg aggaatagag tatccttttg 5580

tacacatttt gaaatgcttc ttctgtagtg atagaacaaa taaatgcaac gaatactctg 5640

tctgccctat cccgtgaagt ccacactggc gtaagagaag gcccagcaga gcaggaatct 5700

gcctagactt tctcccaatg agatcccaat atgagaggga gaagagatgg gcctcaggac 5760

agctgcaata ccacttggga acacatgtgg tgtcttgatg tggccagcgc agcagttcag 5820

cacaacgtac ctcccatcta caacagtgct ggacgtggga attctaagtc ccagtcttga 5880

gggtgggtgg agatggaggg caacaagaga tacatttcca gttctccact gcagcatgct 5940

tcagtcattc tgtgagtggc cgggcccagg gccctcacaa tttcactacc ttgtctttta 6000

catagtcata agaattatcc tcaacatagc cttttgacgc tgtaaatctt gagtattcat 6060

ttaccctttt ctgatctcct ggaaacagct gcctgcctgc attgcacttc tcttcccgag 6120

gagtggggta aatttaaaag tcaagttata gtttggatgt tagtatagaa ttttgaaatt 6180

gggaattaaa aatcaggact ggggactggg agaccaaaaa tttctgatcc catttctgat 6240

ggatgtgtca caccttttct gtcaaaataa aatgtcttgg aggttatgac tccttggtga 6300

aaaaaaaaaa aaaaaaa 6317

<210> 16
<211> 5451
<212> DNA
<213> Mus <mouse, genus>

<220>
<223> Mouse Ikzf1 (NM_001025597.2)

<400> 16
gtcagggtcc cgaagccgcg tgccgtgcgc gcaggccggg tgggctgtgg gacaagccga 60

gcgggaggcg agtcgcaagc gccaacccaa agtttgcacg gtgcggggcg aggggcgcgc 120

gctccgggct gccgcaggtg gcggcgcggt gagcccgggc caggtgcccc ggcagcgggg 180

cggcgctgtc gtgcgggaca gccgggctgc caggggctcg gagccgggtc ggagcccgcg 240

gggggcgggg agtgtggcga gaaatgggga acaatgcgag tgagcaactt gaggaagtca 300

ttgtgaaaga aagctgggaa ttgctccgca gccaacttag cagggcactc taacaagtgc 360

ctgcgcggcc gcgcccgggc cggggacagg ggcagcccgg cgcagtacag cccatcccgg 420

gacgctcggc cgcggctgcc ggagacccgg taggtcccgc ggggtgcagg agcccccaga 480

tccccggctg ctcttcgcgc cccaggatca ttcttggccc ccaaagcgcg gcgcacaaat 540

ccacataacc tgaagacaat ggatgtcgat gagggtcaag acatgtccca agtttcagga 600

aaggagagcc ccccagtcag tgacactcca gatgaagggg atgagcccat gcctgtccct 660

gaggacctgt ccactacctc tggagcacag cagaactcca agagtgatcg aggcatggcc 720

agtaatgtta aagtagagac tcagagtgat gaagagaatg ggcgtgcctg tgaaatgaat 780

ggggaagaat gtgcagagga tttacgaatg cttgatgcct cgggagagaa aatgaatggc 840

tcccacaggg accaaggcag ctcggctttg tcaggagttg gaggcattcg acttcctaac 900

ggaaaactaa agtgtgatat ctgtgggatc gtttgcatcg ggcccaatgt gctcatggtt 960

cacaaaagaa gtcatactgg tgaacggcct ttccagtgca accagtgtgg ggcctccttt 1020

acccagaaag gcaacctcct gcggcacatc aagctgcact cgggtgagaa gcccttcaaa 1080

tgccatcttt gcaactatgc ctgccgccgg agggacgccc tcaccggcca cctgaggacg 1140

cactccgttg gtaagcctca caaatgtgga tattgtggcc ggagctataa acagcgaagc 1200

tctttagagg agcataaaga gcgatgccac aactacttgg aaagcatggg ccttccgggc 1260

atgtacccag tcattaagga agaaactaac cacaacgaga tggcagaaga cctgtgcaag 1320

ataggagcag agaggtccct tgtcctggac aggctggcaa gcaatgtcgc caaacgtaag 1380

agctctatgc ctcagaaatt tcttggagac aagtgcctgt cagacatgcc ctatgacagt 1440

gccaactatg agaaggagga tatgatgaca tcccacgtga tggaccaggc catcaacaat 1500

gccatcaact acctgggggc tgagtccctg cgcccattgg tgcagacacc ccccggtagc 1560

tccgaggtgg tgccagtcat cagctccatg taccagctgc acaagccccc ctcagatggc 1620

cccccacggt ccaaccattc agcacaggac gccgtggata acttgctgct gctgtccaag 1680

gccaagtctg tgtcatcgga gcgagaggcc tccccgagca acagctgcca agactccaca 1740

gatacagaga gcaacgcgga ggaacagcgc agcggcctta tctacctaac caaccacatc 1800

aacccgcatg cacgcaatgg gctggctctc aaggaggagc agcgcgccta cgaggtgctg 1860

agggcggcct cagagaactc gcaggatgcc ttccgtgtgg tcagcacgag tggcgagcag 1920

ctgaaggtgt acaagtgcga acactgccgc gtgctcttcc tggatcacgt catgtatacc 1980

attcacatgg gctgccatgg ctttcgggat ccctttgagt gtaacatgtg tggttatcac 2040

agccaggaca ggtacgagtt ctcatcccat atcacgcggg gggagcatcg ttaccacctg 2100

agctaaaccc agccaggccc cactgaagca caaagatagc tggttatgcc tccttcccgg 2160

cagctggacc cacagcggac aatgttggga gtggatttgc aggcagcatt tgttctttta 2220

tgttggttgt ttggcgtttg atttgcgttg gaagataagt ttttaatgtt agtgacagga 2280

ttgcattgca tcaggaacat tcacaacatc catccttcta gccagttttg ttcactggta 2340

gctgaggttt cccggatatg tggcttccta acactctccc cacccacccc accccccaaa 2400

acagagcctg aatcttcatg aagtgaataa aacaattatc caagaaggag taaggtggat 2460

cttgccctaa gcagagttta tgccacaaag attctccaaa tcccccaaga cagcacagcc 2520

actggggttg agccatctca gggagctctg caggtgagcc agaggaccag atataaggca 2580

gctggggagg agcagggaca tcagcctgtg cagagaccaa ggccaaaggt tgaactttga 2640

aagactatta agtcatatat tgtatggcaa tatggtgtct ggacaagttg tgcaatgtgc 2700

tgaagggaag ggattggaga gccttgaaga ctcttcttca tttgcctgat caacccgacc 2760

tccagagggt ttgttgccca gtaagacgag ctcagtgctc ttgtgatcat ttttctctta 2820

tcgtttccat gccgttgatg gccctgaagc tcatcactgc attttagaac ccaatcctga 2880

aattgggacc ttttttttaa acttctgata ctgtaaaact tcttggaagc caaagctttc 2940

ttccaagccc catcctcagt tatcctggtt cctgttcttc cccgagctga tagtaccagg 3000

acctgttatt ccacaaaagc acaggcatcc gtcacttcaa ttcaatccct gttcagatta 3060

tagatatgga ctttgctatc ttgataaatg tcttctctat gttattttgt ctgaaaaacc 3120

tataaaacca ttattaagaa tgaccatttt tagatggaag aaatgagccc agcatctcag 3180

tggctaaaac acaaaatatc catgctttta aacaaaattg ttaaatattc cgaagctctc 3240

tagtataaac accaagtagc atgtgttttc acataaagaa gacaggggcc atgcaacctt 3300

tatcaagtgg aggtattaga atgttgtaat gtttggagac acagtgtgac cagtacaggt 3360

tcccagagag gaatgcccac catatcacag aaaggtagag gtgggatctg gtatagccag 3420

accaagacag ggatgtcacg ctgaagccaa gtcagttagc tgaagattct caacaggaag 3480

gcctctctta agagtcagta atagggttgt taccatccac cacctcaaca aaacaaaaag 3540

cttataattg taaatgttta cagcactgtc ttcgcagaaa ctttctgagg tgattccaaa 3600

gaactagagg ggagatggtc tataacagct cttgaagtaa acgaggttct tagtctcagc 3660

tctcctgaca tatagggctt gatcattact ggtagggatt gttctgtgaa ttgcttacta 3720

ctacccctgg tctctcccca gtagatgcca ggaacattct agctgatacc taactgtctt 3780

cccaggtgtt cgagggagca aaccactgat ctaaactcta aacgctgaag tacgcaggtt 3840

ttctaaaaat gacaagccct tgaaaccttt cccagtaggc agcctcgagc tggacttgtg 3900

tctttggaat gctgatgaat tctatagatc agcattgcaa atacacttca aatacgtctg 3960

agttcaagtg cagggactga gttcaccaag gtgtgaaatg tgctcaaaaa gttcaaaagt 4020

gtgtgtttct ttgtttctaa aacattgtgg catctttttc atttgtttct aaaacttttt 4080

ttttagaaac aaatgaagca cttggaaagt gaaagtaaaa ttacaaatat aaggatttac 4140

actgaagaga gaaaaatttt aggaactata gctgtgaaaa gattttgttc aaaaggcagg 4200

ctagccttac ccaaattcat atatggcagg tgtcaacctc ccaagcttac agttagcagg 4260

cagcttttgc tcactcatcc ttagccatga gagccattaa gtgtggtcca agaaagatgg 4320

ctccaaaccc tacccccgac ccaccagtgg tattcagaga ttaaagcaga attgtaaata 4380

gtggcttcag gagctctttt ttagaatgct ttgccccttc ctctcactgc cttttttagc 4440

caatataaat gtcaatttgc acaccttttg ttgtggtttt atattgtaac agcatttttt 4500

tgaaactatt gtatttaaga taaggtttca tattatgtcc acaagtaatt aaattatgtt 4560

tgaaggtggc tatatgctgt atcagaagtt gatgatgttt ttctttagct ggtaaaggag 4620

ggttttgcat gacctcactg tttgttctgt ggtttgttct gttgtatgat gtgtgtcttg 4680

agttttgctg tgtgatgaag tgcgctgaga ttccagtgcc ctcaagttgt gttttaagta 4740

gctatcagag gcaagagggt tcctaagagc aggttgacct gttggcgaca gatggcaatc 4800

accatttctc attccttctt ctccctgtta ccccagcttc ctgtcccagg tcccttctgt 4860

gattcttacc ttagtgtgca tgtgtgtctg tcctggtgag agtcaggagc atcgatatgt 4920

tatcattgca ttatcaccaa gggcacgcac agcctagcac ctgttgcttc agataccgtc 4980

acactctgtt tccaatttag atacaaccac ataataaaat gttagagtct tcaatgggaa 5040

gcagaggtgc ttgttataaa gatgggggct tatgcttgtg tcacattttg tgttcttttc 5100

ttcttttgtt tggttttaac ttaattgtga cccttgtaac atcatcttgc caaaaaaaaa 5160

aaaaaagttg aactggattt atgtagacat gtcaagacgt actatctatt tctttgtcag 5220

ttatagcaat aagagtggat aaactctaaa atccagatct cccacaatga acatccgtgt 5280

tctttctatg atttttcttt ctttatggtg agccacaatt aaacttgaga tgtacagcca 5340

cccaaaccca ggaagctcat gtgcatctgg tgctatggca ctcactgtga ataagtgtga 5400

ccagatatta atatgcaata ttgtttccaa tcctttctaa tacatttttt c 5451

<210> 17
<211> 1402
<212> DNA
<213> Homo sapiens

<220>
<223> Human ID2 (NM_002166.4)

<400> 17
ggggacgaag ggaagctcca gcgtgtggcc ccggcgagtg cggataaaag ccgccccgcc 60

gggctcgggc ttcattctga gccgagcccg gtgccaagcg cagctagctc agcaggcggc 120

agcggcggcc tgagcttcag ggcagccagc tccctcccgg tctcgccttc cctcgcggtc 180

agcatgaaag ccttcagtcc cgtgaggtcc gttaggaaaa acagcctgtc ggaccacagc 240

ctgggcatct cccggagcaa aacccctgtg gacgacccga tgagcctgct atacaacatg 300

aacgactgct actccaagct caaggagctg gtgcccagca tcccccagaa caagaaggtg 360

agcaagatgg aaatcctgca gcacgtcatc gactacatct tggacctgca gatcgccctg 420

gactcgcatc ccactattgt cagcctgcat caccagagac ccgggcagaa ccaggcgtcc 480

aggacgccgc tgaccaccct caacacggat atcagcatcc tgtccttgca ggcttctgaa 540

ttcccttctg agttaatgtc aaatgacagc aaagcactgt gtggctgaat aagcggtgtt 600

catgatttct tttattcttt gcacaacaac aacaacaaca aattcacgga atcttttaag 660

tgctgaactt atttttcaac catttcacaa ggaggacaag ttgaatggac ctttttaaaa 720

agaaaaaaaa aatggaagga aaactaagaa tgatcatctt cccagggtgt tctcttactt 780

ggactgtgat attcgttatt tatgaaaaag acttttaaat gccctttctg cagttggaag 840

gttttcttta tatactattc ccaccatggg gagcgaaaac gttaaaatca caaggaattg 900

cccaatctaa gcagactttg ccttttttca aaggtggagc gtgaatacca gaaggatcca 960

gtattcagtc acttaaatga agtcttttgg tcagaaatta cctttttgac acaagcctac 1020

tgaatgctgt gtatatattt atatataaat atatctattt gagtgaaacc ttgtgaactc 1080

tttaattaga gttttcttgt atagtggcag agatgtctat ttctgcattc aaaagtgtaa 1140

tgatgtactt attcatgcta aactttttat aaaagtttag ttgtaaactt aaccctttta 1200

tacaaaataa atcaagtgtg tttattgaat ggtgattgcc tgctttattt cagaggacca 1260

gtgctttgat ttttattatg ctatgttata actgaaccca aataaataca agttcaaatt 1320

tatgtagact gtataagatt ataataaaac atgtctgaag tcaaaaaaaa aaaaaaaaaa 1380

aaaaaaaaaa aaaaaaaaaa aa 1402

<210> 18
<211> 1289
<212> DNA
<213> Mus <mouse, genus>

<220>
<223> Mouse Id2 (NM_010496.3)

<400> 18
tcattctgaa ccgagcctgg tgccgcgcag tcagctcagc cccctgtggc ggctccctcc 60

cggtcttcct cctacgagca gcatgaaagc cttcagtccg gtgaggtccg ttaggaaaaa 120

cagcctgtcg gaccacagct tgggcatctc ccggagcaaa accccggtgg acgacccgat 180

gagtctgctc tacaacatga acgactgcta ctccaagctc aaggaactgg tgcccagcat 240

cccccagaac aagaaggtga ccaagatgga aatcctgcag cacgtcatcg attacatctt 300

ggacctgcag atcgccctgg actcgcatcc cactatcgtc agcctgcatc accagagacc 360

tggacagaac caggcgtcca ggacgccgct gaccaccctg aacacggaca tcagcatcct 420

gtccttgcag gcatctgaat tcccttctga gcttatgtcg aatgatagca aagtactctg 480

tggctaaata aatggcattt ggggactttt tttttctttt ttactttctc tttttctttt 540

gcacaagaag aagtctacaa gatcttttaa gacttttgtt atcagccatt tcaccaggag 600

aacacgttga atggaccttt ttaaaaagaa agcggaagga aaactaagga tgatcgtctt 660

gcccaggtgt cgttctccgg cctggactgt gataccgtta tttatgagag actttcagtg 720

ccctttctac agttggaagg ttttctttat atactattcc caccatgggg agcgaaaacg 780

ttaaaaaaaa aagaaaaaaa tcacaaggaa ttgcccaatg taagcagact ttgccttttc 840

acaaaggtgg agcgtgaata ccagaaggac ccagtattcg gttacttaaa tgaagtcttc 900

ggtcagaaat ggcctttttg acacgagcct actgaatgct gtgtatatat ttatatataa 960

atatatatat attgagtgaa ccttgtggac tctttaatta gagttttctt gtatagtggc 1020

agaaataacc tatttctgca ttaaaatgta atgacgtact tatgctaaac tttttataaa 1080

agtttagttg taaacttaac ccttttatac aaaataaatc aagtgtgttt attgaatgtt 1140

gattgcttgc tttatttcag acaaccagtg ctttgatttt ttttatgcta tgttataact 1200

gaacccaaat aaataccagt tcaaatttat gtagactgta ttaagattat aataaaatgt 1260

gtctgacatc aaaaaaaaaa aaaaaaaaa 1289

<210> 19

<211> 5946
<212> DNA
<213> Homo sapiens

<220>
<223> Human BCL11A (NM_022893.3)

<400> 19
tttttttttt ttttttgctt aaaaaaaagc catgacggct ctcccacaat tcatcttccc 60

tgcgccatct ttgtattatt tctaatttat tttggatgtc aaaaggcact gatgaagata 120

ttttctctgg agtctccttc tttctaaccc ggctctcccg atgtgaaccg agccgtcgtc 180

cgcccgccgc cgccgccgcc gccgccgccg cccgccccgc agcccaccat gtctcgccgc 240

aagcaaggca aaccccagca cttaagcaaa cgggaattct cgcccgagcc tcttgaagcc 300

attcttacag atgatgaacc agaccacggc ccgttgggag ctccagaagg ggatcatgac 360

ctcctcacct gtgggcagtg ccagatgaac ttcccattgg gggacattct tatttttatc 420

gagcacaaac ggaaacaatg caatggcagc ctctgcttag aaaaagctgt ggataagcca 480

ccttcccctt caccaatcga gatgaaaaaa gcatccaatc ccgtggaggt tggcatccag 540

gtcacgccag aggatgacga ttgtttatca acgtcatcta gaggaatttg ccccaaacag 600

gaacacatag cagataaact tctgcactgg aggggcctct cctcccctcg ttctgcacat 660

ggagctctaa tccccacgcc tgggatgagt gcagaatatg ccccgcaggg tatttgtaaa 720

gatgagccca gcagctacac atgtacaact tgcaaacagc cattcaccag tgcatggttt 780

ctcttgcaac acgcacagaa cactcatgga ttaagaatct acttagaaag cgaacacgga 840

agtcccctga ccccgcgggt tggtatccct tcaggactag gtgcagaatg tccttcccag 900

ccacctctcc atgggattca tattgcagac aataacccct ttaacctgct aagaatacca 960

ggatcagtat cgagagaggc ttccggcctg gcagaagggc gctttccacc cactcccccc 1020

ctgtttagtc caccaccgag acatcacttg gacccccacc gcatagagcg cctgggggcg 1080

gaagagatgg ccctggccac ccatcacccg agtgcctttg acagggtgct gcggttgaat 1140

ccaatggcta tggagcctcc cgccatggat ttctctagga gacttagaga gctggcaggg 1200

aacacgtcta gcccaccgct gtccccaggc cggcccagcc ctatgcaaag gttactgcaa 1260

ccattccagc caggtagcaa gccgcccttc ctggcgacgc ccccccctcc tcctctgcaa 1320

tccgcccctc ctccctccca gcccccggtc aagtccaagt catgcgagtt ctgcggcaag 1380

acgttcaaat ttcagagcaa cctggtggtg caccggcgca gccacacggg cgagaagccc 1440

tacaagtgca acctgtgcga ccacgcgtgc acccaggcca gcaagctgaa gcgccacatg 1500

aagacgcaca tgcacaaatc gtcccccatg acggtcaagt ccgacgacgg tctctccacc 1560

gccagctccc cggaacccgg caccagcgac ttggtgggca gcgccagcag cgcgctcaag 1620

tccgtggtgg ccaagttcaa gagcgagaac gaccccaacc tgatcccgga gaacggggac 1680

gaggaggaag aggaggacga cgaggaagag gaagaagagg aggaagagga ggaggaggag 1740

ctgacggaga gcgagagggt ggactacggc ttcgggctga gcctggaggc ggcgcgccac 1800

cacgagaaca gctcgcgggg cgcggtcgtg ggcgtgggcg acgagagccg cgccctgccc 1860

gacgtcatgc agggcatggt gctcagctcc atgcagcact tcagcgaggc cttccaccag 1920

gtcctgggcg agaagcataa gcgcggccac ctggccgagg ccgagggcca cagggacact 1980

tgcgacgaag actcggtggc cggcgagtcg gaccgcatag acgatggcac tgttaatggc 2040

cgcggctgct ccccgggcga gtcggcctcg gggggcctgt ccaaaaagct gctgctgggc 2100

agccccagct cgctgagccc cttctctaag cgcatcaagc tcgagaagga gttcgacctg 2160

cccccggccg cgatgcccaa cacggagaac gtgtactcgc agtggctcgc cggctacgcg 2220

gcctccaggc agctcaaaga tcccttcctt agcttcggag actccagaca atcgcctttt 2280

gcctcctcgt cggagcactc ctcggagaac gggagtttgc gcttctccac accgcccggg 2340

gagctggacg gagggatctc ggggcgcagc ggcacgggaa gtggagggag cacgccccat 2400

attagtggtc cgggcccggg caggcccagc tcaaaagagg gcagacgcag cgacacttgt 2460

gagtactgtg ggaaagtctt caagaactgt agcaatctca ctgtccacag gagaagccac 2520

acgggcgaaa ggccttataa atgcgagctg tgcaactatg cctgtgccca gagtagcaag 2580

ctcaccaggc acatgaaaac gcatggccag gtggggaagg acgtttacaa atgtgaaatt 2640

tgtaagatgc cttttagcgt gtacagtacc ctggagaaac acatgaaaaa atggcacagt 2700

gatcgagtgt tgaataatga tataaaaact gaatagaggt atattaatac ccctccctca 2760

ctcccacctg acaccccctt tttcaccact ccccttcccc atcgccctcc agccccactc 2820

cctgtaggat ttttttctag tcccatgtga tttaaacaaa caaacaaaca aacagaagta 2880

acgaagctaa gaatatgaga gtgcttgtca ccagcacacc tgtttttttt ctttttcttt 2940

ttcttttttc tttttccttt tttttttttt tcctttatgt tctcaccgtt tgaatgcatg 3000

atctgtatgg ggcaatacta ttgcatttta cgcaaacttt gagcctttct cttgtgcaat 3060

aatttacatg ttgtgtatgt ttttttttaa acttagacag catgtatggt atgttatggc 3120

tattttaaat tgtccctaat tcgttgctga gcaaacatgt tgctgtttcc agttccgttc 3180

tgagagaaaa agagagagag agagaaaaag accatgctgc atacattctg taatacatat 3240

catgtacagt tttattttat aacgtgagga ggaaaaacag tctttggatt aaccctctat 3300

agacagaata gatagcactg aaaaaaaatc tctatgagct aaatgtctgt ctctaaaggg 3360

ttaaatgtat caattggaaa ggaagaaaaa aggccttgaa ttgacaaatt aacagaaaaa 3420

cagaacaagt ttattctatc atttggtttt aaaatatgag tgccttggat ctattaaaac 3480

cacatcgatg gttctttcta cttgttataa acttgtagct taattcagca ttgggtgagg 3540

taataaacct taggaactag catataattc tatattgtat ttctcacaac aatggctacc 3600

taaaaagatg acccattatg tcctagttaa tcatcatttt tcctttagtt taattttata 3660

aacaaaactg attataccag tataaaagct actttgctcc tggtgagagc ttaaaagaaa 3720

tgggctgttt tgcccaaagt tttatttttt ttaaacaatg attaaattga atgtgtaatg 3780

tgcaaaagcc ctggaacgca attaaataca ctagtaagga gttcatttta tgaagatatt 3840

tgctttaata atgtcttttt aaaaatactg gcaccaaaag aaatagatcc agatctactt 3900

ggttgtcaag tggacaatca aatgataaac tttaagacct tgtataccat attgaaagga 3960

agaggctgac aataaggttt gacagagggg aacagaagaa aataatatga tttattagca 4020

caacgtggta ctatttgcca tttaaaacta gaacaggtat ataagctaat attgatacaa 4080

tgatgattaa ctatgaattc ttaagacttg catttaaatg tgacattctt aaaaaaagaa 4140

gagaaagaat tttaagagta gcagtatata tgtctgtgct ccctaaaagt tgtacttcat 4200

ttcttttcca tacactgtgt gctatttgtg ttaacatgga agaggattca ttgtttttat 4260

ttttattttt ttaatttttt cttttttatt aagctagcat ctgccccagt tggtgttcaa 4320

atagcacttg actctgcctg tgatatctgt atcttttctc taatcagaga tacagaggtt 4380

gagtataaaa taaacctgct cagataggac aattaagtgc actgtacaat tttcccagtt 4440

tacaggtcta tacttaaggg aaaagttgca agaatgctga aaaaaaattg aacacaatct 4500

cattgaggag cattttttaa aaactaaaaa aaaaaaaact ttgccagcca tttacttgac 4560

tattgagctt acttacttgg acgcaacatt gcaagcgctg tgaatggaaa cagaatacac 4620

ttaacataga aatgaatgat tgctttcgct tctacagtgc aaggattttt ttgtacaaaa 4680

cttttttaaa tataaatgtt aagaaaaatt ttttttaaaa aacacttcat tatgtttagg 4740

ggggaactgc attttagggt tccattgtct tggtggtgtt acaagacttg ttatccattt 4800

aaaaatggta gtggaaattc tatgccttgg atacacaccg ctcttcaggt tgtaaaaaaa 4860

aaaaacatac attggggaaa ggtttaagat tatatagtac ttaaatatag gaaaatgcac 4920

actcatgttg attcctatgc taaaatacat ttatggtctt ttttctgtat ttctagaatg 4980

gtatttgaat taaatgttca tctagtgtta ggcactatag tatttatatt gaagcttgta 5040

tttttaactg ttgcttgttc tcttaaaagg tatcaatgta ccttttttgg tagtggaaaa 5100

aaaaaagaca ggctgccaca gtatattttt ttaatttggc aggataatat agtgcaaatt 5160

atttgtatgc ttcaaaaaaa aaaaaaagag agaaacaaaa aagtgtgaca ttacagatga 5220

gaagccatat aatggcggtt tgggggagcc tgctagaatg tcacatggat ggctgtcata 5280

ggggttgtac atatcctttt ttgttccttt ttcctgctgc catactgtat gcagtactgc 5340

aagctaataa cgttggtttg ttatgtagtg tgctttttgt ccctttcctt ctatcaccct 5400

acattccagc atcttacctt catatgcagt aaaagaaaga aagaaaaaaa aaggaaaaaa 5460

aaaaaaaaac caatgttttg cagttttttt cattgccaaa aactaaatgg tgctttatat 5520

ttagattgga aagaatttca tatgcaaagc atattaaaga gaaagcccgc tttagtcaat 5580

acttttttgt aaatggcaat gcagaatatt ttgttattgg ccttttctat tcctgtaatg 5640

aaagctgttt gtcgtaactt gaaattttat cttttactat gggagtcact atttattatt 5700

gcttatgtgc cctgttcaaa acagaggcac ttaatttgat cttttatttt tctttgtttt 5760

tatttttttt tttatttaga tgaccaaagg tcattacaac ctggcttttt attgtatttg 5820

tttctggtct ttgttaagtt ctattggaaa aaccactgtc tgtgtttttt tggcagttgt 5880

ctgcattaac ctgttcatac acccattttg tccctttatt gaaaaaataa aaaaaattaa 5940

agtaca 5946

<210> 20
<211> 3425
<212> DNA
<213> Mus <mouse, genus>

<220>
<223> Mouse Bcl11a (NM_016707.3)

<400> 20
gacgttcaag ttcgcaggga cgtcacgtcc gcacttgaac ttgcagctca gggggcttt 60

tgccattttt ttcatctctc tctctccctc tatccctctt ctctcttcct ctctctcttt 120

tttttcctta aaaaaaaaaa agccatgacg gctctcccac aattcatctt ccctgcgcca 180

tctttgtatt atttctaatt tattttggat gtcaaaaggc actgatgaag atattttctc 240

tggagtctcc ttctttctaa cccggctctc ccgatgtgaa ccgagccgtc gtccgcacgc 300

cgccgccgcc gccgccgccc gccccgcagc ccaccatgtc tcgccgcaag caaggcaaac 360

cccagcactt aagcaaacgg gaattctcgc ccgaacctct tgaagccatt cttacagatg 420

atgaaccaga ccatggcccg ttgggagctc cagaagggga ccacgacctt ctcacctgtg 480

ggcagtgcca gatgaatttc ccactggggg acattcttat ttttatcgag cacaaacgga 540

aacaatgcaa tggcagcctc tgcttagaaa aaggtgtgga taagccgcct tccccttctc 600

ccatcgagat gaaaaaggca tccaatcctg tggaggttgg catccaggtc acgccagagg 660

atgacgattg tttatcaacg tcatctagag gaatttgccc caaacaggaa cacatagcag 720

ataaacttct gcactggagg ggcctgtcct ctcctcggtc tgcacacgga gctctaatcc 780

ccacgcccgg gatgagtgca gaatatgccc cgcagggtat ttgtaaagat gagcccagca 840

gctacacatg tacaacttgc aaacagccat tcaccagtgc atggtttctc ttgcaacacg 900

cacagaacac tcatggatta agaatctact tagaaagtga acacggaagt cccctgaccc 960

cgcgggttgg tatcccttca ggactaggtg cagaatgtcc ttcccagcca cctctccatg 1020
ggattcatat tgcagacaat aaccccctta acctgctaag aataccagga tcagtatcga 1080
gagaggcttc cggcctggca gaagggcgct ttccacccac tccccccctg tttagtccac 1140
caccgagaca tcacttggac ccccaccgca tagagcgcct gggggcggaa gagatggccc 1200
tggccaccca tcacccgagt gcctttgaca gggtgctgcg gttgaatcca atggctatgg 1260
agcctcccgc catggatttc tctaggagac ttagagagct ggcagggaac acgtctagtc 1320
caccgctgtc cccaggccgg cccagtccta tgcaaaggtt actgcaacca ttccagccag 1380
gtagcaagcc acccttcctg gcgacgcccc ccctccctcc tctgcaatcc gcccctcctc 1440
cctcccaacc cccggtcaag tccaagtcat gcgagttctg cggcaagacg ttcaaatttc 1500
agagcaactt ggtggttcac cgacgcagcc atactggtga gaagccctat aagtgcaacc 1560
tgtgcgacca cgcgtgcaca caggccagca agctgaagcg tcacatgaag acacacatgc 1620
acaaatcgtc ccccatgaca gtcaagtccg acgatggcct ctccacagcc agctccccgg 1680
aacctggtac cagcgacctg gtgggcagcg ccagcagtgc gctcaagtca gtggtggcca 1740
agttcaagag tgagaacgac cccaacttga tcccagagaa cggggatgag gaggaagagg 1800
aggacgacga ggaagaagaa gaagaggagg aagaggagga ggaggagctg acggagagcg 1860
agagggtgga ctacggcttc gggctgagcc tggaggctgc acgccaccat gagaacagct 1920
ctcggggcgc agtggtgggc gtgggcgacg agggccgcgc cctgcccgat gtcatgcagg 1980
gcatggtgct cagctccatg cagcacttca gcgaggcctt ccaccaggtc ctgggcgaaa 2040
agcataagcg tagccacctg gccgaggccg agggccatag ggacacttgt gatgaagact 2100
cggtggccgg tgagtcagac cgcatagacg atggcactgt taatggtcgt ggctgctccc 2160
ccggcgaatc ggcttcgggg ggtctgtcca aaaagctgct gctgggtagc cccagctcgc 2220
tgagcccctt ctccaagcgc atcaagctgg agaaggagtt tgacctgccc ccggccgcga 2280
tgcctaacac ggagaacgtg tattcgcagt ggctcgctgg ctatgcggcc tccaggcagc 2340
tcaaagatcc cttccttact ttcggagact ccagacaatc gccttttgcc tcctcatcag 2400
agcactcctc ggagaacggg agcttgcgct tctccacacc gcccggggag ctggacggag 2460
ggatctcagg gcgcagcggc acaggaagtg gagggagcac gccccatatt agtggtccgg 2520
gcccgggcag gcccagctca aaagagggca gacgcagcga cacttgtcct tcacacaccc 2580
ccgttcggcg tagtaccccg cgagctcaag atgtgtggca gttttcggat ggaagctcaa 2640
gaacccttaa gttctgagaa actttgaagc ccccaagggc ggggcggaca tgcgccgccc 2700
agccgacgtc aacgtgctcc gttatcctgc tagattgtga tgttttctga cagtagcctc 2760
caagaagaca agagtcctgc cgagtcctcc cagcctgggc ctgcagtgcc attttattta 2820
tattttttaa taaaacgtaa aaacaaaaaa aaccagaccc acattggaac agtgaacccg 2880

tcccatccag agggccctag gactgccgca gttggagcga cgtccaaccc ttttgaaacc 2940

agccaaccta attacccgta ctgtggaaat gagcatgacc cctgacccct tgtttctata 3000

cattctatgt tgtcttttaa aaagtgtgct taacattgac ataataaatg ttggagcttt 3060

aggcggtgtg tgcttgtttt ttaattttta atgctcgtaa gacaatgtgg ctgcttcagg 3120

ctttatgtct gtgtactttt tttccttcag aagctcatag ggtgagcaga aggaccagac 3180

tcaagtgcca ggcaggagac ctagaaaagg aagtaggctt ttcagatggc atacattttc 3240

aaagaaaatc aaaatgcaaa gctaggggat aaatcatagt aatatcataa ttaatgtagt 3300

agtattgctg tttattaatg ctgacgtgtg tttttcctct ctgacttata atttgcatac 3360

cattaaataa tgcataaata tggcacactg aatccttttt caaatacacg cttttggtga 3420

ctacc 3425

<210> 21
<211> 2297
<212> DNA
<213> Homo sapiens

<220>
<223> Human RELB (NM_006509.3)

<400> 21
ggccccgcgc cccgcgcagc cccgggcgcc gcgcgtcctg cccggcctgc ggccccagcc 60

cttgcgccgc tcgtccgacc cgcgatcgtc caccagaccg tgcctcccgg ccgcccggcc 120

ggcccgcgtg catgcttcgg tctgggccag cctctgggcc gtccgtcccc actggccggg 180

ccatgccgag tcgccgcgtc gccagaccgc cggctgcgcc ggagctgggg gccttagggt 240

cccccgacct ctcctcactc tcgctcgccg tttccaggag cacagatgaa ttggagatca 300

tcgacgagta catcaaggag aacggcttcg gcctggacgg gggacagccg ggcccgggcg 360

aggggctgcc acgcctggtg tctcgcgggg ctgcgtccct gagcacggtc accctgggcc 420

ctgtggcgcc cccagccacg ccgccgcctt ggggctgccc cctgggccga ctagtgtccc 480

cagcgccggg cccgggcccg cagccgcacc tggtcatcac ggagcagccc aagcagcgcg 540

gcatgcgctt ccgctacgag tgcgagggcc gctcggccgg cagcatcctt ggggagagca 600

gcaccgaggc cagcaagacg ctgcccgcca tcgagctccg ggattgtgga gggctgcggg 660

aggtggaggt gactgcctgc ctggtgtgga aggactggcc tcaccgagtc cacccccaca 720

gcctcgtggg gaaagactgc accgacggca tctgcagggt gcggctccgg cctcacgtca 780

gcccccggca cagttttaac aacctgggca tccagtgtgt gaggaagaag gagattgagg 840

ctgccattga gcggaagatt caactgggca ttgaccccta caacgctggg tccctgaaga 900

accatcagga agtagacatg aatgtggtga ggatctgctt ccaggcctca tatcgggacc 960

agcagggaca gatgcgccgg atggatcctg tgctttccga gcccgtctat gacaagaaat 1020

ccacaaacac atcagagctg cggatttgcc gaattaacaa ggaaagcggg ccgtgcaccg 1080

gtggcgagga gctctacttg ctctgcgaca aggtgcagaa agaggacata tcagtggtgt 1140

tcagcagggc ctcctgggaa ggtcgggctg acttctccca ggccgacgtg caccgccaga 1200

ttgccattgt gttcaagacg ccgccctacg aggacctgga gattgtcgag cccgtgacag 1260

tcaacgtctt cctgcagcgg ctcaccgatg gggtctgcag cgagccattg cctttcacgt 1320

acctgcctcg cgaccatgac agctacggcg tggacaagaa gcggaaacgg gggatgcccg 1380

acgtccttgg ggagctgaac agctctgacc cccatggcat cgagagcaaa cggcggaaga 1440

aaaagccggc catcctggac cacttcctgc ccaaccacgg ctcaggcccg ttcctcccgc 1500

cgtcagccct gctgccagac cctgacttct tctctggcac cgtgtccctg cccggcctgg 1560

agccccctgg cgggcctgac ctcctggacg atggctttgc ctacgaccct acggccccca 1620

cactcttcac catgctggac ctgctgcccc cggcaccgcc acacgctagc gctgttgtgt 1680

gcagcggagg tgccggggcc gtggttgggg agacccccgg ccctgaacca ctgacactgg 1740

actcgtacca ggccccgggc cccggggatg gaggcaccgc cagccttgtg ggcagcaaca 1800

tgttccccaa tcattaccgc gaggcggcct ttgggggcgg cctcctatcc ccggggcctg 1860

aagccacgta gccccgcgat gccagaggag gggcactggg tggggaggga ggtggaggag 1920

ccgtgcaatc ccaaccagga tgtctagcac ccccatcccc ttggcccttc ctcatgcttc 1980

tgaagtggac atattcagcc ttggcgagaa gctccgttgc acgggtttcc ccttgagccc 2040

attttacaga tgaggaaact gagtccggag aggaaaaggg acatggctcc cgtgcactag 2100

cttgttacag ctgcctctgt ccccacatgt gggggcacct tctccagtag gattcggaaa 2160

agattgtaca tatgggagga gggggcagat tcctggccct ccctccccag acttgaaggt 2220

ggggggtagg ttggttgttc agagtcttcc caataaagat gagtttttga gcctccggga 2280

aaaaaaaaaa aaaaaaa 2297

<210> 22
<211> 2218
<212> DNA
<213> Mus <mouse, genus>

<220>
<223> Mouse RelB (NM_009046.2)

<400> 22
   gcccgccgcc cgctcggccc ggcgcccccc gcagccccgg gcgccgtgcg tccagcccgg 60
   ccttcggccc tagccttgca ccgcttgccc ggccggtgat cgtcccagcc gaccgggctg 120
   tccgactgcc cggcttgccc tcgtgtgcgc tcgccctgag ctggccttcg ggctgtcggt 180
   ccctacgggc cgggccatgc cgagtcgccg cgctgccaga gagtccgcgc ccgagctagg 240
   ggccttgggt tccagtgacc tctcttccct gtcactaacg gtctccagga ccacagatga 300
   attggaaatc atcgacgaat acattaagga gaacggcttt ggcctggacg ggacacagct 360
   gagtgagatg ccgcgcctgg tgccccgcgg gcccgcctca ctgagcagcg tcacgctggg 420
   ccctgctgca ccaccgcctc cggccacgcc gtcctggagc tgcacactgg gcaggctggt 480
   gtcacccggc ccgtgcccac ggccgtacct ggtcatcaca gagcagccaa agcagcgtgg 540
   catgcgcttc cgctacgagt gcgagggccg ctcggccggc agcatcctcg gggagagcag 600
   caccgaagcc agcaagaccc tgcccgccat cgagcttcga gactgtggcg ggctgcggga 660
   ggtggaggtg acggcgtgcc tggtgtggaa ggactggcca caccgggtac acccacatag 720
   cctcgtgggg aaagactgca cggacggcgt ctgcagggtg cggctgcggc ctcacgtcag 780
   cccccggcac agctttaaca acctgggcat ccagtgtgtt aggaagaagg aaattgaagc 840
   tgccattgag cggaagatcc agctgggaat tgacccctac aatgctggct ccctgaagaa 900
   ccatcaggag gtcgacatga atgtcgtcag gatctgcttc caggcctcct atcgggacca 960
   gcagggacat ctgcaccgca tggaccccat cctctctgag cctgtctacg acaagaagtc 1020
   caccaacaca tcggagctgc ggatttgccg aatcaacaag gagagcgggc cgtgcacagg 1080
   tggtgaggag ctgtacttgc tctgtgacaa ggtgcaaaaa gaggacatat ccgtggtgtt 1140
   cagcacagct tcctgggaag gccgtgccga cttctctcaa gctgatgtgc accggcagat 1200
   cgccattgtg ttcaaaacgc caccctacga ggacctggag atctcagagc ccgtgactgt 1260
   caatgtgttc ttgcagcggc tcacggatgg ggtgtgcagc gagccgctgc ccttcacgta 1320
   cctgcctcgg gatcatgaca gctacggtgt ggacaagaag cgaaagcggg gactgcctga 1380
   tgtccttgga gagttgagca gctctgatcc acatggaatc gagagcaaac gaaggaaaaa 1440
   gaaaccagtg ttcttggacc acttcctgcc tggccacagc tcaggcctgt tcctcccacc 1500
   atcggctctg cagccggcag actctgattt cttccctgct tccatatccc ttcctgggct 1560
   ggagcctcct ggtggacccg atctcctgga cgatggcttt gcctatgatc cttctgcccc 1620
   cacgctcttc actatgttgg acctgctgcc cccagcacca ccacttgcca gtgctgtggt 1680
   gggtagcggg ggtgcagggg ccaccgttgt ggagtcttct ggcccagagc ccctatcact 1740
   ggactctttt gcagcgccgg gccccgggga tgttggtact gctagccttg tgggcagcaa 1800
   catgtttccc aaccagtacc gagaggcagc tttcgggggt ggcctcctat ctccagggcc 1860
   tgaagccacg tagcctctga ggtaacagag gaggcactgg gtgaggtatg tggtatagca 1920
   ctccattccg aagccaacct tgatcagtct tccagcttcc tcatcctgaa tcggacatct 1980
   gcagcgctgg tgggaagatg gggagcactc cggttctctt tgagcccatt ttacagaatg 2040
   ctgagtccga agaggaaaag gggctcctgc agatggaccc cttctcagga cagattctca 2100
   gagattgtac ataggggagg agggagcagg tccccagcct tctcccctaa tcctgaagaa 2160
   ggcagtggat tgttcagttt tcccaataaa aattagtttt ttgaaaaaaa aaaaaaaa 2218

<210> 23
<211> 5233
<212> DNA
<213> Homo sapiens

<220>
<223> Human ZBTB46 (NM_025224.3)

<400> 23
tgaccttctc tcctccctgt gtcttttttt ttcctttaga gatgggggtc ttgctctgtc 60

actccagcta gagcacagtg gtgcaatcgg agctcactga ctgcagcctg caactcctgg 120

gctcaagtga ttctgccgcc tcagcctctc aacgtgctgc gattagagag tctgtagaag 180

aggcgacacc agggcttcca aatgaacaac cgaaaggaag atatggaaat cacgtcccac 240

taccggcacc tgctgcggga gctcaacgag cagaggcagc acggcgtcct gtgcgacgtc 300

tgcgtggtcg tggagggcaa ggtcttcaag gcgcacaaga acgtcctgct gggcagcagc 360

cgctacttca agacgctcta ctgccaggtg cagaagacgt cggagcaggc cacggtcacg 420

cacctggaca tcgtcacggc ccagggcttc aaggccatca tcgacttcat gtactcagcg 480

cacctggcgc tcaccagcag gaacgtcatc gaggtgatgt cagccgccag cttcctgcag 540

atgacggaca tcgtgcaagc ctgccacgac ttcatcaagg cggcgctgga catcagcatc 600

aagtcggacg cctcagatga gcttgcggag ttcgagatcg gcgcctcgtc cagcagcagc 660

acggaagctc tcatctcggc cgtgatggct gggaggagca tctccccgtg gctggcacgg 720

cgaacgagtc ctgccaattc ttccggagac tcggccatcg ccagctgtca cgacggaggg 780

agcagctacg ggaaagagga tcaggagccc aaggccgatg gccctgatga tgtttcttca 840

cagcctctat ggcctggaga cgtgggctac gggcctctgc gcatcaagga agagcaggtt 900

tcaccgtctc agtacggagg gagcgagctg ccttctgcca aggacggtgc agtacagaac 960

tctttctcag agcagagtgc tggtgatgcc tggcagccca cgggccgaag gaagaatcgg 1020

aaaaacaaag agaccgtccg gcacatcaca cagcaggtgg aagatgacag ccgggccagc 1080

tccccggtgc cgtccttcct gccgacgtcg gggtggccgt tcagcagccg agactcaaat 1140

gcggacctgt ccgtcaccga agccagcagc tccgacagcc gaggagagag ggccgagctc 1200

tatgcacagg tggaggaggg tctcctggga ggagaagcca gctatctggg ccctcccctc 1260

accccagaga aggacgacgc cctgcatcag gccaccgcgg tggccaacct gcgcgcggcg 1320

ctcatgagta agaacagcct gctgtcgctg aaggccgacg tgctggggga tgacggctcc 1380

ctgctgttcg agtacctgcc cagaggggcc cactcgctgt ccctgaatga gttcacggtg 1440

atcaggaaga agttcaagtg tccgtactgc agcttctcgg ccatgcacca gtgcatcctc 1500

aagcgacaca tgcgctcgca cacgggagag cggccctacc cctgcgagat ctgcgggaag 1560

aagttcacgc ggcgcgagca catgaagcgc cacacgctgg tccacagcaa ggacaagaag 1620

tatgtgtgca aggtgtgcag ccgcgtcttc atgtccgccg ccagcgtggg catcaggcat 1680

ggctccaggc gccacggtgt gtgcaccgac tgtgctggcc gcggcatggc cgggcccctg 1740

gaccatggcg gcggaggcgg cgagggctct ccagaggcgc tgttcccagg cgacggcccc 1800

tatctggagg accctgagga cccacgaggg gaggcggagg agctgggcga ggacgacgag 1860

ggcctggccc ctgaggatgc gctgttggcg gacgacaagg atgaggaaga ctcgccgcgg 1920

ccgcgcagcc ccccaggagg ccctgacaag gacttcgcct ggctctccta ggcccgcccg 1980

ccggcagggt cggtggctgc ctcgctccgt ccacccgtgt gtgtgtccgg tgggtctcca 2040

ctgcggggcc agggccacgc tcacccctct cgcggcccct cctctgcttc cccctgaacc 2100

caccccccac ggaaaccagc cctgcgggct aagcaggtgc gaccccagca agaggggtgc 2160

tctgggacca gacatgaagt gagttggggg agggcacagg gtgggtttga gtgaagggag 2220

agcacggtcc taagtcccca gcaggtggtg cgggtgtgtg agtggcccct gtgatggcca 2280

gcctggcttg gacacgtgat gggcctgtgg ccgggtccag tgggcactgg gcggggtggt 2340

gtgttcggcc cagaggcccc tggcctgagc aggtgcagag ttttacagac acccggtcag 2400

cccggctgga gcccgccctc ccctccccta gcagccaggt cactgcctgt ggctgcagcc 2460

gtggcccgtg gtgcagccgt ggcccatggc agcctgtaga atccaggtgc acagagagcc 2520

ctgccaccgt cttacctctg ggctttggtg cttaacacac aacacagctg cagaccctgc 2580

tggaggccga gggctccagg tgctatctta ggtggacaca gccctggggg ctccttccag 2640

gaggggaccc tcagccctgt gccccccacc acttcaggcc acaccaggtt ccctctgcaa 2700

gggcctcggc tcagtcgtgt gcacttcctc ggagagcctt gggctgccac gtccaccccg 2760

ggctctgccc gtcctgttct gcccatggcc cagcccggcc gctcctgctg acccctcctg 2820

gacgggctgg agctgggctc ctgcctttgc tgctaacact ggaggcggtg ttcctaactg 2880

cagtgtgctg cttacacctc cccgcgtggg taaccaaatt tttaagtagt cagagacata 2940

tcgaggtagt tacataaaat tattttgttt ggcattattt ttctcactcg aagaaactat 3000

atagggttgt tttcctttta gcttgtgctc aagtcctctt gctgtgtttt cagaagcact 3060

cacatgttct ttcttttcct gagtgaaaag caaaggtccc acggtgtgtg ctgtggtgca 3120

ccgcctggct ttgggggtcc cggaggcagg ctgcctagac tcacagcctc gggaccgttg 3180

ccacggcctg tcttctcgtt caggcctgcc tctgacagca ctcaccatga ggacattcca 3240

tccttcaccc cctcctctgg cacaggccac cactgcggtg ctgtgccttc agatgggagg 3300

tgggcgcggt ggcctcctcc ttccctccag gacctgcccg tgtgaagacc ccccggagtg 3360

ctgagcttca gggctgcgtg gaaagagttt ttactctctt tttctagcct gtataccagg 3420

cttttcccca cattgtcagg tagagcacca gcttccctga ccgctgctgc tcggggaggg 3480

ctggggctgg ccgggggtcc tgtggaggag tacatggagg actccaggta cagcgcagga 3540

gtcacggctt ttgttttttg acattggccc ccggttctac caatgacagg gtgccctggc 3600

tggagctgtc atcacacaca cccctcagct cggaggctgt gggctcctca aagctggaga 3660

aagaggccaa gatttttctg cacacggagt gtggggatag gagccgggcc aagcgctggc 3720

ccctcagcgg tgagccctgc ccactcttac cgagcaaggt gggtggctct ggcacgagtc 3780

ccccaggggg agagcatggc taccagggag ctgcagcgga gccctccagc cctcacccca 3840

ggccagcccc accccggcct ctttgagaat tctcagaact ttgtaccttt cccctgattt 3900

ttaaaccctt tttctaaaca gactgacttt cttacaaaat gcatttggaa accagacctt 3960

tgctacccac caatgtctct gggttttgta ccagtccctg ctctcaggcc accctgccca 4020

ggacccaggc ccgcctcccc ctccacactc aggatgtcct gctccatctg gccggctcac 4080

tccgtgtggc ctgcctttgc tgaccgtttt ggggttcccc gccggagcta caggggcatt 4140

ttcttcccta aaaccaacag tgtcccactg acctccccaa gtgtttgctg cgtggcagat 4200

ttcctgttct tgttcgcagt ttgccgactg aagagtgtgg gatttccgag gcccaggtga 4260

gcacgtccat ctcaggaggc gtggagggaa aagacatgtc atgaagggtt ttttttatgt 4320

gactgatttt tttttaaatc gatgttcaaa ctaataaata tttttttatg aagaggaaaa 4380

atgtgtagat tacatttcac attttgtatt tttgtttgtg tctgtttgta ttttggtgtt 4440

tacaacacca aagtgggaaa tacagtccat tggggatggt gttatttggg ggcggggagg 4500

gggcagggac accacgattt ttctgtcaag ctctggatcc tgaccaggtt gtacactggg 4560

gctctctgag ctttgggaca caggacactg ccagggctac gtagggaact gactcagaag 4620

acgcagctta ctgcttccaa ctttgcacat cttcctcttt aaaaaactga gaaaatgcaa 4680

aaactggaac tttttgcaat attataaaag aagtaatctt attttagctc attctgtgac 4740

atgtgcgact cttaagaaag ccatacttaa tggtggtggt tttttttaga tcttatattg 4800

tgttttgtat gcagcccttt tagaactact tgtagtgagg gtgctgtgtg tgcttttctt 4860

aaatatttat ttttttcaac atgctttcaa cctgtcaaca aaaacaaaac acacaaaaaa 4920

agggcagtgt ttgaagattg ttgatttttt tctggggata atctatatta tattgacttc 4980

ctattactta ttataaacct gtgtttgtat tggagatgtg tctactattg ggggaagagg 5040

ttctcgtaat cgctcggtgg gaaatcatgg ctctgccgtc ctgcctctct gtggccgtgg 5100

gttcacgtgg cctctgcggt gagtctccaa gtttctgcct aggcgcctgt gcgtttcctt 5160

tctgtgacgg gattagctta gacatccttg caaagcgatc actttcaata aattgggaaa 5220

ttgctgctcc agc 5233

<210> 24
<211> 2014
<212> DNA
<213> Mus <mouse, genus>

<220>
<223> Mouse Zbtb46 (NM_027656.2)

<400> 24
agtctataga agacacagca gcaagactcc cagatgaaca accgaaagga agatatggaa 60

atcacttctc actaccggca tctgcttcga gagctcaatg agcagaggca gcacggagtc 120

ctctgtgatg cgtgcgtcgt ggtggagggc aaggtcttca aggcacataa gaacgtcttg 180

cttgggagca gccgctactt taagacgctc tactgccagg tacagaagac atctgaccag 240

gccaccgtca ctcacttgga cattgttaca gcccagggct tcaaggccat tattgacttc 300

atgtactccg cccatctggc tctcactagt aggaatgtca tcgaggtgat gtcagctgcc 360

agcttcctac agatgactga cattgtgcag gcctgccatg atttcatcaa ggctgcactg 420

gacatcagca taaagtcaga tgcctccgat gaactctcag aatttgagat tggcacccca 480

gccagcaaca gtacagaggc gttgatctca gctgtgatgg ctggaaggag tatctcccca 540

tggttggctc ggagaacaag tcctgccaat tcttctggag actctgccat tgccagctgt 600

catgaaggag gaagcagcta tgggaaggag gaccaggaac ccaaagctga tggccctgat 660

gacgtttctt cacagtcttt gtggcctgga gatgtaggct atgggtctct gcgcatcaag 720

gaagaacaga tttcaccatc acattatgga gggagtgagc ttccatcttc caaggacact 780

gcaatacaga attctttatc agaacagggt tctggggatg gctggcagcc cacaggccgg 840

aggaagaatc ggaaaaacaa agagactgtc cgacacatca cccagcaggt ggaggaggac 900

agccaggctg gctctccagt accttcattc ctacccacat cgggatggcc tttcagcagc 960

cgagactcaa atgtagacct gacggtcact gaggccagca gcttggacag ccgaggcgag 1020

agagcagagc tctatgctca catcgatgag ggcctactag gaggagaaac cagctacttg 1080

ggcccacccc tcaccccaga gaaggaagaa gcactacacc aggctactgc agtggccaat 1140

cttcgtgctg cactcatgag taagaacagt ctgctgtcac tcaaggctga cgtgctcggt 1200

gatgatggct cacttctgtt cgagtacctg cccaaaggtg cccactcact gtctctgaac 1260

gaattcacgg tgatcaggaa gaagttcaag tgcccctact gcagcttctc agccatgcac 1320

cagtgcatcc ttaagcgaca catgcgctca catactggag agcggcccta cccttgtgag 1380

atctgtggca agaagttcac taggagagag cacatgaagc gacatactct ggtccacagc 1440

aaggacaaga agtacgtgtg caaagtgtgc agccgtgtgt tcatgtctgc agccagcgtg 1500

ggcattaagc acggctctcg tcgccatggg gtgtgtgcag actgtgctgg ccggggtgtg 1560

ggcacgccac tggaccatgg tggaggtggc gagggctccc ctgaggcgct gtttgctggt 1620

gaagggccgt acctggagga cccggatgat ccacgagggg aggctgagga ggagctggtt 1680

gaggatgagg atgaggacgt ggccaagtgg aaggatgacg tgggcttggc ccatgaggat 1740

gcactgctgg gggatgacaa ggatgatgaa gactctccac aggggcctca cagcccctct 1800

ggggagcccg ataaagactt tgcctggatc tcctaggggc tctgctctag tgggcagggt 1860

gagtgatggc tgaatcgctt tgtccacctg tgtgtgtgtc ttagtggatc tttacttacc 1920

caggagcagg gctatgctta ctccttgaag ctcctcctaa aggcctctcc tccatgctat 1980

acccctctcc attacagaaa ctggtcctgt gagc 2014

<210> 25
<211> 4340
<212> DNA
<213> Homo sapiens

<220>
<223> Human RUNX3 (NM_001031680.2)

<400> 25
attcattcat tccccgtggc actggaggcg gcccactctg ctctgtcagc ttcggagctc 60

ctccaccctg gctgccgaaa gccccttccc gccatctaat gatacactct gcatacgctt 120

ctgttgagaa tttgtggcta gacattcctg tgggaccggg aatccaaatt cttgggtaca 180

aacagaaact tactttcctt ggggattttt ttctctctct cactcacaca cactctcgcg 240

ttctttcctt ttttcttttt cgtagcagca gggggaaaaa aagagacaaa aacaaaacaa 300

aaaacaacaa aaagcaacac cccccccttt tattttcaaa agtagctaga ggaaaaaaaa 360

ataaaacaac agccaaccaa gtgaatccca acccaacccc ctgaagggct gaaaattctc 420

gccttcttca gagcggggca tggcatcgaa cagcatcttc gactccttcc cgacctactc 480

gccgaccttc atccgcgacc caagcaccag ccgccgcttc acacctccct ccccggcctt 540

cccctgcggc ggcggcggcg gcaagatggg cgagaacagc ggcgcgctga gcgcgcaggc 600

ggccgtgggg cccggagggc gcgcccggcc cgaggtgcgc tcgatggtgg acgtgctggc 660

ggaccacgca ggcgagctcg tgcgcaccga cagccccaac ttcctctgct ccgtgctgcc 720

ctcgcactgg cgctgcaaca agacgctgcc cgtcgccttc aaggtggtgg cattggggga 780

cgtgccggat ggtacggtgg tgactgtgat ggcaggcaat gacgagaact actccgctga 840

gctgcgcaat gcctcggccg tcatgaagaa ccaggtggcc aggttcaacg accttcgctt 900

cgtgggccgc agtgggcgag ggaagagttt caccctgacc atcactgtgt tcaccaaccc 960

cacccaagtg gcgacctacc accgagccat caaggtgacc gtggacggac cccgggagcc 1020

cagacggcac cggcagaagc tggaggacca gaccaagccg ttccctgacc gctttgggga 1080

cctggaacgg ctgcgcatgc gggtgacacc gagcacaccc agcccccgag gctcactcag 1140

caccacaagc cacttcagca gccagcccca gaccccaatc caaggcacct cggaactgaa 1200

cccattctcc gacccccgcc agtttgaccg ctccttcccc acgctgccaa ccctcacgga 1260

gagccgcttc ccagacccca ggatgcatta tcccggggcc atgtcagctg ccttccccta 1320

cagcgccacg ccctcgggca cgagcatcag cagcctcagc gtggcgggca tgccggccac 1380

cagccgcttc caccatacct acctcccgcc accctacccg ggggccccgc agaaccagag 1440

cgggcccttc caggccaacc cgtcccccta ccacctctac tacgggacat cctctggctc 1500

ctaccagttc tccatggtgg ccggcagcag cagtgggggc gaccgctcac ctacccgcat 1560

gctggcctct tgcaccagca gcgctgcctc tgtcgccgcc ggcaacctca tgaaccccag 1620

cctgggcggc cagagtgatg gcgtggaggc cgacggcagc cacagcaact cacccacggc 1680

cctgagcacg ccaggccgca tggatgaggc cgtgtggcgg ccctactgac cgccctggtg 1740

gactcctccc gctggaggcg gggaccctaa caaccttcaa gaccagtgat gggccggctc 1800

cgaggctccg ggcgggaatg ggacctgcgc tccagggtgg tctcggtccc agggtggtcc 1860

cagctggtgg gagcctctgg ctgcatctgt gcagccacat ccttgtacag aggcataggt 1920

taccaccccc accccggccc gggatactgc ccccggccca gatcctggcc gtctcatccc 1980

atacttctgt ggggaatcag cctcctgcca cccccccgga aggacctcac tgtctccagc 2040

tatgcccagt gctgcatggg acccatgtct cctgggacag aggccatctc tcttccagag 2100

agaggcagca ttggcccaca ggataagcct caggccctgg gaaacctccc gacccctgca 2160

ccttcgttgg agcccctgca tcccctgggt ccagccccct ctgcatttac acagatttga 2220

gtcagaactg gaaagtgtcc cccaccccca ccaccctcga gcggggttcc cctcattgta 2280

cagatggggc aggacccagc acgctgctgg cagagatggt ttgagaacac atccaagcca 2340

gtccccccag cccagcttcc cctccgttcc taactgttgg ctttccccca gccgcacggg 2400

tcccaggccc cagagaagat gagtctatgg catcaggttc ttaaacccag gaaagcacct 2460

acagaccggc tcctccatgc actttaccag ctcaacgcat ccactctctg ttctcttggc 2520

agggcggggg aggggggata ggaggtcccc tttcccctag gtggtctcat aattccattt 2580

gtggagagaa caggagggcc agatagatag gtcctagcag aaggcattga ggtgagggat 2640

cattttgggt cagacatcaa tgtccctgtc ccccctgggt ccagccaagc tgtgccccat 2700

cccccaagcc tcctgggagg atccagccaa atcttgcgac tcctggcaca cacctgtctg 2760

taacctgttt tgtgctctga aagcaaatag tcctgagcaa aaaaaaaaaa aaaacaaaaa 2820

aacaaaaaaa aaacaaaaca gtttttaaaa ctgattttag aaaaagaagc ttaatctaac 2880

gttttcaaac acaaggtctc ttacaggtat agttccgtga ttatgatagc tctgtgatta 2940

taagcaacat ccccgccccc tctccccccc gcggaccccc agctgcctcc tgagggtgtg 3000

gggttattag ggtctcaata ctttctcaag gggctacact ccccatcagg cagcatccca 3060

ccagcctgca ccacaggctc ccctgggagg acgagggaaa cgctgatgag acgctgggca 3120

tctctcctct gtggctctag gacatctgtc caggaggctg ggcggaggtg ggcaggatgt 3180

gagaggtggg gagtactggc tgtgcgtggc aggacagaag cactgtaaag ggctctccag 3240

ccgcagctca gctgcactgc gttccgaggt gaagtcttgc ccctgaattt tgcaaaatgg 3300

gaaagtgggc gcttgcccaa gggccaggct gcatggattc tcacatcaga gttctctggc 3360

cctagaaagg cttagaaaag gcgtaaggga actcataaag gctagcagca tgcggtattt 3420

taactttctg cctcggcctc tgtggatgca gaaatctgcc ctacaaaatg ctcttcattg 3480

gttgtctctg tgagagcact gtccccaccc aacctgtcac aacggccaga accatacacc 3540

agagacacac tggcaggtta ggcagtcctt ctggtgatcc tattccattc cctcctgctg 3600

cggtttctct tggcctgtcc tcactggaaa aacagtctcc atctcctcaa aatagttgct 3660

gactccctgc acccaagggg cctctccatg ccttcttagg aagcagctat gaatccattg 3720

tccttgtagt ttcttccctc ctgttctctg gttatagctg gtcccaggtc agcgtgggag 3780

gcacctttgg gttcccagtg cccagcactt tgtagtctca tcccagatta ctaacccttc 3840

ctgatcctgg agaggcaggg atagtaaata aattgctctt cctaccccat cccccatccc 3900

ctgacaaaaa gtgacggcag ccgtactgag tctgtaaggc ccaaagtggg tacagacagc 3960

ctgggctggt aaaagtaggt ccttatttac aaggctgcgt taaagttgta ctaggcaaac 4020

acactgatgt aggaagcacg aggaaaggaa gacgttttga tatagtgtta ctgtgagcct 4080

gtcagtagtg ggtaccaatc ttttgtgaca tattgtcatg ctgaggtgtg acacctgctg 4140

cactcatctg atgtaaaacc atcccagagc tggcgagagg atggagctgg gtggaaactg 4200

ctttgcacta tcgtttgctt ggtgtttgtt tttaacgcac aacttgcttg tacagtaaac 4260

tgtcttctgt actatttaac tgtaaaatgg aattttgact gatttgttac aataatataa 4320

ctctgagatg tgtggaagga 4340

<210> 26
<211> 3891
<212> DNA
<213> Mus <mouse, genus>

<220>
<223> Mouse Runx3 (NM_019732.2)

<400> 26
attcattcat tcatccaggc tgctgagtct ggactggctg ctgcagcccc gggctcctcc 60

gccctgactg cccagagccc cttcccacca tttaatgata ctctgcacat agtgctgttg 120

aaaatttgtg gctagacatt cctgtgggac tgggaagcca aattcttggg tacaaaccga 180

aactttcttt ccttggagat tttcttctct ctcgcccaca gacgctctcg cgttctttcc 240

ccattttttt tctttctctt cttttttttc tttttttttt tttttttgta gcagcagggg 300

gagaaaaaaa gaaaccaaac tttaaaaaaa aaaagtcaac acctcccctt ttttattttc 360

gaaagcaact tgaaaaaata tatataacaa aacagcagcc aaccaagtgg gtctgaaccc 420

aaccccctga ggccgagtcc ttgccactgt cagcgtgcga catggcttcc aacagcatct 480

ttgactcctt ccccaactat acaccaacct tcatacgaga cccgagcacc agccgccgct 540

tcactccccc ctccacggcc ttcccctgcg gcggcggcgg cggcggcaag atgggcgaga 600

acagcggcgc gctaagcgcg caggcaaccg cgggccccgg cggccgcacc cggcccgaag 660

tgcgctcgat ggtggacgtg ctggccgacc acgcgggaga gctcgtgcgc accgacagcc 720

ccaacttcct ctgctccgtg ctgccctcgc actggcgctg caacaagacg ctgccggtcg 780

ccttcaaggt ggtggccctg ggggatgtgc cggatggaac ggtggtgacc gtgatggccg 840

gcaatgatga gaactactcc gccgagctgc gcaacgcttc cgctgtcatg aagaaccaag 900

tggccaggtt caacgacctt cgattcgtgg gccgcagtgg gcgagggaag agtttcacgc 960

tcacaatcac cgtgttcacc aaccctaccc aagtggctac ctaccaccga gccatcaagg 1020

tcactgtgga tggaccccgg gaaccccgac ggcaccggca gaagatagaa gaccagacca 1080

aggccttccc cgaccgcttt ggagacctgc gcatgcgtgt aacaccaagc acacccagcc 1140

cccgtggctc tctcagcacc acgagccact tcagcagcca ggcccagacc ccaatccaag 1200

gctcctcaga cctgaacccc ttctccgacc cccgccagtt tgaccgctcc ttccctacgc 1260

tgcagagcct cacagagagc cgcttcccgg accccaggat gcactacccg ggagccatgt 1320

ctgccgcctt cccctacagc gccacaccat cgggcaccag cctgggcagc ctgagcgtgg 1380

cgggcatgcc ggccagcagc cgcttccacc acacctacct ccctccgccc taccccgggg 1440

ccccacagag ccagagcggg ccctttcagg ccaaccccgc gccctaccac ctcttttacg 1500

gcgcctcctc cggctcctac cagttctcca tggcagccgc gggaggtggt gagcgctcgc 1560

ccacccgcat gctgacctcc tgccccagcg gcgcttcggt gtcagcaggc aacctcatga 1620

accccagcct gggccaggct gatggcgtgg aagccgacgg cagccacagc aactcgccca 1680

cggccctgag cacgccgggc cgcatggacg aggccgtgtg gcggccctac taagcaccct 1740

ggagactctc gggccagccg aatccttccc tccagcccga gactacaaga agaaacagac 1800

tttggcctgg tcccaggggc cagagctggc agtggctccc agagctctgt ccagccacaa 1860

gtttgagcag aggatgtggg gacctcccac aggacactgc tccaacccaa atcaatgtcc 1920

tctctagctg tgcatcccaa acccacctct tgtctccagc catgcccagg atcaattcta 1980

gaatcagagg ctacccctgc ctgtgtgggg agggcctttg ctggcaagag aagctcaggc 2040

tcagagatgt ctggactttc tctccctccc tctctctctc tctctctctc tctctctctc 2100

tctctctctc tctctctctc acacacacac acacacacac acacacacac acacacacac 2160

actgccctgg ggaccatcca tgcccatcaa accaaaggga aactcaaacc cctgggaagg 2220

tttcactccc ctgaagagag agaggagcca tatctctctt tcacatacac acacacacac 2280

acacacacac acacacacac acacgtcccc aacttgactt ccccctggtc ccaggccagc 2340

agcccctgat cagtcctaac ttacctgaaa acaccaggcc ccagagcaga ctcatccggg 2400

gtggcaggtc cctaaagcaa ggaaggcctc acactgtcca gcatactcca gcctgaattg 2460

cctactgggt tctcaggggg cagacaggtc ctggccgaaa gtcttaccat ggggatcgat 2520

agggtggtta gccctgcgca ctaggtccag ccaatctgtg ctctgggcct tgcctcccag 2580

gccacagtcc tgctcccaac ttgtttgtcc ctagaagcag acaactttga accaaaagca 2640

tctcagtcaa gcatctaaaa cagggggcag tctgtgtata caagagccca cctcctgctg 2700

tgtgatgtca tcaggacacc cccaccctca ccccaccacc agggtcctga cttcacattc 2760

ttcacagaag gggacaagac ctccaggagg ccctgtggag tctctggtga ggtgtgcgat 2820

ggcagagcag catccaagca gagatcagat gtcctcttca gtctgtgggc gggcgattgt 2880

cagtgggatt ggttaaaaag ctcttaccag agctggctgg cccacggctg gtttttcaaa 2940

ctgagtgagc cccgtagtga tggcctctgc cttgtgcttg gccttcccca ttgcctgcac 3000

ctgaaacagc cacccacata cacactctgg taggtcaggg ggtccccgac aatcctggac 3060

aatcctgagc agaatacctc ctgttacagt gtctcttgtc ccacccttac tggaggaaaa 3120

aaaaaaaaaa aactctatct cttggaagta gctgaggtaa ccttccagct ccctgcaccc 3180

agagggggcc ttctcacctc ttctcaggaa gcagctacga cccattgccc tcctacctgc 3240

ttcgctactg gtccatggta cagactgttc cagatcacca agaaaggtac tttcaggatc 3300

ccagtgccta gcactgggta agctacccca gacaactagc cttccttata cccaaacgct 3360

ggtcggtgga aatagaaagt cacccctccc ccatcgcctc tttctcatcc ctgattaaca 3420

gtgaagtttg agtcttgtcg tgagtcctac agtgagcaca ggcagctggg gctaataaaa 3480

aaagaagaag aagaagaaaa aggagaggag agaaaaaaaa caaaaatgaa aggaaaaggg 3540

tccttatttg caaggctact ttaaagtgct acttgaaagc acaggctaga cagaaagcac 3600

aaggaaccta gggggttgat acaatgttac tgtaattgtg tgggtgggtg tcatcctttg 3660

tgacattgtt accctgaggt atactccctg cagtgagctg gtcttcctga agtgggcctg 3720

ctttgcacta taggctgctt gtatgtttgg tttgttgttt ttgttttttt gtttttttta 3780

atgcacaact tgcttgtaca ggtcaactgt ccatcttggg tactatttaa ctgtcaaaca 3840

gaggaatctg tgtttgttac aataatataa cagatgcctt gaaggacccg c 3891

<210> 27
<211> 2802
<212> DNA
<213> Homo sapiens

<220>
<223> Human GFI1 (NM_005263.3)

<400> 27
gagggtgcgc ccaccggtcc cgccgggcgc ccgcgggacg cgccgccagg gccctctccg 60
ccgggggctc ggcgctcgcc cacctcttcc aaatttaacc attacctaaa tccgaaggga 120
aatgagcaaa cctctcggat tgggtgtcaa ggtctcctcc gggctggggc tgagcaagcc 180
ctcggagtga ccgtgggtga cagcggctcc agggactctt ggggcgcagt ggggaaagtg 240
ccggaccacc atgccgcgct catttctcgt caaaagcaag aaggctcaca gctaccacca 300
gccgcgctcc ccaggaccag actattccct ccgtttagag aatgtaccgg cgcctagccg 360
agcagacagc acttcaaatg caggcggggc gaaggcggag ccccgggacc gtttgtcccc 420
cgaatcgcag ctgaccgaag ccccagacag agcctccgca tccccagaca gctgcgaagg 480
cagcgtctgc gaacggagct cggagtttga ggacttctgg aggcccccgt caccctccgc 540
gtctccagcc tcggagaagt caatgtgccc atcgctggac gaagcccagc ccttccccct 600
gcctttcaaa ccgtactcat ggagcggcct ggcgggttct gacctgcggc acctggtgca 660
gagctaccga ccgtgtgggg ccctggagcg tggcgctggc ctgggcctct tctgcgaacc 720
cgccccggag cctggccacc cggccgcgct gtacggcccg aagcgggctg ccggcggcgc 780
gggggccggg gcgccaggga gctgcagcgc aggggccggt gccaccgctg gccctggcct 840
agggctctac ggcgacttcg ggtctgcggc agccgggctg tatgagaggc ccacggcagc 900
ggcgggcttg ctgtaccccg agcgtggcca cgggctgcac gcagacaagg gcgctggcgt 960
caaggtggag tcggagctgc tgtgcacccg cctgctgctg ggcggcggct cctacaagtg 1020
catcaagtgc agcaaggtgt tctccacgcc gcacgggctc gaggtgcacg tgcgcaggtc 1080
ccacagcggt accagaccct ttgcctgcga gatgtgcggc aagaccttcg ggcacgcggt 1140
gagcctggag cagcacaaag ccgtgcactc gcaggaacgg agctttgact gtaagatctg 1200
tgggaagagc ttcaagaggt catccacact gtccacacac ctgcttatcc actcagacac 1260
tcggccctac ccctgtcagt actgtggcaa gaggttccac cagaagtcag acatgaagaa 1320
acacactttc atccacactg gtgagaagcc tcacaagtgc caggtgtgcg gcaaggcatt 1380
cagccagagc tccaacctca tcacccacag ccgcaaacac acaggcttca agcccttcgg 1440
ctgcgacctc tgtgggaagg gtttccagag gaaggtggac ctccgaaggc accgggagac 1500

gcagcatggg ctcaaatgag caccctggct ggctgcaagc agcagctaca caacactaca 1560

gagggcagcc tccctgcttg ccaccactct gctccctgct tgcctccact cccttctgac 1620

tttccagacc ccaggtccag tctgcagatc ctaccaggtt gctcctcctt cgccttacct 1680

cctggagctg ccagaagaaa tgaggtacct tttcaaagtg cagccgagag tgagaaccaa 1740

gtgactctct aggcttcgga cacaaatagg ctcctctaca cctgaagaca aaggcaaagt 1800

caaatgggga ccagaataaa tcttagaccc cacagtcctt cccatttcca gccctaatct 1860

acagacagga atgcccttca ggtttcttcc ctcccccctc ttgacctacc ccagatattt 1920

gtgtggaaga ggaggaatca ccatttacaa ggtggacaaa tgctaatatt tttatctaga 1980

aagaagagtg agtgttaact tttatttttt tccttctggg gggtctgttg actcctttct 2040

tttgggtgct gcctataaat cttggaggaa tcatttctcc tcctcaaaaa ctgattcaga 2100

aactgacttg gggaaggaat ttaatacttt gaagtcatga gatgcaccat cgaggctacc 2160

cccaagaaga agcagaagag aagttggtaa tgagagggga ttagaggtcc tcccttcagt 2220

agggctgtga aaacctcatc actggaggta aaagcacaag caatgcctgt ggacaagatg 2280

tcattcattc actcagcaaa tgttcatgga tcaccggcta ccaaggtacc aggcaccatg 2340

ctaggtattg gggaagagag actgaagtca caacccctga ctgctcctca aaagctaacg 2400

gttgcacctc caagtggctg ggtctgttct tactcttgga gggaattctg agaagacagc 2460

acagaattgt aaaccttccc ttttgaccct tttggatttt atcaggtgta aacaaaaagc 2520

tgaacagtta cttcaaagat atgtgtgtat attcagtttt ttattgttaa gctgatattt 2580

taaagatttc tgagctagca ggcatgtggg aaggaaggct ctgtcttcaa ctctttgacc 2640

ctccatgtgt accatagagg ggggaaaggt ggtattttca ctttgatgag gttggtaaat 2700

gtttttagat cttctggtaa gcattatgtt tgttaataca tatttattag agtgatgttt 2760

taagttaata aagtattaag agtattaaaa aaaaaaaaaa aa 2802

<210> 28
<211> 2862
<212> DNA
<213> Mus <mouse, genus>

<220>
<223> Mouse GFI1 (NM_010278.2)

<400> 28
ctccaagccc cttccgggtc ccaaacactg atgccccctg actggctaaa ctaagccacg 60

catccctggc gtgagccaca acccagaagg gagcaggtgg gcttgcaggg ttgacttggg 120

ataacggacc agttgtggac tactgctctc aggagagtga tgatctagct ttggtaggga 180

aggggagggg ctgaggcgtg ggcagggcag agcaaaggga ccagagccag agcctgggga 240

caggttttac cactgagctg ttgcagtggc ggcggaggcc gggattcgtg ccacctgtcc 300

gagtgccacc tggtgagcgt ggcgcctggg tccaggcccc tcctcccgcg gcttccctct 360

ctcctccctg gcccacactc ttccttggcc tgggaaccta ccacaaccgc catcggtgct 420

gaccctcgtt tccacccaat tttccccctt ctctcagaac tcagagtatc cgagggtcca 480

aacattcgtc cagcggctga ccaccatgcc gcgctcattc ctggtcaaga gcaagaaggc 540

gcacagctat caccagccgc gttctccggg gccggactac tccctgcgcc tggagaccgt 600

gcctgcgccg ggcagagcag agggcggcgc tgtgagtgca ggcgagtcga aaatggagcc 660

ccgagagcgt ttgtcccccg actctcagct taccgaggct cccgacaggg cctccgcgtc 720

ccccaacagc tgcgaaggca gcgtttgtga cccctgctcc gagttcgagg acttttggag 780

gcccccttct ccctccgtgt ctccagcgtc ggagaagtca ctgtgccgct ctctggacga 840

agcccagccc tacacgctgc ctttcaagcc ctatgcatgg agcggtcttg ctgggtctga 900

cctgcggcac ctggtgcaga gctatcggca gtgcagcgcg ctggagcgca gcgcgggcct 960

gagcctcttc tgcgagcgcg gctcggagcc gggccgcccg gcagcgcgct acggccccga 1020

gcaggctgcg ggcggagccg gtgcgggaca gccagggagc tgcggggtcg ccgggggcgc 1080

caccagcgct gcgggcctgg ggctctacgg cgacttcgcg cctgcggcgg ccgggctgta 1140

cgagcggccg agcacagcag caggccggct gtaccaagat catggccacg agctgcacgc 1200

ggacaagagc gtgggcgtca aggtggagtc ggagctgctt tgcacccgtc tgctgctggg 1260

cggcggctcc tacaaatgca tcaaatgcag caaggtgttc tccacaccgc acgggctgga 1320

ggtgcacgtg cgccggtccc acagcggcac aagacccttt gcgtgcgaga tgtgcggcaa 1380

gaccttcggg cacgcggtga gcctggagca acacaaggca gtgcactccc aggaacgcag 1440

ctttgactgt aagatctgtg gcaagagctt caagaggtca tccacgctgt ccacacatct 1500

gctcattcac tcggacaccc ggccctatcc ctgtcagtac tgtggcaaaa gattccacca 1560

gaagtcagat atgaagaaac acaccttcat ccacacaggt gagaagcccc acaaatgcca 1620

ggtgtgcggc aaagccttca gtcagagctc caacctcatc actcatagca gaaagcacac 1680

aggcttcaag ccctttggct gtgacctgtg tgggaagggc ttccagagga aggtggatct 1740

caggaggcac cgagagactc agcatggact caaatgagta ccctggcagc ccgcaacacc 1800

agctgtgtaa cactaccgtg agggatgtct tccctgcctc cctccagccc cttctcaggc 1860

cctgagtcca gtgtgcaaag ctcatcatgg ttagtcccct tcaccttcct tcccggagct 1920

gctggaggag atgaactccc gtttctaagg tcaacccaga gtgggaaccg cagcagcagc 1980

agcagtcgtc tgtgctttgg gcttccctac agctgaagat ggggatcaaa tgagatcttg 2040

cacctcccag ttcttccctt ttttgctgtc tcacaggcca gaatgaactc tgggcagctg 2100

ctacaagagg aggcatcacc tcttaagctt tgagcgccac tgatgcattt attgagaacg 2160

aatgaacatt aattttctct tctggggaga ctgctgactc ctttatcctc caccagactc 2220

tggtttaggg aaggaacctc gttcctttga aatcatcaga tgcaccatca agcctgccac 2280

gagaagaagg ggacttggtg atgagaggga gtcagaggtc ctgtggtgcc atcagaggag 2340

gtgaagctgt ggagcagctc cggggaacag ggtcttcact tactcagcga gtgattattg 2400

gccgcaggtt atcagagtaa ggaactgtgc taggtatggg agagccagag attaaagtca 2460

taggccctaa cccccaaaag ctatcagttg gacttcaaca tagctagagc ctgtgttctg 2520

tgcttccaag ggagttctga agaaggccac acaaacattg ggacttcttt ttgacactta 2580

cggagttttt caagtgtaaa caaataggaa ctggaggatt atttctaaag ttcatgagta 2640

aatccagctt ttattgttag gtgggacttt attagatgcc tgcgctggga gatgtggggt 2700

gaaggctatg gcctcagctc ctgcccctta tcatcttagg aacaacttat ttttcctgtg 2760

agggttgtag acgttcctaa atcttcttga gtgcattatg tattagcata atcatattta 2820

ttagaatgtt gttttaactt aataaagtat taagattatt ac 2862

<210> 29
<211> 2302
<212> DNA
<213> Homo sapiens

<220>
<223> Human IRF2 (NM_002199.3)

<400> 29
agccgcggct cttcgcagtt tcctctcctt gttttgcttt cgatctggac tgttctcagg 60

caagccgggg agtaactttt agttttgctc ctgcgattat tcaactgacg ggctttcatt 120

tccatttcac ataccctagc aacacttata ccttgcggaa ttgtattggt agcgtgaaaa 180

aagcacactg agagggcacc atgccggtgg aaaggatgcg catgcgcccg tggctggagg 240

agcagataaa ctccaacacg atcccggggc tcaagtggct taacaaggaa aagaagattt 300

ttcagatccc ctggatgcat gcggctagac atgggtggga tgtggaaaaa gatgcaccac 360

tctttagaaa ctgggcaatc catacaggaa agcatcaacc aggagtagat aaacctgatc 420

ccaaaacatg gaaggcgaat ttcagatgcg ccatgaattc cttgcctgat attgaagaag 480

tcaaggataa aagcataaag aaaggaaata atgccttcag ggtctaccga atgctgcccc 540

tatcagaacg gccttctaag aaaggaaaga aaccaaagac agaaaaagaa gacaaagtta 600

agcacatcaa gcaagaacca gttgagtcat ctctggggct tagtaatgga gtaagtgatc 660

tttctcctga gtatgcggtc ctgacttcaa ctataaaaaa tgaagtggat agtacggtga 720

acatcatagt tgtaggacag tcccatctgg acagcaacat tgagaatcaa gagattgtca 780

ccaatccgcc agacatttgc caagttgtag aggtgaccac tgagagcgac gagcagccgg 840

tcagcatgag cgagctctac cctctgcaga tctcccccgt gtcttcctat gcagaaagcg 900

aaacgactga tagtgtgccc agcgatgaag agagtgccga ggggcggcca cactggcgga 960

agaggaatat tgaaggcaaa cagtacctca gcaacatggg gactcgaggc tcctacctgc 1020

tgcccggcat ggcgtccttc gtcacttcca acaaaccgga cctccaggtc accatcaaag 1080

aggagagcaa tccggtgcct tacaacagct cctggccccc ttttcaagac ctcccccttt 1140

cttcctccat gaccccagca tccagcagca gtcggccaga ccgggagacc cgggccagcg 1200

tcatcaagaa aacatcggat atcacccagg cccgcgtcaa gagctgttaa gcctctgact 1260

ctccgcggtg gttgttgggg cttcttggct ttgttttgtt gtttgtttgt attttatttt 1320

tttctctctg acacctattt tagacaaatc taagggaaaa agccttgaca atagaacatt 1380

gattgctgtg tccaactcca gtactggagc ttctctttaa ctcaggactc cagcccattg 1440

gtagacgtgt gtttctagag cctgctggat ctcccagggc tactcactca agttcaagga 1500

ccaacaaggg cagtggaggt gctgcattgc ctgcggtcaa ggccagcaag gtggagtgga 1560

tgcctcagaa cggacgagat aatgtgaact agctggaatt ttttattctt gtgaatatgt 1620

acataggcag cactagcgac attgcagtct gcttctgcac cttatcttaa agcacttaca 1680

gataggcctt cttgtgatct tgctctatct cacagcacac tcagcacccc cttctctgcc 1740

cattccccag cctctcttcc tatcccatcc catcccatcc catcccatcc catcccatcc 1800

cgctcttttc ctacttttcc ttccctcaaa gcttccattc cacatccgga ggagaagaag 1860

gaaatgaatt tctctacaga tgtcccattt tcagactgct ttaaaaaaaa tccttctaat 1920

ctgctatgct tgaatgccac gcggtacaaa ggaaaaagta tcatggaaat attatgcaaa 1980

ttcccagatt tgaagacaaa aatactctaa ttctaaccag agcaagcttt tttatttttt 2040

atacagggga atattttatt caaggtaaaa ttctaaataa aatataattg ttttttatct 2100

tttctacagc aaatttataa ttttaagatt ccttttcttg tttatcagca gttgttatta 2160

catccttgtg gcacattttt ttttttaatt ttgtaaaggt gaaaaaagct tttatgagct 2220

catgtagcaa tcagattttc ctgtggattg ataataaatg aatatgatat atagttaaat 2280

ttttaaaaaa aaaaaaaaaa aa 2302

<210> 30
<211> 2484
<212> DNA
<213> Mus <mouse, genus>

<220>
<223> Mouse Irf2 (NM_008391.4)

<400> 30

gctcctcgca gtttcctctc cttgttttgc tttcgatctg gactgttctc aggcaagccg 60

gggactaact tttagttttg ctcctgcgat tattcaactg acgggctttc atttccattt 120

tacacaccct aacaacactc acaccttgcg ggattgtatt ggtagcgtgg aaaaaaaaaa 180

gcacattgag agggtaccat gccggtggaa cggatgcgaa tgcgcccgtg gctggaggag 240

cagataaatt ccaatacgat accagggcta aagtggctga acaaggagaa gaagattttc 300

cagatcccct ggatgcatgc ggctcggcac ggatgggacg tggaaaagga tgctccgctc 360

ttcagaaact gggcgatcca tacaggaaag catcaaccag gaatagataa accagatcca 420

aaaacatgga aagcaaattt tcgatgtgcc atgaattccc tgcccgacat tgaggaagtg 480

aaggacagaa gcataaagaa aggaaacaac gccttcagag tctaccggat gctgccctta 540

tccgaacgac cttccaagaa aggaaagaaa ccaaagacag aaaaagaaga gagagttaag 600

cacatcaagc aagaaccagt tgagtcatct ttggggctta gtaatggagt aagtggcttt 660

tctcctgagt atgcggtcct gacttcagct ataaaaaatg aagtggatag tacggtgaac 720

atcatagttg taggacagtc ccatctggac agcaacattg aagatcaaga gatcgtcact 780

aacccgccag acatctgcca ggttgtagaa gtgaccactg agagtgatga ccagccagtc 840

agcatgagtg agctctaccc tctacagatt tctcctgtgt cttcctacgc agaaagcgaa 900

actaccgaca gtgtggccag tgatgaagag aacgcagagg ggagaccaca ctggaggaag 960

aggagcatcg aaggcaagca gtacctcagc aacatgggga cacggaacac ctatctgctg 1020

cccagcatgg cgacctttgt cacctccaac aagccagatc tgcaggtcac catcaaagag 1080

gatagctgtc cgatgcctta caacagctcc tggcccccat ttacagacct tccccttcct 1140

gccccagtga cccccacgcc cagcagcagt cggccagacc gggagacccg ggccagtgtc 1200

atcaagaaga catctgatat cacccaggcc cgtgtcaaga gctgttaagc ctttgactct 1260

ccctggtggt tgttgggatt tcttagcttt gtgttgttct ttgtttgtat tatattattt 1320

tttttctcta tgatacctat cttagacaca tctaagggag aaagccttga cgatagatta 1380

ttgattgctg tgtccaactc cagagctgga gcttcttctt aactcaggac tccagccccc 1440

cccccccctc ggtagatgcg tatctctaga acctgctgga tctgccaggg ctactccctc 1500

aagttcaagg accaacagcc acacgggcag tggaggtgct gcgttgccta cggtcaaggc 1560

cagcatggtg gagtggatgc ctcagaacgg aggagaaaat gtgaactagc tggaattttt 1620

ttattcttgt gaatatgtac atagggcagt acgagcaatg tcgcgggctg cttctgcacc 1680

ttatcttgaa gcacttacaa taggccttct tgtaatcttg ctctccttca cagcacactc 1740

ggcgacccct tctgtgtcca ctaccccact acccacccct ccctcctcaa cccctccatc 1800

ccggtcctct atgcgcccct tccccccaac caatcccatc acaacctctt acctatcctt 1860

tccctcccaa ccccttctat cccagcccac cacctacccc actcctcccc aactcctcca 1920

ttctagccca ttacccacgc ctctctcctc agcccagcct accccatccc accctgttcc 1980

tttcctccag tttcctctcc tcaaaggcaa ggctctacat cttggaggag gaggaggaga 2040

agaaaatgag tttcttcacc gctgtcccat tttaagactg cttgaataat aaaaaaaaaa 2100

tctttctaat ctgctatgct tgaatggcac gcggtacaaa ggaaaactgt catggaaata 2160

ttatgcaaat tcccagatct gaagacggaa aatactctaa ttctaaccag agcaagcttt 2220

tttatttttt tatacaaggg gaatatttta ttcaaggtaa aaaaattcta aataaaatat 2280

aattgttttt tatcttttct acagcaaatt tataatttta agattccttt tcctgttcat 2340

cagcagttgt tattacatcc cttgtggcac attttttttt taattttgta aaggtgaaaa 2400

aaaaactttt atgagctcat gtagcaatca aattatcctg tggattgata ataaatgaat 2460

atggtatata gttaaagatt ttaa 2484

<210> 31
<211> 2247
<212> DNA
<213> Homo sapiens

<220>
<223> Human NFIL3 (NM_001289999.1)

<400> 31
aattgtgcag ggggcggtgt ttgtgcgtgg agctttccct cccggctccg ggccgtcgcg 60

gctctcggga gaggcgccgg gacattttaa tcgctgcctc cgccgcgcag ccctgcgcag 120

ctgcccggcc gcgccaaccc cttccccgcc gcagcgcgcc ccgagtgttg gcagcttgcc 180

agccgccacc ccccgccttc cctcctgccc accccaaggt agagggctcc tctcgggagt 240

gtgcggggaa ggggaggccg aggtccgggc cacgcccggg tagccgcaac ccgcagtgct 300

cagtcggcaa caggtagccc agcaggctgc ggctctcagg aagacaaaaa gcgcctctgc 360

gagcaaataa cgaaggaggc ccaacttcat tcaataagga gcctgacgga tttatcccag 420

acggtagaac aaaaggaaga atattgatgg attttaaacc agagttttta aagagcttga 480

gaatacgggg aaattaattt gttctcctac acacatagat agggtaaggt tgtttctgat 540

gcagctgaga aaaatgcaga ccgtcaaaaa ggagcaggcg tctcttgatg ccagtagcaa 600

tgtggacaag atgatggtcc ttaattctgc tttaacggaa gtgtcagaag actccacaac 660

aggtgaggag ctgcttctca gtgaaggaag tgtggggaag aacaaatctt ctgcatgtcg 720

gaggaaacgg gaattcattc ctgatgaaaa gaaagatgct atgtattggg aaaaaaggcg 780

gaaaaataat gaagctgcca aaagatctcg tgagaagcgt cgactgaatg acctggtttt 840

agagaacaaa ctaattgcac tgggagaaga aaacgccact ttaaaagctg agctgctttc 900

actaaaatta aagtttggtt taattagctc cacagcatat gctcaagaga ttcagaaact 960

cagtaattct acagctgtgt actttcaaga ttaccagact tccaaatcca atgtgagttc 1020

atttgtggac gagcacgaac cctcgatggt gtcaagtagt tgtatttctg tcattaaaca 1080

ctctccacaa agctcgctgt ccgatgtttc agaagtgtcc tcagtagaac acacgcagga 1140

gagctctgtg cagggaagct gcagaagtcc tgaaaacaag ttccagatta tcaagcaaga 1200

gccgatggaa ttagagagct acacaaggga gccaagagat gaccgaggct cttacacagc 1260

gtccatctat caaaactata tggggaattc tttctctggg tactcacact ctcccccact 1320

actgcaagtc aaccgatcct ccagcaactc cccgagaacg tcggaaactg atgatggtgt 1380

ggtaggaaag tcatctgatg gagaagacga gcaacaggtc cccaagggcc ccatccattc 1440

tccagttgaa ctcaagcatg tgcatgcaac tgtggttaaa gttccagaag tgaattcctc 1500

tgccttgcca cacaagctcc ggatcaaagc caaagccatg cagatcaaag tagaagcctt 1560

tgataatgaa tttgaggcca cgcaaaaact ttcctcacct attgacatga catctaaaag 1620

acatttcgaa ctcgaaaagc atagtgcccc aagtatggta cattcttctc ttactccttt 1680

ctcagtgcaa gtgactaaca ttcaagattg gtctctcaaa tcggagcact ggcatcaaaa 1740

agaactgagt ggcaaaactc agaatagttt caaaactgga gttgttgaaa tgaaagacag 1800

tggctacaaa gtttctgacc cagagaactt gtatttgaag caggggatag caaacttatc 1860

tgcagaggtt gtctcactca agagacttat agccacacaa ccaatctctg cttcagactc 1920

tgggtaaatt actactgagt aagagctggg catttagaaa gatgtcattt gcaatagagc 1980

agtccatttt gtattatgct gaattttcac tggacctgtg atgtcatttc actgtgatgt 2040

gcacatgttg tctgtttggt gtctttttgt gcacagatta tgatgaagat tagattgtgt 2100

tatcactctg cctgtgtata gtcagatagt ccatgcgaag gctgtatata ttgaacatta 2160

tttttgttgt tctattataa agtgtgtaag ttaccagttt caataaagga ttggtgacaa 2220

acacagaaaa aaaaaaaaaa aaaaaaa 2247

<210> 32
<211> 2019
<212> DNA
<213> Mus <mouse, genus>

<220>
<223> Mouse Nfil3 (NM_017373.3)

<400> 32
acacatctct cggcgcggcc acggcgcccg cggacccggc gcgcccgctc gtctcccgcg 60

ccgcgccctc gccaccgccc gctgagggcc cggcggagct tgaatcgcgc cccggcgccg 120

ccagcccggt tacagccgcc ctttcttttc cccctcacgg accagggagc agaaccacga 180

taacccatga aaggctcctg acagatttac cctgtgccgg acaacagaag gacccattga 240

tggatgagag gctgaagtcc ttaagacctg aaagaagact ggggaagtga tttgtctcct 300

ccatccggtc tgtaggacga ggaggtcctt tctgatgcag ctgagaaaaa tgcagaccat 360

caaaaaggag cccgcacccc tagatcctac cagcagctca gacaagatgc tgctgctgaa 420

ctctgcctta gctgaggtgg ccgaggacct agcctcaggt gaagatttgc tcctgaacga 480

agggagcatg ggaaaaaaca aatcctcggc gtgtcggaga aaacgggaat tcattccgga 540

cgagaagaaa gacgccatgt attgggagaa acggcggaaa aacaacgaag ctgccaaaag 600

atctcgggag aagcgccgcc tcaatgacct ggttttggag aacaagctga tcgccctggg 660

agaagaaaat gccactttaa aagctgagct gctctccctg aaattaaagt ttggtttaat 720

tagctccacg gcgtatgccc aagaaatcca gaaactcagt aattccacag ctgtctactt 780

tcaggactac cagacatcca aggctgccgt gagctctttt gtggacgagc atgagcctgc 840

gatggtagcc ggaagttgca tctcagtcat caagcactct ccccagagct cgctctccga 900

tgtgtcagag gtgtcctcgg tggagcacac tcaggaaagc cccgcacagg gaggctgccg 960

gagccctgag aacaagttcc ctgtgatcaa gcaggagccc gtggagttgg agagctttgc 1020

cagggaggcc agggaggagc ggggcacgta ttccacctcc atctaccaga gctacatggg 1080

aagctctttc tccacttact cccactcccc acccctcttg caggtccatg ggtccactag 1140

caactcccca agaacctcag aggccgatga gggtgtagtg ggcaagtctt ctgatgggga 1200

agacgaacaa caggtcccta agggccccat ccattctcca gtggagctgc aacgggtcca 1260

cgccacggtg gtgaaggttc cggaagtgaa cccttctgcc ttaccgcaca agcttcggat 1320

taaagccaag gccatgcagg tcaaagtgga ggctttggac agcgagtttg aaggcatgca 1380

gaaactctct tcacccgccg atgcgatcgc caaaagacat tttgacctgg agaaacatgg 1440

aacctcgggt atggcccatt cctccctccc tcctttctca gtgcaggtga cgaacattca 1500

agattggtcc ctcaaatcgg aacactggca tcacaaagaa ctgagcagca aaactcagag 1560

tagcttcaaa acaggtgtgg tggaagtcaa agacggtggc tataaggttt ccgaagctga 1620

gaatttgtat ttgaagcagg gaatagcaaa cttatctgca gaggtggtct cgctcaagag 1680

attcatagcc acacaaccga tctcggcttc ggactccagg taaatggctg ctgaccgagc 1740

tatgcatgga ggaggaggct gttggtacca tactgaattt ccactggacc tctaaagtca 1800

tttcactgta gtgtgcacaa cggcgtctgt ctgggtgtcc ttgtgtgcac gcgctgaaga 1860

cttgatgccc tcactctgcc tggcgtgtag tcagatagcc ccccacagag gctgtacata 1920

ctgaacgtta tttttgctct attataaagt gtgtatgttg ccagtttcaa taaaggattg 1980

gtggcaagca caaaaaaaaa aaaaaaaaaa aaaaaaaaa 2019

<210> 33

<211> 3575
<212> DNA
<213> Homo sapiens

<220>
<223> Human BCL6 (NM_001706.4)

<400> 33
accatcgtct tgggcccggg gagggagagc caccttcagg cccctcgagc ctcgaaccgg 60

aacctccaaa tccgagacgc tctgcttatg aggacctcga aatatgccgg ccagtgaaaa 120

aatcttgtgg ctttgagggc ttttggttgg ccaggggcag taaaaatctc ggagagctga 180

caccaagtcc tcccctgcca cgtagcagtg gtaaagtccg aagctcaaat tccgagaatt 240

gagctctgtt gattcttaga actggggttc ttagaagtgg tgatgcaaga agtttctagg 300

aaaggccgga caccaggttt tgagcaaaat tttggactgt gaagcaaggc attggtgaag 360

acaaaatggc ctcgccggct gacagctgta tccagttcac ccgccatgcc agtgatgttc 420

ttctcaacct taatcgtctc cggagtcgag acatcttgac tgatgttgtc attgttgtga 480

gccgtgagca gtttagagcc cataaaacgg tcctcatggc ctgcagtggc ctgttctata 540

gcatctttac agaccagttg aaatgcaacc ttagtgtgat caatctagat cctgagatca 600

accctgaggg attctgcatc ctcctggact tcatgtacac atctcggctc aatttgcggg 660

agggcaacat catggctgtg atggccacgg ctatgtacct gcagatggag catgttgtgg 720

acacttgccg gaagtttatt aaggccagtg aagcagagat ggtttctgcc atcaagcctc 780

ctcgtgaaga gttcctcaac agccggatgc tgatgcccca agacatcatg gcctatcggg 840

gtcgtgaggt ggtggagaac aacctgccac tgaggagcgc ccctgggtgt gagagcagag 900

cctttgcccc cagcctgtac agtggcctgt ccacaccgcc agcctcttat tccatgtaca 960

gccacctccc tgtcagcagc ctcctcttct ccgatgagga gtttcgggat gtccggatgc 1020

ctgtggccaa ccccttcccc aaggagcggg cactcccatg tgatagtgcc aggccagtcc 1080

ctggtgagta cagccggccg actttggagg tgtcccccaa tgtgtgccac agcaatatct 1140

attcacccaa ggaaacaatc ccagaagagg cacgaagtga tatgcactac agtgtggctg 1200

agggcctcaa acctgctgcc ccctcagccc gaaatgcccc ctacttccct tgtgacaagg 1260

ccagcaaaga agaagagaga ccctcctcgg aagatgagat tgccctgcat ttcgagcccc 1320

ccaatgcacc cctgaaccgg aagggtctgg ttagtccaca gagcccccag aaatctgact 1380

gccagcccaa ctcgcccaca gagtcctgca gcagtaagaa tgcctgcatc ctccaggctt 1440

ctggctcccc tccagccaag agccccactg accccaaagc ctgcaactgg aagaaataca 1500

agttcatcgt gctcaacagc ctcaaccaga atgccaaacc agagggccct gagcaggctg 1560

agctgggccg cctttcccca cgagcctaca cggccccacc tgcctgccag ccacccatgg 1620

agcctgagaa ccttgacctc cagtccccaa ccaagctgag tgccagcggg gaggactcca 1680
ccatcccaca agccagccgg ctcaataaca tcgttaacag gtccatgacg ggctctcccc 1740
gcagcagcag cgagagccac tcaccactct acatgcaccc cccgaagtgc acgtcctgcg 1800
gctctcagtc cccacagcat gcagagatgt gcctccacac cgctggcccc acgttccctg 1860
aggagatggg agagacccag tctgagtact cagattctag ctgtgagaac ggggccttct 1920
tctgcaatga gtgtgactgc cgcttctctg aggaggcctc actcaagagg cacacgctgc 1980
agacccacag tgacaaaccc tacaagtgtg accgctgcca ggcctccttc cgctacaagg 2040
gcaacctcgc cagccacaag accgtccata ccggtgagaa accctatcgt tgcaacatct 2100
gtggggccca gttcaaccgg ccagccaacc tgaaaaccca cactcgaatt cactctggag 2160
agaagcccta caaatgcgaa acctgcggag ccagatttgt acaggtggcc cacctccgtg 2220
cccatgtgct tatccacact ggtgagaagc cctatccctg tgaaatctgt ggcacccgtt 2280
tccggcacct tcagactctg aagagccacc tgcgaatcca cacaggagag aaaccttacc 2340
attgtgagaa gtgtaacctg catttccgtc acaaaagcca gctgcgactt cacttgcgcc 2400
agaagcatgg cgccatcacc aacaccaagg tgcaataccg cgtgtcagcc actgacctgc 2460
ctccggagct ccccaaagcc tgctgaagca tggagtgttg atgctttcgt ctccagcccc 2520
ttctcagaat ctacccaaag gatactgtaa cactttacaa tgttcatccc atgatgtagt 2580
gcctctttca tccactagtg caaatcatag ctgggggttg ggggtggtgg gggtcggggc 2640
ctgggggact gggagccgca gcagctcccc ctcccccact gccataaaac attaagaaaa 2700
tcatattgct tcttctccta tgtgtaaggt gaaccatgtc agcaaaaagc aaaatcattt 2760
tatatgtcaa agcaggggag tatgcaaaag ttctgacttg actttagtct gcaaaatgag 2820
gaatgtatat gttttgtggg aacagatgtt tcttttgtat gtaaatgtgc attcttttaa 2880
aagacaagac ttcagtatgt tgtcaaagag agggctttaa tttttttaac caaaggtgaa 2940
ggaatatatg gcagagttgt aaatatataa atatatatat atataaaata aatatatata 3000
aacctaaaaa agatatatta aaaatataaa actgcgttaa aggctcgatt ttgtatctgc 3060
aggcagacac ggatctgaga atctttattg agaaagagca cttaagagaa tattttaagt 3120
attgcatctg tataagtaag aaaatatttt gtctaaaatg cctcagtgta tttgtatttt 3180
tttgcaagtg aaggtttaca atttacaaag tgtgtattaa aaaaaacaaa aagaacaaaa 3240
aaatctgcag aaggaaaaat gtgtaatttt gttctagttt tcagtttgta tatacccgta 3300
caacgtgtcc tcacggtgcc ttttttcacg gaagttttca atgatgggcg agcgtgcacc 3360
atcccttttt gaagtgtagg cagacacagg gacttgaagt tgttactaac taaactctct 3420
ttgggaatgt ttgtctcatc ccattctgcg tcatgcttgt gttataacta ctccggagac 3480
agggtttggc tgtgtctaaa ctgcattacc gcgttgtaaa atatagctgt acaaatataa 3540

gaataaaatg ttgaaaagtc aaactggaaa aaaaa 3575

<210> 34
<211> 3527
<212> DNA
<213> Mus <mouse, genus>

<220>
<223> Mouse Bcl6 (NM_009744.4)

<400> 34
gtagccgcgc gcgcccgcct cgggaccccc ggtgttccgg gcggcggtgc tgcgggaacc 60

acgatccgca ctatagggcg gcgagcggcg cggccccgag gcattctgcc ggccgggagg 120

gcgctgcgga gcgcgccgct gccaccgagt cctcccctgc tgcggagcaa tggtaaagcc 180

cgcagctcaa attccgagaa ttgagctctg ttgattctta gaactggggt tcttagaagt 240

ggtgatgcaa gaagtttcta ggaaaggccg gacaccagtt ttaaagcaaa attttggact 300

gtgaagcaag gcactgggca aacacaacat ggcctccccg gctgacagct gtatccagtt 360

tacccggcac gctagtgatg ttcttctcaa ccttaatcgc ctccggagtc gggacatctt 420

gacggacgtt gtcatcgtgg tgagccgtga gcagtttaga gcccataaga cagtgctcat 480

ggcctgcagc ggcctgttct acagtatctt cactgaccag ttgaaatgca accttagtgt 540

aatcaatcta gatcctgaaa tcagccctga ggggttttgc atcctcctgg acttcatgta 600

cacatctagg ctcaacctga gggaaggcaa tatcatggcg gtgatgacca cagccatgta 660

cctgcagatg gagcatgttg tcgacacatg caggaagttc atcaaggcca gtgaagcaga 720

aatggcccct gcacttaaac ctccccgtga agagttcctg aacagccgga tgctgatgcc 780

ccatgacatc atggcctacc gaggtcgtga ggtcgtggag aacaatatgc cactgagaaa 840

tactcccggg tgtgagagca gagcttttgc tcctcctctg tacagtggcc tgtcaacacc 900

accagcctct tatcccatgt acagccatct cccgctcagc accttcctct tctctgatga 960

ggagctccga gatgcccccc gaatgcctgt ggccaaccct tttcccaagg agcgtgccct 1020

cccctgcgac agtgccaggc aagtccctaa tgagtatagc aggccagcca tggaggtgtc 1080

ccccagtttg tgtcacagca acatctactc gcccaaggag gcagtcccag aggaggctcg 1140

gagtgacata cactacagtg tgcctgaggg ccccaagcct gctgtccctt ctgctcggaa 1200

tgctccatac ttcccctgtg acaaagccag caaagaagaa gagagacctt cttcggagga 1260

tgagattgcc ctgcatttcg agccccccaa tgcacccttg aaccggaagg gtctggttag 1320

tccccagagt ccccagaaat ccgactgcca gcccaactca cccacagagt cctgcagcag 1380

caagaacgcc tgcatccttc aggcctctgg ctctccgcca gccaagagcc ccactgaccc 1440

gaaagcctgc aactggaaga agtataagtt catcgttctc aacagcctca atcagaatgc 1500

caaacccgag ggctctgagc aggcagagct gggtcgcctc tcccctcgag cctaccctgc 1560

accgcccgct tgccagccgc ctatggagcc cgcgaacctt gatctccagt ccccgaccaa 1620

gctcagtgcc agtggggagg actctaccat cccccaagcc agccggctca ataatctcgt 1680

gaacaggtcc ctggcaggct ccccccgaag cagcagtgag agtcactcac cactctacat 1740

gcacccccca aagtgcacat cctgcggctc tcagtcccca cagcatacag agatgtgcct 1800

ccatactgct gggcccacgt tcccggagga gatgggggaa acccagtcag agtattcgga 1860

ttctagctgt gagaatggga ccttcttctg caacgaatgt gactgccgtt tctctgagga 1920

ggcctcgctc aagaggcaca cgctgcagac gcacagtgac aaaccataca aatgtgatcg 1980

ctgccaggcc tccttccgct acaagggcaa cctcgccagc cacaagactg tccacacggg 2040

tgagaaaccc tatcgctgta acatttgtgg agcgcagttc aatcggccag ccaacctgaa 2100

gacccacact cgaattcact ctggagaaaa gccctacaaa tgtgaaacct gtggggccag 2160

gtttgttcag gtggcccacc tccgtgccca cgtgctcatc cacactggag agaagccgta 2220

cccctgtgaa atctgtggca ctcgcttccg gcaccttcag actctgaaga gccatctgcg 2280

catccacaca ggagagaaac cttaccattg tgagaagtgt aacctgcact ttcgtcacaa 2340

aagccaactg cgacttcatt tgcgccagaa gcacggcgcc atcaccaaca ccaaggtgca 2400

ataccgcgtg tcggccgctg acctgcctcc ggagctcccc aaagcctgct gaatgaagca 2460

tggagtgttc ctcgcccttt cctctccagc cccttctcag aatctaccca aaggatgctg 2520

taacacttta tacaaaggtc atcccatgat gtagtgcctc tctcatccac tagtgcaaat 2580

catagttggg gtgggggtgg gggtggggtt tgcgggaccg ggagccaagg cagctcccct 2640

tcccacactg ccataaaaca ttaagaaaat actattgctt cttctcctat gtgtaaggca 2700

aaccctgtca gcaaaaagca aattcatttt ctatatcaaa gtaggggaga atgcagaagt 2760

tctgacttga caggttgcaa ccgaggaatg taaatgatgt gtgggaacag aggtctcttt 2820

tgtatgtaaa tgtgtattgt ttttaaagac aagacttcag tatgctgtca aagagaaggc 2880

tttaattttt ttaaccaaag gtgaaggaat atatggcaga gttgtaaata tataaatata 2940

tatatatata atataaatat ataaacctaa aaagatatat tgaaaatata aaactgtgtt 3000

aaaggctcga ttttgtatct gcaggcagac acggatctga gaatctttat tgagaaagag 3060

cacttaagag actattttaa gtattgcgtc tgtataagta agaaaatatt ttgtctaaaa 3120

tgcctccgtg tatttgtatt tttttgcaag tgaaggttta caaatttaca aagtgtgtat 3180

taaaaacaaa aagaacaaaa aaaaaagctg cagaaggaga aatgtatact tttgttccag 3240

ttttcagttt gtacatacct gtaatgtgtc ctcacggtgc ctttttacac ggaagttttc 3300

aatgatggac gggtgtgcgc catccctttt tgaagtgtag gcagacacag ggacttgaag 3360

ttgtcactaa ctaaactctc tttgggaatg tttgactcct cccacattct gcgtcatgct 3420

tgtcgttata attactccgg agacagggtt tggctgtgtc taaactgcat tagtgcgttg 3480

taaaatagag ctgtacaaaa cataagaata aaacattgaa aagtcga 3527

<210> 35
<211> 3623
<212> DNA
<213> Homo sapiens

<220>
<223> Human L-MYC (NM_001033081.2)

<400> 35
aatgcgcctg cagctcgcgc tcccgcgccg atcccgagag cgtccgggcc gccgtgcgcg 60

agcgagggag ggcgcgcgcg cggggggggc gcgcttgtga gtgcgggccg cgctctcggc 120

ggcgcgcatg tgcgtgtgtg ctggctgccg ggctgccccg agccggcggg gagccggtcc 180

gctccaggtg gcgggcggct ggagcgaggg aggctgcggg tggccagggc acgggcgcgg 240

gtcccgcggt gcgggctggc tgcaggctgc cttctgggca cggcgcgccc ccgcccggcc 300

ccgcccggcc ctgggagctg cgctccgggc ggcgctggca aagtttgctt tgaactcgct 360

gcccacagtc gggtccgcgc gctgcgattg gcttccccta ccactctgac ccggggcccg 420

gcttcccggg acgcgaggac tgggcgcagg ctgcaagctg gtggggttgg ggaggaacga 480

gagcccggca gccgactgtg ccgagggacc cggggacacc tccttcgccc ggccggcacc 540

cggtcagcac gtcccccctt ccctcccgca gggagcggac atggactacg actcgtacca 600

gcactatttc tacgactatg actgcgggga ggatttctac cgctccacgg cgcccagcga 660

ggacatctgg aagaaattcg agctggtgcc atcgcccccc acgtcgccgc cctggggctt 720

gggtcccggc gcaggggacc cggcccccgg gattggtccc ccggagccgt ggcccggagg 780

gtgcaccgga gacgaagcgg aatcccgggg ccactcgaaa ggctggggca ggaactacgc 840

ctccatcata cgccgtgact gcatgtggag cggcttctcg gcccgggaac ggctggagag 900

agctgtgagc gaccggctcg ctcctggcgc gccccggggg aacccgccca aggcgtccgc 960

cgccccggac tgcactccca gcctcgaagc cggcaacccg gcgcccgccg ccccctgtcc 1020

gctgggcgaa cccaagaccc aggcctgctc cgggtccgag agcccaagcg actcggagaa 1080

tgaagaaatt gatgttgtga cagtagagaa gaggcagtct ctgggtattc ggaagccggt 1140

caccatcacg gtgcgagcag accccctgga tccctgcatg aagcatttcc acatctccat 1200

ccatcagcaa cagcacaact atgctgcccg ttttcctcca gaaagctgct cccaagaaga 1260

ggcttcagag aggggtcccc aagaagaggt tctggagaga gatgctgcag gggaaaagga 1320

agatgaggag gatgaagaga ttgtgagtcc cccacctgta gaaagtgagg ctgcccagtc 1380

ctgccacccc aaacctgtca gttctgatac tgaggatgtg accaagagga agaatcacaa 1440

cttcctggag cgcaagaggc ggaatgacct gcgttcgcga ttcttggcgc tgagggacca 1500

ggtgcccacc ctggccagct gctccaaggc ccccaaagta gtgatcctaa gcaaggcctt 1560

ggaatacttg caagccctgg tgggggctga gaagaggatg gctacagaga aaagacagct 1620

ccgatgccgg cagcagcagt tgcagaaaag aattgcatac ctcactggct actaactgac 1680

caaaaagcct gacagttctg tcttacgaag acacaagttt attttttaac ctccctctcc 1740

cctttagtaa tttgcacatt ttggttatgg tgggacagtc tggacagtag atcccagaat 1800

gcattgcagc cggtgcacac acaataaagg cttgcattct tggaaacctt gaaacccagc 1860

tctccctctt ccctgactca tgggagtgct gtatgttctc tggcgccttt ggcttcccag 1920

caggcagctg actgaggagc cttggggtct gcctagctca ctagctctga agaaaaggct 1980

gacagatgct atgcaacagg tggtggatgt tgtcaggggc tccagcctgc atgaaatctc 2040

acactctgca tgagctttag gctaggaaag gatgctccca actggtgtct ctggggtgat 2100

gcaaggacag ctgggcctgg atgctctccc tgaggctcct ttttccagaa gacacacgag 2160

ctgtcttggg tgaagacaag cttgcagact tgatcaacat tgaccattac ctcactgtca 2220

gacactttac agtagccaag gagttggaaa cctttatata ttatgatgtt agctgacccc 2280

cttcctccca ctcccaatgc tgcgaccctg ggaacactta aaaagcttgg cctctagatt 2340

ctttgtctca gagccctctg ggctctctcc tctgagggag ggacctttct ttcctcacaa 2400

gggacttttt tgttccatta tgccttgtta tgcaatgggc tctacagcac cctttcccac 2460

aggtcagaaa tatttcccca agacacaggg aaatcggtcc tagcctgggg cctggggata 2520

gcttggagtc ctggcccatg aacttgatcc ctgcccaggt gttttccgag gggcacttga 2580

ggcccagtct tttctcaagg caggtgtaag acacctcaga gggagaactg tactgctgcc 2640

tctttcccac ctgcctcatc tcaatccttg agcggcaagt ttgaagttct tctggaacca 2700

tgcaaatctg tcctcctcat gcaattccaa ggagcttgct ggctctgcag ccacccttgg 2760

gccccttcca gcctgccatg aatcagatat ctttcccaga atctgggcgt ttctgaagtt 2820

ttggggagag ctgttgggac tcatccagtg ctccagaagg tggacttgct tctggtgggt 2880

tttaaaggag cctccaggag atatgcttag ccaaccatga tggattttac cccagctgga 2940

ctcggcagct ccaagtggaa tccacgtgca gcttctagtc tgggaaagtc acccaaccta 3000

gcagttgtca tgtgggtaac ctcaggcacc tctaagcctg tcctggaaga aggaccagca 3060

gcccctccag aactctgccc aggacagcag gtgcctgctg gctctgggtt tggaagttgg 3120

ggtgggtagg gggtggtaag tactatatat ggctctggaa aaccagctgc tacttccaaa 3180

tctattgtcc ataatggttt ctttctgagg ttgcttcttg gcctcagagg accccagggg 3240

atgtttggaa atagcctctc tacccttctg gagcatggtt tacaaaagcc agctgacttc 3300

tggaattgtc tatggaggac agtttgggtg taggttactg atgtctcaac tgaatagctt 3360

gtgttttata agctgctgtt ggctattatg ctgggggagt cttttttttt tatattgtat 3420

ttttgtatgc cttttgcaaa gtggtgttaa ctgtttttgt acaaggaaaa aaactcttgg 3480

ggcaatttcc tgttgcaagg gtctgattta ttttgaaagg caagttcacc tgaaattttg 3540

tatttagttg tgattactga ttgcctgatt ttaaaatgtt gccttctggg acatcttcta 3600

ataaaagatt tctcaaacat gtc 3623

<210> 36
<211> 3686
<212> DNA
<213> Mus <mouse, genus>

<220>
<223> Mouse L-myc (NM_008506.3)

<400> 36
agtcgggaaa caatgcgcct gcagatcgcg ctcccgcgtc gatcccggga gcgtcctggc 60

tgccgtgtgc gagcgaggcg gggggcgcgc gcacgggggg cgcgctcgtg agtgcggggc 120

cgcgcgctcg gtggcgcgca tgtgtgtgtg tgcgggctgc cgggcttccc cgagccggcg 180

gggagccgct ccgctccagg tggcgggcgg cgggagcgag gtgaggctgc gggtggcccg 240

ggcaggggtc cccaggggac tggcgggctg caaggctgca gactgccttc gagacagcgc 300

gcccccgccc ggccctgctg tgcccccgga gctgagctcc gggcggtgct ggcaaagttt 360

gctttgaact cgctcccctc agcctggtcg gcccgttgcg agctgccctg agcgagctga 420

ccccaggcca ggcttcccag gagcagggac cagggcgcgg gctgcaagct ggtgggcctg 480

gggagagacc agagccccgc agccagctgc agcgagggac tcggagccgc ctcttccctc 540

ggcgggcacc gcagtcagct cgtctccccc ttccctcccg cagggagcgg acatggactt 600

cgactcgtat cagcactatt tctacgacta tgactgcgga gaggatttct accgctccac 660

ggcgcccagc gaggacatct ggaagaaatt cgagctggtg ccgtcgcccc ccacgtcgcc 720

gccctggggc tccggtcccg gcgccgtgga cccagcctct gggattaatc ccggggagcc 780

gtggcctgga gggggtgccg gggacgaggc ggaatctcgg ggccattcga aagcctgggg 840

caggaattat gcttccatca ttcgccgtga ctgcatgtgg agcggcttct ccgcccgaga 900

acggctggag agagtggtga gcgacaggct ggccccaggc gcgccccggg ggaacccgcc 960

caaagcgccc gctaccccgg acggcactcc tagtctggaa gccagtaacc cggcgcccgc 1020

cacccaatgt cagctgggcg agcccaagac tcaggcctgc tccgggtccg agagccccag 1080

cgattctgaa ggtgaagaga ttgacgtggt gaccgtggag aagaggcgat ctctggacat 1140

ccgaaagcca gtcaccatca cggtgcgagc agaccccctg gacccctgca tgaagcactt 1200

ccatatctct atccaccaac agcagcataa ctatgctgcc cgttttcctc cagaaagttg 1260

ctctcaagag ggggatcctg agccaggtcc ccaggaagag gctccggaga tagaagctcc 1320

caaggagaaa gaggaggagg aagaggaaga ggaggaagaa gagattgtga gccccccacc 1380

tgtcggaagt gaggctcccc agtcctgcca ccccaaacct gtcagttctg acactgagga 1440

cgtgaccaag aggaagaacc ataacttctt ggaacgaaaa aggaggaatg acctccgctc 1500

ccggttccta gccctgcggg accaggttcc caccctggcc agctgctcta aggcccccaa 1560

agtcgtgatc ctcagcaagg cgttagaata cttgcaggct ttggtggggg ctgaaaagaa 1620

aatggctaca gagaaaaggc agctccggtg tcggcaacag caactgcaaa agagaatcgc 1680

gtacctcagt ggctactaac cgaccagaac gcctgacttc ttggtctcac agacacaagc 1740

ttattgttta acctctctct cccttttagt aatttgcaca ttttggttac agcggggggg 1800

gcagtctgga cagtagatcc cagaatgcat tgcagccggt gtgcgcacac aataagggct 1860

tgcattcttg gtaacctcga aacccaattc tccctcttcc ccgaccgact catgggaatg 1920

ctgtccttct ctggcgcctt tggcttctca gcaggcagct actgaggaga tttggggtct 1980

gcttagctca ctagctcctg acgaaaggct gacagatgct atgcaacagg tggtggacgt 2040

tgttggggct gcagcctacg tgaaatctca cactgtgctg gggcttcagg ctaggaaagg 2100

atgctgctct cactgctgtc tctggggatg atctgaggac agctgggcct ggatactgtc 2160

ccccaggctc cgttttccag gaggcaagcg agctgtcccg ggcgaagaca agctcgcaga 2220

cttgatcagc atggagcatt acctcaccgt cagacacttt acagtagctg tggagtggaa 2280

acctttaaga tagatttgga tggtaggcca cacccttccc tgcacgctca atgctatgac 2340

tttgagaaag ggcttggcct ctatgtagag tctttgtctc agagttctct gggccttctc 2400

agagagggac ctttctatcc tcacaaggga cctttttgtt tcttcctgcc tttgttatgc 2460

aatggccacc acagcaccct ttcacaccga ccagaaatat ttccccagga catagggaaa 2520

tgggtcacag cccaggacct ggggaagcct tggcatcccc actcatgacc aacggtcctt 2580

gcccaggttt tctgcagggc tatttgaggc ccagcttgga accttttctc gaggcagata 2640

gttacaaggt gcctctgaag gacaagccct atcgcttcct ctttcccacc tgcctctctg 2700

tcagatcttg actctgtcta caatctgctg gaacagtgca aacctgtcct tctcgagcaa 2760

ctttgctggc tctgcagcca ccatcctgat tctctgccgg cctgagtcac atcctttccc 2820

ctggaatctg ggccttacag agagattcag aggggcaccg cttgcattca cctgatgccc 2880

ccagaaggta aacttacttc ctggtgggtt gtcagtgtac ctctaggaac gctactcagc 2940

caacaaggag agtttgctcc agctgtgttc tgcaactccc tgtggaatca aagtacagcc 3000

ctctatcctg ggaaagtcac caagctagca gccgtcacgt gagcatcttt caggagatcc 3060

taagctttgc ctgaaagaag agccagcctt tccagaactc tacccaggaa agcagatctg 3120

ttcctgctgg ccctgggctt ggaagtaggg gtacagtgtg ggggacagac agtaagtaac 3180

aacatgtggc tctcaaaaac cagctaccac ttccaaattg gctcccaact gtgatggcct 3240

ccaattactt cctggcctca agtcctagag gaagcttcgg aagttgctgt tgtacctgtt 3300

ggggcaggac ttctaggcac caagggactc ctggaactat cttgggagga caagtggtga 3360

acaggctaaa gtctcatctg aatggcttgt gttttataag ctgctgcggg gttgtatgct 3420

gtgggcgtct tttgtttttg tttttgcttt tttttttaat actgtatttt tgtatgcttt 3480

tttgcaaagt ggtgttaact gtttttgtat aagaaaaaca aaaaacaaaa accctcctgt 3540

tgcaagggtc tggtttattt tgaaaggtgc atttacctga aattttgtat ttagttgtaa 3600

tcattaattg cttgatttta aactgttgcc ttctgggaca tcttctaata aaaagatttc 3660

tcaaaaaaaa aaaaaaaaaa aaaaaa 3686

<210> 37
<211> 5635
<212> DNA
<213> Homo sapiens

<220>
<223> Human NR4A3 (NM_006981.3)

<400> 37
ataaatgacg tgccgagaga gcgagcgaac gcgcagccgg gagagcggag tctcctgcct 60

cccgcccccc acccctccag ctcctgctcc tcctccgctc cccatacaca gacgcgctca 120

cacccgctcc ctcactcgca cacacagaca caagcgcgca cacaggctcc gcacacacac 180

ttcgctctcc cgcgcgctca cacccctctt gccctgagcc cttgccggtg cagcgcggcg 240

ccgcagctgg acgcccctcc cgggctcact ttgcaacgct gacggtgccg gcagtggccg 300

tggaggtggg aacagcggcg gcatcctccc ccctggtcac agcccaagcc aggacgcccg 360

cggaacctct cggctgtgct ctcccatgag tcgggatcgc agcatccccc accagccgct 420

caccgcctcc gggagccgct gggcttgtac accgcagccc ttccgggaca gcagctgtga 480

ctccccccca gtgcagattt cgggacagct ctctagaaac tcgctctaaa gacggaaccg 540

ccacagcact caaagcccac tgcggaagag ggcagcccgg caagcccggg ccctgagcct 600

ggacccttag cggtgccggg cagcactgcc ggcgcttcgc ctcgccggac gtccgctcct 660

cctacactct cagcctccgc tggagagacc cccagcccca ccattcagcg cgcaagatac 720

cctccagata tgccctgcgt ccaagcccaa tatagccctt cccctccagg ttccagttat 780

gcggcgcaga catacagctc ggaatacacc acggagatca tgaaccccga ctacaccaag 840

ctgaccatgg accttggcag cactgagatc acggctacag ccaccacgtc cctgcccagc 900

atcagtacct tcgtggaggg ctactcgagc aactacgaac tcaagccttc ctgcgtgtac 960
caaatgcagc ggcccttgat caaagtggag gaggggcggg cgcccagcta ccatcaccat 1020
caccaccacc accaccacca ccaccaccat caccagcagc agcatcagca gccatccatt 1080
cctccagcct ccagcccgga ggacgaggtg ctgcccagca cctccatgta cttcaagcag 1140
tccccaccgt ccacccccac cacgccggcc ttccccccgc aggcgggggc gttatgggac 1200
gaggcactgc cctcggcgcc cggctgcatc gcacccggcc cgctgctgga cccgccgatg 1260
aaggcggtcc ccacggtggc cggcgcgcgc ttcccgctct tccacttcaa gccctcgccg 1320
ccgcatcccc ccgcgcccag cccggccggc ggccaccacc tcggctacga cccgacggcc 1380
gctgccgcgc tcagcctgcc gctgggagcc gcagccgccg cgggcagcca ggccgccgcg 1440
cttgagagcc acccgtacgg gctgccgctg gccaagaggg cggccccgct ggccttcccg 1500
cctctcggcc tcacgccctc ccctaccgcg tccagcctgc tgggcgagag tcccagcctg 1560
ccgtcgccgc ccagcaggag ctcgtcgtct ggcgagggca cgtgtgccgt gtgcggggac 1620
aacgccgcct gccagcacta cggcgtgcga acctgcgagg gctgcaaggg ctttttcaag 1680
agaacagtgc agaaaaatgc aaaatatgtt tgcctggcaa ataaaaactg cccagtagac 1740
aagagacgtc gaaaccgatg tcagtactgt cgatttcaga agtgtctcag tgttggaatg 1800
gtaaaagaag ttgtccgtac agatagtctg aaaggaggga gaggtcgtct gccttccaaa 1860
ccaaagagcc cattacaaca ggaaccttct cagccctctc caccttctcc tccaatctgc 1920
atgatgaatg cccttgtccg agctttaaca gactcaacac ccagagatct tgattattcc 1980
agatactgtc ccactgacca ggctgctgca ggcacagatg ctgagcatgt gcaacaattc 2040
tacaacctcc tgacagcctc cattgatgta tccagaagct gggcagaaaa gattccggga 2100
tttactgatc tccccaaaga agatcagaca ttacttattg aatcagcctt tttggagctg 2160
tttgtcctca gactttccat caggtcaaac actgctgaag ataagtttgt gttctgcaat 2220
ggacttgtcc tgcatcgact tcagtgcctt cgtggatttg gggagtggct cgactctatt 2280
aaagactttt ccttaaattt gcagagcctg aaccttgata tccaagcctt agcctgcctg 2340
tcagcactga gcatgatcac agaaagacat gggttaaaag aaccaaagag agtcgaagag 2400
ctatgcaaca agatcacaag cagtttaaaa gaccaccaga gtaagggaca ggctctggag 2460
cccaccgagt ccaaggtcct gggtgccctg gtagaactga ggaagatctg caccctgggc 2520
ctccagcgca tcttctacct gaagctggaa gacttggtgt ctccaccttc catcattgac 2580
aagctcttcc tggacaccct acctttctaa tcaggagcag tggagcagtg agctgcctcc 2640
tctcctagca cctgcttgct acgcagcaaa gggataggtt tggaaaccta tcatttcctg 2700
tccttcctta agaggaaaag cagctcctgt agaaagcaaa gactttcttt tttttctggc 2760
tcttttcctt acaacctaaa gccagaaaac ttgcagagta ttgtgttggg gttgtgtttt 2820

atatttaggc attgggggat ggggtgggag ggggttatag ttcatgaggg ttttctaaga 2880
aattgctaac aaagcacttt tggacaatgc tatcccagca ggaaaaaaaa ggataatata 2940
actgttttaa aactctttct ggggaatcca attatagttg ctttgtattt aaaaacaaga 3000
acagccaagg gttgttcgcc agggtaggat gtgtcttaaa gattggtccc ttgaaaatat 3060
gcttcctgta tcaaaggtac gtatgtggtg caaacaaggc agaaacttcc ttttaatttc 3120
cttcttcctt tattttaaca aatggtgaaa gatggaggat tacctacaaa tcagacatgg 3180
caaaacaata atggctgttt gcttccataa acaagtgcaa ttttttaaag tgctgtctta 3240
ctaagtcttg tttattaact ctcctttatt ctatatggaa ataaaaagga ggcagtcatg 3300
ttagcaaatg acacgttaat atccctagca gaggctgtgt tcaccttccc tgtcgatccc 3360
ttctgaggta tggcccatcc aagactttta ggccattctt gatggaacca gatccctgcc 3420
ctgactgtcc agctatcctg aaagtggatc agattataaa ctggattaca tgtaactgtt 3480
ttggttgtgt tctatcaacc ccaccagagt tccctaaact tgcttcagtt atagtaactg 3540
actggtatat tcattcagaa gcgccataag tcagttgagt atttgatccc tagataagaa 3600
catgcaaatc agcaggaact ggtcatacag ggtaagcacc agggacaata aggattttta 3660
tagatataat ttaatttttg ttattggtta aggagacaat tttggagagc aagcaaatct 3720
ttttaaaaaa tagtatgaat gtgaatacta gaaaagattt aaaaaatagt atgagtgtga 3780
gtactaggaa ggattagtgg gctgcgtttc aacattccgt gttcgtactc ccttttgtat 3840
gtttctactg ttaatgccat attactatga gataatttgt tgcatagtgt ccttatttgt 3900
ataaacattt gtatgcacgt tatattgtaa tagctttgcc tgtatttatt gcaagaccac 3960
cagctcctgg aagctgagtt acagagtaat taaatggggt gttcacagtg acttggatac 4020
accaattaga aattaaataa gcaaatatat atatatatat aaatatagca ggttacatat 4080
atatatttat aatgtgtctt tttattaacc atttgtacaa taaatgtcac ttcccatgcc 4140
gttattttat ggttcatttg cagtgacttt taaggcagta ctgtttagca ctttgatatt 4200
aaaattttgc ttatgttttg ctaaattcga ataatgtttg aagattttta ggtctaaaag 4260
tctttatatt atatactctg tatcaagtca aaatatcttt ggccattttg ctaagaaaca 4320
aactttgaat gtcaaactga tgtcacagta gtttttgtta gctttaaatc atttttgctt 4380
tagtcttttt aaaggaaaat aacaaaacta tgctgtttat attgtcatta aattatacaa 4440
tcaaacaaat gccaaatgaa ttgcctaatt gctgcaaagt ataacccaga taggaaatca 4500
tatgtttttt tccaagagtc attctaatat ttgattatgt tatgtgtgct tttatgaaag 4560
attgttattt ttatatatca agatgataga acctggaatg ttaggatttt gaaatgttag 4620
acttggaagg ggcctggtct gtcaactagt ccaacccctt aaaattcata gaggagcaaa 4680

ctggggccca ttgaagggtg aagagttact caaggtcaaa cagctggtaa cagaatcaag 4740

actaagacct aatttacctt tccatactct ttttttttct caacttcatc tatataaaat 4800

caggctttta aacataacca ctaatattta cctgaagata accatgagta aagtatactt 4860

ttgcattaat tttttgagct tatatgcaaa cataataaat attattaaat atcaggaaag 4920

ctaacatttc atacaagata gcttcagacc aaattcaaat tgaatttgaa taaattagaa 4980

atactgtgca tacataacct tcttgtgcac catgagtatt tggaaagtta atccttgttt 5040

ttgtcgtgtc tataaaggaa gaacaaaaca aaataaaaac agagccctag agaaatgctg 5100

ttacttttta tttttacacc catcagattt aaggaaaaga ctttttagcc attataatct 5160

agtggttgga aggaatgaag aagctttttt agtaataggt ccagatatga gtgctaaaaa 5220

taaagatgat agcatgttct tctgtcttcc atagttatta caactatgag agcctcccaa 5280

gtcatcttat caactcaact cccttttttt tgtcttaatg ttgcacataa gtttatacag 5340

agtggatgac cacactagca cagaagagaa caacatgtat taaagcaggt gattcctccc 5400

cttggcggga gagctctctc agtgtgaaca tgccttctgt gggcggaaat caggaagcca 5460

ccagctgtta atggagagtg ccttgctttt atttcagaca gcagagtttt ccaaagtttc 5520

tctgctcctc taacagcatt gctctttagt gtgtgttaac ctgtggtttg aaagaaatgc 5580

tcttgtacat taacaatgta aatttaaatg attaaattac attttatcaa tggca 5635

<210> 38
<211> 5573
<212> DNA
<213> Mus <mouse, genus>

<220>
<223> Mouse Nr4a3 (NM_015743.3)

<400> 38
cacacgcgcg ctcacacgct ccgcacacac actccactct ctcccgcgcg ctcacacccc 60

tctcacccgg ctccctcgcc agtgtcgcgc cgcgccgcgc cgcagccgga cgcccctcca 120

gggctcactt tgcaacgctg acagagcccg cagtgtccgt ggaggtggga aacgtggcga 180

catcctaccc cctggtcgca gccggagact ggatgcctgc ggaacctctc ggcggcgctc 240

tcccatgagt tgggatcgca gcatcccccg ccagccgctc accgcctctg ggagccgctg 300

ggtttgtgca ccgcagccct tccgggacag cagctgtgac tctcccccaa tccagatttc 360

ggggtcgctc tctagaaact cgctctaaag acggaaccgc cacagcaccc aaagcccact 420

gcgggagagc gcagcccgac aagcccgggc gctgagcctg gaccctcagc agagcgggcc 480

agcacagcgg ctgctgcttc gcctatcccg acgtccccgc ctcctacact ctcagcctcc 540

gctggagaga cccccagccc caccattcag cgcgcaagat accctccaga tatgccctgc 600

gtgcaagccc agtatagccc ttcacctccg gggtccactt acgccacgca gacttatggc 660
tcggaataca ccacagaaat catgaacccc gactacacca agctgaccat ggacctcggt 720
agcacgggga tcatggccac cgccactaca tccctgccca gcttcagtac cttcatggag 780
ggctacccca gcagctgcga actcaagccc tcctgcctgt accaaatgcc gccttctggg 840
cctcggcctt tgatcaagat ggaagagggt cgcgagcatg gctaccacca ccaccatcac 900
catcaccatc atcaccacca ccaccagcaa cagcagccgt ccattcctcc tccctccggc 960
cccgaggacg aggtactgcc cagcacctcc atgtacttca agcagtctcc gccttctaca 1020
ccgaccactc caggcttccc cccgcaggcg ggggcgctgt gggacgacga gctgccctct 1080
gcgcctggct gcatcgctcc gggaccgctg ctggacccgc agatgaaggc ggtacccccc 1140
atggccgctg ctgcgcgctt cccgatcttc ttcaagccct caccgccaca ccctcccgcg 1200
cccagtccag ccggcggcca ccacctcggc tatgacccca cggccgcagc tgcactcagt 1260
ctgcccctgg gagccgcggc cgcagcaggc agccaagctg ctgcgctcga gggccaccca 1320
tacgggctcc cgctggccaa gaggacggcc acgctgacct tccctccgct gggcctcaca 1380
gcctccccca ccgcgtccag cctgctggga gagagcccca gcctcccatc gccacccaat 1440
aggagctcat catctgggga aggcacatgt gccgtgtgcg gcgacaacgc tgcctgccag 1500
cactacggag tccgcacctg cgagggctgc aagggcttct tcaagagaac ggtgcagaaa 1560
aatgcaaaat atgtttgcct ggcaaataaa aactgcccag tggacaagag acgccgaaac 1620
cgatgtcagt actgcagatt tcagaagtgt ctcagtgtcg ggatggttaa ggaagttgtg 1680
cgtacagaca gtctgaaagg gaggagaggt cgtctgcctt ccaaaccaaa gagcccacta 1740
caacaggagc cctcgcagcc ctccccgcca tctcctccga tctgtatgat gaatgccctt 1800
gtccgagctt taacagatgc aacacccaga gatcttgatt attccagata ctgtcccacc 1860
gaccaggcca ctgcaggcac agatgctgag cacgtgcaac agttctacaa ccttctgacg 1920
gcctccattg acgtgtccag aagctgggca gaaaagatcc caggattcac tgatctcccc 1980
aaagaagatc agacgttact tatagaatca gcctttttgg agctgtttgt tcttagactt 2040
tccatcaggt caaacactgc tgaagataag tttgtgttct gcaatggact tgtcctgcat 2100
cgacttcagt gccttcgagg atttggggag tggctcgact ccattaaaga cttttcttta 2160
aacttgcaga gcctgaacct tgatatccaa gccttagcct gcctgtcagc actgagtatg 2220
atcacagagc gacatgggtt aaaagaacca aagagagtgg aggagctatg caccaagatc 2280
acaagcagct taaaggacca ccagaggaag ggacaggctc tggagccctc ggagcctaag 2340
gtcctgcgcg cgctggtaga actgagaaag atctgtaccc agggcctcca gcgcatcttc 2400
tacctgaagc tagaggactt ggtacctcca ccttctgtca tcgacaagct cttccttgac 2460
accctgcctt tctgagcagg ggaagcctga gcagagagct acttgctctg ctggcacctg 2520

atcattaagt gagcaaaaga atgggtttga acaccttcca cactcactgt ccttccttca 2580

ggggaaaagc agctcccgta gaaagcaaag actttttttt cttttcctgg cacctttcct 2640

tacaacctaa agccagaaac cttgcagagt atcgtgttgg ggttgtgttt tatatttagg 2700

ctttggtggg tgggttggga gggggtaaat agttcatgag gcttttctaa gaaattgctg 2760

acaaagcact tttggatgat gctatcccag cagtggggtg gggagaaagg ataatataac 2820

tgttttgttt ttgtttttgt ttttgttttt aaaaaaaaac tctttctggg gaatatgatt 2880

atggttgctt tgtatttaaa aataagaaca gccaagggct gttcaccagg gtagggctgt 2940

gtcctacgac ggatcccttt aggatatact tcctgtgtca agggtagata cgtggtgcaa 3000

atgaggcaga aaattccctt ttcttcattt ctttcttaaa aaaatggcaa aagatgaaag 3060

attatctaca aatcagactt agcaaaaatg ataatggcta tttgcttcca tatacaagtg 3120

caatttttta gagtgctgtc ttactaagtc ttgtttgtga actccccctt attttacatg 3180

aaaataagaa ggaggcagtc atgctatgaa atggcgcgct cattttccta gctaaggctt 3240

gatccacctg ccctgtagaa cccttcggag gtgtggccct tctaagactt tcaggccatt 3300

ctcgatggaa ttcgacccct gatgactatc cagccatcct gaagggggat cgggttataa 3360

agtggattgc atacaactgt cttcgctgtg ttttgtcaac ctggacagag ttctctaaac 3420

ctacttcagt tgtagcaagt tcctgattca tccactcaga agcccaatga gcactggttg 3480

actcaatcaa gtgttaaccc taggagaaca agcaaataag taggaactgg gtcatacagg 3540

gtaaacacca gagatgataa ggatatatat atatatatat atatatatat atatatatat 3600

atgtatatgt atatatatat acatattaat ttttgttatt ggttagagac aattttggaa 3660

agcaagagaa tcatcttttt ttaaaaaaga gagaagaaga agaagaaaag atagtattaa 3720

cgtgagttct agagaaggtt agtgggttgc ggttcaaaat tccgtgtttg tgcccccttt 3780

tatatgtttc tactgttgat gccatattat tatgaaatga tttgttgcat agtgtcctta 3840

tttgtatgaa catttgtatg cacgttatat tgtaatagct ttgcctgtat ttattgcaag 3900

accaccagct cctggaggct gagatacaga gtaatcaaat ggggtgttcg tggtgacttg 3960

gatacaccaa ttagaaatta aataagcata tatatatata aacatagccg gttacatata 4020

tatttataat gtgtcttttt attaaccatt tgtacaagaa atgtcacttc ccacgcagtt 4080

actttaccct tcacttgcag tgacctttaa ggcagcactg tttagcactt tgatatgaaa 4140

tttttgctta tgttttgcta aattctaata atgtttgaag attttaaggt ctaaaagtct 4200

ttatattata tactctgtat caagtcaaga tccctttggc cgttttgcta agactcaaaa 4260

actttgaatg tcaaactgat gtcacagtag cttttgttag cttttaatca tttttgcttt 4320

agtttttttt ttaaaaaaaa taacaaacta tactgtttat attgtcatta aattatacaa 4380

tcaaacaaat gccaaatgaa ttgcctaatt gctgcaaagt gtaacccaga taacaaatca 4440

tattctctcc tctttttgca actcattcag atgtttgatc ttgtgttttg tttgtataaa 4500

gattgtatga actatatcac cttcctatca aggtgacagg gcttgagatg ttaagatttt 4560

tgaacttcag acttggaagg ggcctggctc atcatctggt cagatctatt aaaattcaca 4620

aaggagaaaa gtgggggccc gttgcagggc aaacagctag caacaggata aaggctcaga 4680

cttaattcac cttcccttag tctcccccac atcttactgc atctgtagga aaataaactt 4740

ttaaacatag tcacgcatac taacacttta ccagcagaca gccttgaata aagtagtact 4800

ttggcattaa tttgttgagc ttatatgcaa acataataaa cattattaaa tatcaggaaa 4860

gctaacattt cacacgagat cgcttcagac caaattcaat ttgaatttga ataaattaga 4920

agtactgagc gtacgtaacc ttcttgtgca ccttgagtat tgcaaagttg ctccttggtt 4980

gttggttttt tttgttgttt cttttcgtcc tgtctgaaag gcaaagcaaa acagcagcac 5040

ggtgctctgg gaaatgctgt tcccccttct gtcctcacac ccacagattg aagggaaaga 5100

actgtctaag catctatctg ctggtttgaa gaatgatgaa gccgttttag ttaagggcca 5160

gacactagtg ctgaaagaaa agactataca gccagtgttc ttctgtcttc catagttatc 5220

acaactatga gagcctctta ggtcatcatt ttgaaatgtt gacacccaag tgtacccaga 5280

gtggaaggca cactagctct gcagaggaca agcattaatg caggtgattc ctcccgctga 5340

ctggagtgca gtctcagtgt gaacccgccc tcagcaggaa atgggaatcc acttgctgtt 5400

cctggagagt gccttgcttt tgtttcaaat ggccgagtct cccaagccct tctcctctaa 5460

cagcattgct ttttagtaag tgtgtgttaa cctgtggttt gaaagaaatg ctcttgtaca 5520

ttaacaatgt aaatttaaat gattaaatta aattacattt taccaatgga aaa 5573

<210> 39
<211> 5989
<212> DNA
<213> Homo sapiens

<220>
<223> Human ETV6 (NM_001987.4)

<400> 39
gcgtcccggg tccccgcgcc gcgccgcgac ctgcagaccc cgccgccgcg ctcgggcccg 60

tctcccacgc ccccgccgcc ccgcgcgccc aactccgccg gccgccccgc cccgccccgc 120

gcgctccaga cccccggggc ggctgccggg agagatgctg gaagaaactt cttaaatgac 180

cgcgtctggc tggccgtgga gcctttctgg gttggggaga ggaaaggaaa gtggaaaaaa 240

cctgagaact tcctgatctc tctcgctgtg agacatgtct gagactcctg ctcagtgtag 300

cattaagcag gaacgaattt catatacacc tccagagagc ccagtgccga gttacgcttc 360

ctcgacgcca cttcatgttc cagtgcctcg agcgctcagg atggaggaag actcgatccg 420

cctgcctgcg cacctgcgct tgcagccaat ttactggagc agggatgacg tagcccagtg 480

gctcaagtgg gctgaaaatg agttttcttt aaggccaatt gacagcaaca cgtttgaaat 540

gaatggcaaa gctctcctgc tgctgaccaa agaggacttt cgctatcgat ctcctcattc 600

aggtgatgtg ctctatgaac tccttcagca tattctgaag cagaggaaac ctcggattct 660

tttttcacca ttcttccacc ctggaaactc tatacacaca cagccggagg tcatactgca 720

tcagaaccat gaagaagata actgtgtcca gaggaccccc aggccatccg tggataatgt 780

gcaccataac cctcccacca ttgaactgtt gcaccgctcc aggtcaccta tcacgacaaa 840

tcaccggcct tctcctgacc ccgagcagcg gcccctccgg tcccccctgg acaacatgat 900

ccgccgcctc tccccggctg agagagctca gggacccagg ccgcaccagg agaacaacca 960

ccaggagtcc taccctctgt cagtgtctcc catggagaat aatcactgcc cagcgtcctc 1020

cgagtcccac ccgaagccat ccagcccccg gcaggagagc acacgcgtga tccagctgat 1080

gcccagcccc atcatgcacc ctctgatcct gaacccccgg cactccgtgg atttcaaaca 1140

gtccaggctc tccgaggacg ggctgcatag ggaagggaag cccatcaacc tctctcatcg 1200

ggaagacctg gcttacatga accacatcat ggtctctgtc tccccgcctg aagagcacgc 1260

catgcccatt gggagaatag cagactgtag actgctttgg gattacgtct atcagttgct 1320

ttctgacagc cggtacgaaa acttcatccg atgggaggac aaagaatcca aaatattccg 1380

gatagtggat cccaacggac tggctcgact gtggggaaac cataagaaca gaacaaacat 1440

gacctatgag aaaatgtcca gagccctgcg ccactactac aaactaaaca ttatcaggaa 1500

ggagccagga caaaggcttt tgttcaggtt tatgaaaacc ccagatgaaa tcatgagtgg 1560

ccgaacagac cgtctggagc acctagagtc ccaggagctg gatgaacaaa tataccaaga 1620

agatgaatgc tgaaggaacc aacagtccac ctcagcgggc cagcagccca gggaacccct 1680

gcccaccagg attgctggaa gtgtgacgga gcaggcgggc tgaggagagt ggaaaaggaa 1740

gcgacccaga aatggcaggg acacttctct tgcagaccaa gagggaccct ggagcacctt 1800

agacaaacta cccagcacag gcggggctgg aattctggcg gagggcatga gcctgggact 1860

ccatgtcacg tttccttctg atttggaatc tctccatctg taattcctca ccctcaccct 1920

tccaccgttg ttagtatcat ggtgtttttg tttttgtttt tgttttaaga acctgcagtt 1980

tgactcttca tcgttcatct aggggaagac atctgatgtt gttttcctat ggaaatatat 2040

atctattata tatatatttt ttgcaaatct cacaaagtgc ggcaagccca gctggtcagg 2100

aaagagaata cttgcagagg ggttcaggtt cctctttttc ctgccacgtg gatcaggtct 2160

gttcctgtta ctgttgggtc ttggctgaaa aaaaaaaatg cttttaaaaa agataaaatg 2220

aaaaggagag ctctcttttt ctctctcttg ctctgttctt cccttggtcc cctctgtcct 2280

cccgccctgc ctgcagttga gattcagatg ccttctgaca gagttcagcc tcttggagag 2340

tcttggggat tgttggcacc taaacagaat cagtgacccg ggtgctttgt ggccagcagc 2400

acagaatcaa acccgcatcc cagcattggg ccacccatct gagggaggcc aaaatcatca 2460

cagatgctgc tgtgctgcag acagatacat gctagtccag agagccgccc ctgagatggc 2520

tgtgagaacc atgtgtctaa ggcgtaagat aaggatggaa ggctgtccaa gttatttgga 2580

aggcctcggc agcttgggat tagcttggga gcgcagcgct gcaaagtgga aaatatgaaa 2640

agaccacaca ggcccagcag tccagaaact gggcaaaaat attctgcagt ggggatttat 2700

ttttccaaag caggtaacag aggctagtga gaaagaaaag ctcctctctg ctccattcca 2760

aaggccatct tgtggtcagt ttcatgccct cacctgattt tttttttttt tttttttttt 2820

caattcctaa ccttttttaa agtttcctgg tctccactgg acacagagct ttggagacgg 2880

aggatcccag agggcagtct cagttgcaat cagtgtgtgc ccagcctggg cagacaggaa 2940

attcctcgga tacattattt tttctttctt tcatagctgt gtctcagaaa ggacccattt 3000

gtggctcttt ttcacctcaa aataagatcg atggtatctt gtaaaatgag ggtagtgcca 3060

cttcttagta tttttgaaag ctgttttaga tttttttttt ttttcctttt ctagccatct 3120

aaattgactc ttccaatata ggtctcagaa atccaatatt tggagtacaa tttcttttaa 3180

tccagattac acctgcctta caaagcaccc cctccttgtt cccctctgtt tcctctactc 3240

agttggggga gaaactcaca gctcctccgg gatacatatg tgccctcagc agcagctccc 3300

aggtgaagtt accagacccc tgggcttctc cccagctttt tctgagttga gtcagacatg 3360

tagagtttgg gtcacacagg caagaggaat tttccctcgg ccttactgac aaggacacca 3420

acctagggtg caaacagatg gactatggtt caaggacact ggaattgagg agctgatcaa 3480

ggctctcttc agccttgctc tgtccctgcc tcttatcaga gcacaggtag acacacgggc 3540

atagccagcc cactcctact gtcacaggcg ccccaccatt caaccttccg ggaggtcagg 3600

gaccttctat atgaggcgag tgggtctcag tctgcttgaa tggtgatgag attctgctgg 3660

atctcagcac gctgcaggtg tcttttgaga gcattcagta ggacatggtg atccctattt 3720

cagcctctaa gatgactggt attctatctg aaatgcagag attaagccaa atacctgatg 3780

tattgtgaaa gccactgatt ttaagaatgg agagaaaggg attttttact gcatccctct 3840

gtatgaatat gaaatcagag accagggcat gatgttgcta ggattagagc ctctcagtct 3900

ggcctcttca cccaagtgca agaactcagt ctcttactgt tcaaagaatc ttaacagttg 3960

aattatggag ggaaattccc ttttgcccca agcatttcta tatttaaagc aatatcccag 4020

gagaatatgt tagacttagg atgatacctt cagccacttg aagaagaaat agaaggcgct 4080

cattccaata tagtctttat ttcccattca gatacaggtt gagcatccct aatctgaaca 4140

gttaaaaccc ccaaatgccc caaaatccaa accttcctga acgctatgac accatgagtg 4200

gaaaattcca cacctaacaa acacatttgc tttcttatgg ttcaatgtac acaaactgtt 4260

ttatatagaa aatgatttca aatatcataa aattaccttc aggctatgtg tataaagtat 4320

atatgagcca taaatgaatt ttgtgtttag actttgtgtc catccccaag atctctcatt 4380

ttatatatat atatatatat atatatatat atatatatat atatatatac acacacacac 4440

acatacacaa atattccagg atacaaaaaa aaacatttaa aaatccgaga cccagaacac 4500

ttctggtccc aagcatttca gataagggat atcaatctgt actaccaata aggatttcgt 4560

aattccccta actgcaaatg tcctcttcat ttgttcttta tgagaaaacc cgggtagtgc 4620

cagcacctgg atacagtatt tacaccctgc agaccctaaa gatttcagat tcagttagca 4680

aaccttgatg aagcacctgc tggacactga gggacccaaa gctcaatcag ccataatccc 4740

tgctttcaga gtttatattg tacctgccta atccacccgg cgtgactcat ttcaacacta 4800

agtactaggg gtgttgtcag gagacaaatc tgaagtcagg agaggaaaat gcaaaggagc 4860

cctgccgtgt gatggatgtg cattctcact tgggtcttga agttctcatt cctacatctc 4920

aagctagcca ggcagtctcc tctctatcag aagaaagcac tggtaattgg ctagactggc 4980

tatgttgaag gtaacatgaa ctctaagatc ttgacccagg gcgacttggt tttgcttaag 5040

gtggcatcac caatgttcca aatcctttag ggagatgagg gtatccccac agaaaaagag 5100

gaataataga ccaatggatt ttctcctttc accagtatgt ttggaaccct ctgatccaat 5160

gtcttttgat actgatctct tgtccaaatg agaatgtcgc tttagctgaa attcaaatgg 5220

ctgtgacaat ttaccgaaat gatgaagtaa ccaccattcc cacctttcac tgcctaggct 5280

ccaagtctga atacattttt gaaataggaa ctcccttttg caaaaaagaa acctgggtgt 5340

cagggaggtg aagtgacttg ccctaggagc agacagcatg ccaagaatgg aattaggctc 5400

aggatccagc ctgggctcac cctgtgtggc tcattcccac ccaggaaact gaagataaaa 5460

gatttgggaa aacacaccaa gaaaaagggg cagttttctt tgcccaagca tttggtgcta 5520

gttagaggct gttcactctc tcctgctcct cttcggagta gaaataaagg ctgtgacaca 5580

aggaagccag tggggtggga gggaggcacc ataatccctc cctaaaaccc acagaagact 5640

aacctgatac tcttttgacc caactgcatc aacactaaac agctgcagac cccctgaatc 5700

tttcacacat gccaagtgaa cattcttgat gatttctctt tgtgaccgca accacctgca 5760

aaccagaacg actctagaat ttccttcccc gccccccttt ttgtttagtt tctaatctct 5820

tgtttatgag gtgtggggtt tataagggac tgaatcaaat gaatgtaaca aaaaagaaaa 5880

aaaaaacaaa aaaaaatgcc ttttctcagg gccagtgagt tgcaaataat ttttaaagaa 5940

aagcctataa ttacatcatc tcaataaatt ttttataaaa aaaaaaaaa 5989

<210> 40
<211> 5548
<212> DNA
<213> Mus <mouse, genus>

<220>
<223> Mouse ETV6 (NM_007961.4)

<400> 40
ttctgccttt cagtttctct cttccaggaa ggaaaacatt cgagaaagag agagggaggg 60

agggagcgag ggagcgagag agcgagcgag cgagcgccgc gggagggcgg gcgcgccggc 120

cgcgggtggg aggagacacg ccaggccggc ctgctgcgcc cgggtcgccg cgccgcgacc 180

cgcacacccc acgccgtgct cgccggctcc tacgcccccg cacctcgcgc ccaatccgcg 240

cgttgagaag agccccccct cccccctcgg cggccgccgc cagcgatgct gcaagaaact 300

tcttaaacga ccgcatcccg ctgccccgcc gagcgtttct gggttgggga gaggaaagga 360

aagtggaaaa aaactgagaa cttcctgatc cctttcgctg tgagacatgt ctgagactcc 420

tgctcagtct agcattaagc aggaacgaat ttcatacacg cccccagaga gtccagtggc 480

aagccaccgt tcctcgactc cgcttcatgt tcacacagtg cctcgagcgc tcaggatgga 540

ggaagactcg atccacctgc caacacacct gcgtttgcag ccgatttact ggagcagaga 600

tgacgtagcc cagtggctca agtgggcaga aaatgagttt tctttaaggc ccattgagag 660

caacaagttc gaaatgaatg gcaaggccct cctgctgctg accaaagagg atttccgcta 720

ccgatctcct cattcaggcg acgtgctcta tgaactcctt cagcatatcc tgaagcagag 780

gaaatctcga atgctcttct caccattctt cccccctggg gactctatcc acaccaagcc 840

agaggtcctc ctgcatcaga accatgacga agataactgt gtccagagga cacccaggac 900

gcccgcggag agcgtgcacc acaaccctcc caccatcgaa ctcttacatc gccctaggtc 960

acccatcacc acaaaccaca ggccttctcc tgaccccgaa cagcagcggc cccagcggtc 1020

ccccctagac aacatgagcc gccgcctctc gccagtggag aaagcccagg ggcccaggct 1080

acagcaggag aacaaccacc aggaaacgta ccccctgtca gtgtctcctg tcgagaataa 1140

tcactgcctg ccctcaagcc cctggcagga gagcactcga gtgatccagc tgatgcccag 1200

ccccatcatg caccctttga tcctgaaccc ccggcactcg cactcggtgg acttcaaaca 1260

gtcccggcac tccgaggatg ggatgaatcg ggaagggaag cccatcaacc tgtctcatcg 1320

ggaggacctg gcttacttga accacatcat ggtctctatg tccccaccgg aagagcacgc 1380

catgcccatt gggagaatag cagactgtag actgctttgg gattatgtct atcagttgct 1440

gtctgacagc cggtacgaaa acttcatccg atgggaggac aaagaatcca aaatattccg 1500

gatagtggat cccaacggac tggctcgact ctggggaaac cataagaaca gaacaaacat 1560

gacctatgag aaaatgtcca gagccctgcg ccactactac aaactaaaca ttatcaggaa 1620

ggagcccgga caaaggcttt tgttcaggtt catgaaaacc ccagatgaga tcatgagtgg 1680

ccggacagac cgtctagaac acctcgagtc tcaagtgctg gatgaacaaa cgtaccaaga 1740

ggatgaacct accatagcct caccggtggg ctggccaaga ggaaacctgc ccacggggac 1800

cgcaggaggc gtgatggaag caggcgagct aggggtggct gtaaaggaag agacccggga 1860

atagcaggga cacttctcct gccgatcaag agggacccag agcaccttag acaagccacc 1920

gagcaatggc agggctgaag ttctggcgga gggcacaagc ctgagactca cacgtcacgt 1980

tcgcttctcc ttctgatctc ttgtctgtaa ctctcaccct ctcccttccc ctacacctgt 2040

tgtagtctca tggtgtttct ggtttcgttt tttgtttttg ttttttaaga acatgcagtt 2100

tgactattca ttgttcatac agggaagaca tcacatgttg ttttcctatg gaaatatatc 2160

tattatatat atattattaa tttttttgtt gttgttgcaa atctcaccaa gtacggccag 2220

cttggctggt caggaaagag aaaacttgca gaaggaatca ggttcctctt tttcctgcca 2280

catagatctg gtgtgtcctg tccaagtcag gtcttagatg agaaaggaaa acaaaatgag 2340

agagatactt tcaaacaatg gaaaagaaga gttctgtctc catctccctc tctcgccgct 2400

catccccgcc cctttctcct tcccccttct ctgcctgcta tgcacttcac aggcttttca 2460

tcagagctag cctcactagc acctgaacta cacagtgacc cggctgctct gtgacagtca 2520

gaccccttgg gcacccatct gaggaaggcc agacatctcc ccccaccaaa tgctgctgta 2580

ttctagaaag aacaatggga ggatcgagca taagatagga cagagggtgg ccacaggatt 2640

tgcaaggccc aggcagtttg ggattgtcct gaaagtgcta cacattagga aaatatgaga 2700

gaccacacag gcctggtagt ttaggagcaa gaacaggtca ccaatgttgt cccaagacat 2760

catgcccttg tgggattgtc accaccccac acacacactc caagtcctag cctttttctt 2820

ctatgctggt cctcatggaa cacagaacac acatgaggga ggatgctgga atttagtctc 2880

aggcacagcc ggtatttgcg cagacaggaa attcctcaga tttagacttt ttcctttttt 2940

ccgtctgtca tggctgtatt tcaggaggac atatctgtgg ctcttctcca cctcgacata 3000

aggtcagtag catcttccaa gatgagggta atgctattcc ttaggatttt tgatagcagg 3060

gattttattt tatttattta tttatttttg ggattttttt ttttgagaca gggtttttct 3120

gtgtatccct ggaattcact ctgtagacca gactaggact cagagatcta cctgcctctg 3180

cctcccaagc actgagatta aaggtgtgcg cccccatgct tagctttttt tccttttcta 3240

gccatctaag tggactcttt gaagttcact gttttaaaac ccagataatg aggcctgcct 3300

ttcaaaacca ccccttgtcc ttcctgtgtt ctcccacatc atggaatgag cctctcccag 3360

ccctcctggg tgtgtctctg cttggtcaga cgcaggacac aggtgaggct gacagaccag 3420

agctgaccta caactactcc taagtggggc acatagaggc taggaggacc tggacaagtc 3480

actctcccga cgtcagctca caggcggact gtggttcaca gacactggaa ttgagttgac 3540
tgggtatctt tagtcttgct gtgacttccc cgtgcctccc ccacaaccca caatctgcct 3600
tcaaagaggt cagtgacctg ggtgaggggt tccctcttaa tattcatggg gaatgatgag 3660
agtcacgtgc tatgtgtttc ttgaaagcat gacaggggtt cctttttttg gcctctaaaa 3720
atgactggta ccttatatca aatggggaga ttaagccaaa tacttgtgtt gtatgagcca 3780
ctgggaggaa gagcaagtga gaaagggtgt gctttttgtt tttgttttga ctgaggtcct 3840
ttgtgtgaat ataaaatgag aaactacaac acttcatact ggccactttg cccatgcgta 3900
gaactcagtc ctttaactgc ccaaagaatt gtccatatga actgtgtaag gaaattcctc 3960
tgttccctaa ggagtcttgg atgacagctt tggccactta cgttagaaat gctcatggca 4020
gtgccgtctt tatttcacac ctggtgttgc cataattgat ttctttgatt ctcttacctg 4080
caaaagccct tgtttgtttt ttggagagaa aatatggcca gggttgtctg ggttgtcagg 4140
gttgtgcagt gctctcaggc ttcagactca cagaatttag atttagccaa tcagaccttg 4200
gaggaaacac cacaatgggg gtcctgtttt gtctttttgg tttttgagtt ttggtttttt 4260
gtttgtttgt ttttttacaa tcaactatga tcttctagat cgacattaaa gcgacattaa 4320
gttcaagagg tgatagaagg aatctaaaag ctcctttagt ggcctttttc tgcagccatt 4380
gtgggttgag tcaggaaagg aatttgcagg gaagcattgc catgtggagg atgggcattg 4440
ccacctaggt cctgggattc tgtcctgagt cctcggccgg gctgtcttca ggtctccttc 4500
agcagaaacc aggggcagct ggtcagatgt tgacagcagc ttctcttccg ataattttca 4560
tccaaggcca cttagtgcca cttggagaca gggtccctga tgtcccatat ctggttttgg 4620
aaagagaatg gtcctcataa gaaaagaaca aaacaggctt cttgcctagt acattcaggt 4680
ccctctgatc cagcatcttt ttacactaat accttgtcca gataagaatg tcactccagc 4740
tgacatctag atgtctgtga ggagtccctg gaatgaccac tcttgtcttt tcacactgtt 4800
cgggatccaa ccataaacac agtttggccc attcactgtt gttggcagaa gacaacctga 4860
gagaggtcgt gtcactttcc gtgggagcag acagcatgac acacagttgg gatcagtatc 4920
tagccggcct gcacttagca gcaggataag cgttcagaag gcagttagaa attattctgg 4980
tttaaacccc catttgatcc atccccggca aagagacgaa agatgccaag ggtggttttt 5040
actgcccagt cattcggtgc tagttagagc tgctgtgcgt tctcagcctg ctcctcttct 5100
gaacagaaac caaaattgga cgtcatgaga aacccagtgg agcaggggta cagagggaca 5160
ccccagaacc ctgcctatca acaaggcaca cgggactcac ccagcactct tttggcccca 5220
tccgcatcaa cactaaacaa cttcaaatgc gcccgactct tttcaccctc acaggtgaac 5280
cctcttctgg agacttctct ttgtgacggc acccacctgc aagccagatg aatctagaat 5340
gactttttgt tgttgttgtt gtttagtttc taatctcttg tttatgaggt gtggggttta 5400

taagggactg aatcaaatga atgtaacaaa aaaagaaaaa aaaacaaaca aaaaatgcct 5460

tttctcaggg ccagtgagtt gcaaataatt tttaaagaaa aacctataat tacatcatct 5520

caataaattt tttataaaaa aaaaaaaa 5548

<210> 41
<211> 5967
<212> DNA
<213> Homo sapiens

<220>
<223> Human RUNX1 (NM_001754.4)

<400> 41
ctttgggcct cataaacaac cacagaacca caagttgggt agcctggcag tgtcagaagt 60

ctgaacccag catagtggtc agcaggcagg acgaatcaca ctgaatgcaa accacagggt 120

ttcgcagcgt ggtaaaagaa atcattgagt cccccgcctt cagaagaggg tgcattttca 180

ggaggaagcg atggcttcag acagcatatt tgagtcattt ccttcgtacc cacagtgctt 240

catgagagaa tgcatacttg gaatgaatcc ttctagagac gtccacgatg ccagcacgag 300

ccgccgcttc acgccgcctt ccaccgcgct gagcccaggc aagatgagcg aggcgttgcc 360

gctgggcgcc ccggacgccg gcgctgccct ggccggcaag ctgaggagcg gcgaccgcag 420

catggtggag gtgctggccg accacccggg cgagctggtg cgcaccgaca gccccaactt 480

cctctgctcc gtgctgccta cgcactggcg ctgcaacaag accctgccca tcgctttcaa 540

ggtggtggcc ctaggggatg ttccagatgg cactctggtc actgtgatgg ctggcaatga 600

tgaaaactac tcggctgagc tgagaaatgc taccgcagcc atgaagaacc aggttgcaag 660

atttaatgac ctcaggtttg tcggtcgaag tggaagaggg aaaagcttca ctctgaccat 720

cactgtcttc acaaacccac cgcaagtcgc cacctaccac agagccatca aaatcacagt 780

ggatgggccc cgagaacctc gaagacatcg gcagaaacta gatgatcaga ccaagcccgg 840

gagcttgtcc ttttccgagc ggctcagtga actggagcag ctgcggcgca cagccatgag 900

ggtcagccca caccacccag cccccacgcc caaccctcgt gcctccctga accactccac 960

tgcctttaac cctcagcctc agagtcagat gcaggataca aggcagatcc aaccatcccc 1020

accgtggtcc tacgatcagt cctaccaata cctgggatcc attgcctctc cttctgtgca 1080

cccagcaacg cccatttcac ctggacgtgc cagcggcatg acaaccctct ctgcagaact 1140

ttccagtcga ctctcaacgg cacccgacct gacagcgttc agcgacccgc gccagttccc 1200

cgcgctgccc tccatctccg acccccgcat gcactatcca ggcgccttca cctactcccc 1260

gacgccggtc acctcgggca tcggcatcgg catgtcggcc atgggctcgg ccacgcgcta 1320

ccacacctac ctgccgccgc cctaccccgg ctcgtcgcaa gcgcagggag gcccgttcca 1380

agccagctcg ccctcctacc acctgtacta cggcgcctcg gccggctcct accagttctc 1440

catggtgggc ggcgagcgct cgccgccgcg catcctgccg ccctgcacca acgcctccac 1500

cggctccgcg ctgctcaacc ccagcctccc gaaccagagc gacgtggtgg aggccgaggg 1560

cagccacagc aactccccca ccaacatggc gccctccgcg cgcctggagg aggccgtgtg 1620

gaggccctac tgaggcgcca ggcctggccc ggctgggccc cgcgggccgc cgccttcgcc 1680

tccgggcgcg cgggcctcct gttcgcgaca agcccgccgg gatcccgggc cctgggcccg 1740

gccaccgtcc tggggccgag ggcgcccgac ggccaggatc tcgctgtagg tcaggcccgc 1800

gcagcctcct gcgcccagaa gcccacgccg ccgccgtctg ctggcgcccc ggccctcgcg 1860

gaggtgtccg aggcgacgca cctcgagggt gtccgccggc cccagcaccc aggggacgcg 1920

ctggaaagca aacaggaaga ttcccggagg gaaactgtga atgcttctga tttagcaatg 1980

ctgtgaataa aaagaaagat tttataccct tgacttaact ttttaaccaa gttgtttatt 2040

ccaaagagtg tggaattttg gttggggtgg ggggagagga gggatgcaac tcgccctgtt 2100

tggcatctaa ttcttatttt taatttttcc gcaccttatc aattgcaaaa tgcgtatttg 2160

catttgggtg gtttttattt ttatatacgt ttatataaat atatataaat tgagcttgct 2220

tctttcttgc tttgaccatg gaaagaaata tgattccctt ttctttaagt tttatttaac 2280

ttttcttttg gacttttggg tagttgtttt tttttgtttt gttttgtttt tttgagaaac 2340

agctacagct ttgggtcatt tttaactact gtattcccac aaggaatccc cagatattta 2400

tgtatcttga tgttcagaca tttatgtgtt gataattttt taattattta aatgtactta 2460

tattaagaaa aatatcaagt actacatttt cttttgttct tgatagtagc caaagttaaa 2520

tgtatcacat tgaagaaggc tagaaaaaaa gaatgagtaa tgtgatcgct tggttatcca 2580

gaagtattgt ttacattaaa ctccctttca tgttaatcaa acaagtgagt agctcacgca 2640

gcaacgtttt taataggatt tttagacact gagggtcact ccaaggatca gaagtatgga 2700

attttctgcc aggctcaaca agggtctcat atctaacttc ctccttaaaa cagagaaggt 2760

caatctagtt ccagagggtt gaggcaggtg ccaataatta catctttgga gaggatttga 2820

tttctgccca gggatttgct caccccaagg tcatctgata atttcacaga tgctgtgtaa 2880

cagaacacag ccaaagtaaa ctgtgtaggg gagccacatt tacataggaa ccaaatcaat 2940

gaatttaggg gttacgatta tagcaattta agggcccacc agaagcaggc ctcgaggagt 3000

caatttgcct ctgtgtgcct cagtggagac aagtgggaaa acatggtccc acctgtgcga 3060

gaccccctgt cctgtgctgc tcactcaaca acatctttgt gttgctttca ccaggctgag 3120

accctaccct atggggtata tggcttttta cctgtgcacc agtgtgacag gaaagattca 3180

tgtcactact gtccgtggct acaattcaaa ggtatccaat gtcgctgtaa attttatggc 3240

actattttta ttggaggatt tggtcagaat gcagttgttg tacaactcat aaatactaac 3300
tgctgatttt gacacatgtg tgctccaaat gatctggtgg ttatttaacg tacctcttaa 3360
aattcgttga aacgatttca ggtcaactct gaagagtatt tgaaagcagg acttcagaac 3420
agtgtttgat ttttatttta taaatttaag cattcaaatt aggcaaatct ttggctgcag 3480
gcagcaaaaa cagctggact tatttaaaac aacttgtttt tgagttttct tatatatata 3540
ttgattattt gttttacaca catgcagtag cactttggta agagttaaag agtaaagcag 3600
cttatgttgt caggtcgttc ttatctagag aagagctata gcagatctcg gacaaactca 3660
gaatatattc actttcattt ttgacaggat tccctccaca actcagtttc atatattatt 3720
ccgtattaca tttttgcagc taaattacca taaaatgtca gcaaatgtaa aaatttaatt 3780
tctgaaaagc accattagcc catttccccc aaattaaacg taaatgtttt ttttcagcac 3840
atgttaccat gtctgacctg caaaaatgct ggagaaaaat gaaggaaaaa attatgtttt 3900
tcagtttaat tctgttaact gaagatattc caactcaaaa ccagcctcat gctctgatta 3960
gataatcttt tacattgaac ctttactctc aaagccatgt gtggaggggg cttgtcacta 4020
ttgtaggctc actggattgg tcatttagag tttcacagac tcttaccagc atatatagta 4080
tttaattgtt tcaaaaaaaa tcaaactgta gttgttttgg cgataggtct cacgcaacac 4140
atttttgtat gtgtgtgtgt gtgcgtgtgt gtgtgtgtgt gtgaaaaatt gcattcattg 4200
acttcaggta gattaaggta tctttttatt cattgccctc aggaaagtta aggtatcaat 4260
gagaccctta agccaatcat gtaataactg catgtgtctg gtccaggaga agtattgaat 4320
aagccatttc tactgcttac tcatgtccct atttatgatt tcaacatgga tacatatttc 4380
agttctttct ttttctcact atctgaaaat acatttccct ccctctcttc cccccaatat 4440
ctcccttttt ttctctcttc ctctatcttc caaaccccac tttctccctc ctccttttcc 4500
tgtgttctct taagcagata gcacataccc ccacccagta ccaaatttca gaacacaaga 4560
aggtccagtt cttccccctt cacataaagg aacatggttt gtcagccttt ctcctgttta 4620
tgggtttctt ccagcagaac agagacattg ccaaccatat tggatctgct tgctgtccaa 4680
accagcaaac tttcctgggc aaatcacaat cagtgagtaa atagacagcc tttctgctgc 4740
cttgggtttc tgtgcagata aacagaaatg ctctgattag aaaggaaatg aatggttcca 4800
ctcaaatgtc ctgcaattta ggattgcaga tttctgcctt gaaatacctg tttctttggg 4860
acattccgtc ctgatgattt ttatttttgt tggtttttat ttttgggggg aatgacatgt 4920
ttgggtcttt tatacatgaa aatttgtttg acaataatct cacaaaacat attttacatc 4980
tgaacaaaat gcctttttgt ttaccgtagc gtatacattt gttttgggat ttttgtgtgt 5040
ttgttgggaa ttttgttttt agccaggtca gtattgatga ggctgatcat ttggctcttt 5100
ttttccttcc agaagagttg catcaacaaa gttaattgta tttatgtatg taaatagatt 5160

ttaagcttca ttataaaata ttgttaatgc ctataacttt ttttcaattt ttttgtgtgt 5220

gtttctaagg acttttcttt aggtttgcta aatactgtag ggaaaaaaat gcttctttct 5280

actttgttta ttttagactt taaaatgagc tacttcttat tcacttttgt aaacagctaa 5340

tagcatggtt ccaatttttt ttaagttcac tttttttgtt ctaggggaaa tgaatgtgca 5400

aaaaaagaaa aagaactgtt ggttatttgt gttattctgg atgtataaaa atcaatggaa 5460

aaaaataaac tttcaaattg aaatgacggt ataacacatc tactgaaaaa gcaacgggaa 5520

atgtggtcct atttaagcca gcccccacct agggtctatt tgtgtggcag ttattgggtt 5580

tggtcacaaa acatcctgaa aattcgtgcg tgggcttctt tctccctggt acaaacgtat 5640

ggaatgcttc ttaaagggga actgtcaagc tggtgtcttc agccagatga catgagagaa 5700

tatcccagaa ccctctctcc aaggtgtttc tagatagcac aggagagcag gcactgcact 5760

gtccacagtc cacggtacac agtcgggtgg gccgcctccc ctctcctggg agcattcgtc 5820

gtgcccagcc tgagcagggc agctggactg ctgctgttca ggagccacca gagccttcct 5880

ctctttgtac cacagtttct tctgtaaatc cagtgttaca atcagtgtga atggcaaata 5940

aacagtttga caagtacata caccata 5967

<210> 42
<211> 5803
<212> DNA
<213> Mus <mouse, genus>

<220>
<223> Mouse Runx1 (NM_001111021.2)

<400> 42
gtgaaaactt ctttggacct cataaacaac cacagaacca caagttgggt agcctggcag 60

tgtcagaagt gtaagcccag cacagtggtc agcaggcagg acgaatcaca ctgaatgcaa 120

accacaggct ttcgcagagc ggtgaaagaa attatagaat cccccgcctt caggagaggt 180

gcgttttcga aaggaaacga tggcttcaga cagcattttt gagtcatttc cttcatatcc 240

acagtgcttc atgagagatg ccagcacgag ccgccgcttc acgccgcctt ccaccgcgct 300

gagccccggc aagatgagcg aggcgctgcc gctgggcgcc ccggatggcg gcgccgccct 360

ggccagcaag ctgaggagcg gcgaccgcag catggtggag gtactagctg accaccctgg 420

cgagctagtg cgcaccgaca gccccaactt cctctgctcc gtgctaccca ctcactggcg 480

ctgcaacaag accctgccca tcgctttcaa ggtggtggca ctgggggacg tcccggatgg 540

cactctggtc accgtcatgg caggcaacga tgaaaactac tcggcagaac tgagaaatgc 600

taccgcggcc atgaagaacc aggtagcgag attcaacgac ctcaggtttg tcgggcggag 660

cggtagaggc aagagcttca ctctgaccat caccgtcttt acaaatccgc cacaagttgc 720

cacctaccat agagccatca aaatcacagt ggacggcccc cgagaacccc gaagacatcg 780

gcagaaacta gatgatcaga ccaagcccgg gagtttgtcc ttttccgagc ggctcagtga 840

attggagcag ctgcggcgca cggccatgag ggtcagcccg caccacccag cccccacgcc 900

caaccctcgg gcctccttga accactccac tgcctttaac cctcagcctc aaagtcagat 960

gcaggatgcc aggcagatcc agccatcccc accgtggtcc tatgaccagt cctaccagta 1020

cctgggatcc atcacctctt cctctgtcca cccagcgaca cccatttcac ccggccgtgc 1080

cagcggcatg accagcctct ctgcagaact ttccagtcga ctctcaacgg ctccggacct 1140

gaccgccttc ggcgacccac gccagttccc tactctgccg tccatctccg acccgcgcat 1200

gcactaccca ggcgccttca cctactcgcc gcccgtcacg tcgggcatcg gcatcggcat 1260

gtcagccatg agctcggcct ctcgctacca cacctacctg ccgccgccct accccggctc 1320

atcacaggcg caggccgggc ccttccagac cggctcgccc tcctaccatc tatactacgg 1380

cgcctcggcc ggttcctacc agttctccat ggtgggcgga gagagatcgc ccccgcgcat 1440

cctgccgccc tgcaccaacg catccaccgg cgccgcgctg ctcaacccca gcctccccag 1500

ccagagcgac gtggtggaga ccgagggcag ccatagcaac tcgcccacca acatgccccc 1560

cgcgcgcctg gaggaggccg tgtggcggcc ctactgagct gagcgccatc gccatcgagg 1620

gactgggcct gccgtccatg cacagacccc gccaggaggg cccttggagg ccaccaggaa 1680

gaatcccgga gggaaactgt gaatgcttct gatttagcaa tgctgtgaat aaaagaaaga 1740

ttttataccc ttgacttcac tttttaacca cgttgtttat tccaaagagt gtggaatgtt 1800

ttcggttcgg ggtggggaag acgcagccca tcctgtttgg catctatttc ttatttcgga 1860

gttttctttt ccgcacctta tcgattgcaa aaatgcctgt ttgcatctgg gtggtcattt 1920

atttttaagt gtgtatagat ttgagcttgc ttttttttct tcctttgacc aactcaaaga 1980

aataaaattc ccttctctgt aaggtttatt taacttttag actttcatgt agctgggggt 2040

tttatttgtg tttggttttt gtttttattt ttaaagagac agctacagct ttgggtcatt 2100

ttttaactac tgtatttcca caaagaaatc cctagatatt tatgtatctt gatgtttgaa 2160

catttacata tgtgttgata cttttttaat tatttaaatg tacttatatt aagaaagata 2220

tcaagtacta catttttctt tataatagcc aaagttaaat attattgcgt tgaagatgtc 2280

tggaaaaaaa agagatcgct tggttaacta gaaatattgt ttacattaaa ctccctttat 2340

gttattcaaa caagttggta ggtaacgcag caatgttttt aattggattg tagacactga 2400

gggtcactcc aaggtcagaa gtacaaaatt ttctgctagg ctcaacaaat agtctcatac 2460

ctggctcctt cccttcaaaa agagaggcaa actctgtcct gaaagggttc agagaggtgc 2520

caaggatttg ctctgaagag gatttcattt tggcctggag atatacttgc cccaaggcct 2580

cctcattctg gcatgcttta tcacagagct caaccaagta agctgttggt caggggttta 2640

cttacatagt atttacatag acccaaacca ctgaatgtga tttttaaatt gccttccatt 2700

aatagtaccc gttcattgat gaaaaccaaa acttgaggct gtaccccaaa gatccaaata 2760

gaagagttaa gaccaggtgt ctttgaggcc taaaggctga gttttaagag agtgtacccc 2820

aaaagtctga aggagccggt ttccttctcc cagtcttagt ggaatcagtc atgggaggca 2880

gatgccacgc ccacctgtgc aggatgctcc tcagaagctg ccccttcacc agcatcttct 2940

cccaccaggc cgagcccctg acctttgggg tgcatcagtg tgatagatcc tggtctctgc 3000

agtccgccat ggctacggtt cagatgtgca tcgtgtcact gtaaatgtaa tggtactgtt 3060

gttacagtgg aggacttggt caaaatccag ttgttctaca acgtatgaag cctaaccgct 3120

ggttctgaca tacatgtgct caaaatgatc tggttgtttg gatttttctt ttgttgtttt 3180

gttttttaat gtacctctta aattagttga agtgatgtca ggtcaactcc gaagagcgtt 3240

tgaaagcagg acttcagcac agtgtttgat ttttttatta ttattaatat tattttataa 3300

atttaagcat tcagattaga tctttggctg caggcagcaa aaacggctgg acttatttaa 3360

aaaaaataca gcttgttttt tgagttatct atatctatat ctatatgttg attctttgtc 3420

ttacatagag cagcagcact ttggtaacct gtgataccag gttgctcttg tctggagaag 3480

agcgctagca ggattcagag aaactcagaa tagatcttca tatcagccat accttcctcc 3540

tccatccggt ctccactcag ttattccaca gaacactttg acagctgtgt tgtcagaaaa 3600

ataaaaaaaa atttaatttc tcaaaaggag tttgtttctc caacattaga tgttcctctt 3660

accataggct gccgtatctg gcctgagaaa acggtaggga aggacgaagg aaagagattt 3720

ctattttttc atattaattt tgatatctaa agatacgcta gccctcagag gagcagataa 3780

tctcacacat tgaattttcg ccctgggcac catgcatcaa gaaggcttgt cactgtgtta 3840

gagccattta gtgcttccta aacttttatc aacataggca gtatttagtc tcagagaaaa 3900

aaaaatccat caggcacatg tagtcttgga gatagattcc acggggcagg tatttctcta 3960

cctgagaaat tgtgttcatt gccttcgggt gcttccagcg gtctcctcat tcgctgtctt 4020

caaggaagac ccataagcca attctgagat aatggagctg ttgggaatac tggtccagag 4080

aaagaaaaat gggataagcc attcttactg cttattcaag cccctattta taattttaac 4140

acactttcca ttccttctgg ttttctcgcc gtctatatcc tcccaatagc ccttctcact 4200

tttcttttcc ctcctgcaaa cacacacaca cacacacaca cacacacaca taaggcacac 4260

acacacacat cctctccccc ataccaagtg tccagaacac agaaagtcca gttcttctcc 4320

gtttattaaa gaacagggtg agtcagccat tctcttgctc acgggttttt ttccccaaca 4380

gaacagaggc gttgccagcc attttgggtc tgctttctgt ccagatactg cagcaaaaac 4440

tcttgaggat cacaacccgt tggctgagca gctgtgctgc tgcccaaacg tcctgcgcag 4500

acaaacgcac gctgggaccg gaaggggtgt ctctccttct gcctcttttc tttcatacgt 4560

ttctctcgaa aggcctcaac tgaggactgc aaatttcttt cttgaaataa ctttccccca 4620

gggacattcg gtcttaggga ttttttggtt ttgatgggtt ttgttttgtt ttggtttttt 4680

tggttcttct catttctttt gtaggagaag gcatgagatg ttgagggtct ttcatacatg 4740

aaaataaata gtttgacagc aatctcagaa tatatttttt ccttatttga acaaagtact 4800

gttttgttta ctctacagta cacctttatt tggtgggttt ggctgttggt cggaaatgcc 4860

ttccctcttt cagccaggtc ggtatagcca aagctgatca tttgtctgtt ttttcttttt 4920

tttcctcaga agagttgtat ccacaaactc aattgtattt atgtatgtaa atagatttca 4980

tgcttcatta taaaatattg ttaatgcctg taataacttt tttcacattt tgtgtgtgtt 5040

tctaaggact ttttcttatg ttcgctaaat attgtagaag aaaaatgctt ctcttaactt 5100

gtttatttta gattttaaaa aaaccaagct acttcttact cacttttata aacataataa 5160

acaagcatgg ttccaatggg ttttttttaa gttcacctta tgttctagga gaaatgaatg 5220

tgcaaaaaaa aaaatcaatc ttcaaatgaa ctgttgggta tttctgttac tctggatgga 5280

aaaagatcaa tggaaaacaa aaaacaaaaa aacaaacttc caaattgaaa tagcagtaaa 5340

acacatgtat tgggggtaaa aaaaaaaaaa aaaaacacac ctcagaaaat gcagttctcc 5400

agcctccagc tactgcccac acatgtggca agtgttagat gtggtcgtga ggaatcccaa 5460

aatcagtgcc cgggcctttt ccttcctggt actaacatta agaacacctc ctaaggggca 5520

ctgtcaggct caggtcttaa agcacataga taagagagaa tgtcccagaa cgattctcag 5580

tagcccaggc cagcaggtac tcacggcact gcacctgctc actgcacaca gtcgctgagg 5640

cagacccctc tcctgggggc catgcagagc cagggcaggg cagccctaag ctactgatgt 5700

tttctttcct ttgtactatg gtcttttctg taactccaat attcaaaccc atgtgaatgg 5760

caaataaaca atttgacaag cacctaaaaa aaaaaaaaaa aaa 5803

<210> 43
<211> 2475
<212> DNA
<213> Homo sapiens

<220>
<223> Human GFI-1B (NM_004188.6)

<400> 43
ctttattgcg aggctgagta tgatgggctc catccagcac gaacacaaag ccctgtgaga 60

ggaggcagag tgagatgaag tcgaactgtg ggatatgaaa ggcattttga aggagtaggc 120

atcaggctgg tttttgttcg aggaaagcta ctcaggagct tgggaggatt gcttgagacc 180

aggagttcaa gaccagcctg ggctacagag caagacccca tctttttgaa acttcctgca 240

actcgcatct ggaaaagtgc tgttatttga aagggtgccg acattgtgaa aaacacggag 300

tattgatctc tgaagctgag atttttgtcc taaagaatac tcaagatgtg tccctggctg 360

ggcacagcag ctcgtgcctg taatcccagc actttgggag gctgaggcag gaggatcgct 420

tgaggccagg agttcaagac cagcctattg tgaatagtac cactataaat agagaaagcg 480

tgccgctcca agtgtcgagg gttagaaatg agaattcgaa gtcttgtgtc ctggaaagtt 540

ttgataagca aatacggctg agctcccgcg ctctcttcat tggctgcttg ttcaccgcca 600

gattttgaca caaataatca gattgaaaat cagggagggg aacagaagag gaaaaacaca 660

cagagagaca gagcaaaaag gagaagtatc tatttgtgca aagagtcaca cagttgacag 720

agtggaggcc agtcccgaga gaggctttgc agttcccacc tcgggaagct ccggcagaac 780

ccaggcgagg gacagctccg gacaggtgtg gggtgcacac tgaaaatgcc acgctccttc 840

ctggtgaaga gcaagaaggc tcacacctac caccagcccc gtgtgcagga agatgaaccg 900

ctctggcctc ctgcccttac cccggtgccc agagaccagg ctccaagcaa cagccctgtc 960

cttagcactc tattcccaaa ccagtgcctg gactggacca acctcaaacg agagccggag 1020

ctggagcagg accagaactt ggccaggatg gccccggcac cagagggccc cattgtgctg 1080

tcccgacccc aggatgggga ctctccactg tccgactcac ccccattcta caagcctagc 1140

ttctcctggg acaccttggc cacaacctat ggccacagct accggcaggc cccctccacc 1200

atgcagtcag ccttcctgga gcactccgtc agcctgtacg gcagtcctct tgtgcccagc 1260

actgagcccg ccttggactt cagcctccgc tactccccag gcatggatgc gtaccactgt 1320

gtgaagtgca acaaggtctt ctccacccct cacgggctcg aagtgcatgt gcgacgctcc 1380

catagtggga cccggccctt cgcctgtgac atctgcggca aaaccttcgg ccacgctgtg 1440

agcctggagc agcacacgca cgtccactcc caggagcgca gcttcgagtg ccgcatgtgc 1500

ggcaaggcct tcaagcgctc gtccacgctg tccacccacc tgctcatcca ctcagacacg 1560

cggccctacc cctgccagtt ctgcggcaag cgtttccacc agaagtccga catgaagaag 1620

cacacctaca tccacacagg tgagaagccg cacaagtgcc aggtgtgcgg aaaggccttc 1680

agccagagct ccaacctcat cacccacagc cgcaagcaca caggcttcaa gcccttcagc 1740

tgtgagctgt gcaccaaagg cttccagcgc aaggtggacc tgcggcggca ccgcgagagc 1800

cagcacaatc tcaagtgagg ctgcgccggc tcccagctcc tggccagcct gccctgcggt 1860

cctgtcacct ggaggccagc ctcacatgcc caaatctcca gtctcctgga ggtgggactg 1920

gacaggagtc taccagcttg ttttgagact catgaaattg ctgtgtgacc ttgggcaagt 1980

cacttaccct gtctggatca acatttctcc tgctgccaag tgtgggagcc tggctgggtc 2040

tttctcagca gaagttgttt ccaggtgtgc tcaagtgcct tcctctagca gagcacagaa 2100

agctagaata cccccaggga gacagggatg ccaagagtag accagagctg ggacccacag 2160

acagaacctc cacctgcctg ctgcccactg agctgggacc tggtcacctt ggattttagc 2220

cggcctcttt ctggctataa caggcagagt cggagctgcc tcccacccca gtcagaagcc 2280

tggcaccccc tctgcttcgg ccagatgtgc tggctgactc cgacttccga ccagcactca 2340

gctggcctct ggggattcta gctccacaac caggccgtga ggctggagaa actggcagtt 2400

attgctgtca aaagcctgtt ctctcaactg ctgtcaataa aattaaagat acagatttgc 2460

tgccaaaaaa aaaaa 2475

<210> 44
<211> 1724
<212> DNA
<213> Mus <mouse, genus>

<220>
<223> Mouse Gfi-1b (NM_008114.3)

<400> 44
cacagaaacg aaaaggagaa gtgtctgtct gtgcagagac tcataacgtt gaccgagccg 60

agagcagtcc ccagggacag tgtggaggtt cgtggctctc gggcagaact cagaagaggg 120

acagctccct gacggtgtgg cgtgcacgca gaaaaatgcc acggtccttt ctagtgaaga 180

gtaagaaggc acacacttac caccagcccc gggcacaggg tgatgagctg gtctggcctc 240

ctgctgtaat tcctgtggca aaagagcata gccagagtgc cagccctctt ctcagcacac 300

cgcttccaag ccagaccttg gactggaaca caatcaaaca ggagcgggag atgttgctga 360

accagagcct tcccaagatg gcctcagccc cagaggggcc tctcgtgaca ccccaacccc 420

aggatgggga atcaccactc tctgagtcac cccctttcta caagcccagc ttctcctggg 480

ataccttggc ctcctcctac agccacagct acacacagac cccctccacc atgcagtccg 540

ccttcctgga gcgctccgtg aggctgtacg gcagccccct cgtgcccagc acagagtctc 600

ccttggactt ccgcctccgc tactctccag gcatggacac ttaccactgt gtcaagtgca 660

acaaggtgtt ctccacccct catgggctag aagtgcatgt ccgccgctct cacagcggaa 720

cccggccctt tgcctgtgat gtctgtggca aaacctttgg ccacgctgtg agcttggagc 780

agcatactca cgtccactca caggagcgaa gcttcgagtg ccggatgtgt ggcaaagcct 840

tcaagcgttc atccaccctg tccacccacc tgctcatcca ctcggacact cggccctacc 900

cctgccagtt ctgtgggaag cgcttccacc agaagtcgga catgaagaaa cacacctaca 960

tccacacagg tgagaagccc cacaagtgcc aggtgtgtgg gaaagccttc agccagagct 1020

ccaacctcat cacccacagc cgcaagcaca caggcttcaa gccgttcagc tgtgagctgt 1080

gcaccaaggg cttccagcgc aaggtggacc tgcgacgtca ccgtgagagt caacacaatc 1140

tcaagtgaga cggttggccg cctgctttag tgtgtctcgc ctgaaggcca gcctctcctt 1200

tccaatcctg atcccagtcc tcctggaagc agcattgccc atgagcctct ctgcttcttt 1260

tgagactgga tgacctcaac caagccacac tcctcctctg accacagctg atgtgggagt 1320

cagttcgggc ctttctgaac tgaggcagtc acagacatgc tctggtctca ttcaatgcca 1380

gagcacagac agctacaaag ccctcgtgga catggcaaaa ccaggcaaga gccaggatcc 1440

acagagaaac cctcttgctt gcttctctgg gtctagtgac tgaattttta gggattctct 1500

ttttggctgc cacagtcaga gttgtcccct ggcctagagg tctggcgccc ctctgcttcg 1560

gccagatgtg ctctctgtcc cccacctctc accacatcca tccgcccact gggagtcagg 1620

ctcctgaggt gagccacgag gctatgaaat cagccatgat agtagaagcc tgctcctatc 1680

agttgatgta aatgtaaata aaggtgggtt tatttacatg tgaa 1724

<210> 45
<211> 2076
<212> DNA
<213> Homo sapiens

<220>
<223> Human HOX-A9 (NM_152739.3)

<400> 45
agttgttaca tgaaatctgc agtttcataa tttccgtggg tcgggccggg cgggccaggc 60

gctgggcacg gtgatggcca ccactggggc cctgggcaac tactacgtgg actcgttcct 120

gctgggcgcc gacgccgcgg atgagctgag cgttggccgc tatgcgccgg ggaccctggg 180

ccagcctccc cggcaggcgg cgacgctggc cgagcacccc gacttcagcc cgtgcagctt 240

ccagtccaag gcgacggtgt ttggcgcctc gtggaaccca gtgcacgcgg cgggcgccaa 300

cgctgtaccc gctgcggtgt accaccacca tcaccaccac ccctacgtgc acccccaggc 360

gcccgtggcg gcggcggcgc cggacggcag gtacatgcgc tcctggctgg agcccacgcc 420

cggtgcgctc tccttcgcgg gcttgccctc cagccggcct tatggcatta aacctgaacc 480

gctgtcggcc agaaggggtg actgtcccac gcttgacact cacactttgt ccctgactga 540

ctatgcttgt ggttctcctc cagttgatag agaaaaacaa cccagcgaag gcgccttctc 600

tgaaaacaat gctgagaatg agagcggcgg agacaagccc cccatcgatc ccaataaccc 660

agcagccaac tggcttcatg cgcgctccac tcggaaaaag cggtgcccct atacaaaaca 720

ccagaccctg gaactggaga aagagtttct gttcaacatg tacctcacca gggaccgcag 780

gtacgaggtg gctcgactgc tcaacctcac cgagaggcag gtcaagatct ggttccagaa 840

ccgcaggatg aaaatgaaga aaatcaacaa agaccgagca aaagacgagt gatgccattt 900

gggcttattt agaaaaaagg gtaagctaga gagaaaaaga aagaactgtc cgtccccctt 960

ccgccttctc ccttctctca cccccaccct agcctccacc atccccgcac aaagcggctc 1020

taaacctcag gccacatctt ttccaaggca aaccctgttc aggctggctc gtaggcctgc 1080

cgctttgatg gaggaggtat tgtaagcttt ccattttcta taagaaaaag gaaaagttga 1140

ggggggggca ttagtgctga tagctgtgtg tgttagcttg tatatatatt tttaaaaatc 1200

tacctgttcc tgacttaaaa caaaaggaaa gaaactacct ttttataatg cacaactgtt 1260

gatggtaggc tgtatagttt ttagtctgtg tagttaattt aatttgcagt ttgtgcggca 1320

gattgctctg ccaagatact tgaacactgt gttttattgt ggtaattatg ttttgtgatt 1380

caaacttctg tgtactgggt gatgcaccca ttgtgattgt ggaagataga attcaatttg 1440

aactcaggtt gtttatgagg ggaaaaaaac agttgcatag agtatagctc tgtagtggaa 1500

tatgtcttct gtataactag gctgttaacc tatgattgta aagtagctgt aagaatttcc 1560

cagtgaaata aaaaaaaatt ttaagtgttc tcggggatgc atagattcat cattttctcc 1620

accttaaaaa tgcgggcatt taagtctgtc cattatctat atagtcctgt cttgtctatt 1680

gtatatataa tctatatgat taaagaaaat atgcataatc agacaagctt gaatattgtt 1740

tttgcaccag acgaacagtg aggaaattcg gagctataca tatgtgcaga aggttactac 1800

ctagggttta tgcttaattt taattggagg aaatgaatgc tgattgtaac ggagttaatt 1860

ttattgataa taaattatac actatgaaac cgccattggg ctactgtaga tttgtatcct 1920

tgatgaatct ggggtttcca tcagactgaa cttacactgt atattttgca atagttacct 1980

caaggcctac tgaccaaatt gttgtgttga gatgatattt aactttttgc caaataaaat 2040

atattgattc ttttctaaaa aaaaaaaaaa aaaaaa 2076

<210> 46
<211> 3229
<212> DNA
<213> Mus <mouse, genus>

<220>
<223> Mouse HOX-A9 (NM_010456.3)

<400> 46
gaaaaaacag aagagggaag gataccagag cggttcatac agggcccaga aactaggcga 60

ggtgacccct cagcaagaca aacacctctt gatgttgact ggcgattttc cccatctcca 120

gtctggggag cgggactagg catacagatg atggagctta gaacccgctg gctagggaat 180

aaaattcgct gggcagtttg tgctcaaaga agtgggccag ggcgcttgtg acacaatcag 240

ggcgtttgtg acacaaaccc ttgagggttg gcagttctct ccttggcggt tgctctggtt 300

gctctgtggg gccttccctg tggagcaagg gtgatctggc cgatgtgcaa gcgcctggct 360

ggctttccag tctgactagg gtcggtagcc cattttaggt ggttgtatca tcgacggtgc 420

gtcgcgacag gggcggtggt cactctgttt gaggtggaga gagccttgta tttgactttt 480
ctaggccggc cctgggggcg cgcgcgggcg gggggctcac atctctgagg actgcaagga 540
ttatttacag ggtattcacc aaccaaacac aacagtctaa tttaaccttt ccaagtcctc 600
ataaattttt acagggagcc acagcgaggc aaacgaatct gttggtcgct cctgactttc 660
caccagcctg tgtggcttcc gaaacaataa ctccttatga aatatcataa atatagattt 720
aaatacagta gagtgagaat gcgatttggc tgctttttta tggcttcaat tattgtctaa 780
ttttatgtga ggggctctgc tggccgtgct cacacgcggg acccgcgcct tcctgatggc 840
gtgattaatt gtgatataaa atagtccgct taagaagtgt gtgtgtctgg tatgtgtgtg 900
tgttgggggg gtggcaaggg agagtacaga ggcaaggcca gatttgatct tttaatcttc 960
gttggccaca attaaaacaa accagatcgt ggagctgcgc gatccctttg cataaaaaca 1020
tatggctttt gctataaaaa ttatgactgc aaaacaccgg gccattaata gcgtgcggag 1080
tgatttacgc gttattgttc tgccgggcgg acacgtgacg cgcgtggcca atgggggcgc 1140
gggcgccggc aacttattag gtgactgtac ttcacccccc cctggtgcca ccaagttgtt 1200
acatgaaatc tgcagtttca taatttcggc gggtcgggct gggccggcca ggcgcgggct 1260
actgcaatgg ccaccaccgg ggccctgggc aactactatg tggactcctt cctgctgggc 1320
gccgacgctg ctgatgagct gggtgcggga cgctacgctc cagggaccct gggtcaaccc 1380
ccaaggcagg cggcagctct ggccgaacac cccgacttca gtccttgcag cttccagtcc 1440
aaggcggcgg tgtttggtgc ctcgtggaac ccagtgcacg cggcgggcgc caatgcggtg 1500
cctgctgcag tgtatcatca ccaccaccac ccctacgtgc atccccaggc gcccgtggcg 1560
gcggcggcgc cggacggcag gtatatgcgc tcctggctgg aacccacgcc cggtgcgctc 1620
tccttcgcgg gcttaccctc cagccggcct tatggcatta aacctgaacc gctctcggcc 1680
agaaggggtg actgtcccac gcttgacact cacactttgt ccctgactga ctatgcttgt 1740
ggttctcctc cagttgatag agaaaaacaa cccagcgaag gcgccttctc cgaaaacaat 1800
gccgagaatg agagcggcgg agacaagccc cccatcgatc ccaataaccc ggctgccaac 1860
tggctacatg ctcgctccac tcggaagaag cgatgcccct acacaaaaca ccagacgctg 1920
gaactggaga aggagtttct gtttaacatg tacctcacac gggaccgcag gtacgaggtg 1980
gcccggctgc tcaacctcac cgaaaggcag gtcaagatct ggttccagaa ccgcaggatg 2040
aaaatgaaga aaatcaacaa ggaccgagca aaagacgagt gagcctttta ggggctcatt 2100
taaaaagaga gcaagctaga aagaaaaaga aaggactgtc cgtctccctc tgtctcctct 2160
cccccaaacc cagcctccac ccgcacaaag gggctctaaa tcccaggcct catctcccca 2220
ctggcagtcc gtgctcaggc tggctcttag gcctgcggct ttgatggagg aggtattgta 2280
agctttccat tttatagaag gcacacacac acacaaggga gggcattagc gctattggct 2340

gtatgtgcta gcttgtatat atatatatat atttaaaaaa aatctacctg cttctgactt 2400

taagcaaaag gaaagaaaac taccttttta tataatgcac aactgttgat gactggctgt 2460

atagttttta gtctctgtag ctaatttaat ttgctcttcg tgtggcagat cattctgcca 2520

aaatacttga acactgtgtt ttattgtggt aattatgttt tgtgactcaa acttctgtgc 2580

tgggtgaagt acccattgtg attgtggaag ctagaattca atttgaactc aggttgttta 2640

tggggggaga gaagctgtgg cataatgtat agctctgtag tcttctgtgt agctagactg 2700

ttaacctacg attgtaaact agctgtaagg atttcccagt gaaatcagat ttggaaagaa 2760

aagagagata gttctccggg atgcatagat tcatgattta tctacttttg aaatgtgggc 2820

ttcttcatct ttacgttcta gactccaatt ttgtccaatg tatatagaat ctccatatta 2880

aagaaatatt cataatcaga aagcttgaat attgtttttg caccagagga gaagtgagga 2940

aattcggagc tatacgtgtg tgcagagggt tgctacctat gggtttacgc ttaattttaa 3000

ttgggagaaa gtgagtgctt attgtaatgg agttaatttt attgataata aattatacac 3060

catgaaactg ctcttgggct actgtagatt tgtatccttg gtgaatttgg ggttcccatc 3120

ggattgaaca tacactgtat attttgcaat agttacctca aggcctactg accaaattgt 3180

tgtgtcgaga tatttaactt ttgccaaata aaatatattg attcttttta 3229

<210> 47
<211> 3484
<212> DNA
<213> Homo sapiens

<220>
<223> Human GATA2 (NM_001145661.1)

<400> 47
actgggtcaa gcacagccct gagcggccgc gtgtccgagg cccaggtgcc ctctagagcc 60

ctgtagttcc tgcccctctc tgcccctctc ggctcctgct gttccgccgc tgtcgtccga 120

accatcccaa cccccagtcc acccagacag cgcccgagct aggggaggga acggtctggg 180

agtcggcagc tggcgccagg gcggccggag gatgccgagg ggccggagcc gggagggccc 240

gaggccgagg cgcactctac ccccagctcc taccctgtaa gccccgccag cctccggacg 300

tgctgtccct gggcccgtcg ccctcggggc tcccgccgga actccttcac tctcagaggc 360

cgagtccctc ccctccccac ggctgcgtgt ggccgttgcc gtctgcaccc agaccctgag 420

ccgccgccgc cggccatgga ggtggcgccc gagcagccgc gctggatggc gcacccggcc 480

gtgctgaatg cgcagcaccc cgactcacac cacccgggcc tggcgcacaa ctacatggaa 540

cccgcgcagc tgctgcctcc agacgaggtg gacgtcttct tcaatcacct cgactcgcag 600

ggcaacccct actatgccaa ccccgctcac gcgcgggcgc gcgtctccta cagccccgcg 660

cacgcccgcc tgaccggagg ccagatgtgc cgcccacact tgttgcacag cccgggtttg 720

ccctggctgg acgggggcaa agcagccctc tctgccgctg cggcccacca ccacaacccc 780

tggaccgtga gccccttctc caagacgcca ctgcacccct cagctgctgg aggccctgga 840

ggcccactct ctgtgtaccc aggggctggg ggtgggagcg ggggaggcag cgggagctca 900

gtggcctccc tcacccctac agcagcccac tctggctccc accttttcgg cttcccaccc 960

acgccaccca aagaagtgtc tcctgaccct agcaccacgg gggctgcgtc tccagcctca 1020

tcttccgcgg ggggtagtgc agcccgagga gaggacaagg acggcgtcaa gtaccaggtg 1080

tcactgacgg agagcatgaa gatggaaagt ggcagtcccc tgcgcccagg cctagctact 1140

atgggcaccc agcctgctac acaccacccc atccccacct acccctccta tgtgccggcg 1200

gctgcccacg actacagcag cggactcttc caccccggag gcttcctggg gggaccggcc 1260

tccagcttca cccctaagca gcgcagcaag gctcgttcct gttcagaagg ccgggagtgt 1320

gtcaactgtg gggccacagc cacccctctc tggcggcggg acggcaccgg ccactacctg 1380

tgcaatgcct gtggcctcta ccacaagatg aatgggcaga accgaccact catcaagccc 1440

aagcgaagac tgtcggccgc cagaagagcc ggcacctgtt gtgcaaattg tcagacgaca 1500

accaccacct tatggcgccg aaacgccaac ggggaccctg tctgcaacgc ctgtggcctc 1560

tactacaagc tgcacaatgt taacaggcca ctgaccatga agaaggaagg gatccagact 1620

cggaaccgga agatgtccaa caagtccaag aagagcaaga aaggggcgga gtgcttcgag 1680

gagctgtcaa agtgcatgca ggagaagtca tcccccttca gtgcagctgc cctggctgga 1740

cacatggcac ctgtgggcca cctcccgccc ttcagccact ccggacacat cctgcccact 1800

ccgacgccca tccacccctc ctccagcctc tccttcggcc acccccaccc gtccagcatg 1860

gtgaccgcca tgggctaggg aacagatgga cgtcgaggac cgggcactcc cgggatgggt 1920

ggaccaaacc cttagcagcc cagcatttcc cgaaggccga caccactcct gccagcccgg 1980

ctcggcccag caccccctct cctggagggc gcccagcagc ctgccagcag ttactgtgaa 2040

tgttccccac cgctgagagg ctgcctccgc acctgaccgc tgcccaggtg gggtttcctg 2100

catggacagt tgtttggaga acaacaagga caactttatg tagagaaaag gaggggacgg 2160

gacagacgaa ggcaaccatt tttagaagga aaaaggatta ggcaaaaata atttattttg 2220

ctcttgtttc taacaaggac ttggagactt ggtggtctga gctgtcccaa gtcctccggt 2280

tcttcctcgg gattggcggg tccacttgcc agggctctgg gggcagattt gtggggacct 2340

cagcctgcac cctcttctcc tctggcttcc ctctctgaaa tagccgaact ccaggctggg 2400

ctgagccaaa gccagagtgg ccacggccca gggagggtga gctggtgcct gctttgacgg 2460

gccaggccct ggagggcaga gacaatcacg ggcggtcctg cacagattcc caggccaggg 2520

ctgggtcaca ggaaggaaac aacattttct tgaaagggga aacgtctccc agatcgctcc 2580

cttggctttg aggccgaagc tgctgtgact gtgtcccctt actgagcgca agccacagcc 2640

tgtcttgtca ggtggaccct gtaaatacat cctttttctg ctaacccttc aaccccctcg 2700

cctcctactc tgagacaaaa gaaaaaatat taaaaaaatg cataggctta actcgctgat 2760

gagttaattg ttttattttt aaactctttt tgggtccagt tgattgtacg tagccacagg 2820

agccctgcta tgaaaggaat aaaacctaca cacaaggttg gagctttgca attctttttg 2880

gaaaagagct gggatcccac agccctagta tgaaagctgg gggtggggag gggcctttgc 2940

tgcccttggt ttctgggggc tggttggcat ttgctggcct ggcagggggt gaaggcagga 3000

gttgggggca ggtcaggacc aggacccagg gagaggctgt gtccctgctg gggtctcagg 3060

tccagcttta ctgtggctgt ctggatcctt cccaaggtac agctgtatat aaacgtgtcc 3120

cgagcttaga ttctgtatgc ggtgacggcg gggtgtggtg gcctgtgagg ggcccctggc 3180

ccaggaggag gattgtgctg atgtagtgac caagtgcaat atgggcgggc agtcgctgca 3240

gggagcacca cggccagaag taacttattt tgtactagtg tccgcataag aaaaagaatc 3300

ggcagtattt tctgttttta tgttttattt ggcttgtttt attttggatt agtgaactaa 3360

gttattgtta attatgtaca acatttatat attgtctgta aaaaatgtat gctatcctct 3420

tattccttta aagtgagtac tgttaagaat aataaaatac tttttgtgaa tgcccaaaaa 3480

aaaa 3484

<210> 48
<211> 3258
<212> DNA
<213> Mus <mouse, genus>

<220>
<223> Mouse Gata2 (NM_008090.5)

<400> 48
gtctgtgcag gagtcggcag ctggcgccag ggcggccgga ggatgcagag gggccggagc 60

cgggcgggcc ggaggccgag acgcgcgctg tcccccaccc ctatcccgtg aatccgccgg 120

ccctggaacg cgctgtcgct gggcccgccg tacccgggct ctcctggtgt ctcttactct 180

ctactgctga gccctcccct tcccgcgccg ctgcgagtgg ccgccccacc ttcgcctggt 240

tcccaagaca cagtagtgga ccatggaggt ggcgcctgag cagcctcgct ggatggcgca 300

ccccgccgta ttgaatgcgc agcaccccga ctcgcaccat ccgggcctgg cgcataacta 360

catggagcca gcacagctgc tgcctcccga cgaggtggat gtcttcttca accatctcga 420

ctcgcagggc aacccttact acgccaaccc ggcccacgcg cgcgcgcgcg tttcctacag 480

cccggcgcat gcccgtctca ccggaggcca gatgtgccga ccacacttgt tgcacagccc 540

aggcttgccg tggctggacg ggggcaaagc agctctctct gccgccgctg cccatcacca 600

cagtccctgg accgtcagcc cgttctccaa gaccccgctg cacccctcag ctgctggagc 660

acccggaggg cctctgtctg tttacccagg ggctgcgggt gggagcgggg gaggcagtgg 720

gagctccgtg gcctccctca cccccactgc agcccactcg ggctcccatc tcttcggctt 780

cccacccacg ccacccaaag aagtgtctcc agaccccagc acaacaggag ctgcttcccc 840

ggcctcttct tctgcagggg gtagtgtagc ccggggtgag gacaaggatg gcgtcaagta 900

ccaagtgtca ctctccgaga gcatgaagat ggaaggcggc agtcccctgc gcccgggcct 960

agctaccatg ggcacccagc ctgcaacaca ccacccgata cccacctatc cctcctatgt 1020

gcccgccgca gctcatgact atggcagcag tctcttccat ccaggaggct tcctgggtgg 1080

ccccgcctcc agcttcaccc ctaagcagag aagcaaggct cgctcctgct cagaaggccg 1140

ggagtgtgtc aactgtggtg ccacagccac ccctctctgg cgacgagatg gcacgggcca 1200

ctacctgtgc aatgcctgtg ggctctacca caagatgaat ggacagaacc ggccgctcat 1260

caagcccaag cggaggctgt ctgctgccag aagagcgggc acctgttgtg caaattgtca 1320

gacgacaacc accaccttat ggcgccggaa cgccaacggg gaccctgtgt gcaacgcctg 1380

tggcctctac tacaagctgc acaatgttaa caggccactg accatgaaga aggaagggat 1440

ccagacccgg aatcggaaga tgtccagcaa atccaagaag agcaagaaag gggctgaatg 1500

tttcgaggag ctctccaagt gcatgcaaga gaagtcaccg cccttcagtg cggctgccct 1560

ggctggacac atggcacctg tgggacacct cccacctttt agtcactctg gacacatcct 1620

acccacgccc acgcctatcc acccttcctc cagtctctct tttggccacc cccacccgtc 1680

cagcatggtg actgccatgg gctaggcaag cctcccactg gacagacatg gacatcaagg 1740

gtggtttggc agaaccagag cgaggctggg cactcccagg atgggtggaa catactcttg 1800

gctcccgccc atcccaagag acccacttcc tcctgccagc ctagcctggc cgaagccacc 1860

tctccttgga ggactcccag ccttgtgccg ccattactgt gaatatttct aactgggctg 1920

cagctcgcgt gtgcccgggg tgctgcccag aaaagtgttt tcacggagag tgtttgtttg 1980

gagagcaaaa tggacaggtt tacagattta tagcaagaag agactgggga tagaaaaatg 2040

aaaccttttt ttttcttttt ctttttttct tcttctgttt tatttttttg atggagaaag 2100

gagtaggcaa gaagaaaaat aatttatttt gctcttattt cttacaagaa cgtgaagaca 2160

tggaggcgtg tgctatttgt gttcttgggg tccttctttg ggacctcctg ccaccagtca 2220

gggctctcgg gggcagactt agaggtcctc agcctgagcc tccttcaccc cagcctgcct 2280

gcagggtagc ccctgccctg acgcagccct agagggcaga gacaattgca ggcggtcctg 2340

cgcagattcc caggccaggg ctgggtcaca ggaaggaaac attctctgga aaggggaaac 2400

gtctcccaga tcattcccct ggcttccaga ggccaaagct ggtgtgaccc aaatgggcca 2460

gagctgcagc ctgtgctcta ggccagtcgg acccctgtaa atacaacctt cttttctgct 2520

aaaccctcgg ccccctcccc ctctaagata aataagaaaa tactcaaagc gaaaaccaaa 2580

ctgcataagc ttaacccgct gatgagtggt tttattttga aactcgtttt ttgggtccag 2640

tcaattgtac gttgccacag aagccccgct atggaaaaaa ataaataaaa cctacaaacc 2700

aggcctgagc ttcacagtcc tttgagtggt tcttgggtcc cacagccctg gcagggggct 2760

cgggacaagg gggaatctta tgctcttggt ttctgggaga cagggggcag gcaggcagtg 2820

gccctgtgat cccaggcttc tgttctgctg tggctggctg aatccttcaa ggtacagttg 2880

tacataaaaa gtgtcccaag cttcgattct gtgtgtggtg gtggcagtgg tgcagcagcc 2940

agcaaggggg ccccgagtga gcccagggag acgattgtgc tgagtcaacc aagtgcaata 3000

tcggtgtcca gttgctgcag agcaccctaa ccggaagtaa cttattttgt gctagtaccc 3060

gcataagaga agaatcggca gtattttctg tttttatgtt ttgggcttgt tttattttga 3120

attagtgacc taagttattg ttaactgtgt acaacattta aatattgtct gtaaaaattg 3180

tatgctaccc tcttattcct ttaaagtgaa tactgttaaa aataataaaa tactttttgt 3240

gaaaaaaaaa aaaaaaaa 3258

<210> 49
<211> 3198
<212> DNA
<213> Homo sapiens

<220>
<223> Human MEIS1 (NM_002398.2)

<400> 49
atttgaggtg ttctgaccag aagaagacag agcggatgat cattcattca ccacgttgac 60

aacctcgcct gtgattgaca gctggagtgg cagaaagcca tgagatttgg tagttgggtc 120

tgaggggcgc tctttttttt ccttttcttt ctttctttct tttttttttt ttaaactgat 180

ttttggggga gagaagatct gctttttttt gcccccgctg ctgtcttgga aacggagcgc 240

ttttatgctc agtgactcgg gcgctttgct tcaggtcccg tagaccgaag atctgggacc 300

agtagctcac gttgctggag acgttaaggg atttttcgtc gtgctttttt tttttttttt 360

ttttttttcc gggggagttt gaatatttgt ttcttttcac actggcctta aagaggatat 420

attagaagtt gaagtaggaa gggagccaga gaggccgatg gcgcaaaggt acgacgatct 480

accccattac gggggcatgg atggagtagg catcccctcc acgatgtatg gggacccgca 540

tgcagccagg tccatgcagc cggtccacca cctgaaccac gggcctcctc tgcactcgca 600

tcagtacccg cacacagctc ataccaacgc catggccccc agcatgggct cctctgtcaa 660

tgacgcttta aagagagata aagatgccat ttatggacac cccctcttcc ctctcttagc 720

actgattttt gagaaatgtg aattagctac ttgtaccccc cgcgagccgg gggtggcggg 780

cggggacgtc tgctcgtcag agtcattcaa tgaagatata gccgtgttcg ccaaacagat 840

tcgcgcagaa aaacctctat tttcttctaa tccagaactg gataacttga tgattcaagc 900

catacaagta ttaaggtttc atctattgga attagagaag gtacacgaat tatgtgacaa 960

tttctgccac cggtatatta gctgtttgaa agggaaaatg cctatcgatt tggtgataga 1020

cgatagagaa ggaggatcaa aatcagacag tgaagatata acaagatcag caaatctaac 1080

tgaccagccc tcttggaaca gagatcatga tgacacggca tctactcgtt caggaggaac 1140

cccaggccct tccagcggtg gccacacgtc acacagtggg gacaacagca gtgagcaagg 1200

tgatggcttg gacaacagtg tagcttcccc cagcacaggt gacgatgatg accctgataa 1260

ggacaaaaag cgtcacaaaa agcgtggcat ctttcccaaa gtagccacaa atatcatgag 1320

ggcgtggctg ttccagcatc taacacaccc ttacccttct gaagaacaga aaaagcagtt 1380

ggcacaagac acgggactca ccatccttca agtgaacaat tggtttatta atgcccggag 1440

aagaatagtg cagcccatga tagaccagtc caaccgagca gtaagtcaag gaacacctta 1500

taatcctgat ggacagccca tgggaggttt cgtaatggac ggtcagcaac atatgggaat 1560

tagagcacca ggacctatga gtggaatggg catgaatatg ggcatggagg ggcagtggca 1620

ctacatgtaa ccttcatcta gttaaccaat cgcaaagcaa gggggaaggc tgcaaagtat 1680

gccaggggag tatgtagccc ggggtggtcc aatgggtgtg agtatgggac agccaagtta 1740

tacccaaccc cagatgcccc cccatcctgc tcagctgcgt catgggcccc ccatgcatac 1800

gtacattcct ggacaccctc accacccaac agtgatgatg catggaggac cgccccaccc 1860

tggaatgcca atgtcagcat caagccccac agttcttaat acaggagacc caacaatgag 1920

tggacaagtc atggacattc atgctcagta gcttaaggga atatgcattg tctgcaatgg 1980

tgactgattt caaatcatgt tttttctgca atgactgtgg agttccattc ttggcatcta 2040

ctctggacca aggagcatcc ctaattcttc atagggacct ttaaaaagca ggaaatacca 2100

actgaagtca atttggggga catgctaaat aactatataa gacattaaga gaacaaagag 2160

tgaaatattg taaatgctat tatactgtta tccatattac gttgtttctt atagattttt 2220

taaaaaaaat gtgaaatttt tccacactat gtgtgttgtt tccatagctc ttcacttcct 2280

ccagaagcct ccttacatta aaaagcctta cagttatcct gcaagggaca ggaaggtctg 2340

atttgcagga tttttagagc attaaaataa ctatcaggca gaagaatctt tcttctcgcc 2400

taggatttca gccatgcgcg cgctctctct ctttctctct cttttcctct ctctccctct 2460

ttctagcctg gggcttgaat ttgcatgtct aattcattta ctcaccatat ttgaattggc 2520

ctgaacagat gtaaatcggg aaggatggga aaaactgcag tcatcaacaa tgattaatca 2580

gctgttgcag gcagtgtctt aaggagactg gtaggaggag gcatggaaac caaaaggccg 2640

tgtgtttaga agcctaattg tcacatcaag catcattgtc cccatgcaac aaccaccacc 2700

ttatacatca cttcctgttt taagcagctc taaaacatag actgaagatt tatttttaat 2760

atgttgactt tatttctgag caaagcatcg gtcatgtgtg tattttttca tagtcccacc 2820

ttggagcatt tatgtagaca ttgtaaataa attttgtgca aaaaggactg gaaaaatgaa 2880

ctgtattatt gcaatttttt tttgtaaaag tagcagtttg gtatgagttg gcatgcatac 2940

aagatttact aagtgggata agctaattat actttttgtt gtggataaac aaatgcttgt 3000

tgatagcctt tttctatcaa gaaaccaagg agctaattat taataacaat cattgcacac 3060

tgagtcttag cgtttctgat ggaaacagtt tggattgtat aataacgcca agcccagttg 3120

tagtcgtttg agtgcagtaa tgaaatctga atctaaaata aaaacaagat tatttttgtc 3180

aaaaaaaaaa aaaaaaaa 3198

<210> 50
<211> 3346
<212> DNA
<213> Mus <mouse, genus>

<220>
<223> Mouse Meis1 (NM_010789.3)

<400> 50
agcattctgg tcggaatcca cctctccgcc tgtgcaacac acactttaca cacgcacggc 60

gactgcaagc gggcagcatc gatcgtggct cctttaagac aaactcagac agacattttt 120

ttaaccctcc tctctaatct cccttcagtg cagcagttgc aaagagggag agagaaagag 180

aaagagagcg agagaagaga gaaactgatt aggaattagg actgattcaa ggaaagcggg 240

cgctagggct ttgtgcattt gaatattaac atttgaggtg ttctgaccag aagaagacag 300

aacggacgat cattcattca ccacgttgac aacctcgcct gtgattgaca gctggagtgg 360

cagaaagcca tgagatttgg tagttgggtc tgaggggcgc tctttttttt ttttcttttt 420

cttttctttt ctttcttttt ttttaaactg atttttttgg gggggagaga agatctgctt 480

tttttttccc ctcccactgc tgtcttggtg gaaccagagc gcttttatgc tcagcgacgc 540

gggcgccttg cttcaggtcc ggtagaccga agatctggga ccagtaattc acactgctgg 600

agacgcaaag ggattttttt ttgttattgt tgtgctttat ttttttttcc gggggagttt 660

gcatatttgt ttcttttcac actggcctta aagaggatat attagaagtt gaagtaggaa 720

gggagccaga gaggccgatg gcgcaaaggt acgacgacct accccattat gggggtatgg 780

atggagtagg catcccctcc acgatgtatg gggacccgca tgcagccagg tccatgcaac 840

cggtccacca cctgaaccac gggcctcctc tgcactcgca tcagtacccg cacacagctc 900

acaccaacgc catggccccc agcatgggtt cctcggtcaa tgacgcttta aagagagata 960

aagatgccat ttatggacac cccctcttcc ctctcttagc actgattttt gagaaatgtg 1020

aattagctac ttgtaccccc cgcgagccgg gggtggcggg cggggacgtc tgctcgtcag 1080

agtcattcaa tgaagatata gcggtgttcg ccaaacagat tcgcgcagaa aaacctctat 1140

tctcttctaa tccagaactg gataacttga tgattcaagc catacaagtg ttaaggtttc 1200

atctgttgga attagagaag gtacacgaat tatgtgacaa tttctgccac cggtatatta 1260

gctgtttgaa agggaaaatg cctatcgatt tggtgataga tgatagagaa ggaggatcaa 1320

aatcagacag tgaagatgta acaagatcag caaatctaac tgaccagccc tcttggaata 1380

gagaccatga tgacacggca tccactcgtt caggaggaac cccgggccct tccagcggtg 1440

gccatacttc acacagtggg gataacagca gtgagcaagg tgatggcttg gacaacagtg 1500

tagcttcccc cagcacaggt gacgatgatg accctgataa ggacaaaaag cgtcacaaaa 1560

agcgtggcat ctttcccaaa gtagccacca atatcatgag ggcgtggctg ttccagcatc 1620

taacacaccc ttacccttct gaagaacaga aaaagcagtt ggcacaagat acaggactta 1680

ccatccttca agtgaacaat tggtttatta atgcccggag aagaatagtg cagcccatga 1740

tagaccagtc caaccgagca gtcagccaag ggacacctta taaccctgat ggacagccaa 1800

tgggaggttt tgtaatggac ggtcagcagc acatgggcat cagagcgcca gggctgcaaa 1860

gtatgccagg ggagtatgta gcccggggtg gcccaatggg tgtgagtatg ggacagccga 1920

gttataccca agcccagatg cccccccatc ctgctcagct gcgtcatggg ccccccatgc 1980

atacgtacat tcctggacac cctcaccacc ccgcagtgat gatgcatgga ggacagcccc 2040

accctggaat gccaatgtca gcctcaagcc cctcggttct taacacagga gacccgacaa 2100

tgagtgcaca agtcatggac attcacgctc agtagcttaa gggaatatgc gttgtctaca 2160

atggtgactg atctcgaatc aggtcttttc ctgcaaggac tatggagttc cattcttgac 2220

atctactttg gaccaaggag catccctagt tcttcatagg gactcttaaa atgcaggaga 2280

accatccgaa gtcaactcgg gggacatgca aaaataacta tataagacat taaaagaaca 2340

aagagtgaaa tattgtaaat gctattatac tgttatccat attacgttgt ttcttataga 2400

ttttttaaaa aaaatgtgaa atttttccac actatgtgtg ttgtttccat agctgttcac 2460

ttcctccaga agcctcctta cattaaaaag ccttacagtt atcctgcaag ggacaggaag 2520

gtctgatttg caggattttt agagcattaa aataactatc aggcagaaga atctttcttc 2580

ttgcctagga tttcagccat gtgcgtgcgc tctctctctc tctctctctc tctctctctc 2640

tctctctctc tctctctctc tctctcttct cctccctctc cctctttcta gcctggggct 2700

tgaatttgca tgtctaattc atttactcac catatttgaa ttggcctgaa cagatgtaaa 2760

tcgggaagga tgggaaaaac tgcagtcacc aacaatgatt aatcagctgt tgcaggcagt 2820

gtcttaagga gactggtagg aggaggcatg gaaacccaaa ggccgtgtgt gtttagaagc 2880

ctaactgtca catcaagcat catcgtcccc atgcaacaac aaccatcacc ttatacatca 2940

cttcctgttt tatgcagctc aaaaaacata gactgaagat ttatttttta atatgttgac 3000

tttgtttctc agcaaagcat tggtcatgtg tgtatttttc catagtccca ccttggagca 3060

tttatgtaga cattgtaaat aaattttgtg caaaaaggac tggaaagatg aactgtatta 3120

ttgcatttat tttgtttttt tttttgtgaa agtagcagat tagtgcaagg tggcatgcat 3180

ataaggttaa ctgagtgggg tacgctaatt atactttttg ttgtggataa aaaatgcttg 3240

tcaatagcct ttttctatca agaaaacaag gagctaatta ttaataacag tcatggcaca 3300

ctgtgtccta tcttagcatt taaaagtatg caaactccag gtccag 3346

<210> 51
<211> 4414
<212> DNA
<213> Homo sapiens

<220>
<223> Human STAT5A (NM_001288718.1)

<400> 51
agatggccgg agtaaaagaa ggagggaggt gctgcggtgg tgggggtgat cttggcttca 60

ctagaatccc cagttcttcc cctctctaca gttttgtctc tgaggtcaca aaacctgtgg 120

cccccaagac acacatgcgc acacacgcgc gtgcacacac acaccccaca catttatttt 180

ttaatctagg ggctcaaaag atgacacgcg ccagagctgg aaggcgtcgc caattggtcc 240

acttttccct cctccctttt tgcggatgag aaaactgagg cccaggtttg ggatttccag 300

agcccgggat ttcccggcaa cgcccgacaa ccacattccc ccggctattc tgacccgccc 360

cggttccggg acgctccctg ggagccgccg ccgagggcct gctgggactc ccgggggacc 420

ccgccgtcgg ggcagccccc acgcccggcg ccgcccgccg ggaacggccg ccgctgttgc 480

gcacttgcag gggagccggc gactgagggc gaggcaggga gggagcaagc ggggctggga 540

gggctgctgg cgcgggctcg cgcgctgtgt atggtctatc gcaggcagct gacctttgag 600

gaggaaatcg ctgctctccg ctccttcctg tagtaacagc cgccgctgcc gccgccgcca 660

ggaaccccgg ccgggagcga gagccgcggg gcgcagagcc ggcccggctg ccggacggtg 720

cggccccacc aggtgaacgg ccatggcggg ctggatccag gcccagcagc tgcagggaga 780

cgcgctgcgc cagatgcagg tgctgtacgg ccagcacttc cccatcgagg tccggcacta 840

cttggcccag tggattgaga gccagccatg ggatgccatt gacttggaca atccccagga 900

cagagcccaa gccacccagc tcctggaggg cctggtgcag gagctgcaga agaaggcgga 960

gcaccaggtg ggggaagatg ggtttttact gaagatcaag ctggggcact acgccacgca 1020

gctccagaaa acatatgacc gctgccccct ggagctggtc cgctgcatcc ggcacattct 1080

gtacaatgaa cagaggctgg tccgagaagc caacaattgc agctctccgg ctgggatcct 1140

ggttgacgcc atgtcccaga agcaccttca gatcaaccag acatttgagg agctgcgact 1200

ggtcacgcag gacacagaga atgagctgaa gaaactgcag cagactcagg agtacttcat 1260

catccagtac caggagagcc tgaggatcca agctcagttt gcccagctgg cccagctgag 1320

cccccaggag cgtctgagcc gggagacggc cctccagcag aagcaggtgt ctctggaggc 1380

ctggttgcag cgtgaggcac agacactgca gcagtaccgc gtggagctgg ccgagaagca 1440

ccagaagacc ctgcagctgc tgcggaagca gcagaccatc atcctggatg acgagctgat 1500

ccagtggaag cggcggcagc agctggccgg gaacggcggg ccccccgagg gcagcctgga 1560

cgtgctacag tcctggtgtg agaagttggc cgagatcatc tggcagaacc ggcagcagat 1620

ccgcagggct gagcacctct gccagcagct gcccatcccc ggcccagtgg aggagatgct 1680

ggccgaggtc aacgccacca tcacggacat tatctcagcc ctggtgacca gcacattcat 1740

cattgagaag cagcctcctc aggtcctgaa gacccagacc aagtttgcag ccaccgtacg 1800

cctgctggtg ggcgggaagc tgaacgtgca catgaatccc ccccaggtga aggccaccat 1860

catcagtgag cagcaggcca agtctctgct taaaaatgag aacacccgca acgagtgcag 1920

tggtgagatc ctgaacaact gctgcgtgat ggagtaccac caagccacgg gcaccctcag 1980

tgcccacttc aggaacatgt cactgaagag gatcaagcgt gctgaccggc ggggtgcaga 2040

gtccgtgaca gaggagaagt tcacagtcct gtttgagtct cagttcagtg ttggcagcaa 2100

tgagcttgtg ttccaggtga agactctgtc cctacctgtg gttgtcatcg tccacggcag 2160

ccaggaccac aatgccacgg ctactgtgct gtgggacaat gcctttgctg agccgggcag 2220

ggtgccattt gccgtgcctg acaaagtgct gtggccgcag ctgtgtgagg cgctcaacat 2280

gaaattcaag gccgaagtgc agagcaaccg gggcctgacc aaggagaacc tcgtgttcct 2340

ggcgcagaaa ctgttcaaca acagcagcag ccacctggag gactacagtg gcctgtccgt 2400

gtcctggtcc cagttcaaca gggagaactt gccgggctgg aactacacct tctggcagtg 2460

gtttgacggg gtgatggagg tgttgaagaa gcaccacaag ccccactgga atgatggggc 2520

catcctaggt tttgtgaata agcaacaggc ccacgacctg ctcatcaaca agcccgacgg 2580

gaccttcttg ttgcgcttta gtgactcaga aatcgggggc atcaccatcg cctggaagtt 2640

tgactccccg gaacgcaacc tgtggaacct gaaaccattc accacgcggg atttctccat 2700

caggtccctg gctgaccggc tgggggacct gagctatctc atctatgtgt ttcctgaccg 2760

ccccaaggat gaggtcttct ccaagtacta cactcctgtg ctggctaaag ctgttgatgg 2820

atatgtgaaa ccacagatca agcaagtggt ccctgagttt gtgaatgcat ctgcagatgc 2880

tgggggcagc agcgccacgt acatggacca ggccccctcc ccagctgtgt gcccccaggc 2940

tccctataac atgtacccac agaaccctga ccatgtactc gatcaggatg gagaattcga 3000

cctggatgag accatggatg tggccaggca cgtggaggaa ctcttacgcc gaccaatgga 3060

cagtcttgac tcccgcctct cgccccctgc cggtcttttc acctctgcca gaggctccct 3120

ctcatgaatg tttgaatccc acgcttctct ttggaaacaa tatgcaatgt gaagcggtcg 3180

tgttgtgagt ttagtaaggc tgtgtacact gacacctttg caggcatgca tgtgcttgtg 3240

tgtgtgtgtg tgtgtgtgtc cttgtgcatg agctacgcct gcctcccctg tgcagtcctg 3300

ggatgtggct gcagcagcgg tggcctcttt tcagatcatg gcatccaaga gtgcgccgag 3360

tctgtctctg tcatggtaga gaccgagcct ctgtcactgc aggcactcaa tgcagccaga 3420

cctattcctc ctgggcccct catctgctca gcagctattt gaatgagatg attcagaagg 3480

ggaggggaga caggtaacgt ctgtaagctg aagtttcact ccggagtgag aagctttgcc 3540

ctcctaagag agagagacag agagacagag agagagaaag agagagtgtg tgggtctatg 3600

taaatgcatc tgtcctcatg tgttgatgta accgattcat ctctcagaag ggaggctggg 3660

gttcattttc gagtagtatt ttatacttta gtgaacgtgg actccagact ctctgtgaac 3720

cctatgagag cgcgtctggg cccggccatg tccttagcac aggggggccg ccggtttgag 3780

tgagggtttc tgagctgctc tgaattagtc cttgcttggc tgcttggcct tgggcttcat 3840

tcaagtctat gatgctgttg cccacgtttc ccgggatata tattctctcc cctccgttgg 3900

gccccagcct tctttgcttg cctctctgtt tgtaaccttg tcgacaaaga ggtagaaaag 3960

attgggtcta ggatatggtg ggtggacagg ggccccggga cttggagggt tggtcctctt 4020

gcctcctgga aaaaacaaaa acaaaaaact gcagtgaaag acaagctgca aatcagccat 4080

gtgctgcgtg cctgtggaat ctggagtgag gggtaaaagc tgatctggtt tgactccgct 4140

ggaggtgggg cctggagcag gccttgcgct gttgcgtaac tggctgtgtt ctggtgaggc 4200

cttgctccca accccacacg ctcctccctc tgaggctgta ggactcgcag tcaggggcag 4260

ctgaccatgg aagattgaga gcccaaggtt taaacttctc tgaagggagg tggggatgag 4320

aagaggggtt tttttgtact ttgtacaaag accacacatt tgtgtaaaca gtgttttgga 4380

ataaaatatt tttttcataa aaaaaaaaaa aaaa 4414

<210> 52
<211> 3628
<212> DNA
<213> Mus <mouse, genus>

<220>
<223> Mouse Stat5a (NM_001164062.1)

<400> 52

taacagcctc caccgccgcc gccgtcaaga gccgtcagga gccgtcagaa gccccggcct 60

ggagcgacag ccgcaggcgc tccgcagcac caggtaaaca gccatggcgg gctggattca 120

ggcccagcag cttcagggag atgccctgcg ccagatgcaa gtgttgtatg ggcagcattt 180

ccccatcgag gtccggcact acctggccca gtggatcgag agccagccgt gggatgctat 240

tgacttggat aatccccagg accgaggtca ggccacccaa ctcctggagg gcctggtgca 300

ggagctgcag aagaaggcgg agcaccaggt gggggaagat gggtttttgc tgaagatcaa 360

gctggggcac tatgccacac agctccagaa cacgtatgac cgctgtccca tggagctggt 420

tcgctgtatc cgtcacattc tgtacaacga acagaggctg gttcgcgaag ccaacaattg 480

cagctcccct gctggtgtcc tggttgacgc catgtcccag aagcaccttc agatcaacca 540

aaggtttgag gagctgcgcc tgatcacaca ggacacggag aacgagctga agaagctgca 600

gcagacccaa gagtacttca tcatccagta ccaggagagc ctgcggatcc aagctcagtt 660

tgcccagctg ggccagctga acccccagga gcgcatgagc agggagacgg ccctccagca 720

gaagcaagtg tccctggaga cctggctgca gcgagaggca cagacactgc agcagtaccg 780

agtggagctg gctgagaagc accagaagac cctgcagctg ctgcggaagc agcagaccat 840

catcctggac gacgagctga tccagtggaa gcggagacag cagctggccg ggaacggggg 900

tccccccgag ggcagcctgg acgtgctgca gtcctggtgt gagaagctgg ccgagatcat 960

ctggcagaac cggcagcaga tccgcagggc tgagcacctg tgccagcagc tgcccatccc 1020

aggccccgtg gaggagatgc tggctgaggt caacgccacc atcacggaca tcatctcagc 1080

tctggtcacc agcacgttca tcatcgagaa gcagcctcct caggtcctga agacccagac 1140

caagtttgcg gccaccgtgc gcctgctggt ggggggaaag ctgaatgtgc acatgaaccc 1200

cccgcaggtg aaggcgacca tcatcagcga gcagcaggcc aagtccctgc tcaagaatga 1260

gaacacccgc aatgagtgca gcggcgagat cctgaacaac tgttgcgtca tggagtacca 1320

ccaggccact ggcacgctca gcgcccactt cagaaacatg tcactgaaaa gaatcaagcg 1380

cgccgacagg cgtggtgcag agtcggtgac ggaggagaag ttcacagtcc tgtttgagtc 1440

tcagttcagc gttggcagca acgagctggt gttccaggtg aagaccctgt ccctccctgt 1500

ggtcgttatc gtccatggca gccaggacca caatgctact gccaccgtgc tgtgggacaa 1560

tgcctttgct gagccgggca gggtgccatt tgctgtgcct gacaaggtgc tgtggccgca 1620

gctgtgtgaa gcgctcaaca tgaaattcaa ggctgaagta cagagcaacc ggggcttgac 1680

caaagagaac ctcgtgttcc tggcacagaa actgttcaac atcagcagca accacctcga 1740

ggactacaac agcatgtctg tgtcctggtc ccagttcaac cgggagaact tgcccggctg 1800

gaactacacc ttctggcagt ggttcgacgg ggtgatggag gtgctgaaga agcaccataa 1860

gccccattgg aatgatgggg ctatcctggg tttcgtgaac aagcaacagg cccacgacct 1920

gctcatcaac aagccggacg ggaccttcct gctgcgcttc agtgactcgg aaatcggggg 1980

catcaccatt gcttggaagt ttgactctcc ggaccgaaac ctctggaatc tgaagccatt 2040

cacgacgcga gatttctcca ttcggtccct ggccgaccgg ctgggggacc tgaactacct 2100

tatctacgtg ttcccagacc gacccaagga cgaggtcttt gccaagtatt acactcctgt 2160

acttggttcg tcaatgcatc cacagatgcc ggagccagcg ccacctacat ggaccaggct 2220

ccttccccag tcgtgtgccc tcaacctcac tacaacatgt acccacccaa ccctgaccct 2280

gtccttgacc aagatggcga gtttgacctg gatgagagca tggatgttgc caggcacgtg 2340

gaagaacttt tacgccggcc catggacagt ctcgacgccc gcctctcccc acctgctggt 2400

ctcttcacct ccgctagaag ctccctgtcc tgaacgctgg actccatgct tctcttggaa 2460

aaccaccttc agtgtgagga gcccacgtca gttgtagtat ctctgttcat accaacaatg 2520

gctttgcacg tttcacaggg ctaccttgcc cacacagttc tgggtttgtg gctaaagcgg 2580

tggtgacctt tttgttcaga cctcaagggc ccccagggcc tctcgtgtaa gagctgaacc 2640

tatcattgct gacaaaccta tttctccggt gtcctttttc tgtccaatgg ccatttcagt 2700

gaaattctag aaaaggcagg gaggcaggtt taggcaacta agttggagtt ttactcctaa 2760

gctagaagct tcgcccagac cggtgtgctc ctgtcctcgc acaggtggaa gattggggtt 2820

catcttagtg accctttata cctttgtgta tacatacggg ctgcagactt tgtgattgct 2880

cggtgtgctt aagctgttcc cttcaacaca gcagagggct gccacagccg agtgtcagtt 2940

cttgcgccag ggtggatgga cgtgagattc aagtctaacg gccttgtcca cgttcccacc 3000

atcccttttc tccattcgat atcctcaccc ttccagatgg attcatcctt cttgcttttt 3060

tttttttttt tatgtttttg ctttgctttt ttgagacaag gtctctccat atatcccaga 3120

ctatccatga atgatcctcc tacctgtttc tagagtgcta ggattacagg catgcatgac 3180

cacacgtggc ctcatccttt cttcctttcc tgtttgcaac cttgcttatt atatcagaaa 3240

ggaggggaat actgggggtc tgggaggagg aaacctgggg cgaaaaacct gtagcacaca 3300

aaacctgtac acactgtctg aaggaagggg gtggggagcg ctcagtctgc tccactctgc 3360

tgggcggcaa gacctagaac gggccctgca ctgtcccacc atgggttgtg gtctcagaaa 3420

tcgcttcagc agctcttccc tggaggctgt gacagagcag ccaggaggag ccgactagga 3480

gtcctggctt ccaacctctc tgaaggaagc agagggatgc tttttatatt ttgtacatag 3540

aactcaacat ttatgtaaac agtgtttttg aataaagttt ttgttggttt ggtttttgca 3600

aatctaaaaa aaaaaaaaaa aaaaaaa 3628

<210> 53
<211> 8044
<212> DNA

<213> Homo sapiens

<220>
<223> Human BCL11b (NM_138576.3)

<400> 53
tgcgctttcc acctaccaga ccctgaaaga aagtgtcagg agccggtgca aaacccagtt 60

taagttcaag aagacatttg caagtgcaag aggccaagca gtttgaagaa gtgtaagaga 120

ttttttttcc ttcgaaagaa tatattttta aagaaaccag ccagtccgcg gaaagcaaca 180

gcagtttttt ttttttttgc ctctttttct tattttagat cgagaggttt ttcttgcttt 240

tcttcccttt tttttctttt tgcaaacaaa acaaaaaaca gcatagaaga aagagcaaaa 300

taaagaagaa gaagaggagg aagagaggga aagagaggaa gggaaaaaaa acaccaaccc 360

gggcagagga ggaggtgcgg cggcggcggc ggcggcggca gcggcggcag cggcgcggcg 420

gcggctcgga ccccctcccc cggctccccc catcagtgca gctctccggg cgatgccaga 480

atagatgccg gggcaatgtc ccgccgcaaa cagggcaacc cgcagcactt gtcccagagg 540

gagctcatca ccccagaggc tgaccatgtg gaggccgcca tcctcgaaga agacgagggt 600

ctggagatag aggagccaag tggcctgggg ctgatggtgg gtggccccga ccctgacctg 660

ctcacctgtg gccagtgtca aatgaacttc cccttggggg acatcctggt ttttatagag 720

cacaaaagga agcagtgtgg cggcagcttg ggtgcctgct atgacaaggc cctggacaag 780

gacagcccgc caccctcctc acgctccgag ctcaggaaag tgtccgagcc ggtggagatc 840

gggatccaag tcacccccga cgaagatgac cacctgctct cacccacgaa aggcatctgt 900

cccaagcagg agaacattgc agggccgtgc aggcctgccc agctgccagc ggtggccccc 960

atagctgcct cctcccaccc tcactcatcc gtgatcactt cacctctgcg tgccctgggc 1020

gctctcccgc cctgcctccc cctgccgtgc tgcagcgcgc gcccggtctc gggtgacggg 1080

actcagggtg agggtcagac ggaggctccc tttggatgcc agtgtcagtt gtcaggtaaa 1140

gatgagcctt ccagctacat ttgcacaaca tgcaagcagc ccttcaacag cgcgtggttc 1200

ctgctgcagc acgcgcagaa cacgcacggc ttccgcatct acctggagcc cgggccggcc 1260

agcagctcgc tcacgccgcg gctcaccatc ccgccgccgc tcgggccgga ggccgtggcg 1320

cagtccccgc tcatgaattt cctgggcgac agcaacccct tcaacctgct gcgcatgacg 1380

ggccccatcc tgcgggacca cccgggcttc ggcgagggcc gcctgccggg cacgccgcct 1440

ctcttcagtc ccccgccgcg ccaccacctg gacccgcacc gcctcagtgc cgaggagatg 1500

gggctcgtcg cccagcaccc cagtgccttc gaccgagtca tgcgcctgaa ccccatggcc 1560

atcgactcgc ccgccatgga cttctcgcgg cggctccgcg agctggcggg caacagctcc 1620

acgccgccgc ccgtgtcccc gggccgcggc aaccctatgc accggctcct gaaccccttc 1680

cagcccagcc ccaagtcccc gttcctgagc acgccgccgc tgccgcccat gccccctggc 1740

ggcacgccgc ccccgcagcc gccagccaag agcaagtcgt gcgagttctg cggcaagacc 1800

ttcaagttcc agagcaatct catcgtgcac cggcgcagtc acacgggcga gaagccctac 1860

aagtgccagc tgtgcgacca cgcgtgctcg caggccagca agctcaagcg ccacatgaag 1920

acgcacatgc acaaggccgg ctcgctggcc ggccgctccg acgacgggct ctcggccgcc 1980

agctcccccg agcccggcac cagcgagctg gcgggcgagg gcctcaaggc ggccgacggt 2040

gacttccgcc accacgagag cgacccgtcg ctgggccacg agccggagga ggaggacgag 2100

gaggaggagg aggaggagga ggagctgcta ctggagaacg agagccggcc cgagtcgagc 2160

ttcagcatgg actcggagct gagccgcaac cgcgagaacg gcggtggtgg ggtgcccggg 2220

gtcccgggcg cgggggcggg cgcggccaag gcgctggctg acgagaaggc gctggtgctg 2280

ggcaaggtca tggagaacgt gggcctaggc gcactgccgc agtacggcga gctcctggcc 2340

gacaagcaga agcgcggcgc cttcctgaag cgtgcggcgg gcggcgggga cgcgggcgac 2400

gacgacgacg cgggcggctg cggggacgcg ggcgcgggcg gcgcggtcaa cgggcgcggg 2460

ggcggcttcg cgccaggcac cgagcccttc cccgggctct tcccgcgcaa gcccgcgccg 2520

ctgcccagcc ccgggctcaa cagcgccgcc aagcgcatca aggtggagaa ggacctggag 2580

ctgccgcccg ccgcgctcat cccgtccgag aacgtgtact cgcagtggct ggtgggctac 2640

gcggcgtcgc ggcacttcat gaaggacccc ttcctgggct tcacggacgc acgacagtcg 2700

cccttcgcca cgtcgtccga gcactcgtcc gagaacggca gcctgcgctt ctccacgccg 2760

cccggggacc tgctggacgg cggcctctcg ggccgcagcg gcacggccag cggaggcagc 2820

accccgcacc tgggcggccc gggccccggg cggcccagct ccaaggaggg ccgccgcagc 2880

gacacgtgcg agtactgcgg caaggtgttc aagaactgca gcaacttgac ggtgcaccgg 2940

cggagccaca ccggcgagcg gccttacaag tgcgagctgt gcaactacgc gtgcgcgcag 3000

agcagcaagc tcacgcgcca catgaagacg cacgggcaga tcggcaagga ggtgtaccgc 3060

tgcgacatct gccagatgcc cttcagcgtc tacagcaccc tggagaaaca catgaaaaag 3120

tggcacggcg agcacttgct gactaacgac gtcaaaatcg agcaggccga gaggagctaa 3180

gcgcgcgggc cccggcgccc cgcacctgta cagtggaacc gttgccaacc gagagaatgc 3240

tgacctgact tgcctccgtg tcaccgccac cccgcacccc gcgtgtcccc ggggcccagg 3300

ggaggcggca ctccaaccta acctgtgtct gcgaagtcct atggaaaccc gagggttgat 3360

taaggcagta caaattgtgg agccttttaa ctgtgcaata atttctgtat ttattgggtt 3420

ttgtaatttt tttggcatgt gcaggtactt tttattatta ttttttctgt ttgaattcct 3480

ttaagagatt ttgttgggta tccatccctt ctttgttttt tttttaaccc ggtagtagcc 3540

tgagcaatga ctcgcaagca atgttagagg ggaagcatat cttttaaatt ataatttggg 3600

gggaggggtg gtgctgcttt tttgaaattt aagctaagca tgtgtaattt cttgtgaaga 3660

agccaacact caaatgactt ttaaagttgt ttactttttc attccttcct tttttttgtc 3720

ctgaaataaa aagtggcatg cagttttttt tttaattatt ttttaatttt ttttttggtt 3780

tttgtttttg gggtgggggg tgtggatgta cagcggataa caatctttca agtcgtagca 3840

ctttgtttca gaactggaat ggagatgtag cactcatgtc gtcccgagtc aagcggcctt 3900

ttctgtgttg atttcggctt tcatattaca taagggaaac cttgagtggt ggtgctgggg 3960

gaggcacccc acagactcag cgccgccaga gatagggttt ttggagggct cctctgggaa 4020

atggcccgac agcattctga ggttgtgcat gaccagcaga tactatcctg ttggtgtgcc 4080

ctggggtgcc atggctgcta ttcgctgtag attaggctac ataaaatggg ctgagggtac 4140

ctttttgggg agatggggtg gcctgcagtg acacagaaag gaagaaacta gcggtgttct 4200

tttaggcgtt ttctggcttg acggcttctc tcttttttta aatcaccccc accacataaa 4260

tctcaaatcc tatgttgcta caaggggtca tccatcattt cccaagcaga cgaatgccct 4320

aattaattga agttagtgtt ctctcattta atgcacactg atgatattgt agggatgggt 4380

ggggtgggga tcttgcaaat ttctattctc ttttactgaa aaagcagggg atgagttcca 4440

tcagaaggtg cccagcgcta cttcccaggt ttttattttt tttttcctat ctcattaggt 4500

tggaaggtac taaatattga actgttaaga ttagacattt gaattctgtt gacccgcact 4560

ttaaagcttt tgtttgcatt taaattaaat ggcttctaaa caagaaattg cagcatattc 4620

ttctctttgg cccagaggtg ggttaaactg taagggacag ctgagattga gtgtcagtat 4680

tgctaagcgt ggcattcaca atactggcac tataaagaac aaaataaaat aataatttat 4740

aggacagttt ttctactgcc attcaatttg atgtgagtgc cttgaaaact gatcttccta 4800

tttgagtctc ttgagacaaa tgcaaaactt tttttttgaa atgaaaagac tttttaaaaa 4860

agtaaaacaa gaaaagtaca ttctttagaa actaacaaag ccacatttac tttaagtaaa 4920

aaaaaaaaaa attctggttg aagatagagg atatgaaatg ccataagacc caatcaaatg 4980

aagaaataaa cccagcacaa ccttggacat ccattagctg aattatcctc agcccctttt 5040

gtttttggga caacgctgct tagatatgga gtggaggtga tttactgctg aattaaaact 5100

caagtgacac aagttacaag ttgatatcgt tgaatgaaaa gcaaaacaaa aacaattcag 5160

gaacaacggc taattttttc taaagttaaa tttagtgcac tctgtcttaa aaatacgttt 5220

acagtattgg gtacatacaa gggtaaaaaa aaaattgtgt gtatgtgtgt tggagcgatc 5280

tttttttttc aaagtttgct taataggtta tacaaaaatg ccacagtggc cgcgtgtata 5340

ttgttttctt ttggtgacgg ggttttagta tatattatat atattaaaat ttcttgatta 5400

ctgtaaaagt ggaccagtat ttgtaataat cgagaatgcc tgggcatttt acaaaacaag 5460

aaaaaaaata ccctttcttt ttccttgaaa atgttgcagt aaaatttaaa tggtgggtct 5520

ataaatttgt tcttgttaca gtaactgtaa agtcggagtt ttagtaaatt tttttctgcc 5580

ttgggtgttg aatttttatt tcaaaaaaaa tgtatagaaa cttgtatttg gggattcaaa 5640

ggggattgct acaccatgta gaaaaagtat gtagaaaaaa agtgcttaat attgttattg 5700

ctttgcagaa aaaaaaaaaa tcacatttct gacctgtact tatttttctc ttcccgcctc 5760

cctctggaat ggatatattg gttggttcat atgatgtagg cacttgctgt atttttactg 5820

gagctcgtaa ttttttaact gtaagcttgt ccttttaaag ggatttaatg tacctttttg 5880

ttagtgaatt tggaaataaa aagaaaaaaa aaacaaaaac aaacaggctg ccataatata 5940

tttttttaat ttggcaggat aaaatattgc aaaaaaaaca catttgtatg ttaagtccta 6000

ttgtacagga gaaaaagggt tgtttgacaa cctttgagaa aaagaaacaa aaggaagtag 6060

ttaaatgctt tggttcacaa atcatttagt tgtatatatt ttttgtcgga attggcctac 6120

acagagaacc gttcgtgttg ggcttctctc tgaacgcccc gaaccttgca tcaaggctcc 6180

ttggtgtggc cacagcagac cagatgggaa attatttgtg ttgagtggaa aaaaatcagt 6240

ttttgtaaag atgtcagtaa cattccacat cgtcctccct ttctctaaga ggccatctct 6300

aagatgtcag atgtagagga gagagagcga gagaacatct tccttctcta ccatcactcc 6360

tgtggcggtc accaccacca cctctcccgc ccttaccagc agaaagcaat gcaaactgag 6420

ctgctttagt ccttgagaaa ttgtgaaaca aacacaaata tcataaaagg agctggtgat 6480

tcagctgggt ccaggtgaag tgacctgctg ttgagaccgg tacaaattgg atttcaggaa 6540

ggagactcca tcacagccag gacctttcgt gccatggaga gtgttggcct cttgtctttc 6600

ttccctgctt tgctgctttg ctctctgaaa cctacattcc gtcagtttcc gaatgcgagg 6660

gcctgggatg aatttggtgc ctttccatat ctcgttctct ctccttcccc tgcgtttcct 6720

ctccatcctt catcctccat tggtcctttt tttttctttc atttttattt taatttcttt 6780

tcttcctgtc tgttcctccc ctaatcctct attttatttt tattttttgt aaagccaagt 6840

agctttaaga taaagtggtg gtcttttgga tgagggaata atgcattttt aaataaaata 6900

ccaatatcag gaagccattt tttatttcag gaaatgtaag aaaccattat ttcaggttat 6960

gaaagtataa ccaagcatcc ttttgggcaa ttccttacca aatgcagaag cttttctgtt 7020

cgatgcactc tttcctcctt gccacttacc tttgcaaagt taaaaaaaag gggggaggga 7080

atgggagaga aagctgagat ttcagtttcc tactgcagtt tcctacctgc agatccaggg 7140

gctgctgttg cctttggatg ccccactgag gtcctagagt gcctccaggg tggtcttcct 7200

gtagtcataa cagctagcca gtgctcacca gcttaccaga ttgccaggac taagccatcc 7260

caaagcacaa gcattgtgtg tctctgtgac tgcagagaag agagaatttt gcttctgttt 7320

tgtgtttaaa aaaccaacac ggaagcagat gatcccgaga gagaggcctc tagcatgggt 7380

gacccagccg acctcaggcc ggtttccgca ctgccacaac tttgttcaaa gttgccccca 7440

attggaacct gccacttggc attagagggt ctttcatggg gagagaagga gactgaatta 7500

ctctaagcaa aatgtgaaaa gtaaggaaat cagcctttca tcccggtcct aagtaaccgt 7560

cagccgaagg tctcgtggaa cacaggcaaa cccgtgattt tggtgctcct tgtaactcag 7620

ccctgcaaag caaagtccca ttgatttaag ttgtttgcat ttgtactggc aaggcaaaat 7680

atttttatta ccttttctat tacttattgt atgagctttt gttgtttact tggaggtttt 7740

gtcttttact acaagtttgg aactatttat tattgcttgg tatttgtgct ctgtttaaga 7800

aacaggcact tttttttatt atggataaaa tgttgagatg acaggaggtc atttcaatat 7860

ggcttagtaa aatatttatt gttcctttat tctctgtaca agattttggg cctctttttt 7920

tccttaatgt cacaatgttg agttcagcat gtgtctgcca tttcatttgt acgcttgttc 7980

aaaaccaagt ttgttctggt ttcaagttat aaaaataaat tggacattta acttgatctc 8040

caaa 8044

<210> 54
<211> 7922
<212> DNA
<213> Mus <mouse, genus>

<220>
<223> Mouse Bcl11b (NM_001079883.1)

<400> 54
aatttatttt agccttttct ctattttaga gcaagccatc ttcctggctt tttcttcttt 60

ctttttgcaa aaacaaaaac aaaaacacag cccagaagaa agagcaaaat aaagacgaag 120

aagaagaaga ggaggaggaa gaggaagaga ggaagggcag aggcaccaac ccgggcggag 180

gaggaggcgc ggcggcggcg gcggctcaga ccccctcccc ggcccgcatc tgtgcagctt 240

tccgggcgat gccagaatag atgccggggc aatgtcccgc cgcaaacagg gcaacccgca 300

gcacttgtcc cagagggaac tcatcacgcc agaggctgac catgtggagg ctaccatcct 360

cgaggaagac gagggtctgg agatagagga gcctagcagc ctggggctga tggtgggagg 420

ccccgaccct gatctactca cctgtggcca gtgtcagatg aacttcccgc tgggggacat 480

cctggttttt atagagcaca agaagaaaca gtgtggaggc ctgggcccct gctacgacaa 540

ggtcctggac aagagcagtc cacctccctc ctctcgctct gagctcagga gagtatctga 600

gccagtggag atcgggatcc aggtcacccc tgatgaagat gaccacctac tgtcacccac 660

gaaaggcatc tgtcccaagc aggagaacat tgcagggccg tgcaggcctg cccagctgcc 720

atcgatggcc cccatagctg cctcctcttc ccaccctccc acctccgtga ttacttcacc 780

tctgcgtgcc ctgggcgttc tcccaccctg tttcccgctg ccttgctgtg gtgcacgccc 840

catctcgggc gacgggactc agggtgaggg tcagatggag gctccctttg gatgccagtg 900

tgagttgtca ggtaaagatg agccttccag ctacatttgc acaacatgca agcagccctt 960

caacagcgcc tggttcctgc tgcagcacgc acagaacaca catggcttcc gaatctacct 1020

ggagcctggg ccggccagca cctcgctcac gcccaggctc accatcccgc caccgctcgg 1080

gccggagacc gtggcgcagt ccccactcat gaatttcctg ggggacagca atcctttcaa 1140

cctgctgcgc atgacgggcc ccatcctgcg ggaccaccct ggcttcggtg agggccgctt 1200

gccaggtacg ccaccgctct tcagcccacc gccacgccat cacttggacc cacaccgcct 1260

cagtgcagag gagatggggc tcgtggccca gcaccccagt gccttcgacc gagtcatgcg 1320

cctgaacccc atggccatag actctcctgc catggacttc tcccggcggc tgcgagaact 1380

ggccggcaac agctccacgc cgccgcccgt gtccccaggc cgtggcaacc ctatgcaccg 1440

gctgctgaac cctttccagc ccagtcccaa gtccccgttc ctcagcacgc caccgctgcc 1500

acccatgcct gcgggcacac cgccaccgca gccgcctgcc aagagcaagt cctgtgagtt 1560

ctgcggcaag accttcaagt tccagagcaa tctcatcgtg caccggcgca gccacacggg 1620

cgagaagccc tacaagtgcc agctgtgcga ccatgcgtgc tcgcaggcga gcaagctcaa 1680

gcgccacatg aagacgcaca tgcacaaggc gggctctctg gctggccgct cagacgacgg 1740

gctctcagct gccagctccc ctgagccggg caccagcgag ctgccaggtg acctgaaagc 1800

ggccgatggc gacttccgcc accatgagag cgacccatct ctgggccccg agcctgagga 1860

cgacgaggac gaggaggagg aagaagagga gctgctgctg gagaacgaga gccggcctga 1920

gtcgagcttc agcatggact cggagctggg ccgtggccgc gagaacggag gtggcgtgcc 1980

accgggggtg gcgggcgcag gggctgcagc tgcggctctg gcggatgaga aggctctggc 2040

cctgggcaag gtgatggagg acgcagggct gggcgcactg ccgcagtatg gggagaagcg 2100

gggcgccttc ctgaagcgtg caggcgacac gggtgatgcc ggagctgttg gctgtgggga 2160

cgcgggtgca ccgggtgcag tgaacgggcg cggcggggcc ttcgcgccag gcgcagagcc 2220

ctttccagct ctcttcccac gcaagccagc accgctgccc agccctgggc tcggtggtcc 2280

cgcgctgcac gcggccaagc gcatcaaggt ggagaaagac ctggagctgc cacctgccgc 2340

cctcatccca tctgagaacg tgtactcgca gtggctcgtg ggctacgcag catcgcgcca 2400

cttcatgaag gacccattcc tgggcttcac ggatgcgcgc cagtcgcctt tcgccacatc 2460

gtcggaacat tcctctgaga acggcagcct gcgcttctca acgccacccg gggacctgct 2520

ggacggcggg ctgtccgggc gcagtggcac ggcgagcggg ggcagcacac ctcacctggg 2580

tggtccgggt cctgggaggc cgagctccaa ggagggccgc cgcagcgaca catgtgagta 2640

ctgcggcaag gtcttcaaga actgtagcaa cctgacggtg caccggagga gccacaccgg 2700

cgagcggcct tacaagtgcg agctgtgcaa ctacgcgtgc gcgcagagca gcaagctcac 2760

gcgccacatg aagacgcacg ggcagatcgg caaggaggtg taccgctgcg acatctgcca 2820

gatgcccttc agcgtctaca gcaccctgga gaaacacatg aaaaagtggc acggtgaaca 2880

cttgctgact aatgatgtca aaatcgagca ggctgagagg agctaagcgc atacgtgggg 2940

gacactgcgt gcgtgcgtct gtacagcgtg accatcgcca accttcgcca acgggaccgg 3000

tgaccggact ggcctctgca tccccggggc ccagggaggc ggcagtccag cctaacctgt 3060

gtctgcgaag tcctatggaa acctgagggt tgattaaggc agtaaaaaat taaaaaaaaa 3120

aaaacaaaaa aacaaaaaaa tttgtggagc cttttaactg tgcaataatt tctgtattta 3180

ttgggttttg taattttttt ggcatgtacg tgcaggtacc tattattatt attatttctg 3240

tttaaattcc tttaaaagat tttgttgagt atccatccct tcattttttt ttttaataac 3300

ccagtagtag tctgagcaat gactcgcaag tgacgtagag ggaagctatc ttttaaatta 3360

taattttgtg tgtgtgtgtg ggggggtgct gcttttttga aatttaagct aagcatgtgt 3420

aatttcttgt caagaagcca acacttaaat gacttttaaa gttgtttgcg ttttcatcat 3480

ttactttgtc ctgaaatgaa aagtggcatg tgggattttt ttccgggggg gaggggtggc 3540

tttcaaaacc attttttttt tccgggaggt ggatgtacag cggattacaa tctttaaaat 3600

tgtagcactt tgtcttagca tcgcattgga gagttagcac tgatgtcctc ctgagccaca 3660

gaggcgtctc tgtgtggaat tcaactttca tgctatggaa ggaaaatgtt gaggggggcg 3720

tggcaaacga gcctctcccc cacttgggac cccgagaatc ttttccaggg tgactctagg 3780

aacagccccc acatcctgca attgtgtgtt accaggagat atgataatgt ccttttctgg 3840

gggagcttgg cgtattggga agccgcctct gccctttgct gtagatttgg ctgcataaaa 3900

tgggccgagg atacccactg ggtagacagc tgtggcctgt agtggcggga aagaagagac 3960

taacaatgtt ctctcaggct tctcctggca agatggcttc tcttttcagc cccagcccta 4020

cagataaatc ccaggtgtcc attgctacag tggtaacttg actgtgtccc aagcagacaa 4080

ctggccctga tggtgaactg gagaagttag ctgcggggtc gctcatctca cgctgatggg 4140

cactgtaggg caaaggaatc tcacccgttc tctcttcccg gaggagctgg taagagttct 4200

gtccaaggtg ccccacgctg cttccgtttc tgtgttttgt cttttccttc ctccaattaa 4260

gttgggacat actatacatt ggacttgttg ggatttgatg tttgaattct gttgacccgc 4320

actttaaagc ttttgtttgc atttaaatta aatggcttct aaacaagaaa ttgcagcata 4380

ttcttgtctt tggcccagag gtgggttaaa ctgtaaggga cagctgagat tgagtgtcag 4440

tgttgctaag cgtggcattc acaatactgg cactataaag aacaaaatta aataatttat 4500

tggacagttt ctctactgcc attcaatttg atgtgcgtgc cttgaaaacc gatcttccta 4560

tttgagtctc ttgagacaaa tgcaaaactc tctttttttt ttgagatgaa aaaaaaaaaa 4620

gactttttaa aaagcaaaca agagaagtac attctttaga aacaaagcca catttacttt 4680

aaattaaaaa aaaaaatcct ggttgaagag agaggacggg aaaatgccat aagaccagtc 4740

aaatgaagaa ataaacccag cacagccctg gacatctatt agcagaatcg ttttcagacc 4800

cccctccccc ttttttgggg caacgctgct agatatggag tggaggtgac ttactgctga 4860

attaaaactc acgtgacaca agccgatatt gttgaatgaa aaaacaaaca acaacaacaa 4920

caacaaaaaa caattcagga acaatggctg attttttttt tcctcctaaa tttaaattta 4980

gtgcactctg tcttaaaaat acgtttacag tattggatac atacaagggt aaaataaatc 5040

gtgtgtatgt gtgttggagc gatcttttta ttttatttta ttttcaaagt ctgcttaata 5100

ggttatacag aatgccacaa tggctaggtg tatattgttt tcttttggtg atggggtttt 5160

agtatacatt atatatattg aaatttcttg attactgtca aagtggacca gtatttgtaa 5220

taattgagac tgcctgggca ttttacaaaa tgagaagaaa aaaaagtcct ttttcttttc 5280

cttgaaaccg ttacagtaag ttttaaatgg tgggtctata aaatctgctc tgtcacagca 5340

acctgtaaag tcggagtttt agtaaatttt tttctgcctt gggtgttgaa tttttatttt 5400

gaaaacaaaa tgtatagaaa cttgtatttg gggtttaaag ggggattgct gcaatgtgta 5460

gaaaaagtat gtagagaaag tgcttaatat tgttactgct ttgcagaaga agaaaaaaaa 5520

aatcacactt ctgacctgtg cgtatttttc tctcccacct ccctctggaa tggatctatt 5580

ggtcggttca tatgatgtag gctctcgctg tatttttaac tggagcttgt aattttttaa 5640

ctgtaagctt gtccttttaa agggatttaa atgtaccttt ttgttagtga atttggaaat 5700

aaaaagagaa aaaaaaaaca aaaacaaaca ggctgccata atatattttt ttaatttggc 5760

aggataaaat attacaaaaa aaaaatcccc cacaaaacaa caaacaaaca aaaatcccac 5820

ccacatttgt atgttaagtc ctcttgtatg gggtgggggg agggttgttt gacaaccttc 5880

aagagaaaaa gacacagaaa gagacatgct ttgattcaca agtcatttta gttgtatata 5940

gtctgtcaga attggcctac tcaaaaagaa ctgttggctt tgggctttca ttcgaatgcc 6000

ccaactttgc atctggcctc cttggtgagg ccacagccga cttgatggca agtccttact 6060

tgtgttgagt ggagaaaaga aagcccttcc tccaagaggc agtctataag atggtagcgc 6120

ggtgagagag caagggaggt taccttctcc gctgtggcta ctgcggccac cccctccacc 6180

ccctggccac gctcaggagc agagagaatc gacagctgct tttgtccttg agcaaacctg 6240

gatggagcag cgaagcttct gagaggagca gctgggccca ggggagcctc tgccactgag 6300

actggtgcag gcgggacaga ggagagcctt gcacgcaggt cctgttccct tggaatgtct 6360

gctccatttt ccccctctgt ctctctgctt ttctctgcaa ttccatccgt ttcagaccag 6420

tgaggcctgg aatgaaggaa ttctgtgcct tcccatctat gttccctccg tcctccccag 6480

gctctgccta ctctttgtgg actcctcttt aattttcttc ctcctttgcc ccctctcccc 6540

aaaccctctt ttctcttcct ttcttctctc cttcttttct tcctcctcct cctcttttct 6600

cctcctcctc ctcctcctcc tcctcctcct cctcctcctc ttcttcttct tcttcttctt 6660

cttcttcttt ttcttcttct cctcctcctt cttcttcttt tttttttttt tgtaaagcca 6720

agtagcttta agataataaa atggttgttt attggatgag ggaataatga tacactccac 6780

aaatacctat atcaggaagc catttttttt taatttcagg aaatgtaaga aaccatcatt 6840

tcaggttatg aaagcgtgat tgtgcatctt tccaggcaga tctttactaa atgcagagtc 6900

tgtctcactg tttcccattt gacacagtta aaagatgggg gcctagtggg aggccaggcc 6960

taagtctcag tgtcctgcat gcctgcccta cggcccaggg ttaccctgcc cttttgacac 7020

cctgcctctc tgctacctgg gagcctctgt gtggttctcc tgtgctcata aaagctagcc 7080

agtgctcacc agctcaccta tcaccagaat gccaggacta agccatccta aagcacaagg 7140

acttgtgtgt ctctgtcaac tgcagagaga agaggatttt tgcttctctt atcaaagaga 7200

aaaatcaacc aaccaaccta cccgaagcag gtgacccagc ctaagggctc taccgcgggt 7260

gacccagccg acctcaggtc gggttccaca ctgccacaac tttacccaaa gttgctccct 7320

attggaacct gccacttgcc attggagggt catggggggg tgggggagga ggagactgga 7380

ttactcgaag caaaatgtga accctgaagc gaacacagcc ttccacctgg ccctccccgt 7440

ccgaggtttg attccgtgat tttggtgctt tctgtgtctg ccctgcatag ccaaggcaca 7500

ttgatctctg cggtttgctt ttgtactggc aaggcggaat atttttatta ccttttctat 7560

tacgtatcgt atgagctttt gttgtttgcc tagagggttt gtctcttaca gcaagtttgg 7620

agctatttat tattgcttgg tatttgtgct ctgcttttaa gaatgggcac ttggttttat 7680

ttttgtttgt ttgttttttt gttgtttttt aattatggat aaaatgttga gatgacaaaa 7740

ggtcatttca atatggctta gtaaaaatat ttattgtttc ttctctgtac aagatttggg 7800

gcctcttttt tcccccttaa tgtcacaatg ttgagttcag catgtgtctg ccatttcatt 7860

tgttcaaaac caagtttgtt ctggtttcaa gttatgaaaa taaattggga catttaactt 7920

ga 7922

<210> 55
<211> 7530
<212> DNA
<213> Homo sapiens

<220>
<223> Human NFATC2 (or NFAT1) (NM_012340.4)

<400> 55
agcaggaagc tcgcgccgcc gtcgccgccg ccgctcagct tccccgggcg cgtccaggac 60

ccgctgcgcc aggcgcgccg tccccggacc cggcgtgcgt ccctacgagg aaagggaccc 120

cgccgctcga gccgcctccg ccagccccac tgcgaggggt cccagagcca gccgcgcccg 180

ccctcgcccc cggccccgca gccttcccgc cctgcgcgcc atgaacgccc ccgagcggca 240

gccccaaccc gacggcgggg acgccccagg ccacgagcct gggggcagcc cccaagacga 300

gcttgacttc tccatcctct tcgactatga gtatttgaat ccgaacgaag aagagccgaa 360

tgcacataag gtcgccagcc caccctccgg acccgcatac cccgatgatg tcctggacta 420

tggcctcaag ccatacagcc cccttgctag tctctctggc gagccccccg gccgattcgg 480

agagccggat agggtagggc cgcagaagtt tctgagcgcg gccaagccag caggggcctc 540

gggcctgagc cctcggatcg agatcactcc gtcccacgaa ctgatccagg cagtggggcc 600

cctccgcatg agagacgcgg gcctcctggt ggagcagccg cccctggccg gggtggccgc 660

cagcccgagg ttcaccctgc ccgtgcccgg cttcgagggc taccgcgagc cgctttgctt 720

gagccccgct agcagcggct cctctgccag cttcatttct gacaccttct ccccctacac 780

ctcgccctgc gtctcgccca ataacggcgg gcccgacgac ctgtgtccgc agtttcaaaa 840

catccctgct cattattccc ccagaacctc gccaataatg tcacctcgaa ccagcctcgc 900

cgaggacagc tgcctgggcc gccactcgcc cgtgccccgt ccggcctccc gctcctcatc 960

gcctggtgcc aagcggaggc attcgtgcgc cgaggccttg gttgccctgc cgcccggagc 1020

ctcaccccag cgctcccgga gcccctcgcc gcagccctca tctcacgtgg caccccagga 1080

ccacggctcc ccggctgggt acccccctgt ggctggctct gccgtgatca tggatgccct 1140

gaacagcctc gccacggact cgccttgtgg gatccccccc aagatgtgga agaccagccc 1200

tgacccctcg ccggtgtctg ccgccccatc caaggccggc ctgcctcgcc acatctaccc 1260

ggccgtggag ttcctggggc cctgcgagca gggcgagagg agaaactcgg ctccagaatc 1320

catcctgctg gttccgccca cttggcccaa gccgctggtg cctgccattc ccatctgcag 1380

catcccagtg actgcatccc tccctccact tgagtggccg ctgtccagtc agtcaggctc 1440

ttacgagctg cggatcgagg tgcagcccaa gccacatcac cgggcccact atgagacaga 1500

aggcagccga ggggctgtca aagctccaac tggaggccac cctgtggttc agctccatgg 1560

ctacatggaa aacaagcctc tgggacttca gatcttcatt gggacagctg atgagcggat 1620

ccttaagccg cacgccttct accaggtgca ccgaatcacg gggaaaactg tcaccaccac 1680

cagctatgag aagatagtgg gcaacaccaa agtcctggag atacccttgg agcccaaaaa 1740

caacatgagg gcaaccatcg actgtgcggg gatcttgaag cttagaaacg ccgacattga 1800

gctgcggaaa ggcgagacgg acattggaag aaagaacacg cgggtgagac tggttttccg 1860

agttcacatc ccagagtcca gtggcagaat cgtctcttta cagactgcat ctaaccccat 1920

cgagtgctcc cagcgatctg ctcacgagct gcccatggtt gaaagacaag acacagacag 1980

ctgcctggtc tatggcggcc agcaaatgat cctcacgggg cagaacttta catccgagtc 2040

caaagttgtg tttactgaga agaccacaga tggacagcaa atttgggaga tggaagccac 2100

ggtggataag gacaagagcc agcccaacat gctttttgtt gagatccctg aatatcggaa 2160

caagcatatc cgcacacctg taaaagtgaa cttctacgtc atcaatggga agagaaaacg 2220

aagtcagcct cagcacttta cctaccaccc agtcccagcc atcaagacgg agcccacgga 2280

tgaatatgac cccactctga tctgcagccc cacccatgga ggcctgggga gccagcctta 2340

ctacccccag cacccgatgg tggccgagtc cccctcctgc ctcgtggcca ccatggctcc 2400

ctgccagcag ttccgcacgg ggctctcatc ccctgacgcc cgctaccagc aacagaaccc 2460

agcggccgta ctctaccagc ggagcaagag cctgagcccc agcctgctgg gctatcagca 2520

gccggccctc atggccgccc cgctgtccct tgcggacgct caccgctctg tgctggtgca 2580

cgccggctcc cagggccaga gctcagccct gctccacccc tctccgacca accagcaggc 2640

ctcgcctgtg atccactact cacccaccaa ccagcagctg cgctgcggaa gccaccagga 2700

gttccagcac atcatgtact gcgagaattt cgcaccaggc accaccagac ctggcccgcc 2760

cccggtcagt caaggtcaga ggctgagccc gggttcctac cccacagtca ttcagcagca 2820

gaatgccacg agccaaagag ccgccaaaaa cggacccccg gtcagtgacc aaaaggaagt 2880

attacctgcg ggggtgacca ttaaacagga gcagaacttg gaccagacct acttggatga 2940

tgagctgata gacacacacc ttagctggat acaaaacata ttatgaaaca gaatgactgt 3000

gatctttgat ccgagaaatc aaagttaaag ttaatgaaat tatcaggaag gagttttcag 3060

gacctcctgc cagaaatcag acgtaaaaga agccattata gcaagacacc ttctgtatct 3120

gacccctcgg agccctccac agcccctcac cttctgtctc ctttcatgtt catctcccag 3180

cccggagtcc acacgcggat caatgtatgg gcactaagcg gactctcact taaggagctc 3240

gccacctccc tctaaacacc agagagaact cttcttttcg gtttatgttt taaatcccag 3300

agagcatcct ggttgatctt aatggtgttc cgtccaaata gtaagcacct gctgaccaaa 3360

agcacattct acatgagaca ggacactgga actctcctga gaacagagtg actggagctt 3420

ggggggatgg acgggggaca gaagatgtgg gcactgtgat taaaccccag cccttgcgtt 3480

cgtttttcca ggtcacagat acagctcctg taccttttga aggcaaggag ttctcagagc 3540

aaccaaagga acgtgaccca agagcccagc ttacaggctg aagaaaccca aaaccctcga 3600

tagagacaga aactgaactg tcagtcctta gagctcgccc agtccatgcc acaactgggc 3660

cacagctaaa gctttatttt tgaattctca ttccaaaacc aaactgtctt gcccagacaa 3720

gatcacctgt taagacttct tggcgttaag ttatgacatg tatacgcgtt tgttattatt 3780

attttttctg ctttaaaagg ctgaccaggg cacctagccc tggagctgtc ttggcgagct 3840

gttctttaac ccctgcagca cgcagtcctg ctaacacaat ttccatagac ttggggggct 3900
gacccaggct gcagagagca agcacctgtc tgctgcagct gtacaacctg gatgctttgc 3960
aaggttccgg cttgctttct tcctagcagc cagagtgctt ttccgtaaag cggtggagaa 4020
tctcaagcat gtgcatttaa ttgaggaata gcagaagggc taaagcaacc aagaaaagaa 4080
gtgtgggtat ttttgttaag taaaacagcc caagtgcttc tggaggtggg tttctaccaa 4140
gatagaggaa aagggctgaa ttccctctaa gtgggacagc cgagctcagg atgtgcttcc 4200
cagcttcact ggttaatttg acctgaacct atttaaagat cccttctgcc cctgaagacc 4260
tatccgcact caaattctaa catgaagaaa tctactcgaa tgcatccttt actttgaatg 4320
agctctattc ggttgcatgt tatatgtgat ttccttcctc ccaactgttt ccactgagcg 4380
cacccagtct cccctagtct tcctctgtgg gtgtgatttt tgtgattttt acaaacaaaa 4440
cccttgaagt tcttggcaga tgtgtttgtt tctgtttgca tgtactgcag ataccccagg 4500
acaagcgggg gattcatttt tcagccattc agttgtttcc tcaataatcc gcagcaaagt 4560
gaaaatattc ttagcactca gactgtactt agagtgtttt ctcagtccag tctgtacagt 4620
ctgtaggcag aaggcctcag aagaaagtca tggccactca gtgcccactg tgggctttgt 4680
aagtcctggc tctcccgtca aggttaccca gaggtaaaag cttcctggga gtggggccag 4740
gtgtgtttgg cactccagat agaaggcaaa atgctcagat tcgggcctgt gcacttgtat 4800
gcaacctgtc ggtcgatacc tagcatttat ttttccctga caatgaacga cctttccctc 4860
acccacccta agctcaaaga gtttagcaaa attctctttt aaataaacag aatgccagta 4920
agaggttgac ccctaccatg gaacttctgg gatgctaaat acttcctcat gaacaaaata 4980
agttccttat tataagttcc ttatactagc agcttcacct aaagaatttt ctctccagca 5040
atattgactt cactggggaa aagccaagag tgtgtggtga gtgatttgtt ctcactcgac 5100
ctggctagga ctggctagga gctgtttttt gtacatgagg gaatttgggc tttcctcagt 5160
tatctgaatg ttttacccaa gtgccttcct gctattgtag caaagtagct cagcttcctt 5220
gtccacaggg tgaaaaagga ctaatgcatt ttccatcagt tttctaacta tgttagcaaa 5280
aacggcctcc tggtagctca acctcctgta cgcgtgtgtg tgtgtaatac acacacaaat 5340
aaacccctct gtttttctaa gacatcttag ctggatatta taggaagcac tttcataaac 5400
aactgtaaca aatcgcaaag gaaagagaaa caaaagcatt agatttgaga cataaacagg 5460
caagagaaag tgtattagga actgacagct atcaaggaag ttttgtcagt tacaaatgct 5520
aggaggaaat tttgccaaga aggatggctc atgaaatatt tccagtacgg gaagaggcaa 5580
taagatcctc taagagaatg agaaagtagg ggtgtctaaa tggtaaagat gggtgtgttg 5640
cacgtgtgtt agaaggatct cagttgagtg aaggtttgca ctgctacatc taagttaatg 5700
taaatatgta gcactctgac aggtctaccg tgttgctgaa tgtagtatat ttccaaagtt 5760

tgcaagtctt cctgtattgt acaaagatgc tgctgcttga taatatgtat agcaatccag 5820

attagtatgt tattaaattt tattttctta cctgtatttt tatgcttttt acctgtcctc 5880

aaaatattac acccctgttg gaattagatt tatatttata aatggtcaga aatcttttta 5940

agtgtctctt tttacacata ggttgatttt tttttcttaa gagaaatgat gtattcttga 6000

aacatttgtt actcattcca ggaaacaaaa acccatataa taaaaccccc actcagagcc 6060

tgttagtcac ctctctagaa gatggcatct caggagaagg aatggctttg tggaagaagg 6120

aatcaccttt ttcttgctca agaattatgc tgacttcagc cctgagcctg gatctggtca 6180

ctgagaatca tcaagtgtct agatcctccc cccaaaataa ctaatttagt aggtgatttt 6240

gattttaaaa aattgacacc aaaaccctgc ctgcattgta atggaattcg aaaagaattc 6300

atgttcacag aactcaacgt tcaggctaat atttacagaa gggaccaaat ctaaatcctg 6360

gtagataact cctgtatgct ttatccaaag gacacccaca gttttccagc atagatataa 6420

ccaaggatga attgattcct tcaaagaact gggaggcacg gatattgcat tttttgttta 6480

catccagtag ccaagacgcc tcagtgagcc agtcttgggc agaggctgtc acatttaggc 6540

agattggaag ttggtatgtt ctaattctca ctctggacta cagtgaggct gaatttatca 6600

tgtcaaaaaa aaaaaaaaaa aaagaccttt ccaagtgctt tctattgctc agaattgaaa 6660

gaatgttttc atttcaagtt tacaagaggc atggatggag ttgtgacgtt cttgacaagc 6720

tgggctaacc tttcccgaac ttgtttcccg gaggcaaggt gctcggtgac ccagcgcatc 6780

ttaaccttgg gtctcctagg ctcgaggcta gggcattacg tttcgtggaa ccaaagcagc 6840

caattgcata gcaagtattt tcctgcattc caattaaatg cttaagaaaa agcagcatcc 6900

tataaaattg tgatcataaa catccatttc cctcagcttt tgtgagtgcc ttgacttaca 6960

gccaacatca ctgtttaact cagtctgttt aaaaacaaac ttttctggtg gttgataaca 7020

gagagttgct ccctgagcca tcagggtcct gggagctgga agtgaaaggg ttattaacat 7080

tctaccttta tgcagctgtt ggctgaccag aataaactcc ctgctgagtt caagctttga 7140

atggaatgga tgcaaatgat gttgtttcca ttagagcagg tgctcacagc attctgattg 7200

gcctgagcag accgaggcta tggctgttgg gacaagctta gcatcctgga catcttgtca 7260

aagaacctca ctcacccctc tggcctctac agccctcaga ggagagaaaa ccaattctcc 7320

aacaaacagg tctctccaac atggtggtgc tggcaggctt aggtttagaa aatcctgact 7380

gttaaaggcg tttgaataca tcacattcct atgcaaatgt ttttaatctc cagtttaatg 7440

tagtttattt ttcctatatg taaagtattt ttatacggct tgtatcatga tagtttagca 7500

ataaaacagt tggaagcaac atctaaaaaa 7530

<210> 56

<211> 6759
<212> DNA
<213> Mus <mouse, genus>

<220>
<223> Mouse Nfatc2 (or Nfat1) (NM_010899.3)

<400> 56
aggctgcaac aggtgccttg agcaggaagc tcgcgccgcc acccgctccc cggacgcgtc 60

caggacccac tgcgccgtga gccatcctcg gcctcaccgt gcgccacaga tacggtgacc 120

cctgctctgc gccccgcgac tctatacgaa cccgcatccc gaacgccccg agccatggac 180

gtcccggagc cgcagcccga ccccgatggc ggggacggcc ccggccacga gcccgggggc 240

agtccccaag acgagctgga cttttccatc ctcttcgatt atgactatct gaaccctatc 300

gaagaagaac cgatcgcaca taaggccatc agctcaccct ccggactcgc atacccggat 360

gatgtcctgg actatggcct caagccatgc aacccccttg ccagtctctc tggcgagccc 420

cctggccggt tcggagagcc ggatagtata gggttccaga actttctgag cccggtcaag 480

ccagcagggg cttcgggccc gagccctcgg atcgagatca ctccatccca cgaactgatg 540

caggcagggg gggccctccg tgggagagac gccggcctgt cccccgagca gccggccctg 600

gccctggccg gcgtggccgc cagcccgagg ttcacactgc ccgtgcccgg ctacgagggc 660

taccgcgagc cgctttgctt gagccccgct agcagcggct cctctgccag cttcatttct 720

gacaccttct ccccctacac ctcgccctgc gtctcaccca ataacgccgg gcccgacgac 780

ctgtgtcccc agtttcaaaa catccctgct cattattccc ccagaacctc tccaataatg 840

tcacctcgaa ccagcctcgc cgaggacagc tgcctgggcc gacactcgcc cgtgccccgt 900

ccggcatccc gctcctcctc acccggtgcc aagcggaggc attcgtgcgc agaggctttg 960

gttgctcctc tgcccgcagc ctcaccccag cgctcccgga gcccctcgcc acagccctcg 1020

cctcacgtgg cactgcagga cgacagcatc cccgctgggt acccccccac ggccggctct 1080

gctgttctca tggatgccct caacaccctg gccaccgact cgccctgcgg gatcccctcc 1140

aagatatgga agaccagtcc tgacccgacg cctgtgtcca ccgctccgtc caaggctggc 1200

ctggcccgcc acatctaccc tactgtggag ttcctggggc catgtgagca ggaggagagg 1260

aggaattccg ctccagagtc catcctgctg gtaccaccta cttggcccaa gcagttggtg 1320

ccggccattc ccatctgcag catccctgtg actgcatccc tcccaccact cgagtggcca 1380

ctctccaatc agtcgggctc ctatgagcta cggattgagg tccaacccaa gccccatcac 1440

cgggcccact atgagacgga gggcagccgt ggcgctgtca aagccccaac aggaggacac 1500

cctgtggtgc agctccacgg ctacatggag aacaagcctc tggggcttca gatcttcatt 1560

gggacagcag atgagaggat ccttaagccg cacgccttct accaagtaca caggatcact 1620

gggaaaacgg tcaccaccac gagctatgag aagatcgtag gcaacaccaa ggtcctggag 1680

atccccctgg agccaaagaa caacatgaga gccaccatcg actgtgcagg catcctgaag 1740

ctccgaaacg ctgacatcga gctgcggaag ggcgagacgg acatcggcag gaagaacacg 1800

cgtgtgcgcc tggtgttccg cgtgcacgtc ccagagccca gtgggcgcat cgtctccctg 1860

caggctgcgt ccaaccccat cgagtgctct cagcgctctg cccacgagct gcccatggtg 1920

gagagacaag acatggacag ctgcctggtc tacgggggcc agcagatgat cctcacgggc 1980

cagaacttca cagcggagtc caaggttgtg ttcatggaga agactacaga tgggcagcag 2040

atttgggaga tggaagctac ggtggataaa gacaagagcc agcctaacat gctttttgtt 2100

gagatccccg agtatcggaa caagcacatc cgcgtgcccg tgaaagtcaa cttctacgtc 2160

atcaacggaa agaggaaacg aagtcagcca cagcacttta cctaccaccc agtccctgcc 2220

atcaagacag agcccagcga tgagtatgaa ccatctttga tctgcagccc cgcccatgga 2280

ggcctgggga gccagccata ttacccacag cacccaatgc tggccgagtc cccctcctgc 2340

cttgtggcta ccatggcccc ctgccaacag ttccgctcgg ggctctcatc ccccgatgct 2400

cgctaccaac agcagagccc cgcagctgcc ctctaccaga gaagcaagag cctgagtccc 2460

ggcctgctgg gctaccagca gccgtccctc ctggcagcac ccttgggtct ggctgatgcc 2520

caccgctctg tgctggtgca tgctggttct caggggcagg ggcagggctc caccctgcca 2580

cacacatcct cggccagcca gcaggcctca cccgtgatcc actactcacc caccaaccag 2640

cagcttcgcg gtgggggtca ccaggagttc cagcatatca tgtactgtga aaacttcggc 2700

cccagctctg ccaggcctgg cccgcctccc atcaaccaag gtcagaggct gagcccgggc 2760

gcctacccca cagtcatcca acaacagact gccccgagcc aaagagctgc caaaaacgga 2820

cccagtgacc agaaggaagc tctgcccacg ggagtgaccg tcaaacagga acagaacctg 2880

gaccagacct acctggatga cgttaatgaa atcatcagga aggagttttc aggacctccc 2940

tcccgaaatc agacctagaa acaaacataa tctcgcgacg ccttctactc tggaccgctt 3000

ctgtctcctc ctgccgtctg tgactggaac actgcgcatg cgcacagtgc acgcagatgg 3060

tgtcagagcc tgggagcctg ctgccgcgca aacagcatct tagccgttgt ccatggtgac 3120

tggacacgaa aagcaactgc ctgctgacca aagcctcatt ctcaactcac caggatgctg 3180

aacctgagac gccaggctgc gctggcagga gaactggaga aactgcgata gcccacagac 3240

atggcttcat tttttttgaa ttttctcttt ccaaagccaa acagccttgc cctgacagga 3300

ccacctgtcc caacttctca gccttaagtt ataacacacg tacgtgtctg ccagcttctg 3360

ggcctttgct gtgttttcct ctgcttcaaa gagccaacca aggaggccgt gggctgagag 3420

ccctctcggc cagctattcc ctaacctgct gcctacacca gactggctgg tggcatccag 3480

tcccggctgc ctgttgggaa ccctgcttct gaaggttccc ctctccaccc cctccaccca 3540

gaggtcggga gaaccttctg gatccttgca ttgactgtta aggagctaaa accaccaaac 3600

ccagtcaaaa cctacacgtg tttgtcatga ccgccaccaa acagaacaaa ggggccgaaa 3660

tctccctttg ctctgaactc aaagcaggaa agtgctggct tcaccctagt ttgttttttg 3720

tttttgtttt ttaacctgaa ttcaggtaaa gcctccatag actcctgaac gctgctgtat 3780

gagctgtgac tcaggcggat ccaccccagc atatgcctta gtgtgatcaa gccctgcctg 3840

gcccggttgt gtgtcctgtg ggattctcca cacacactgc ctgctacagg cggtcctcca 3900

tggtcttcct cctggttcag ttttcttctt ccaacagcgt ttcccccaat catgtacgca 3960

agaccgtcct ctccccttgc cttcatccca gatgtctcta ttgggcatct tcttaaagtg 4020

actttctcgg ctttgtgtgc taggcagagg acccgagaag caagctacag tggctaagta 4080

cccgtggttg gtttcttacg tcctggcttt cttgtcaaag tcatctgaaa ggaaatggct 4140

ggagccgggc cagctgtcgc tggttctcct tacagagacc ccatgcccag ggttcagctt 4200

gaacatgcaa atatgacttt gtggtcagga cctagagtgt ctcccccttg ctgcagccct 4260

gggctttccc tccactgacc ccatgcgtag acctgggcag aattctcgtg tgaacccaga 4320

atgtctgtaa gaagttgagc actaagccgc tatgagtcta agtacttcct gctaagcaag 4380

aaacgttctg ctggcccttt acggccagga gcttctgact ggagcaatgc cgacttctct 4440

ggggaagggt gatgagggtt cggtgagtga cagttcacac ttgccctggc tgagagccac 4500

gttttgcaca tgagggagtc aggcccttcc tcagatatct ccaagtttta cccaagtgcc 4560

ttcctgcaat tgtagtcatt gtaattgtag ctcagctgca ctgtccacag ggagacaaag 4620

ggctaactca cttcccatta gttttcatgt cagcgaaggg gttcctggta gctcagactc 4680

ctatactttc ctctgtgtgt atgtgtgcat gtgtgtgtgt gtgtgtgtgt gtgtatatac 4740

gtgtacacaa ttctcttccc tttatactat cttagccaga tagtctcaga aacttgctga 4800

taaatatccc aaacagccaa ggagagaaag cagcatctgt taccagacaa acaggccata 4860

gaaggtgtat gagcggcaga gagctatgga gaaggtttgg tcagttacag actccagggg 4920

aaacgtggcc gggagtgact gcttatcaac catttccagg atggggagat gcagtctgat 4980

cttagcgaat gagaaaagat gcccgagcag tgacgacgag tgtaccgcac gtgggttaga 5040

ggggcggaag cggaaatgtg tccttgctaa atctgtggtg tcgttccggt gacagttcca 5100

tattgctaaa tgtggcactt tctcttccaa gtccacacat ctccctgact gtacaaaggc 5160

accgctgatg gggagtatat ctatctaccc agatcagtat ggtattaaat tttattttct 5220

tatttgtatt ttgatgcttt ttatctgcct ttaaaatatc tcacccctgt tggagttaga 5280

acatatttat aaatgaccag atgtcttttt ttaagggtct atttcttttt ttaaaaaaaa 5340

tctatatctt cattttctgt aagagaaatg gtatactcct gagttttgtt ttattttgtt 5400

ttgttttgtt ttgctttttg tttctcattc tgggaaacaa atccaaagac tcacctcggg 5460

tcctatcaag acctcaggat gggagagaaa gggactttgt aggagagaac acctttttta 5520

gcttgagaaa gagggcctgc tccttcagct ccatgtcagg gctgtccctg agaaccgtac 5580

gtgaaaggtt tggttggtgg tctcaatcta aagccacaga actctgcctt tccatggaca 5640

agctccttca ggcaggctga cagttgacag agccaaccac gtggagtcct cagaggtgac 5700

actgaggtgc agcatccaaa gaacagccac cttctcccca gcgcagcgta gccacggacc 5760

aagtgacctg ggataacact gttcacatgc agcagcctgg tcatccatta tccagcacca 5820

ccagccgtgg ccagccgccg gcagcactgt cacacaggga ggccagaagc cagtgtgttt 5880

taattagcac tcagaaccta taggaggctg agttggttta tcttcacacc acagtgctca 5940

gcatcaggag aaacgggggc tacgtaacaa ctgtgttcat ttgccgcgct tccgaaaaga 6000

cagaggtggc caggctaggg cgagtattcc tggactcttt gccgtcaggg aaggctggag 6060

gaacccgaca catcctcacc actggcatct cccaagtttg gacaccaacc ttcatggacc 6120

caaaccaggc agctgcagag cgggaggttt ccctgcagcc cagcagttgc ttaaatgagg 6180

caacatcacc ttcacctacg accagcttac ccgtgccttg acagacatac ggccaccatc 6240

gttgtcttaa tccagtttgt ttacagctaa ctctaaatgg tgggccagga gtggtgcttc 6300

cctgagcagg acccctctga gatcacaatg aagtcctggg aactggagtc aaaagtgtta 6360

atgctttcag atgggaataa acgtccctgc cctgcctggc cctgcctggc tctgccctct 6420

caggctttag atggactggg tgcagacagt gctgttgcta tgtgagtagg aaccccagca 6480

ttctgtgaga gcatcccaga catcagacat ccccgtcccc ctgtcatttc tcagccctca 6540

tgggagcctg ctcatcgggc tctgaaggcc ggggtttggg aaaatctcca ccccactacc 6600

tgaggagtcg ggatatatca cattcctatg caaaacgatt ttaatctccg gtttcctgca 6660

gtttattttt gctatatgta aagtattttt atacggctta tatgatgaca gcttagcaat 6720

aaacagttga gagcaactct aaaaaaaaaa aaaaaaaaa 6759

<210> 57
<211> 2042
<212> DNA
<213> Homo sapiens

<220>
<223> Human HOXB4 (NM_024015.4)

<400> 57
ggaaaacgag tcaggggtcg gaataaattt tagtatattt tgtgggcaat tcccagaaat 60

taatggctat gagttctttt ttgatcaact caaactatgt cgaccccaag ttccctccat 120

gcgaggaata ttcacagagc gattacctac ccagcgacca ctcgcccggg tactacgccg 180

gcggccagag gcgagagagc agcttccagc cggaggcggg cttcgggcgg cgcgcggcgt 240

gcaccgtgca gcgctacgcg gcctgccggg accctgggcc cccgccgcct ccgccaccac 300

ccccgccgcc cccgccaccg cccggtctgt cccctcgggc tcctgcgccg ccacccgccg 360

gggccctcct cccggagccc ggccagcgct gcgaggcggt cagcagcagc cccccgccgc 420

ctccctgcgc ccagaacccc ctgcacccca gcccgtccca ctccgcgtgc aaagagcccg 480

tcgtctaccc ctggatgcgc aaagttcacg tgagcacggt aaaccccaat tacgccggcg 540

gggagcccaa gcgctctcgg accgcctaca cgcgccagca ggtcttggag ctggagaagg 600

aatttcacta caaccgctac ctgacacggc gccggagggt ggagatcgcc cacgcgctct 660

gcctctccga gcgccagatc aagatctggt tccagaaccg gcgcatgaag tggaaaaaag 720

accacaagtt gcccaacacc aagatccgct cgggtggtgc ggcaggctca gccggagggc 780

cccctggccg gcccaatgga ggcccccgcg cgctctagtg cccccgcacg cgggagccac 840

gaacctcggg gtgggggtgg gcagtgagtg caggggatgg ggtgggggga caggaggggg 900

ccctggggcc tgggccccgg aaaaatctat ctgccctccc ccacacttta tatacgaata 960

aacgcagaag agggggaggg gaagctttat ttatagaaat gacaatagag ggccacgggg 1020

aggccccccc agaagcaaga ttcaaatctc ttgctttctt tcttaaaaaa aagaaaaaga 1080

aaaagcaaga agaaggaaga aagaaaaaga cagaaagaga aataggagga ggctgcagct 1140

cctcgttttc agctttggcg aagatggatc cacgtttcat ctttaatcac gccaggtcca 1200

ggcccatctg tcttgtttcc tctgccgagg agaagacggg cctcggtggc gaccattacc 1260

tcgacacccg ctaacaaatg aggcccggct cggccgcctc cgcctctgct actgccgctg 1320

ctggaagaca gcctggattt cctttctttg tcccccactc ccgataccca gcgaaagcac 1380

cctctgactg ccagatagtg cagtgttttg gtcacggtaa cacacacaca ctctccctca 1440

tctttcgtgc ccattcactg agggccagaa tgactgctca cccacttcca ccgtggggtt 1500

gggggtgggc aacagaggag gggagcaagt agggaagggg gtggccttga caactcagga 1560

gtgagcagga aaattgagtc caaggaaaaa gagagactca gagacccggg agggccttcc 1620

tctgaaaggc caagccaagc catgcttggc agggtgaggg gccagttgag ttctgggagc 1680

tgggcactac tctgccagtc cagagttgta cagcagaagc ctctctccta gactgaaaat 1740

gaatgtgaaa ctaggaaata aaatgtgccc ctcccagtct gggaggagga tgttgcagag 1800

ccctctccca tagtttatta tgttgcatcg tttattatta ttattgataa tattattatt 1860

actatttttt tgtgtcatgt gagtcctctc tccttttctc tttctgacat tccaaaacca 1920

ggccccttcc tacctctggg gctgcttgag tctagaaccc ttcgtatgtg tgaatatctg 1980

tgtgctgtac agagtgacaa tagaaataaa tgtttggttt cttgtgacca gcaaaaaaaa 2040

aa 2042

<210> 58
<211> 2566
<212> DNA
<213> Mus <mouse, genus>

<220>
<223> Mouse Hoxb4 (NM_010459.7)

<400> 58
agctccctct tatcctgggg aggagatggg gccctatggg agtcagagaa aggttggtgg 60

agattaggta gggcaggggt gggggctaca cccgcagccc caacttctga gggatgcagg 120

gtttgtgctc tgcccagccc cctcaccccc aaacaggccc tccaattaac ctgctcactc 180

tttcttagtt catcacccct ctctgttgtg gagaaagcca ggcgaggagg gggtctcccc 240

gcgggagccc tatgtaaatc ctggtgttgg gtgggtgggg aggggtagag aaggggaaat 300

aaacctcttt ggctggagta gggtccgggt gagcagattt ccttatccgg gaatcgcagg 360

ccgggtggcc attggctcgg aggatcacgt gggcctctaa ctttgttcac ttgacagtaa 420

gtaggagggc tttcggaaac aggaaaacga gtcaggggtc ggaataaatt ttagtatatt 480

ttgtgggcaa ttcccagaaa ttaatggcta tgagttcctt tttgatcaac tcaaactatg 540

tcgaccccaa gttccctccg tgcgaggagt attcacagag cgattaccta cccagcgacc 600

actcgcccgg gtactacgcc ggcggccaga ggcgagagag cggcttccag ccggaggcgg 660

cctttgggcg ccgggcgccg tgcactgtgc agcgctacgc ggcctgccga gaccccgggc 720

ccccgccacc tccgccgccc ccgccgcccc cgccaccgcc cgggctgtcc cctcgggctc 780

cagtgcagcc aacagccggg gccctcctcc cggagcccgg gcagcgcagc gaggcggtca 840

gcagcagccc cccgccgcct ccctgcgccc agaaccccct gcatcccagc ccgtcccact 900

ccgcgtgcaa agagcccgtc gtctacccct ggatgcgcaa agttcacgtg agcacggtaa 960

accccaatta cgccggcggg gagcccaagc gctctcggac cgcctacact cgccagcagg 1020

tcctggagtt ggagaaggag tttcactaca atcgctacct gacgcgccgc cggagggtgg 1080

agatcgccca cgcgctctgc ctgtccgagc gccagatcaa gatctggttc cagaatcggc 1140

gcatgaagtg gaaaaaagac cacaagttgc ccaacaccaa gatccgctcg ggtggcaccg 1200

cgggcgcagc cggaggcccc cctggccggc ccaacggagg cccccctgcg ctctagtgcc 1260

ccccaagcag gagttcgaac atgggggggt ggggggaaca gcgagcaccg aagggggtgc 1320

ggggtatggg agggtccccg ggcttgagcc cagaaaaaat ctatctaccc taccctcact 1380

ttatctataa ggaataaaca cagagaaggg gggtagggaa gccttattta tagaaaggac 1440

aataagggag ccgggtaaag tccttcggag acaagattcg agtctcttgc tttcttcctt 1500

taaaaaaaaa aaagaaagaa agaaagaagg aaagaaagag agagagagaa aagaaaaaga 1560

agaaaggaag gaagcaagaa aaggaggaag aaaggaaaag acagaagaga aatggaggag 1620

tctgctgcgc ctggttttca gctttggtga agatggatcc aggcttcatc tttaatcacg 1680

ccaggcccgg gcccatctgt cttgtttact ctgccgagga gaagacgggc gagcgagctt 1740

cggtggcgac cattacctcg acacttggct aacaaatgag gccaggctcg gccgctgccg 1800

ccgcctctgc tgctgtcgct gctggatcac agcctggatt tttctttctt tgtcccctac 1860

tcctgacacc cagcgaatgc accctcagac tgccagatag cacagtgttt tggccacggt 1920

aacaaacaca cacacatata actttcctcc ctgtctgtac ccactttggg gtgggggtgg 1980

gtgggaagac tgctcactcc cttccaccat agacttagaa gggggaacag aagggaattg 2040

aagggcagtc tgcacaacgt gggttcccaa atccgagccc aagaaataaa tgaaaatgaa 2100

aaaagaaagg tagcaattgg gacacccaag aaggccttct gctagaaggt ccagctaggc 2160

ctggcagggt gaggggcagt tgagttctgg gagctgggaa tgtcttctgg gcagttcaca 2220

gtagtagagt caaggccttc tcttaggtta caaatgaatg tgaaattagg aaataaaata 2280

ctgtggccct cctactctgg aaggacaatg ttgcagaacc ctctcccgtt gttatcattg 2340

ttgcatcgtt tattattatt attattatta ttattattat tattattatt attattatta 2400

ttttatgtca tgtgtgtcct ctctcctgtt ctctttctga cattccaaaa ccaggcccct 2460

tcctacctct ggggctgcct gagcctagaa ccttttgttg gtgtgaaaat ttgtgtcctg 2520

tacagagtga caacagaaat aaatgtttgg tttcttgtga ccatca 2566

<210> 59
<211> 3014
<212> DNA
<213> Homo sapiens

<220>
<223> Human KLF4 (NM_001314052.1)

<400> 59
gcagaggcgg tggcgggcgg cggcggcacc gggagccgcc gagtgaccct cccccgcccc 60

tctggccccc caccctccca cccgcccgtg gcccgcgccc atggccgcgc gcgctccaca 120

caactcaccg gagtccgcgc cttgcgccgc cgaccagttc gcagctccgc gccacggcag 180

ccagtctcac ctggcggcac cgcccgccca ccgccccggc cacagcccct gcgcccacgg 240

cagcactcga ggcgaccgcg acagtggtgg gggacgctgc tgagtggaag agagcgcagc 300

ccggccaccg gacctactta ctcgccttgc tgattgtcta tttttgcgtt tacaactttt 360

ctaagaactt ttgtatacaa aggaactttt taaaaaagac gcttccaagt tatatttaat 420

ccaaagaaga aggatctcgg ccaatttggg gttttgggtt ttggcttcgt ttcttctctt 480

cgttgacttt ggggttcagg tgccccagct gcttcgggct gccgaggacc ttctgggccc 540

ccacattaat gaggcagcca cctggcgagt ctgacatggc tgtcagcgac gcgctgctcc 600

catctttctc cacgttcgcg tctggcccgg cgggaaggga gaagacactg cgtcaagcag 660

gtgccccgaa taaccgctgg cgggaggagc tctcccacat gaagcgactt cccccagtgc 720

ttcccggccg cccctatgac ctggcggcgg cgaccgtggc cacagacctg gagagcggcg 780

gagccggtgc ggcttgcggc ggtagcaacc tggcgcccct acctcggaga gagaccgagg 840

agttcaacga tctcctggac ctggacttta ttctctccaa ttcgctgacc catcctccgg 900

agtcagtggc cgccaccgtg tcctcgtcag cgtcagcctc ctcttcgtcg tcgccgtcga 960

gcagcggccc tgccagcgcg ccctccacct gcagcttcac ctatccgatc cgggccggga 1020

acgacccggg cgtggcgccg ggcggcacgg gcggaggcct cctctatggc agggagtccg 1080

ctccccctcc gacggctccc ttcaacctgg cggacatcaa cgacgtgagc ccctcgggcg 1140

gcttcgtggc cgagctcctg cggccagaat tggacccggt gtacattccg ccgcagcagc 1200

cgcagccgcc aggtggcggg ctgatgggca agttcgtgct gaaggcgtcg ctgagcgccc 1260

ctggcagcga gtacggcagc ccgtcggtca tcagcgtcag caaaggcagc cctgacggca 1320

gccacccggt ggtggtggcg ccctacaacg gcgggccgcc gcgcacgtgc cccaagatca 1380

agcaggaggc ggtctcttcg tgcacccact tgggcgctgg accccctctc agcaatggcc 1440

accggccggc tgcacacgac ttccccctgg ggcggcagct ccccagcagg actaccccga 1500

ccctgggtct tgaggaagtg ctgagcagca gggactgtca ccctgccctg ccgcttcctc 1560

ccggcttcca tccccacccg gggcccaatt acccatcctt cctgcccgat cagatgcagc 1620

cgcaagtccc gccgctccat taccaaggtc agtcccgggg atttgtagct cgggctgggg 1680

agccctgtgt gtgctggccc cacttcggga cacacgggat gatgctcacc ccaccttctt 1740

cacccctaga gctcatgcca cccggttcct gcatgccaga ggagcccaag ccaaagaggg 1800

gaagacgatc gtggccccgg aaaaggaccg ccacccacac ttgtgattac gcgggctgcg 1860

gcaaaaccta cacaaagagt tcccatctca aggcacacct gcgaacccac acaggtgaga 1920

aaccttacca ctgtgactgg gacggctgtg gatggaaatt cgcccgctca gatgaactga 1980

ccaggcacta ccgtaaacac acggggcacc gcccgttcca gtgccaaaaa tgcgaccgag 2040

cattttccag gtcggaccac ctcgccttac acatgaagag gcatttttaa atcccagaca 2100

gtggatatga cccacactgc cagaagagaa ttcagtattt tttacttttc acactgtctt 2160

cccgatgagg gaaggagccc agccagaaag cactacaatc atggtcaagt tcccaactga 2220

gtcatcttgt gagtggataa tcaggaaaaa tgaggaatcc aaaagacaaa aatcaaagaa 2280

cagatggggt ctgtgactgg atcttctatc attccaattc taaatccgac ttgaatattc 2340

ctggacttac aaaatgccaa gggggtgact ggaagttgtg gatatcaggg tataaattat 2400

atccgtgagt tgggggaggg aagaccagaa ttcccttgaa ttgtgtattg atgcaatata 2460

agcataaaag atcaccttgt attctcttta ccttctaaaa gccattatta tgatgttaga 2520

agaagaggaa gaaattcagg tacagaaaac atgtttaaat agcctaaatg atggtgcttg 2580

gtgagtcttg gttctaaagg taccaaacaa ggaagccaaa gttttcaaac tgctgcatac 2640

tttgacaagg aaaatctata tttgtcttcc gatcaacatt tatgacctaa gtcaggtaat 2700

atacctggtt tacttcttta gcatttttat gcagacagtc tgttatgcac tgtggtttca 2760

gatgtgcaat aatttgtaca atggtttatt cccaagtatg ccttaagcag aacaaatgtg 2820

tttttctata tagttccttg ccttaataaa tatgtaatat aaatttaagc aaacgtctat 2880

tttgtatatt tgtaaactac aaagtaaaat gaacattttg tggagtttgt attttgcata 2940

ctcaaggtga gaattaagtt ttaaataaac ctataatatt ttataaaaaa aaaaaaaaaa 3000

aaaaaaaaaa aaaa 3014

<210> 60
<211> 3057
<212> DNA
<213> Mus <mouse, genus>

<220>
<223> Mouse Klf4 (NM_010637.3)

<400> 60
agttccccgg ccaagagagc gagcgcggct ccgggcgcgc ggggagcaga ggcggtggcg 60

ggcggcggcg gcacccggag ccgccgagtg cccctccccg cccctccagc cccccaccca 120

gcaacccgcc cgtgacccgc gcccatggcc gcgcgcaccc ggcacagtcc ccaggactcc 180

gcaccccgcg ccaccgccca gctcgcagtt ccgcgccacc gcggccattc tcacctggcg 240

gcgccgcccg cccaccgccc ggaccacagc ccccgcgccg ccgacagcca cagtggccgc 300

gacaacggtg ggggacactg ctgagtccaa gagcgtgcag cctggccatc ggacctactt 360

atctgccttg ctgattgtct atttttataa gagtttacaa cttttctaag aatttttgta 420

tacaaaggaa cttttttaaa gacatcgccg gtttatattg aatccaaaga agaaggatct 480

cgggcaatct gggggttttg gtttgaggtt ttgtttctaa agtttttaat cttcgttgac 540

tttggggctc aggtacccct ctctcttctt cggactccgg aggaccttct gggcccccac 600

attaatgagg cagccacctg gcgagtctga catggctgtc agcgacgctc tgctcccgtc 660

cttctccacg ttcgcgtccg gcccggcggg aagggagaag acactgcgtc cagcaggtgc 720

cccgactaac cgttggcgtg aggaactctc tcacatgaag cgacttcccc cacttcccgg 780

ccgcccctac gacctggcgg cgacggtggc cacagacctg gagagtggcg gagctggtgc 840

agcttgcagc agtaacaacc cggccctcct agcccggagg gagaccgagg agttcaacga 900

cctcctggac ctagacttta tcctttccaa ctcgctaacc caccaggaat cggtggccgc 960
caccgtgacc acctcggcgt cagcttcatc ctcgtcttcc ccggcgagca gcggccctgc 1020
cagcgcgccc tccacctgca gcttcagcta tccgatccgg gccgggggtg acccgggcgt 1080
ggctgccagc aacacaggtg gagggctcct ctacagccga gaatctgcgc cacctcccac 1140
ggcccccttc aacctggcgg acatcaatga cgtgagcccc tcgggcggct tcgtggctga 1200
gctcctgcgg ccggagttgg acccagtata cattccgcca cagcagcctc agccgccagg 1260
tggcgggctg atgggcaagt ttgtgctgaa ggcgtctctg accacccctg gcagcgagta 1320
cagcagccct tcggtcatca gtgttagcaa aggaagccca gacggcagcc accccgtggt 1380
agtggcgccc tacagcggtg gcccgccgcg catgtgcccc aagattaagc aagaggcggt 1440
cccgtcctgc acggtcagcc ggtccctaga ggcccatttg agcgctggac cccagctcag 1500
caacggccac cggcccaaca cacacgactt ccccctgggg cggcagctcc ccaccaggac 1560
tacccctaca ctgagtcccg aggaactgct gaacagcagg gactgtcacc ctggcctgcc 1620
tcttccccca ggattccatc cccatccggg gcccaactac cctcctttcc tgccagacca 1680
gatgcagtca caagtcccct ctctccatta tcaagagctc atgccaccgg gttcctgcct 1740
gccagaggag cccaagccaa agaggggaag aaggtcgtgg ccccggaaaa gaacagccac 1800
ccacacttgt gactatgcag gctgtggcaa aacctatacc aagagttctc atctcaaggc 1860
acacctgcga actcacacag gcgagaaacc ttaccactgt gactgggacg gctgtgggtg 1920
gaaattcgcc cgctccgatg aactgaccag gcactaccgc aaacacacag ggcaccggcc 1980
ctttcagtgc cagaagtgtg acagggcctt ttccaggtcg gaccaccttg ccttacacat 2040
gaagaggcac ttttaaatcc cacgtagtgg atgtgaccca cactgccagg agagagagtt 2100
cagtattttt ttttctaacc tttcacactg tcttcccacg aggggaggag cccagctggc 2160
aagcgctaca atcatggtca agttcccagc aagtcagctt gtgaatggat aatcaggaga 2220
aaggaagagt tcaagagaca aaacagaaat actaaaaaca aacaaacaaa aaaacaaaca 2280
aaaaaaacaa gaaaaaaaaa tcacagaaca gatggggtct gatactggat ggatcttcta 2340
tcattccaat accaaatcca acttgaacat gcccggactt acaaaatgcc aaggggtgac 2400
tggaagtttg tggatatcag ggtatacact aaatcagtga gcttgggggg agggaagacc 2460
aggattccct tgaattgtgt ttcgatgatg caatacacac gtaaagatca ccttgtatgc 2520
tctttgcctt cttaaaaaaa aaaaaagcca ttattgtgtc ggaggaagag gaagcgattc 2580
aggtacagaa catgttctaa cagcctaaat gatggtgctt ggtgagtcgt ggttctaaag 2640
gtaccaaacg ggggagccaa agttctccaa ctgctgcata cttttgacaa ggaaaatcta 2700
gttttgtctt ccgatctaca ttgatgacct aagccaggta aataagcctg gtttatttct 2760
gtaacatttt tatgcagaca gtctgttatg cactgtggtt tcagatgtgc aataatttgt 2820

acaatggttt attcccaagt atgcctttaa gcagaacaaa tgtgtttttc tatatagttc 2880

cttgccttaa taaatatgta atataaattt aagcaaactt ctattttgta tatttgtaaa 2940

ctacaaagta aaaaaaaatg aacattttgt ggagtttgta ttttgcatac tcaaggtgag 3000

aaataagttt taaataaacc tataatattt tatctgaacg acaaaaaaaa aaaaaaa 3057

<210> 61
<211> 3681
<212> DNA
<213> Homo sapiens

<220>
<223> Human MYB (NM_001130173.1)

<400> 61
aatatcaacc tgtttcctcc tcctccttct cctcctcctc cgtgacctcc tcctcctctt 60

tctcctgaga aacttcgccc cagcggtgcg gagcgccgct gcgcagccgg ggagggacgc 120

aggcaggcgg cgggcagcgg gaggcggcag cccggtgcgg tccccgcggc tctcggcgga 180

gccccgcgcc cgccgcgcca tggcccgaag accccggcac agcatatata gcagtgacga 240

ggatgatgag gactttgaga tgtgtgacca tgactatgat gggctgcttc ccaagtctgg 300

aaagcgtcac ttggggaaaa caaggtggac ccgggaagag gatgaaaaac tgaagaagct 360

ggtggaacag aatggaacag atgactggaa agttattgcc aattatctcc cgaatcgaac 420

agatgtgcag tgccagcacc gatggcagaa agtactaaac cctgagctca tcaagggtcc 480

ttggaccaaa gaagaagatc agagagtgat agagcttgta cagaaatacg gtccgaaacg 540

ttggtctgtt attgccaagc acttaaaggg gagaattgga aaacaatgta gggagaggtg 600

gcataaccac ttgaatccag aagttaagaa aacctcctgg acagaagagg aagacagaat 660

tatttaccag gcacacaaga gactggggaa cagatgggca gaaatcgcaa agctactgcc 720

tggacgaact gataatgcta tcaagaacca ctggaattct acaatgcgtc ggaaggtcga 780

acaggaaggt tatctgcagg agtcttcaaa agccagccag ccagcagtgg ccacaagctt 840

ccagaagaac agtcatttga tgggttttgc tcaggctccg cctacagctc aactccctgc 900

cactggccag cccactgtta acaacgacta ttcctattac cacatttctg aagcacaaaa 960

tgtctccagt catgttccat accctgtagc gttacatgta aatatagtca atgtccctca 1020

gccagctgcc gcagccattc agagacacta taatgatgaa gaccctgaga aggaaaagcg 1080

aataaaggaa ttagaattgc tcctaatgtc aaccgagaat gagctaaaag gacagcaggt 1140

gctaccaaca cagaaccaca catgcagcta ccccgggtgg cacagcacca ccattgccga 1200

ccacaccaga cctcatggag acagtgcacc tgtttcctgt ttgggagaac accactccac 1260

tccatctctg ccagcggatc ctggctccct acctgaagaa agcgcctcgc cagcaaggtg 1320

catgatcgtc caccagggca ccattctgga taatgttaag aacctcttag aatttgcaga 1380

aacactccaa tttatagatt ctgattcttc atcatggtgt gatctcagca gttttgaatt 1440

ctttgaagaa gcagattttt cacctagcca acatcacaca ggcaaagccc tacagcttca 1500

gcaaagagag ggcaatggga ctaaacctgc aggagaacct agcccaaggg tgaacaaacg 1560

tatgttgagt gagagttcac ttgacccacc caaggtctta cctcctgcaa ggcacagcac 1620

aattccactg gtcatccttc gaaaaaaacg gggccaggcc agccccttag ccactggaga 1680

ctgtagctcc ttcatatttg ctgacgtcag cagttcaact cccaagcgtt cccctgtcaa 1740

aagcctaccc ttctctccct cgcagttctt aaacacttcc agtaaccatg aaaactcaga 1800

cttggaaatg ccttctttaa cttccacccc cctcattggt cacaaattga ctgttacaac 1860

accatttcat agagaccaga ctgtgaaaac tcaaaaggaa aatactgttt ttagaacccc 1920

agctatcaaa aggtcaatct tagaaagctc tccaagaact cctacaccat tcaaacatgc 1980

acttgcagct caagaaatta aatacggtcc cctgaagatg ctacctcaga caccctctca 2040

tctagtagaa gatctgcagg atgtgatcaa acaggaatct gatgaatctg gaattgttgc 2100

tgagtttcaa gaaaatggac cacccttact gaagaaaatc aaacaagagg tggaatctcc 2160

aactgataaa tcaggaaact tcttctgctc acaccactgg gaaggggaca gtctgaatac 2220

ccaactgttc acgcagacct cgcctgtggc agatgcaccg aatattctta caagctccgt 2280

tttaatggca ccagcatcag aagatgaaga caatgttctc aaagcattta cagtacctaa 2340

aaacaggtcc ctggcgagcc ccttgcagcc ttgtagcagt acctgggaac ctgcatcctg 2400

tggaaagatg gaggagcaga tgacatcttc cagtcaagct cgtaaatacg tgaatgcatt 2460

ctcagcccgg acgctggtca tgtgagacat ttccagaaaa gcattatggt tttcagaaca 2520

cttcaagttg acttgggata tatcattcct caacatgaaa cttttcatga atgggagaag 2580

aacctatttt tgttgtggta caacagttga gagcagcacc aagtgcattt agttgaatga 2640

agtcttcttg gatttcaccc aactaaaagg atttttaaaa ataaataaca gtcttaccta 2700

aattattagg taatgaattg tagccagttg ttaatatctt aatgcagatt tttttaaaaa 2760

aaacataaaa tgatttatct gtattttaaa ggatccaaca gatcagtatt ttttcctgtg 2820

atgggttttt tgaaatttga cacattaaaa ggtactccag tatttcactt ttctcgatca 2880

ctaaacatat gcatatattt ttaaaaatca gtaaaagcat tactctaagt gtagacttaa 2940

taccatgtga catttaatcc agattgtaaa tgctcattta tggttaatga cattgaaggt 3000

acatttattg taccaaacca ttttatgagt tttctgttag cttgctttaa aaattattac 3060

tgtaagaaat agttttataa aaaattatat ttttattcag taatttaatt ttgtaaatgc 3120

caaatgaaaa acgttttttg ctgctatggt cttagcctgt agacatgctg ctagtatcag 3180

aggggcagta gagcttggac agaaagaaaa gaaacttggt gttaggtaat tgactatgca 3240

ctagtatttc agacttttta attttatata tatatacatt ttttttcctt ctgcaataca 3300

tttgaaaact tgtttgggag actctgcatt ttttattgtg gtttttttgt tattgttggt 3360

ttatacaagc atgcgttgca cttctttttt gggagatgtg tgttgttgat gttctatgtt 3420

ttgttttgag tgtagcctga ctgttttata atttgggagt tctgcatttg atccgcatcc 3480

cctgtggttt ctaagtgtat ggtctcagaa ctgttgcatg gatcctgtgt ttgcaactgg 3540

ggagacagaa actgtggttg atagccagtc actgccttaa gaacatttga tgcaagatgg 3600

ccagcactga acttttgaga tatgacggtg tacttactgc cttgtagcaa aataaagatg 3660

tgcccttatt ttacctacaa a 3681

<210> 62
<211> 3777
<212> DNA
<213> Mus <mouse, genus>

<220>
<223> Mouse Myb (NM_001198914.1)

<400> 62
gggagtgtcc aaacctcttt gtttgatggc atctgtttac agagttacac tttaatatca 60

acctgtttcc tcctcctcct tctcctcctt ctcctcctcc tcctcggtga cctccttctc 120

ctcccctttc tccggagaaa cttcgccccg gcggtgcgga gcgccgctgc gcagccgggg 180

gaggacgcag gcaaggcgga gggcagcggg aggcggcaac cggtgcggtc cccggggctc 240

ttggcggagc cccggcccgc ctcgccatgg cccggagacc ccgacacagc atctacagta 300

gcgatgaaga tgatgaagac attgagatgt gtgaccatga ctacgatggg ctgctgccca 360

aatctggaaa gcgtcacttg ggaaaaacta ggtggacaag ggaagaggat gagaagctga 420

agaagctggt ggaacagaac ggaacagacg actggaaagt cattgccaat tatctgccca 480

accggacaga tgtgcagtgc caacaccggt ggcagaaagt gctgaaccct gaactcatca 540

aaggtccctg gaccaaagaa gaagatcaga gagtcataga gcttgtccag aaatatggtc 600

cgaagcgttg gtctgttatt gccaagcact taaaagggag aattggaaag cagtgtcggg 660

agaggtggca caaccatttg aatccagaag ttaagaaaac ctcctggaca gaagaggagg 720

acagaatcat ttaccaggca cacaagcgtc tggggaacag atgggcagag atcgcaaagc 780

tgctgcccgg acggactgat aatgctatca agaaccactg gaattccacc atgcgtcgca 840

aggtggaaca ggaaggctac ctgcaggagc cttccaaagc cagccagacg ccagtggcca 900

cgagcttcca gaagaacaat catttgatgg ggtttgggca tgcctcacct ccatctcagc 960

tctctccaag tggccagtcc tccgtcaaca gcgaatatcc ctattaccac atcgccgaag 1020

cacaaaacat ctccagtcac gttccctatc ctgtcgcatt gcatgttaat atagtcaacg 1080
tccctcagcc ggctgcggca gccatccaga gacactataa cgacgaagac cctgagaagg 1140
aaaagcgaat aaaggagctg gagttgctcc tgatgtcaac agagaacgag ctgaagggac 1200
agcaggcatt accaacacag aaccacactt gcagctaccc cgggtggcac agcacctcca 1260
ttgtggacca gaccagacct catggggata gtgcacctgt ttcctgtttg ggagaacacc 1320
atgccacccc atctctgcct gcagatcccg gctccctacc tgaagaaagt gcctcaccag 1380
caaggtgcat gatcgtccac cagggcacca ttctggacaa tgttaagaac ctcttagaat 1440
ttgcagaaac actccagttt atagattctg attcttcgtg gtgtgatctc agcagttttg 1500
aattctctga agaagcggca gctttttcac ctagccagca gcccacaggc aaagccttcc 1560
agcttcagca aagagagggc catgggacta gatctgcagg agagcctagc ctgagggtga 1620
ccaggcgagt gctgagcgag gcatccctcg gcccagactc accccaagcg aggcacagca 1680
aggttccgct ggtcgtccta cgaaaaaggc ggggccaggc cagcccccta gccgctggag 1740
agcctagccc ctccctcttt gctgacgtca tcagctcaac tctcaagcgt tcccctgtca 1800
aaagcctacc cttctctccc tcgcagttct tgaacacttc cagcaaccat gaaagctcgg 1860
gcttagatgc acctacctta ccctccactc ctctcattgg tcacaaactg acaccatgtc 1920
gagaccagac tgtgaaaacc cagaaggaaa attccatctt tagaactcca gctatcaaaa 1980
ggtcaatcct cgaaagctct cctcgaactc ccacaccatt caaacatgcc cttgcagctc 2040
aagaaattaa atacggtccc ctgaagatgc tacctcagac cccctcccat gcagtggagg 2100
acctacaaga tgtgattaag caggaatcgg atgaatctgg aattgtggct gagtttcaag 2160
agagtggacc accgttactg aaaaaaatca agcaggaggt ggagtcgcca actgagaaat 2220
cgggaaactt cttctgctca aaccactggg cagagaacag cctgagcacc cagctgttct 2280
cgcaggcgtc tcctgtggca gatgccccaa atattcttac aagctctgtt ttaatgacac 2340
ctgtatcaga agatgaagac aatgtcctca aagcctttac cgtacctaag aacaggcccc 2400
tggtgggtcc cttgcagcca tgcagtggtg cctgggagcc agcatcctgt gggaagacag 2460
aggaccagat gacggcctcc ggtccggctc ggaaatacgt gaacgcgttc tcagctcgaa 2520
ctctggtcat gtgagacatt tccagaaaag cattatggtt ttcagaacac ttaaaagttg 2580
actttcgaca catggctcct cagcgtggag cgctccatgg ctgagagaag agcctgattt 2640
tgttgtggta caacagttga gagcagcacc aagtgcattt ttagttgctt gagatctcac 2700
ttgatttcac acaactaaaa aggatttttt tttttaaaaa taataataat gaataacagt 2760
cttacctaaa ttattaggta atgaattgtg accatttgtt aatatcataa tcagattttt 2820
taaaaaaaat aaaatgattt atttgtattt tagaggatac aacagatcag tatttttgac 2880
tgtggtgaat ttaaaaaaaa aatttacaca aagaaatatc ccagtattcc atgtatctca 2940

gtcactaaac atacacagag agatttttaa aaaccaggag aagcattatt ttgaatgtta 3000

gctaaatccc aagtaatact taatgcaacc ctctaggagc tcatttgtgg ctaataatct 3060

tggaaatatc tttattatac taaaccattt catgaggaga attttgttgt cagcttgctt 3120

gaaaagttat tactgtatga aatagtttta ttgaaaaaat tatattttta ttcagtaatt 3180

taattttgta aatgccaaat ggagaaatgt gttcgctgct atggttttag cctgtagtca 3240

tgctgctagc tagtgtcagg gggcaataga gcttagatgg aaaaaagaga aagagactcg 3300

gtgttagata acggactatg cactagtatt ccagactttt ttatttttat atatatgtac 3360

cttttccttt tgtaattgga aaacttattt gggagaattt tgcatttgtt gtacattttt 3420

gttttttagg attttttttt tttgttgtta ttgtcgattt ataaaagcat tgcacttctt 3480

tttctttttt tgggagattt gtgttgttta tgtcatatgt tttgttttga gttcagcctg 3540

aatgttcatc cgtttgggcg tttttctgac ttggaagaac attctctgta ggtttctaag 3600

tgtacagagc cggaactgcc tcgtggttcc tgggcttcag ggaagacaaa tatggaagtc 3660

aacagccagt ttctgccttg agagcatttg caagaatgct ggccttgaat tctgaaatga 3720

cagtgtatct actgccttgt agcaaaataa agctatcctc ttattttaca tacttcc 3777

<210> 63
<211> 1696
<212> DNA
<213> Homo sapiens

<220>
<223> Human NFE2 (NM_006163.2)

<400> 63
tgttcttggg gatcctcaag catatactgt catcatcttg gaaagaaaag gctgagaacg 60

taaaactgag gacagaggag gaaagcaggg tgacccctga tgttgcccta gaaaatggaa 120

aacaaaacac agcaaaacag aaaaacagaa gatctgactc tgcctttagc caggaaaaca 180

gtttggggga gtaaaaagta ttagggaaaa gagtgggcat tttgcctgga aaaaaggttt 240

ctagagccat ctgggctttc cgggaacctg gaccagactc tggcccagta ggatgtcccc 300

gtgtcctccc cagcagagca ggaacagggt gatacagctg tccacttcag agctaggaga 360

gatggaactg acttggcagg agatcatgtc catcaccgag ctgcagggtc tgaatgctcc 420

aagtgagcca tcatttgagc cccaagcccc agctccatac cttggacctc caccacccac 480

aacttactgc ccctgctcaa tccacccaga ttctggcttc ccacttcctc caccacctta 540

tgagctccca gcatccacat cccatgtccc agatccccca tactcctatg gcaacatggc 600

cataccagtc tccaagccac tgagcctctc aggcctgctc agtgagccgc tccaagaccc 660

cttagccctc ctggacattg ggctgccagc agggccacct aagccccaag aagacccaga 720

atccgactca ggattatccc tcaactatag cgatgctgaa tctcttgagc tggaggggac 780

agaggctggt cggcggcgca gcgaatatgt agagatgtac ccagtggagt acccctactc 840

actcatgccc aactccttgg cccactccaa ctataccttg ccagctgctg agaccccctt 900

ggccttagag ccctcctcag gccctgtgcg ggctaagccc actgcacggg gggaggcagg 960

gagtcgggat gaacgtcggg ccttggccat gaagattcct tttcctacgg acaagattgt 1020

caacttgccg gtagatgact ttaatgagct attggcaagg tacccgctga cagagagcca 1080

gctagcgcta gtccgggaca tccgacgacg gggcaaaaac aaggtggcag cccagaactg 1140

ccgcaagagg aagctggaaa ccattgtgca gctggagcgg gagctggagc ggctgaccaa 1200

tgaacgggag cggcttctca gggcccgcgg ggaggcagac cggaccctgg aggtcatgcg 1260

ccaacagctg acagagctgt accgtgacat tttccagcac cttcgggatg aatcaggcaa 1320

cagctactct cctgaagagt acgcgctgca acaggctgcc gatgggacca tcttccttgt 1380

gccccggggg accaagatgg aggccacaga ctgagctggc ccagaggggt ggaactgctg 1440

atgggatttc cttcattccc ttctgataaa ggtactcccc aaccctgagt cccagaagga 1500

gctgagttct ctagaccaga agaggatgac aatggcaaca agtgtttgga agttccaagg 1560

tgtgttcaaa gaggcttgcc ttgaggaggg ctggaatctg tcttccctga ctcggctcct 1620

caggtcttta gcctccacct tgtctaagct ttggtctata aagtgcgcta cagaaatggc 1680

aaaaaaaaaa aaaaaa 1696

<210> 64
<211> 1896
<212> DNA
<213> Mus <mouse, genus>

<220>
<223> Mouse Nfe2 (NM_001302338.1)

<400> 64
gatctctaag cactcacttc ttgggaagaa agggctgaaa tactaggtgt ggtggtacat 60

gcctttaatt ccagcacttg ggaggcagag gcagtcggat ctctgtgagc tagaagccag 120

cctggtctac atagcaagtt ctaggacaca gccagggaca catagcgaga ccttgtttta 180

aaaaacaaca atgaaagggt tagaccctac gctgaggaca caagaagaag gcaagggtga 240

ctcttgatgt tttcttccag aatggaaaat gaaatttcaa tccccaaacc tgagaaagac 300

tggattttgt ctctggacag gaataccatt tttagggagt aagagaaggc aacttgctga 360

gaaaaagtaa ggatacctac tctgtgtcca gaggactgac tatggtcaga tcaggtgagg 420

tagagagtac tccactgaag aaaagaacat tacagttcat agggtggttg atggcacctg 480

ttgcaggaat cctttgtgct tgtggagacc tggggccatc agctggcaca gtaggatgcc 540

cccgtgtcct cctcagcaga acaggaacag gttatcacag ctgcctgttg gggagcttgg 600

agagatggaa ctgacttggc aagagatcat gtccattact gagctgcagg gtctaaatgt 660

tccaagtgag acatcttttg agcctcaagc acccacccca taccctgggc cactgccacc 720

tccaacatat tgcccctgtt caattcatcc agatgcaggc ttctcccttc ccccaccatc 780

ttatgagctc ccagcatcta ctccccatgt cccagaacta ccatactcct atggtaatgt 840

agccatacca gtgtcaaagc cacttaccct ttcaggcctg ctcaatgagc ccctcccaga 900

ccacttagct ctcctggaca ttgggctgcc agtggggcaa cccaagcccc aagaagaccc 960

agaatctgac tcaggattat ccctcaacta cagtgatgca gaatctcttg agctagaggg 1020

tatggaggct ggcaggcgga ggagcgagta cgcggacatg tacccagtgg agtatcctta 1080

ctcacttatg cccaattctt tggcccatcc caactatact cttccaccca ctgagacacc 1140

cttggcctta gagtcatcct ccggtccagt tcgggctaag cctgctgtcc gtggggaggc 1200

agggagtcgg gacgagcggc gagccctggc catgaagatt cctttcccta cggacaagat 1260

agttaacttg ccggtagatg actttaatga gttgttggca cagtatccgc taacggagag 1320

ccagctggct ctagttcggg acatccgtcg acggggcaag aacaaggtgg cagcccaaaa 1380

ctgtcgcaag agaaaactgg aaaccattgt gcagctggag cgagagctgg agcggctgag 1440

cagtgaaagg gagcggcttc tcagagcccg aggggaggct gaccgcactc tggaggtcat 1500

gcgccaacag ctggcagagc tgtaccatga tattttccag catcttcggg atgaatctgg 1560

caacagttac tcaccagagg aatatgtact gcaacaggct gctgatggtg ccatctttct 1620

ggtaccccgt ggaaccaaaa tggaggctac agattgagct ggtctagaga gtggacactg 1680

gtgctagaga ctccctcatt cccttctgat gaaggcactt cttaatccca caagactcag 1740

agtcctccag accaaaaggc agatgtaacc atatcacctg ggagtatttg gtgaggtttg 1800

cctggaggcc aggtgttggc ttctcaggtc ctcaacccca cctcttgtcc aagctttggt 1860

tcataaagag ctgtgtggaa atagctaaaa aaaaaa 1896

<210> 65
<211> 4093
<212> DNA
<213> Homo sapiens

<220>
<223> Human NF-KB (NM_003998.3)

<400> 65
gtgagagagt gagcgagaca gaaagagaga gaagtgcacc agcgagccgg ggcaggaaga 60

ggaggtttcg ccaccggagc ggcccggcga cgcgctgaca gcttcccctg cccttcccgt 120

cggtcgggcc gccagccgcc gcagccctcg gcctgcacgc agccaccggc cccgctcccg 180

gagcccagcg ccgccgaggc cgcagccgcc cggccagtaa ggcggcgccg ccgcccggcc 240

accgcgcgcc ctgcgcttcc ctccgcccgc gctgcggcca tggcgcggcg ctgactggcc 300

tggcccggcc ccgccgcgct cccgctcgcc ccgacccgca ctcgggcccg cccgggctcc 360

ggcctgccgc cgcctcttcc ttctccagcc ggcaggcccg cgccgcttag gagggagagc 420

ccacccgcgc caggaggccg aacgcggact cgccacccgg cttcagaatg gcagaagatg 480

atccatattt gggaaggcct gaacaaatgt ttcatttgga tccttctttg actcatacaa 540

tatttaatcc agaagtattt caaccacaga tggcactgcc aacagcagat ggcccatacc 600

ttcaaatatt agagcaacct aaacagagag gatttcgttt ccgttatgta tgtgaaggcc 660

catcccatgg tggactacct ggtgcctcta gtgaaaagaa caagaagtct taccctcagg 720

tcaaaatctg caactatgtg ggaccagcaa aggttattgt tcagttggtc acaaatggaa 780

aaaatatcca cctgcatgcc cacagcctgg tgggaaaaca ctgtgaggat gggatctgca 840

ctgtaactgc tggacccaag gacatggtgg tcggcttcgc aaacctgggt atacttcatg 900

tgacaaagaa aaaagtattt gaaacactgg aagcacgaat gacagaggcg tgtataaggg 960

gctataatcc tggactcttg gtgcaccctg accttgccta tttgcaagca gaaggtggag 1020

gggaccggca gctgggagat cgggaaaaag agctaatccg ccaagcagct ctgcagcaga 1080

ccaaggagat ggacctcagc gtggtgcggc tcatgtttac agcttttctt ccggatagca 1140

ctggcagctt cacaaggcgc ctggaacccg tggtatcaga cgccatctat gacagtaaag 1200

cccccaatgc atccaacttg aaaattgtaa gaatggacag gacagctgga tgtgtgactg 1260

gaggggagga aatttatctt ctttgtgaca aagttcagaa agatgacatc cagattcgat 1320

tttatgaaga ggaagaaaat ggtggagtct gggaaggatt tggagatttt tcccccacag 1380

atgttcatag acaatttgcc attgtcttca aaactccaaa gtataaagat attaatatta 1440

caaaaccagc ctctgtgttt gtccagcttc ggaggaaatc tgacttggaa actagtgaac 1500

caaaaccttt cctctactat cctgaaatca aagataaaga agaagtgcag aggaaacgtc 1560

agaagctcat gcccaatttt tcggatagtt tcggcggtgg tagtggtgct ggagctggag 1620

gcggaggcat gtttggtagt ggcggtggag gagggggcac tggaagtaca ggtccagggt 1680

atagcttccc acactatgga tttcctactt atggtgggat tactttccat cctggaacta 1740

ctaaatctaa tgctgggatg aagcatggaa ccatggacac tgaatctaaa aaggaccctg 1800

aaggttgtga caaaagtgat gacaaaaaca ctgtaaacct ctttgggaaa gttattgaaa 1860

ccacagagca agatcaggag cccagcgagg ccaccgttgg gaatggtgag gtcactctaa 1920

cgtatgcaac aggaacaaaa gaagagagtg ctggagttca ggataacctc tttctagaga 1980

aggctatgca gcttgcaaag aggcatgcca atgccctttt cgactacgcg gtgacaggag 2040

acgtgaagat gctgctggcc gtccagcgcc atctcactgc tgtgcaggat gagaatgggg 2100

acagtgtctt acacttagca atcatccacc ttcattctca acttgtgagg gatctactag 2160

aagtcacatc tggtttgatt tctgatgaca ttatcaacat gagaaatgat ctgtaccaga 2220

cgcccttgca cttggcagtg atcactaagc aggaagatgt ggtggaggat ttgctgaggg 2280

ctggggccga cctgagcctt ctggaccgct tgggtaactc tgttttgcac ctagctgcca 2340

aagaaggaca tgataaagtt ctcagtatct tactcaagca caaaaaggca gcactacttc 2400

ttgaccaccc caacggggac ggtctgaatg ccattcatct agccatgatg agcaatagcc 2460

tgccatgttt gctgctgctg gtggccgctg gggctgacgt caatgctcag gagcagaagt 2520

ccgggcgcac agcactgcac ctggctgtgg agcacgacaa catctcattg gcaggctgcc 2580

tgctcctgga gggtgatgcc catgtggaca gtactaccta cgatggaacc acacccctgc 2640

atatagcagc tgggagaggg tccaccaggc tggcagctct tctcaaagca gcaggagcag 2700

atcccctggt ggagaacttt gagcctctct atgacctgga tgactcttgg gaaaatgcag 2760

gagaggatga aggagttgtg cctggaacca cgcctctaga tatggccacc agctggcagg 2820

tatttgacat attaaatggg aaaccatatg agccagagtt tacatctgat gatttactag 2880

cacaaggaga catgaaacag ctggctgaag atgtgaagct gcagctgtat aagttactag 2940

aaattcctga tccagacaaa aactgggcta ctctggcgca gaaattaggt ctggggatac 3000

ttaataatgc cttccggctg agtcctgctc cttccaaaac acttatggac aactatgagg 3060

tctctggggg tacagtcaga gagctggtgg aggccctgag acaaatgggc tacaccgaag 3120

caattgaagt gatccaggca gcctccagcc cagtgaagac cacctctcag gcccactcgc 3180

tgcctctctc gcctgcctcc acaaggcagc aaatagacga gctccgagac agtgacagtg 3240

tctgcgacag cggcgtggag acatccttcc gcaaactcag ctttaccgag tctctgacca 3300

gtggtgcctc actgctaact ctcaacaaaa tgccccatga ttatgggcag gaaggacctc 3360

tagaaggcaa aatttagcct gctgacaatt tcccacaccg tgtaaaccaa agccctaaaa 3420

ttccactgcg ttgtccacaa gacagaagct gaagtgcatc caaaggtgct cagagagccg 3480

gcccgcctga atcattctcg atttaactcg agaccttttc aacttggctt cctttcttgg 3540

ttcataaatg aattttagtt tggttcactt acagatagta tctagcaatc acaacactgg 3600

ctgagcggat gcatctgggg atgaggttgc ttactaagct ttgccagctg ctgctggatc 3660

acagctgctt tctgttgtca ttgctgttgt ccctctgcta cgttcctatt gtcattaaag 3720

gtatcacggt cgccacctgg cattccttct gaccacagca tcattttgca ttcaaattaa 3780

gggttaagaa aagagatatt ttaaaatgag agtcacttga tgtgccattt taaaaaaaaa 3840

ggcatattgc tttttctaat gtggttattt ctctgatttg caaaaaaaaa aaaaaaaaaa 3900

atacttgtca atatttaaac atggttacaa tcattgctga aaatggtatt ttcccccttt 3960

tctgcatttt gctattgtaa atatgttttt tagatcaaat actttaaagg aaaaaatgtt 4020

ggatttataa atgctatttt ttattttact tttataataa aaggaaaagc aaattgatga 4080

cctcaaaaaa aaa 4093

<210> 66
<211> 4128
<212> DNA
<213> Mus <mouse, genus>

<220>
<223> Mouse Nf-kb (NM_008689.2)

<400> 66
gtccgtctgt ctgctctctc tcgacgtcag tgggaatttc cagccaggaa gtgagagagt 60

gagcgagaga ggacgagaga gaagtaccga ggcgagccgg gcaggaagag gaggtttcgc 120

cacccgagca gcccggctgc gcgctgacgg cttcccgtgc cctgcgcgcc cccggcctgc 180

cgccgccgcc gccgccgccg ccgccctcgg cctgctcgcg ggccggctct agcagcgcag 240

gccggagctc agggccccgc cgcgcccggc ccgccccgcg cttctccgcc cgcgccgcag 300

ccatggcgcg ccgctgagcc gcccgcccgc ccgcccgcgc cccgacccgg ctcgggctcc 360

cgccggtccg cgccgctccg cagcgggagc ccgcaggcga ggagaggccg cgccgcatct 420

ccagggtacc ctcagaggcc agaagagggt gtcagagccc ttgtaactgg agtttgacgg 480

tcgtgagctg cgcatcttca ccatggcaga cgatgatccc tacggaactg ggcaaatgtt 540

tcatttgaac actgctttga ctcactcaat atttaatgca gaattatatt caccagaaat 600

accactgtca acagatggcc cataccttca aatattagag caaccaaaac agaggggatt 660

tcgattccgc tatgtgtgtg aaggcccatc acacggaggg cttccgggag cctctagtga 720

gaagaacaag aaatcctacc cacaggtcaa aatttgcaac tatgtggggc ctgcaaaggt 780

tatcgttcag ttggtcacaa atggaaaaaa catccacctg cacgcccaca gcctggtggg 840

caagcactgt gaggacgggg tatgcaccgt aacagcagga cccaaggaca tggtggttgg 900

ctttgcaaac ctgggaatac ttcatgtgac taagaaaaag gtatttgaaa cactggaagc 960

acggatgaca gaggcgtgta ttaggggcta taatcctgga cttctggtgc attctgacct 1020

tgcctatcta caagcagaag gcggaggaga ccggcaactc acagacagag agaaggagat 1080

catccgccag gcagccgtgc agcagaccaa ggagatggac ctgagcgtgg tgcgcctcat 1140

gttcacagcc ttcctccctg acagcactgg cagcttcact cggagactgg agcctgtggt 1200

gtcagacgcc atctatgata gcaaagcccc gaatgcatcc aacctgaaaa tcgtgagaat 1260

ggacagaaca gcaggatgtg tgacgggagg ggaggagatt taccttctct gtgacaaggt 1320

tcagaaagat gacatccaga ttcggtttta tgaagaggaa gaaaatggcg gagtttggga 1380

aggatttggg gacttttccc ccacagatgt tcatagacag tttgccattg tcttcaaaac 1440

gccaaagtat aaggatgtca acattacaaa gccagcttcc gtgtttgttc agcttcggag 1500

gaaatcagac ctggaaacta gtgaaccgaa accctttctc tactaccctg aaatcaaaga 1560

caaagaggaa gtgcaaagga aacgccagaa gcttatgccg aacttctcgg acagcttcgg 1620

tggcggcagt ggagcgggag ccggtggtgg aggcatgttc ggtagtggcg gtggcggagg 1680

gagtaccgga agccctggcc cagggtatgg ctactcgaac tacggatttc ctccctacgg 1740

tgggattaca ttccatcccg gagtcacgaa atccaacgca ggggtcaccc atggcaccat 1800

aaacaccaaa tttaaaaatg gccctaaaga ttgtgccaag agtgatgacg aggagagtct 1860

gactctccct gagaaggaaa ctgaaggtga agggcccagc ctgcccatgg cctgcaccaa 1920

gacggaaccc atcgccttgg catccaccat ggaagacaag gagcaggaca tgggatttca 1980

ggataacctc tttcttgaga aggctctgca gctcgccagg cgacacgcca acgccctttt 2040

cgactacgca gtgacggggg atgtgaagat gttgctggcc gtgcaacgcc atctcaccgc 2100

cgtgcaggat gagaatgggg acagtgtctt acacttagcc atcatccacc tccacgctca 2160

gcttgtgagg gatctgctgg aagtcacatc tggtttgatc tctgatgaca tcatcaacat 2220

gagaaatgac ctgtatcaga cacctctgca cttggccgtg atcaccaagc aggaagatgt 2280

agtagaggat ttgctgaggg ttggggctga cctgagcctt ctggaccgct ggggcaactc 2340

tgtcctgcac ctagctgcca aagaaggaca cgacagaatc ctcagcatcc tgctcaagag 2400

cagaaaagca gcgcccctta tcgaccaccc caatggggaa ggtctaaatg ccatccacat 2460

agctgtgatg agcaatagcc tgccatgtct gctgctgctg gtggctgccg gggcagaagt 2520

caatgctcag gagcagaagt ctgggcgcac agcgctgcac ctggccgtgg agtacgacaa 2580

catctccttg gctggctgcc tgcttctgga gggtgatgcc cacgtggaca gtaccaccta 2640

tgatgggact acacctctgc atatagcggc cggaagaggg tccaccagac tggcagctct 2700

tctcaaagca gcaggagcag accccctggt ggagaacttt gagcctctct atgacctgga 2760

cgactcttgg gagaaggctg gagaagatga gggagtggtg ccaggtacca cacccctgga 2820

catggctgcc aactggcagg tatttgacat actaaatggg aaaccgtatg agcctgtgtt 2880

cacatctgat gatatactac cacaagggga catgaagcag ctgacagaag acacgaggct 2940

acaactctgc aaactgctgg aaattcctga tccagacaaa aactgggcca ctctggcaca 3000

gaagttgggt ctggggatac tgaacaatgc cttccggctg agtcctgctc cttctaaaac 3060

tctcatggac aactatgagg tctctggggg taccatcaaa gagctgatgg aggccctgca 3120

acagatgggc tacacagagg ccattgaagt gatccaggca gccttccgca ccccggcaac 3180

cacagcctcc agccccgtga ccactgctca ggtccactgt ctgcctctct cgtcttcctc 3240

cacgaggcag cacatagatg aactccggga tagtgacagc gtctgtgaca gtggtgtgga 3300

gacatccttc cgcaaactca gctttacaga gtctcttact ggagacagcc cactgctatc 3360

tctgaacaaa atgccccacg gttatgggca ggaaggacct attgaaggca aaatttagcc 3420

tgctggccgt tcccccacac tgtgtaaacc aaagccctga cagtccattg catcgtccca 3480

aaggaggaag gcaaagcgaa tccaaaggtg ctggagaatc gccggcctgc agggtcactc 3540

gatttcattc aaggccttcc gaatttggcg tcctttcttg gttctgaaat gaaatgtagt 3600

tgccacgcac agacggtgtc tagcaatcat ggcgctcgct cgctcagctg cactctatgg 3660

ctcaggtgca gtgtcttgag ctttctctgc tgctactgga tcacatttgc tttgtgttgt 3720

tactgctgtc cctccgctgg gttcctgctg tcattaaaag gtgtcgctgt ccccacccgg 3780

tgtcctttct agccatctac tgtaagttgt gcattcaaat taagattaag gaaaaacata 3840

tttttaaatg agtaccttga tgcgcaataa aaaaaagaca tttctttttt taatgtggtt 3900

tatctgtgat ttaaaaataa aaaacacatg aacttacaat atttaaaaca tgctacaatc 3960

agtgctgaaa atagtatttt ccccgtttta tgcattttac tattgtaaat atgttttcta 4020

aatcaaatac tttaaaagaa gaaatgttga atttataaat gctatttact tttttatttt 4080

acttttataa taaaagtaca agcacattgt tgacctaaaa aaaaaaaa 4128